# EUROPEAN PATENT APPLICATION

(11) **EP 4 527 353 A2**
(43) Date of publication of application: **26.03.2025**
(21) Application number: 25157225.1
(22) Date of filing: 18.05.2020
(51) Int. Cl.: A61F 2/24

(54) **HEART VALVE SEALING DEVICES, DELIVERY DEVICES THEREFOR, AND RETRIEVAL DEVICES**

(30) Priority: 20.05.2019 US 201962850458 P
(62) Divisional of application: 20731671.2
(71) Applicant: Edwards Lifesciences Corporation, Irvine, CA 92614 (US)
(72) Inventor: DIXON, Eric Robert, Irvine, CA 92614 (US); METCHIK, Asher L., Irvine, CA 92614 (US); GOHRES, Rachel Ann, Irvine, CA 92614 (US); NGUYEN, Tam Van, Irvine, CA 92614 (US); MONTOYA, Daniel James, Irvine, CA 92614 (US); KWON, Rhayoung, Irvine, CA 92614 (US); FRESCHAUF, Lauren R., Irvine, CA 92614 (US); STEARNS, Grant Matthew, Irvine, CA 92614 (US); TYLER, Gregory Scott II, Irvine, CA 92614 (US); OBERWISE, Eric Michael, Irvine, CA 92614 (US); FORD, Steven M., Irvine, CA 92614 (US)
(74) Representative: Eisenführ Speiser

(57) **Abstract**

A method and system for implanting and repositioning an implantable device for a native valve of a patient's heart includes a delivery catheter, a collar, a coupler, and an actuation element. The actuation element extends through the delivery catheter and attaches to the device to open the device. The collar is connected to the device and the coupler is connected to the delivery catheter. The collar and coupler are tied together via one or more coupling tethers which can be used to reengage the device after an initial deployment.

## Description

### CROSS-REFERENCE TO RELATED APPLICATIONS

This application claims the benefit of U.S. Provisional Patent Application No. 62/850,458, filed May 20, 2019, which is incorporated by reference herein in its entirety for all purposes.

### BACKGROUND

The native heart valves (i.e., the aortic, pulmonary, tricuspid, and mitral valves) serve critical functions in assuring the forward flow of an adequate supply of blood through the cardiovascular system. These heart valves can be damaged, and thus rendered less effective, for example, by congenital malformations, inflammatory processes, infectious conditions, disease, etc. Such damage to the valves can result in serious cardiovascular compromise or death. Damaged valves can be surgically repaired or replaced during open heart surgery. However, open heart surgeries are highly invasive, and complications may occur. Transvascular techniques can be used to introduce and implant prosthetic devices in a manner that is much less invasive than open heart surgery. As one example, a transvascular technique useable for accessing the native mitral and aortic valves is the trans-septal technique. The trans-septal technique comprises advancing a catheter into the right atrium (e.g., inserting a catheter into the right femoral vein, up the inferior vena cava and into the right atrium). The septum is then punctured, and the catheter passed into the left atrium. A similar transvascular technique can be used to implant a prosthetic device within the tricuspid valve that begins similarly to the trans-septal technique but stops short of puncturing the septum and instead turns the delivery catheter toward the tricuspid valve in the right atrium.

A healthy heart has a generally conical shape that tapers to a lower apex. The heart is four-chambered and comprises the left atrium, right atrium, left ventricle, and right ventricle. The left and right sides of the heart are separated by a wall generally referred to as the septum. The native mitral valve of the human heart connects the left atrium to the left ventricle. The mitral valve has a very different anatomy than other native heart valves. The mitral valve includes an annulus portion, which is an annular portion of the native valve tissue surrounding the mitral valve orifice, and a pair of cusps, or leaflets, extending downward from the annulus into the left ventricle. The mitral valve annulus can form a "D"-shaped, oval, or otherwise out-of-round cross-sectional shape having major and minor axes. The anterior leaflet can be larger than the posterior leaflet, forming a generally "C"-shaped boundary between the abutting sides of the leaflets when they are closed together.

When operating properly, the anterior leaflet and the posterior leaflet function together as a one-way valve to allow blood to flow only from the left atrium to the left ventricle. The left atrium receives oxygenated blood from the pulmonary veins. When the muscles of the left atrium contract and the left ventricle dilates (also referred to as "ventricular diastole" or "diastole"), the oxygenated blood that is collected in the left atrium flows into the left ventricle. When the muscles of the left atrium relax and the muscles of the left ventricle contract (also referred to as "ventricular systole" or "systole"), the increased blood pressure in the left ventricle urges the sides of the two leaflets together, thereby closing the one-way mitral valve so that blood cannot flow back to the left atrium and is instead expelled out of the left ventricle through the aortic valve. To prevent the two leaflets from prolapsing under pressure and folding back through the mitral annulus toward the left atrium, a plurality of fibrous cords called chordae tendineae tether the leaflets to papillary muscles in the left ventricle.

Valvular regurgitation involves the valve improperly allowing some blood to flow in the wrong direction through the valve. For example, mitral regurgitation occurs when the native mitral valve fails to close properly and blood flows into the left atrium from the left ventricle during the systolic phase of heart contraction. Mitral regurgitation is one of the most common forms of valvular heart disease. Mitral regurgitation can have many different causes, such as leaflet prolapse, dysfunctional papillary muscles, stretching of the mitral valve annulus resulting from dilation of the left ventricle, more than one of these, etc. Mitral regurgitation at a central portion of the leaflets can be referred to as central jet mitral regurgitation and mitral regurgitation nearer to one commissure (i.e., location where the leaflets meet) of the leaflets can be referred to as eccentric jet mitral regurgitation. Central jet regurgitation occurs when the edges of the leaflets do not meet in the middle and thus the valve does not close, and regurgitation is present. Tricuspid regurgitation can be similar, but on the right side of the heart.

### SUMMARY

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. The description herein relates to systems, assemblies, methods, devices, apparatuses, combinations, etc. that may be utilized for repairing a valve, such as the mitral valve or tricuspid valve. Various features and steps as described elsewhere in this disclosure may be included in the examples summarized here. Further, the treatment techniques, methods, operations, steps, etc. described or suggested herein can be performed on a living animal or on a non-living simulation, such as on a cadaver, cadaver heart, simulator (e.g. with the body parts, tissue, etc. being simulated), etc.

In some implementations, an example system includes an implantable device, a delivery catheter, a coupler, an actuation element (e.g., actuation wire, actuation shaft, actuation tube, actuation rod, etc.), and a tether. The implantable device has a pair of anchors that are movable between an open position and a closed position to secure the implantable device to a native valve. The coupler is disposed at a distal end of the delivery catheter. The actuation element which extends through the delivery catheter and into the device. The coupler and the collar are tied by the coupling tether which can recouple the device to the coupler after the actuation element has been removed from the device.

In some implementations, an example system includes an implantable device having at least one clasp, a delivery catheter, and a clasp actuation line. The clasp actuation line has two portions that both extend from the delivery catheter and that both pass through a loop of the clasp. A looped end of the clasp actuation line is releasably coupled to at least one of the delivery catheter and the implantable device. The at least one clasp is movable from a closed position to an open position by pulling the clasp actuation line. The at least one clasp is configured to secure the implantable device to a native valve by moving the clasp from the open position to the closed position.

In some implementations, an example system includes an implantable device having at least one clasp, a delivery catheter, a clasp actuation line having a looped end, and a clasp actuation element (e.g., clasp actuation wire, clasp actuation rod, etc.). The clasp actuation line extends from the delivery catheter and passes through a loop of the clasp. The clasp actuation element has an end portion that is moveable from a hooked configuration to a straight configuration. A looped end of the clasp actuation line is releasably coupled to the end portion of the clasp actuation element/wire. The at least one clasp is movable from a closed position to an open position by pulling at least one of the clasp actuation line and/or the clasp actuation element/wire. The at least one clasp is configured to secure the implantable device to a native valve by moving the clasp from the open position to the closed position.

In some implementations, an example system includes an implantable device having at least one clasp, a delivery catheter, and at least one clasp actuation line. The clasp actuation line has a first portion and a second portion that both extend from the delivery catheter and that both pass through a loop of the clasp. A looped end of the first clasp actuation line is tied to at least one of the delivery catheter and the implantable device by a releasable knot. The at least one clasp is movable from a closed position to an open position by pulling the first portion of the clasp actuation line. The releasable knot is untied by pulling the second portion of the clasp actuation line to release the at least one clasp from the clasp actuation line. The at least one clasp is configured to secure the implantable device to a native valve by moving the clasp from the open position to the closed position.

In some implementations, an example system includes an implantable device including a pair of anchors, a collar attached to the device, a delivery catheter, a coupler connected to the catheter, a compressible sleeve, and an actuation element (e.g., actuation wire, actuation shaft, actuation tube, actuation rod, etc.). The compressible sleeve is disposed between the collar and the coupler. The compressible sleeve encases a coupling tether. The actuation wire is coupled (e.g., directly or indirectly) to the pair of anchors for moving the pair of anchors between the open position and the closed position.

In some implementations, an example system includes an implantable device, a delivery catheter, a coupler, a collar, an actuation element (e.g., actuation wire, actuation shaft, actuation tube, actuation rod, etc.), and a pair of clasp actuation lines. The implantable device can have a pair of paddles that are movable between an open position and a closed position and a pair of gripping clasps that secure the implantable device to the native valve. The actuation element extends through the delivery catheter and into the device. The clasp actuation lines each extend through the delivery catheter, through a fastener on one of the pair of gripping clasps, around the actuation element, back through the fastener, and back through the delivery catheter.

In some example methods, a previously implanted valve repair device is observed and recoupled to a native valve of a patient. The previously implanted valve repair device has a pair of anchors that are movable between an open position and a closed position to secure the implantable device to a native valve, and a collar. In the method, an actuation element (e.g., actuation wire, actuation shaft, actuation tube, actuation rod, etc.) is retracted from the implanted valve repair device. A delivery catheter and a coupler are retracted from the implanted valve repair device. Slack is introduced to a coupling tether that couples the implanted valve repair device. The condition of the implanted valve repair device is observed. The coupling tether is pulled, and the coupler is advanced to bring the coupler back to the collar. The actuation element is advanced into the implanted valve repair device. The valve repair device is opened with the actuation element and the valve repair device is repositioned. The pair of anchors are again moved to the closed position. The actuation element is again retracted from the implanted valve repair device. The delivery catheter and the coupler are again retracted from the implanted valve repair device. The coupling tether is decoupled from the valve repair device. The delivery catheter, the actuation element, the coupler, the coupling tether, and the clasp actuation line are removed, leaving the repositioned valve repair device implanted on the native valve.

In some implementations, an example implantable prosthetic device includes a plurality of paddles, a cap, a caption portion, and an extendable coupler. The cap is connected to each of the paddles. A coaption portion is connected to each of the paddles. Movement of the cap relative to the coaption portion opens and closes the plurality of paddles. The extendable coupler is affixed to the cap and the coaption portion such that the extendable coupler extends when the cap moves away from the coaption portion.

In some implementations, an example implantable prosthetic device includes a pair of anchors, a coaption portion, and a collar. The pair of anchors are movable between an open position and a closed position to secure the implantable device to a native valve. The coaption portion is connected to the pair of anchors. The coaption portion is made from a plurality of wires. A plurality of ends of the plurality of wires are gathered into a plurality of bunches. The plurality of bunches are secured in a plurality of openings in the collar to connect the collar to the coaption element.

In some implementations, an example system includes an implantable device, a delivery catheter, and an actuation element (e.g., actuation wire, actuation shaft, actuation tube, actuation rod, etc.). The implantable device has a pair of anchors that are movable between an open position and a closed position to secure the implantable device to a native valve. The actuation element extends through the delivery catheter and is coupled to the device. At least one of the actuation element and the device includes a recapturing feature configured to reconnect the actuation element to the device after an initial disconnection of the actuation element from the device.

In some implementations, an example implantable prosthetic device includes a pair of anchors, a coaption portion, a collar, a cover, and a coupling tether. The pair of anchors are movable between an open position and a closed position to secure the implantable device to a native valve. The coaption portion is connected to the pair of anchors. The collar is connected to the coaption element. The cover is disposed over one or more of the pair of anchors, the coaption portion and the collar. At least a portion of the cover is folded into a tube. The coupling tether extends through the tube.

In some embodiments, an example implantable device has a pair of paddles that are movable between an open position and a closed position and a pair of gripping clasps that secure the implantable device to the native valve. The implantable device includes a delivery catheter, a coupler disposed at a distal end of the delivery catheter, a collar attached to the device; and an actuation element which can extend through the delivery catheter and into the device. In some implementations, the coupler and the collar are coupled via a coupling tether which can recouple the device to the actuation element after the device has been moved to the closed position.

In some implementations, an example implantable device has a pair of paddles that are movable between an open position and a closed position and a pair of gripping clasps that secure the implantable device to the native valve. The implantable device includes a delivery catheter, a coupler disposed at a distal end of the delivery catheter, a collar attached to the device, and an actuation element (e.g., actuation wire, actuation shaft, actuation tube, actuation rod, etc.) which can extend through the delivery catheter and into the device. The coupler and the collar are coupled via a coupling tether which can recouple the device to the actuation element after the device has been moved to the closed position. In some implementations, the coupler and the collar are configured such that pulling an end of the coupling tether results in a 1:2 ratio of movement to a longitudinal position of the collar.

In some embodiments, an example implantable device has a pair of paddles that are movable between an open position and a closed position and a pair of gripping clasps that secure the implantable device to the native valve. The implantable device includes a delivery catheter, a coupler disposed at a distal end of the delivery catheter, a collar attached to the device, and an actuation element (e.g., actuation wire, actuation shaft, actuation tube, actuation rod, etc.) which can extend through the delivery catheter and into the device. The coupler and the collar are coupled via a coupling tether which can recouple the device to the actuation element after the device has been moved to the closed position. In some implementations, the coupler and the collar are configured such that pulling an end of the coupling tether results in a 1:4 ratio of movement to a longitudinal position of the collar.

In some embodiments, an example implantable device has a pair of paddles that are movable between an open position and a closed position and a pair of gripping clasps that secure the implantable device to the native valve. The implantable device includes a delivery catheter having a tether passage disposed at a distal end of the delivery catheter, a collar attached to the device having a tether passage, an actuation element (e.g., actuation wire, actuation shaft, actuation tube, actuation rod, etc.) which can extend through the delivery catheter and into the device, and an outer shaft disposed around the delivery catheter and having a coupling tether extending from a distal end of the outer shaft. The delivery catheter and the collar are coupled via the coupling tether which can recouple the device to the actuation element after the device has been moved to the closed position. In some implementations, the coupling tether extends from the distal end of the outer shaft, through the tether passage of the collar, and through the tether passage of the delivery catheter into the device.

In some implementations, an example implantable device has a pair of paddles that are movable between an open position and a closed position and a pair of gripping clasps that secure the implantable device to the native valve. The implantable device includes a delivery catheter having a tether passage disposed at a distal end of the delivery catheter, a collar attached to the device having a tether passage, an actuation element (e.g., actuation wire, actuation shaft, actuation tube, actuation rod, etc.) which can extend through the delivery catheter and into the device, and an outer shaft disposed around the delivery catheter and having a coupling tether extending from a distal end of the outer shaft. The delivery catheter and the collar are coupled via the coupling tether which can recouple the device to the actuation element after the device has been moved to the closed position. In some implementations, the coupling tether extends from the distal end of the outer shaft, through the tether passage of the delivery catheter, and through the tether passage of the collar into the device.

In some embodiments, an example implantable device has a pair of paddles that are movable between an open position and a closed position and a pair of gripping clasps that secure the implantable device to the native valve. The implantable device has a delivery catheter having a lumen extending longitudinally through the delivery catheter, a collar attached to the device having a tether passage, and an actuation element (e.g., actuation wire, actuation shaft, actuation tube, actuation rod, etc.) which can extend through the delivery catheter and into the device. The delivery catheter and the collar are coupled via the coupling tether which can recouple the device to the actuation element after the device has been moved to the closed position. In some implementations, the coupling tether extends through the lumen of the delivery catheter and through the tether passage of the collar into the device.

In some implementations, an example implantable device has a pair of paddles that are movable between an open position and a closed position and a pair of gripping clasps that secure the implantable device to the native valve. The implantable device can have a delivery catheter, a coupler disposed at a distal end of the delivery catheter, a collar attached to the device, a compressible sleeve disposed between the collar and the coupler which encase a coupling tether, an actuation element (e.g., actuation wire, actuation shaft, actuation tube, actuation rod, etc.) which can extend through the delivery catheter and into the device, or a combination of some or all of these. In some implementations, the coupler and the collar are coupled via the coupling tether which can recouple the device to the actuation element after the device has been moved to the closed position.

In some implementations, an example implantable device has a pair of paddles that are movable between an open position and a closed position and a pair of gripping clasps that secure the implantable device to the native valve. The implantable device can have a delivery catheter, a coupler disposed at a distal end of the delivery catheter and having a flange at a distal end of the coupler, a collar attached to the device, an actuation element (e.g., actuation wire, actuation shaft, actuation tube, actuation rod, etc.) which can extend through the delivery catheter and into the device, or a combination of some or all of these. In some implementations, the coupler and the collar are coupled via a coupling tether which can recouple the device to the actuation element after the device has been moved to the closed position.

In some embodiments, an example method observes and recouples a previously implanted valve repair device from a native valve of a patient, where the previously implanted valve repair device has a pair of paddles that are moveable between an open position and a closed position, a pair of gripping clasps that secure the previously implanted valve repair device to the native valve, and a collar. In some implementations, the method comprises one, some, or all of the following: retracting an actuation element (e.g., actuation wire, actuation shaft, actuation tube, actuation rod, etc.) from the implanted valve repair device, retracting a delivery catheter and a coupler from the implanted valve repair device and introducing slack into a coupling tether which couples the coupler and a collar of the implanted valve repair device, observing the condition of the implanted valve repair device, pulling the coupling tether to bring the coupler back toward the collar and advancing an actuation element into the implanted valve repair device to engage a cap of the implanted valve repair device, opening the valve repair device via the actuation element and a clasp actuation line, repositioning the valve repair device, moving the valve repair device to a closed position, retracting the actuation element from the implanted valve repair device; retracting the delivery catheter and the coupler from the implanted valve repair device, decoupling the coupling tether and the clasp actuation line from the valve repair device, removing the delivery catheter, the actuation element, the coupler, the coupling tether, and the clasp actuation line.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the figures can be drawn to scale for some embodiments, the figures are not necessarily drawn to scale for all embodiments. Embodiments and other features and advantages of the present disclosure will be described and explained with additional specificity and detail through the use of the accompanying drawings in which:
Figure 1 illustrates a cutaway view of the human heart in a diastolic phase;
Figure 2 illustrates a cutaway view of the human heart in a systolic phase;
Figure 3 illustrates a cutaway view of the human heart in a diastolic phase, in which the chordae tendineae are shown attaching the leaflets of the mitral and tricuspid valves to ventricle walls;
Figure 4 illustrates a healthy mitral valve with the leaflets closed as viewed from an atrial side of the mitral valve;
Figure 5 illustrates a dysfunctional mitral valve with a visible gap between the leaflets as viewed from an atrial side of the mitral valve;
Figure 6 illustrates a mitral valve having a wide gap between the posterior leaflet and the anterior leaflet;
Figure 7 illustrates a tricuspid valve viewed from an atrial side of the tricuspid valve;
Figures 8-14M show an example embodiment of an implantable prosthetic device, in various stages of deployment and redeployment;
Figures 15-20M show the example implantable prosthetic device of Figures 8-14M being delivered and implanted, repositioned, and replanted within the native mitral valve;
Figure 21 shows an example embodiment of an implantable prosthetic device;
Figure 21A shows an example embodiment of an implantable prosthetic device;
Figures 22A-22M show an example embodiment of an implantable prosthetic device, in various stages of deployment and redeployment;
Figures 23A-23M show an example embodiment of an implantable prosthetic device, in various stages of deployment and redeployment;
Figures 24A-24E shown an example embodiment of a collar and a coupler which can be used with an implantable prosthetic device, in various stages of deployment;
Figures 25A-25G show an example embodiment of a collar and a coupler which can be used with an implantable prosthetic device, in various stages of deployment;
Figures 26A-26E show an example embodiment of an implantable prosthetic device, in various stages of deployment and redeployment;
Figures 27A-27E show an example embodiment of an implantable prosthetic device, in various stages of deployment and redeployment;
Figures 28A-28F show an example embodiment of an implantable prosthetic device, in various stages of deployment and redeployment;
Figures 29A-29O show an example embodiment of an implantable prosthetic device, in various stages of deployment and redeployment;
Figures 30A-30L show the implantable prosthetic device of Figures 29A-29O being delivered and implanted, repositioned, and replanted within a native valve;
Figures 31A and 31B show a delivery catheter with a compressible sleeve for an implantable prosthetic device;
Figures 31C and 31D show a delivery catheter with a compressible sleeve for an implantable prosthetic device;
Figures 31E and 31F show a delivery catheter with a compressible sleeve for an implantable prosthetic device;
Figures 31G-31I illustrate use of the delivery catheter of Figures 31E and 31F to release an implantable prosthetic device;
Figures 32A and 32B show a compressible sleeve in compressed and elongated positions respectively;
Figures 33A and 33B show a compressible sleeve in elongated and compressed positions respectively;
Figures 34A and 34B show an example embodiment of an implantable prosthetic device;
Figure 35 illustrates an example implantable prosthetic device with an example extendable coupler;
Figure 36 is a cross-sectional view of the example extendable coupler of Figure 35 without the prosthetic device;
Figure 37 illustrates the example extendable coupler of Figure 36 connected to an actuation element;
Figure 38 illustrates the example extendable coupler of Figure 36 moved to a partially extended condition by the actuation element;
Figure 39 illustrates the example extendable coupler of Figure 36 moved to a fully extended condition by the actuation element;
Figure 40 illustrates the example extendable coupler of Figure 39 moved to a partially retracted condition by the actuation element;
Figure 41 illustrates the example extendable coupler of Figure 39 moved to a fully retracted condition by the actuation element;
Figure 42 illustrates the example implantable prosthetic device of Figure 35 with the coupler moved to a partially extended condition by the actuation element;
Figure 43 illustrates the example implantable prosthetic device of Figure 35 with the coupler moved to a fully extended condition by the actuation element;
Figure 44 illustrates the example implantable prosthetic device of Figure 43 with the coupler moved to a partially retracted condition by the actuation element;
Figure 45 illustrates the example implantable prosthetic device of Figure 43 with the coupler moved to a fully retracted condition by the actuation element;
Figure 46 illustrates the example implantable prosthetic device of Figure 35 released from a delivery catheter;
Figure 47 illustrates an example embodiment of an implantable prosthetic device with an example extendable coupler;
Figure 48 illustrates the example implantable prosthetic device of Figure 47 with the coupler moved to a partially extended condition by the actuation element;
Figure 49 illustrates the example implantable prosthetic device of Figure 47 with the coupler moved to a fully extended condition by the actuation element;
Figure 50 illustrates the example implantable prosthetic device of Figure 49 with the coupler moved to a partially retracted condition by the actuation element;
Figure 51 illustrates the example implantable prosthetic device of Figure 49 with the coupler moved to a fully retracted condition by the actuation element;
Figure 52 illustrates an exemplary embodiment of an implantable prosthetic device coupled to a delivery system;
Figure 53 illustrates a control line or suture of the delivery system of Figure 52 looped through a control ring;
Figure 54 illustrate a delivery system with control lines or sutures looped through control rings and wrapped around a paddle control shaft;
Figure 55 illustrate a delivery system with one of the control lines or sutures of Figure 54 to clarify the drawing;
Figure 56 illustrate a delivery system with one of the control lines or sutures of Figure 54 to simplify the drawing;
Figures 57-60 illustrate deployment of an implantable device using the delivery system illustrated by Figure 52;
Figure 61 illustrates an exemplary embodiment of a clasp actuation arrangement;
Figures 62-64 illustrate an exemplary embodiment of an implantable prosthetic device coupled to a delivery system;
Figure 65 illustrates an exemplary embodiment of a keyed coupling for coupling an implantable prosthetic device to a delivery system;
Figure 66 illustrates an exemplary embodiment of a collar and a coupler which can be used with an implantable prosthetic device;
Figure 67 illustrates an exemplary embodiment of a collar and a coupler which can be used with an implantable prosthetic device;
Figure 68 illustrate an exemplary embodiment of an implantable prosthetic device coupled to a delivery system;
Figures 69-71 illustrate release of a control line or suture from a control ring of the delivery system illustrated by Figure 68;
Figures 72-77 illustrate deployment of an implantable device using the delivery system illustrated by Figure 68;
Figure 78 illustrate an exemplary embodiment of an implantable prosthetic device coupled to a delivery system;
Figure 79 illustrates a control line or suture of the delivery system of Figure 78 tied to a control ring;
Figures 80-84 illustrate an exemplary embodiment of a releasable knot;
Figure 85 illustrates an exemplary embodiment of a releasable knot;
Figures 86-89 illustrate deployment of an implantable device using the delivery system illustrated by Figure 78;
Figures 90 and 91 illustrate an exemplary embodiment of a connection between a coaption element and a cap;
Figures 92 and 93 illustrate an exemplary embodiment of a connection between a coaption element and a cap;
Figure 94 illustrates an exemplary embodiment of a cap of a prosthetic device;
Figures 95-97 illustrate an exemplary embodiment of an arrangement for recapturing a prosthetic device;
Figures 98-100 illustrate an exemplary embodiment of an arrangement for recapturing a prosthetic device;
Figure 101 illustrates an exemplary embodiment of a cap of a prosthetic device;
Figures 102-104 illustrate an exemplary embodiment of an arrangement for recapturing a prosthetic device;
Figures 105-107 illustrate an exemplary embodiment of an arrangement for recapturing a prosthetic device;
Figure 108 illustrates an exemplary embodiment of a structure of a cap or collar of a recapturable prosthetic device;
Figure 109 illustrates an exemplary embodiment of a structure of a cap or collar of a recapturable prosthetic device;
Figures 110-112 illustrate an exemplary embodiment of an arrangement for recapturing a prosthetic device;
Figures 113-115 illustrate an exemplary embodiment of an arrangement for recapturing a prosthetic device;
Figure 116 illustrates an exemplary embodiment of a structure of a cap or collar of a recapturable prosthetic device;
Figure 117 illustrates an exemplary embodiment of a structure of a cap or collar of a recapturable prosthetic device;
Figure 118 is a perspective view of the structures illustrated by Figures 116 and 117;
Figures 119 and 120 are perspective views of an exemplary embodiment of a tethered implantable prosthetic device;
Figure 121 is a partial perspective view of an exemplary embodiment of a tetherable implantable prosthetic device;
Figure 122 is a side view of a tether routing structure of the tetherable implantable prosthetic device of Figure 121;
Figure 123 is a partial perspective view of an exemplary embodiment of a tetherable implantable prosthetic device;
Figure 124 is a side view of a tether routing structure of the tetherable implantable prosthetic device of Figure 123;
Figure 125 is a partial perspective view of an implantable prosthetic device with a cover being attached to provide a tether routing structure;
Figure 126 is a partial perspective view of the prosthetic device of Figure 125 with a tether routing structure formed by the cover;
Figures 127-129 illustrate an exemplary embodiment of an implantable prosthetic device with a tether routing structure;
Figure 130 illustrates an exemplary embodiment of an implantable prosthetic device with a tether routing structure; and
Figure 131 illustrates an exemplary embodiment of an implantable prosthetic device with a tether routing structure.

### DETAILED DESCRIPTION

The following description refers to the accompanying drawings, which illustrate specific embodiments of the present disclosure. Other embodiments having different structures and operation do not depart from the scope of the present disclosure.

Example embodiments of the present disclosure are directed to devices and methods for repairing a defective heart valve. Various embodiments of native valve repair devices, systems for delivery of native valve repair devices, and systems for removal of implanted native valve repair devices are disclosed herein, and any combination of these options can be made unless specifically excluded. In other words, individual components of the disclosed devices and systems can be combined unless mutually exclusive or otherwise physically impossible.

As described herein, when one or more components are described as being connected, joined, affixed, coupled, attached, or otherwise interconnected, such interconnection can be direct as between the components or can be indirect such as through the use of one or more intermediary components. Also, as described herein, reference to a "member," "component," or "portion" shall not be limited to a single structural member, component, or element but can include an assembly of components, members, or elements. Also, as described herein, the terms "substantially" and "about" are defined as at least close to (and includes) a given value or state (preferably within 10% of, more preferably within 1% of, and most preferably within 0.1% of).

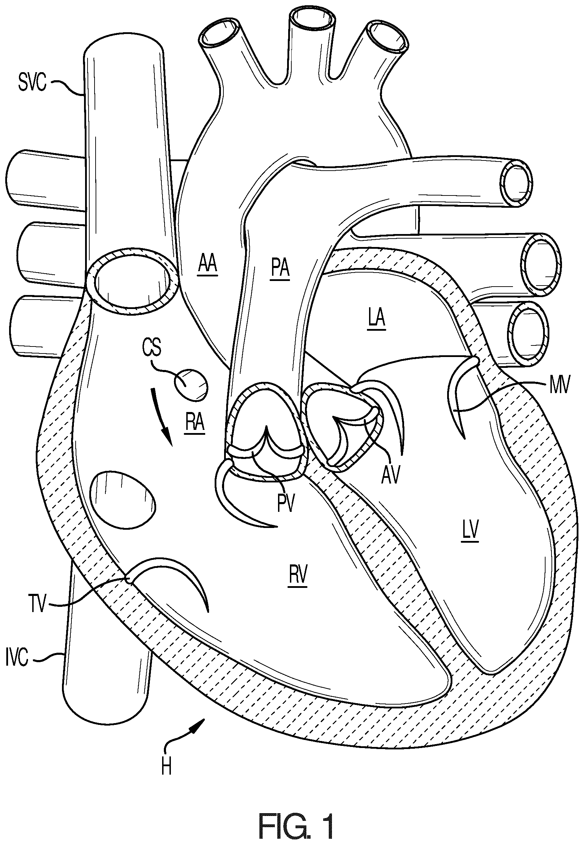

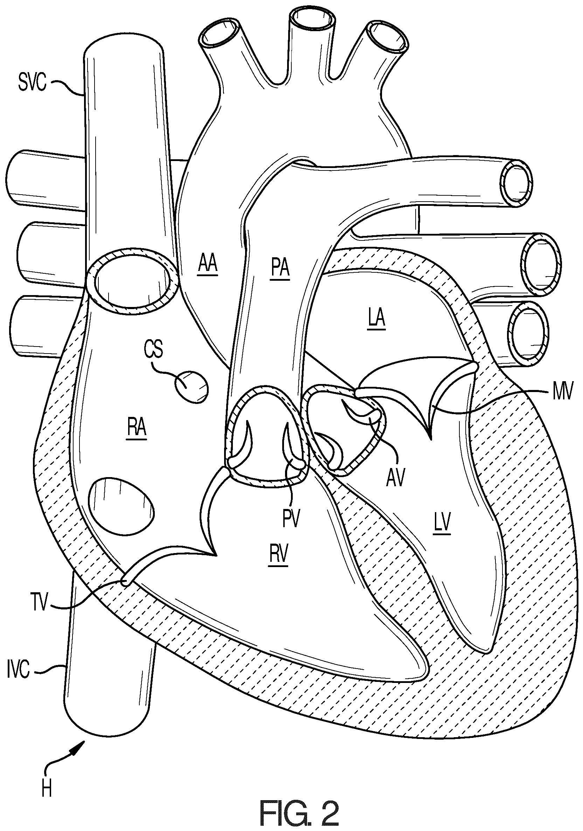

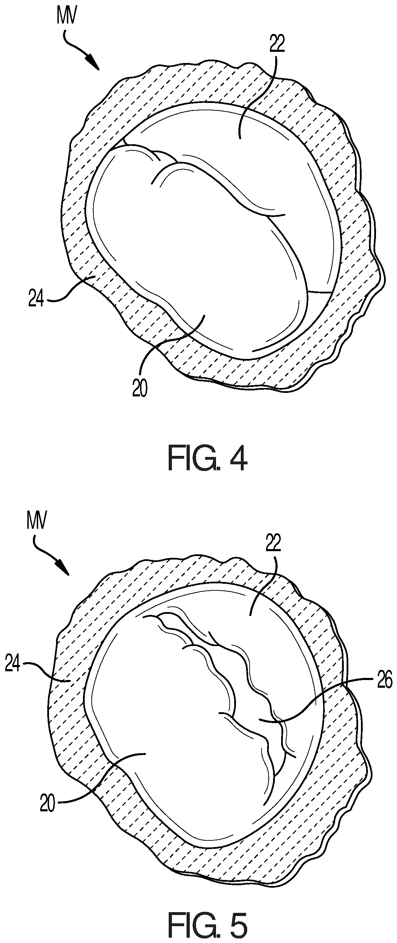

Figures 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta AA, and the pulmonary valve PV separates the right ventricle from the pulmonary artery PA. Each of these valves has flexible leaflets (e.g., leaflets 20, 22 shown in Figures 4 and 5) extending inward across the respective orifices that come together or "coapt" in the flowstream to form the one-way, fluid-occluding surfaces. The native valve repair systems of the present application are frequently described and/or illustrated with respect to the mitral valve MV. Therefore, anatomical structures of the left atrium LA and left ventricle LV will be explained in greater detail. However, it should be understood that the devices described herein may also be used in repairing other native valves, e.g., the devices can be used in repairing the tricuspid valve TV, the aortic valve AV, and the pulmonary valve PV.

The left atrium LA receives oxygenated blood from the lungs. During the diastolic phase, or diastole, seen in Figure 1, the blood that was previously collected in the left atrium LA (during the systolic phase) moves through the mitral valve MV and into the left ventricle LV by expansion of the left ventricle LV. In the systolic phase, or systole, seen in Figure 2, the left ventricle LV contracts to force the blood through the aortic valve AV and ascending aorta AA into the body. During systole, the leaflets of the mitral valve MV close to prevent the blood from regurgitating from the left ventricle LV and back into the left atrium LA, and blood is collected in the left atrium from the pulmonary vein. In one example embodiment, the devices described by the present application are used to repair the function of a defective mitral valve MV. That is, the devices are configured to help close the leaflets of the mitral valve to prevent blood from regurgitating from the left ventricle LV and back into the left atrium LA.

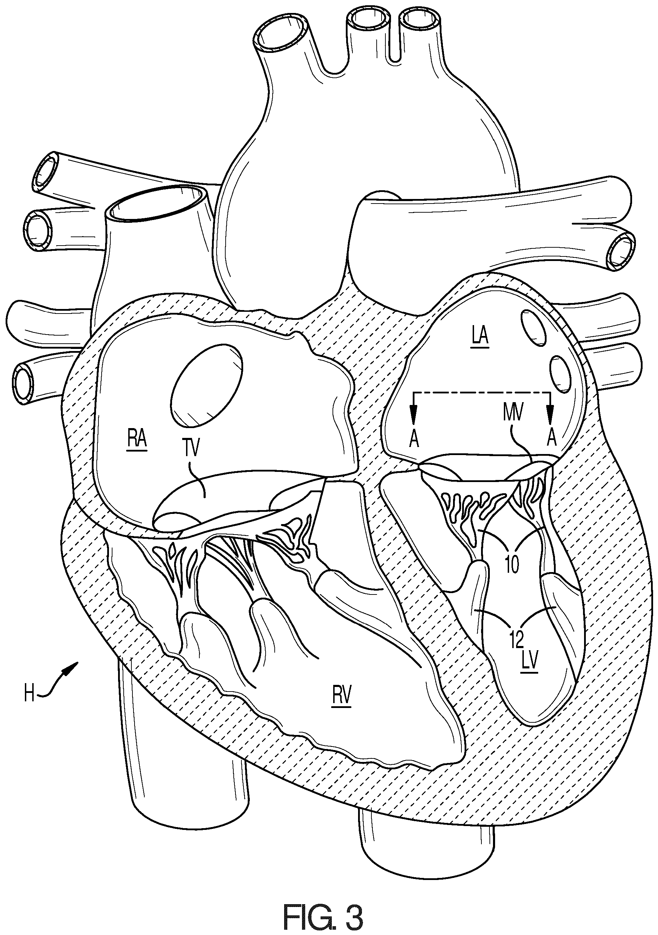

Referring now to Figures 1-6, the mitral valve MV includes two leaflets, the anterior leaflet 20 and the posterior leaflet 22. The mitral valve MV also includes an annulus 24, which is a variably dense fibrous ring of tissue that encircles the leaflets 20, 22. Referring to Figure 3, the mitral valve MV is anchored to the wall of the left ventricle LV by chordae tendineae 10. The chordae tendineae 10 are cord-like tendons that connect the papillary muscles 12 (i.e., the muscles located at the base of the chordae tendineae and within the walls of the left ventricle) to the leaflets 20, 22 of the mitral valve MV. The papillary muscles 12 serve to limit the movements of the mitral valve MV and prevent the mitral valve from being reverted. The mitral valve MV opens and closes in response to pressure changes in the left atrium LA and the left ventricle LV. The papillary muscles do not open or close the mitral valve MV. Rather, the papillary muscles brace the mitral valve MV against the high pressure needed to circulate blood throughout the body. Together the papillary muscles and the chordae tendineae are known as the subvalvular apparatus, which functions to keep the mitral valve MV from prolapsing into the left atrium LA when the mitral valve closes.

Various disease processes can impair proper function of one or more of the native valves of the heart H. These disease processes include degenerative processes (e.g., Barlow's Disease, fibroelastic deficiency, etc.), inflammatory processes (e.g., Rheumatic Heart Disease, etc.), and infectious processes (e.g., endocarditis, etc.). In addition, damage to the left ventricle LV or the right ventricle RV from prior heart attacks (i.e., myocardial infarction secondary to coronary artery disease) or other heart diseases (e.g., cardiomyopathy) can distort a native valve's geometry, which can cause the native valve to dysfunction. However, the majority of patients undergoing valve surgery, such as surgery to the mitral valve MV, suffer from a degenerative disease that causes a malfunction in a leaflet (e.g., leaflets 20, 22) of a native valve (e.g., the mitral valve MV), which results in prolapse and regurgitation.

Generally, a native valve may malfunction in different ways, including: (1) valve stenosis; and (2) valve regurgitation. Valve stenosis occurs when a native valve does not open completely and thereby causes an obstruction of blood flow. Typically, valve stenosis results from buildup of calcified material on the leaflets of a valve, which causes the leaflets to thicken and impairs the ability of the valve to fully open to permit forward blood flow.

Valve regurgitation occurs when the leaflets of the valve do not close completely thereby causing blood to leak back into the prior chamber (e.g., causing blood to leak from the left ventricle to the left atrium). There are three main mechanisms by which a native valve becomes regurgitant-or incompetent-which include Carpentier's type I, type II, and type III malfunctions. A Carpentier type I malfunction involves the dilation of the annulus such that normally functioning leaflets are distracted from each other and fail to form a tight seal (i.e., the leaflets do not coapt properly). Included in a type I mechanism malfunction are perforations of the leaflets, as are present in endocarditis. A Carpentier's type II malfunction involves prolapse of one or more leaflets of a native valve above a plane of coaption. A Carpentier's type III malfunction involves restriction of the motion of one or more leaflets of a native valve such that the leaflets are abnormally constrained below the plane of the annulus. Leaflet restriction can be caused by rheumatic disease (Ma) or dilation of a ventricle (IIIb).

Referring to Figure 4, when a healthy mitral valve MV is in a closed position, the anterior leaflet 20 and the posterior leaflet 22 coapt, which prevents blood from leaking from the left ventricle LV to the left atrium LA. Referring to Figure 5, regurgitation occurs when the anterior leaflet 20 and/or the posterior leaflet 22 of the mitral valve MV is displaced into the left atrium LA during systole. This failure to coapt causes a gap 26 between the anterior leaflet 20 and the posterior leaflet 22, which allows blood to flow back into the left atrium LA from the left ventricle LV during systole. As set forth above, there are several different ways that a leaflet (e.g. leaflets 20, 22 of mitral valve MV) may malfunction, which can thereby lead to regurgitation.

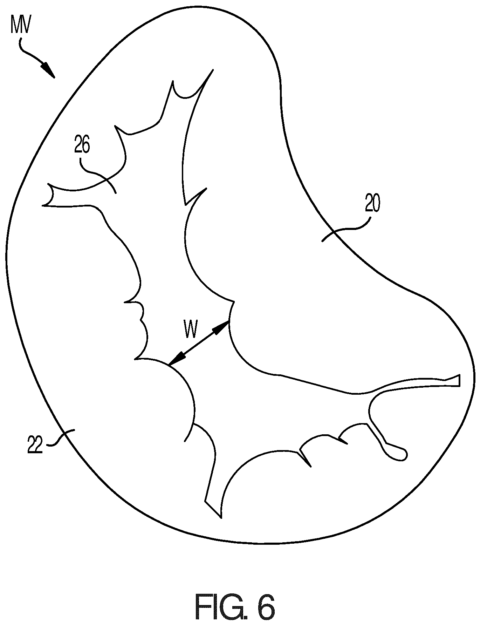

Referring to Figure 6, in certain situations, the mitral valve MV of a patient can have a wide gap 26 between the anterior leaflet 20 and the posterior leaflet 22 when the mitral valve is in a closed position (i.e., during the systolic phase). For example, the gap 26 can have a width W between about 2.5 mm and about 17.5 mm, such as between about 5 mm and about 15 mm, such as between about 7.5 mm and about 12.5 mm, such as about 10 mm. In some situations, the gap 26 can have a width W greater than 15 mm. In any of the above-mentioned situations, a valve repair device is desired that is capable of engaging the anterior leaflet 20 and the posterior leaflet 22 to close the gap 26 and prevent regurgitation of blood through the mitral valve MV.

Although stenosis or regurgitation can affect any valve, stenosis is predominantly found to affect either the aortic valve AV or the pulmonary valve PV, and regurgitation is predominantly found to affect either the mitral valve MV or the tricuspid valve TV. Both valve stenosis and valve regurgitation increase the workload of the heart H and may lead to very serious conditions if left un-treated; such as endocarditis, congestive heart failure, permanent heart damage, cardiac arrest, and ultimately death. Because the left side of the heart (i.e., the left atrium LA, the left ventricle LV, the mitral valve MV, and the aortic valve AV) is primarily responsible for circulating the flow of blood throughout the body and experiences higher pressures, malfunction of the mitral valve MV or the aortic valve AV is particularly problematic and often life threatening.

Malfunctioning native heart valves may either be repaired or replaced. Repair typically involves the preservation and correction of the patient's native valve. Replacement typically involves replacing the patient's native valve with a biological or mechanical substitute. Typically, the aortic valve AV and pulmonary valve PV are more prone to stenosis. Because stenotic damage sustained by the leaflets is irreversible, treatments for a stenotic aortic valve or stenotic pulmonary valve can be removal and replacement of the valve with a surgically implanted heart valve, or displacement of the valve with a transcatheter heart valve. The mitral valve MV and the tricuspid valve TV are more prone to deformation of leaflets and/or surrounding tissue, which, as described above, prevents the mitral valve or tricuspid valve from closing properly and allows for regurgitation or back flow of blood from the ventricle into the atrium (e.g., a deformed mitral valve MV may allow for regurgitation or back flow from the left ventricle LV to the left atrium LA). The regurgitation or back flow of blood from the ventricle to the atrium results in valvular insufficiency. Deformations in the structure or shape of the mitral valve MV or the tricuspid valve TV are often repairable. In addition, regurgitation can occur due to the chordae tendineae 10 becoming dysfunctional (e.g., the chordae tendineae may stretch or rupture), which allows the anterior leaflet 20 and the posterior leaflet 22 to be reverted such that blood is regurgitated into the left atrium LA. The problems occurring due to dysfunctional chordae tendineae can be repaired by repairing the chordae tendineae or the structure of the mitral valve (e.g., by securing the leaflets 20, 22 at the affected portion of the mitral valve).

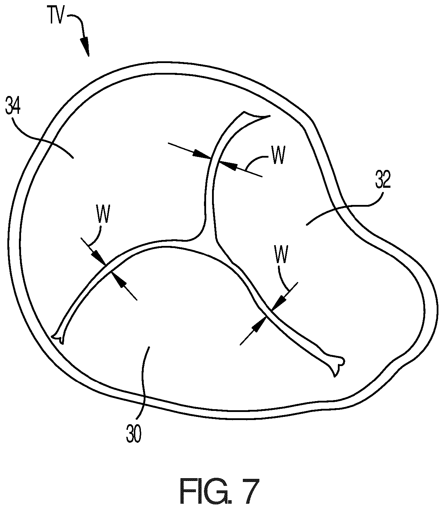

The devices and procedures disclosed herein often make reference to repairing the structure of a mitral valve or removing an implanted repair device from the mitral valve for illustration. However, it should be understood that the devices and concepts provided herein can be used to repair any native valve, as well as any component of a native valve or can be used to remove an implanted repair device from any native valve. For example, referring now to Figure 7, any of the devices and concepts provided herein can be used to repair the tricuspid valve TV or remove an implanted repair device from the tricuspid valve. For example, any of the devices and concepts provided herein can be used between any two of the anterior leaflet 30, septal leaflet 32, and posterior leaflet 34 to prevent or inhibit regurgitation of blood from the right ventricle into the right atrium, and the devices and concepts can be used to remove an implanted repair device from between any two of the anterior leaflet 30, septal leaflet 32, and posterior leaflet 34. In addition, any of the devices and concepts provided herein can be used on all three of the leaflets 30, 32, 34 together to prevent or inhibit regurgitation of blood from the right ventricle to the right atrium, or to remove a repair device from between all three leaflets 30, 32, 34 of the tricuspid valve. That is, the valve repair devices provided herein can be centrally located between the three leaflets 30, 32, 34.

An example implantable prosthetic device can optionally have a coaption element (e.g., spacer, coaptation element, etc.) and at least one anchor (e.g., one, two, three, or more). In some implementations, the coaption element is configured to be positioned within the native heart valve orifice to help fill the space between the leaflets and form a more effective seal, thereby reducing or preventing regurgitation described above. The coaption element can have a structure that is impervious to blood (or that resists blood flow therethrough) and that allows the native leaflets to close around the coaption element during ventricular systole to block blood from flowing from the left or right ventricle back into the left or right atrium, respectively. The prosthetic device can be configured to seal against two or three native valve leaflets; that is, the device can be used in the native mitral (bicuspid) and tricuspid valves. The coaption element is sometimes referred to herein as a spacer because a spacer can be a coaption element that can fill a space between improperly functioning leaflets (e.g., native mitral or tricuspid leaflets, etc.) that do not close completely.

The optional coaption element (e.g., spacer, coaptation element, etc.) can have various shapes. In some embodiments, the coaption element can have an elongated cylindrical shape having a round cross-sectional shape. In some embodiments, the coaption element can have an oval cross-sectional shape, an ovoid cross-sectional shape, a crescent cross-sectional shape, a rectangular cross-sectional shape, or various other non-cylindrical shapes. In some embodiments, the coaption element can have an atrial portion positioned in or adjacent to the atrium, a ventricular or lower portion positioned in or adjacent to the ventricle, and a side surface that extends between the native leaflets. In embodiments configured for use in the tricuspid valve, the atrial or upper portion is positioned in or adjacent to the right atrium, and the ventricular or lower portion is positioned in or adjacent to the right ventricle, and the side surface that extends between the native tricuspid leaflets.

In some embodiments, the anchor can be configured to secure the device to one or both of the native leaflets such that the coaption element is positioned between the two native leaflets. In embodiments configured for use in the tricuspid valve, the anchor is configured to secure the device to one, two, or three of the tricuspid leaflets such that the coaption element is positioned between the three native leaflets. In some embodiments, the anchor can attach to the coaption element at a location adjacent the ventricular portion of the coaption element. In some embodiments, the anchor can attach to an actuation element, such as a shaft or actuation wire, to which the coaption element is also attached. In some embodiments, the anchor and the coaption element can be positioned independently with respect to each other by separately moving each of the anchor and the coaption element along the longitudinal axis of the actuation element (e.g., actuation shaft, actuation rod, actuation tube, actuation wire, etc.). In some embodiments, the anchor and the coaption element can be positioned simultaneously by moving the anchor and the coaption element together along the longitudinal axis of the actuation element, e.g., shaft, actuation wire, etc.). The anchor can be configured to be positioned behind a native leaflet when implanted such that the leaflet is grasped by the anchor.

The prosthetic device can be configured to be implanted via a guide/delivery sheath, a steerable catheter, and/or an implant catheter. The coaption element and the anchor can be compressible to a radially compressed state and can be self-expandable to a radially expanded state when compressive pressure is released. The device can be configured for the anchor to be expanded radially away from the still-compressed coaption element initially in order to create a gap between the coaption element and the anchor. A native leaflet can then be positioned in the gap. The coaption element can be expanded radially, closing the gap between the coaption element and the anchor and capturing the leaflet between the coaption element and the anchor. In some embodiments, the anchor and coaption element are optionally configured to self-expand. The implantation methods for various embodiments can be different and are more fully discussed below with respect to each embodiment. Additional information regarding these and other delivery methods can be found in U.S. Pat. No. 8,449,599 and U.S. Patent Application Publication Nos. 2014/0222136, 2014/0067052, 2016/0331523, US Provisional Patent Application Serial No. 62/744,031 (filed on October 10, 2018), and PCT patent application publication No. WO2020/076898, each of which is incorporated herein by reference in its entirety for all purposes. These methods can be performed on a living animal or on a simulation, such as on a cadaver, cadaver heart, simulator (e.g. with the body parts, heart, tissue, etc. being simulated), etc. *mutatis mutandis.*

The disclosed prosthetic devices can be configured such that the anchor is connected to a leaflet, taking advantage of the tension from native chordae tendineae to resist high systolic pressure urging the device toward the left atrium. During diastole, the devices can rely on the compressive and retention forces exerted on the leaflet that is grasped by the anchor.

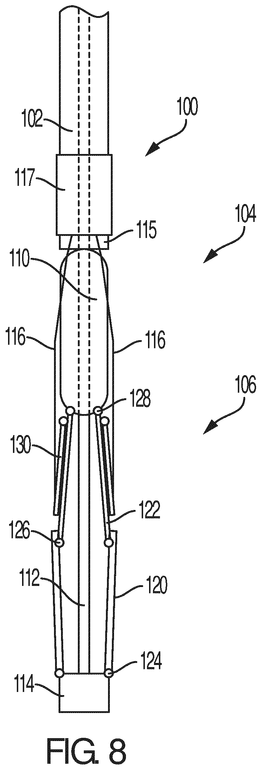

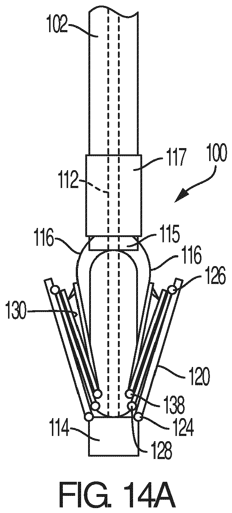

Referring now to Figures 8-14M, a schematically illustrated implantable prosthetic device 100 (e.g., a prosthetic spacer device, valve repair device, etc.) is shown in various stages of deployment. The prosthetic device 100 and other similar prosthetic devices are described in more detail in PCT patent application publication Nos. WO2018/195215, WO2020/076898, and WO 2019/139904, which are incorporated herein by reference in their entirety. The device 100 can include any other features for an implantable prosthetic device discussed in the present application or the applications cited above, and the device 100 can be positioned to engage valve tissue (e.g., leaflets 20, 22) as part of any suitable valve repair system (e.g., any valve repair system disclosed in the present application or the applications cited above). The tethering embodiments disclosed by the present application can be used with any implantable prosthetic device, such as any transcatheter mitral valve repair device and any transcatheter tricuspid valve repair device. The implantable devices disclosed herein are just a few examples of the many implantable devices that the tethering embodiments disclosed herein can be used with. As such, the tethering embodiments disclosed herein can be used with implantable devices that do not include all or any of the features of the implantable devices disclosed herein.

The device 100 is deployed from a delivery or implant catheter/sheath 102 and includes a coaptation portion or coaption portion 104 and an anchor portion 106. One or more additional sheaths or catheters can be disposed around and/or inside the delivery catheter 102. For example, in one example embodiment, the delivery catheter 102 is disposed in a guide or introducing sheath and a positioning catheter. The guide or introducing sheath can be used to position the positioning catheter at a first location, such as the left or right atrium. The positioning catheter can then be extended from the guide or introducing sheath to position the delivery or implant catheter at the delivery site, such as in the left or right ventricle at the leaflets of the mitral valve or tricuspid valve. One or more of the guide sheath, the positioning catheter, and the implant or delivery catheter can optionally be steerable. In the following examples, only the delivery or implant catheter is shown to simplify the drawings.

In some embodiments, the coaption portion 104 of the device 100 includes a means for coapting or coaption element 110 (e.g., spacer, plug, sheet, membrane, etc.) that is adapted to be implanted between leaflets of a native valve (e.g., a native mitral valve, tricuspid valve, etc.) and is slidably attached to an actuation element 112 (e.g., actuation wire, actuation shaft, actuation tube, etc.). The anchor portion 106 includes one or more anchors 108 that are actuatable between open and closed conditions and can take a wide variety of forms, such as, for example, paddles, gripping elements, or the like. Actuation of the means for actuating or actuation element 112 opens and closes the anchor portion 106 of the device 100 to grasp the native valve leaflets during implantation. The means for actuating or actuation element 112 (as well as other means for actuating and actuation elements herein) can take a wide variety of different forms (e.g., as a wire, rod, shaft, tube, screw, suture, line, strip, combination of these, etc.), be made of a variety of different materials, and have a variety of configurations. As one example, the actuation element can be threaded such that rotation of the actuation element moves the anchor portion 106 relative to the coaption portion 104. Or, the actuation element can be unthreaded, such that pushing or pulling the actuation element 112 moves the anchor portion 106 relative to the coaption portion 104.

The anchor portion 106 and/or anchors of the device 100 include outer paddles 120 and inner paddles 122 that are, in some embodiments, connected between a cap 114 and the means for coapting or coaption element 110 by portions 124, 126, 128. The portions 124, 126, 128 can be jointed and/or flexible to move between all of the positions described below. The interconnection of the outer paddles 120, the inner paddles 122, the coaption element 110, and the cap 114 by the portions 124, 126, and 128 can constrain the device to the positions and movements illustrated herein.

In some embodiments, the means for actuating or actuation element 112 extends through the delivery catheter 102 and the means for coapting or coaption element 110 to the distal end (e.g., a cap 114 or other attachment portion at the distal connection of the anchor portion 106). Extending and retracting the actuation element 112 increases and decreases the spacing between the coaption element 110 and the distal end of the device (e.g., the cap 114 or other attachment portion), respectively. In some implementations, a collar or other attachment element removably attaches the coaption element 110 to the delivery catheter 102, either directly or indirectly, so that the means for actuating or actuation element 112 slides through the collar or other attachment element and, in some embodiments, through a means for coapting or coaption element 110 during actuation to open and close the paddles 120, 122 of the anchor portion 106 and/or anchors 108.

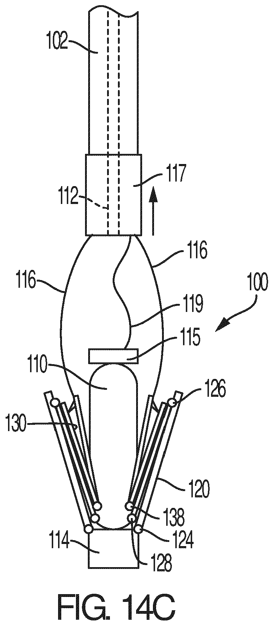

The device 100 can also include a coupler 117 which removably attaches the collar 115 to the delivery catheter 102. The coupler 117 can be removably attached to the collar 115 and can be fixedly or removably attached to the delivery catheter 102. The coupler 117 can attach the delivery catheter 102 to the collar 115 in a wide variety of ways. For example, as will be described with reference to several of the following embodiments one or more tethers (See reference number 119 in Fig. 14C) can pull the coupler 117 against the cap to effectively connect them together. As detailed below, a coupler 117 that utilizes a tether can facilitate the placement, checking for proper deployment, repositioning, and/or replacement of the device 100. Instead or in addition, the coupler 117 can attach the collar 115 to the delivery catheter 102 in any of the ways described in PCT patent application publication WO2020/076898, which are incorporated herein by reference in its entirety.

In some exemplary embodiments, the delivery catheter 102, actuation element 112, coupler 117, and collar 115 can form both a delivery and repositioning system. As will be described later, after the device 100 is connected to valve tissue, if the device 100 needs to be removed from the valve tissue, the coupler 117 and the collar 115 can be used to connect or re-connect to the device 100 such that the actuation element 112 can extend through the coupler 117, the collar 115, and the coaption element 110 to engage the anchor portion 106 to open the paddles 120, 122 and remove the device 100 from the valve tissue.

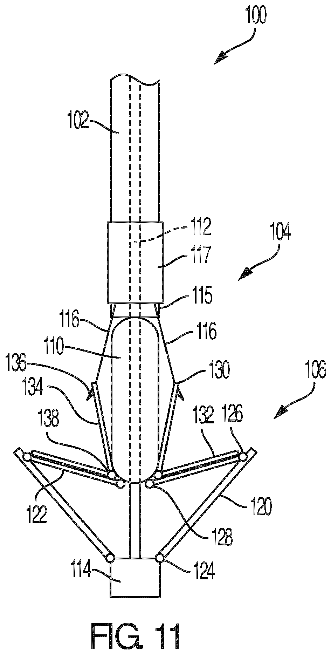

Referring now to Figure 11, the anchor portion 106 and/or anchors include attachment portions or gripping members. The illustrated gripping members can comprise clasps 130 that include a base or fixed arm 132, a moveable arm 134, optional barbs, friction-enhancing elements, or other means for securing 136 (e.g., protrusions, ridges, grooves, textured surfaces, adhesive, etc.), and a joint portion 138. The fixed arms 132 are attached to the inner paddles 122. In some embodiments, the fixed arms 132 are attached to the inner paddles 122 with the joint portion 138 disposed proximate means for coapting or coaption element 110. In some embodiments, the clasps (e.g., barbed clasps, etc.) have flat surfaces and do not fit in a recess of the inner paddle. Rather, the flat portions of the clasps are disposed against the surface of the inner paddle 122. The joint portion 138 provides a spring force between the fixed and moveable arms 132, 134 of the clasp 130. The joint portion 138 can be any suitable joint, such as a flexible joint, a spring joint, a pivot joint, or the like. In some embodiments, the joint portion 138 is a flexible piece of material integrally formed with the fixed and moveable arms 132, 134. The fixed arms 132 are attached to the inner paddles 122 and remain stationary or substantially stationary relative to the inner paddles 122 when the moveable arms 134 are opened to open the clasps 130 and expose the barbs, friction-enhancing elements, or means for securing 136.

In some implementations, the clasps 130 are opened by applying tension to actuation lines 116 attached to the moveable arms 134, thereby causing the moveable arms 134 to articulate, flex, or pivot on the joint portions 138. Other actuation mechanisms are also possible.

During implantation, the paddles 120, 122 can be opened and closed, for example, to grasp the native leaflets (e.g., native mitral valve leaflets, etc.) between the paddles 120, 122 and/or between the paddles 120, 122 and a means for coapting or coaption element 110. The clasps 130 can be used to grasp and/or further secure the native leaflets by engaging the leaflets with barbs, friction-enhancing elements, or means for securing 136 and pinching the leaflets between the moveable and fixed arms 134, 132. The barbs, friction-enhancing elements, or other means for securing 136 (e.g., barbs, protrusions, ridges, grooves, textured surfaces, adhesive, etc.) of the clasps or barbed clasps 130 increase friction with the leaflets or may partially or completely puncture the leaflets. The actuation lines 116 can be actuated separately so that each clasp 130 can be opened and closed separately. Separate operation allows one leaflet to be grasped at a time, or for the repositioning of a clasp 130 on a leaflet that was insufficiently grasped, without altering a successful grasp on the other leaflet. The clasps 130 can be opened and closed relative to the position of the inner paddle 122 (as long as the inner paddle is in an open or at least partially open position), thereby allowing leaflets to be grasped in a variety of positions as the particular situation requires.

The clasps 130 can be opened separately by pulling on an attached actuation line 116 that extends through the means for delivery or delivery catheter/sheath 102 to the clasp 130. The actuation line 116 can take a wide variety of forms, such as, for example, a line, a suture, a wire, a rod, a catheter, or the like. The clasps 130 can be spring loaded so that in the closed position the clasps 130 continue to provide a pinching force on the grasped native leaflet. This pinching force can remain constant or positive regardless of the position of the inner paddles 122. Barbs or means for securing 136 of barbed clasps 130 can pierce the native leaflets to further secure the native leaflets.

Referring now to Figure 8, the device 100 is shown in an elongated or fully open condition for deployment from the delivery catheter 102. The device 100 is loaded in the delivery catheter 102 in the fully open position, because the fully open position takes up the least space and allows the smallest catheter to be used (or the largest device 100 to be used for a given catheter size). In the elongated condition the cap 114 is spaced apart from the means for coapting or coaption element 110 such that the paddles 120, 122 are fully extended. In some embodiments, an angle formed between the interior of the outer and inner paddles 120, 122 is approximately 180 degrees. The clasps 130 are kept in a closed condition during deployment through the means of delivery or delivery catheter/sheath 102 so that the barbs, friction-enhancing elements, or means for securing 136 (Fig. 11) do not catch or damage the catheter or tissue in the patient's heart. The actuation lines 116 can extend through the coupler 117, around the collar 115, and attach to the moveable arms 134.

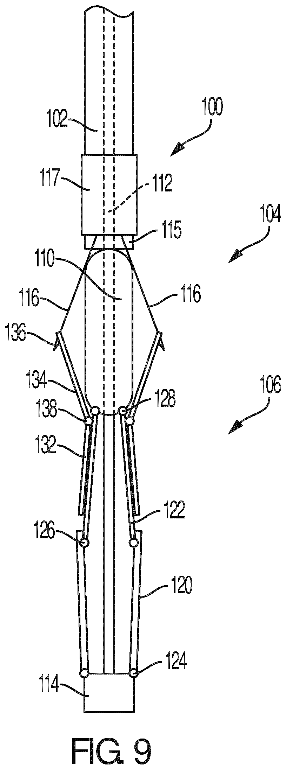

Referring now to Figure 9, the device 100 is shown in an elongated detangling condition, similar to Figure 8, but with the clasps 130 in a fully open position, ranging from about 140 degrees to about 200 degrees, from about 170 degrees to about 190 degrees, or about 180 degrees between fixed and moveable portions of the clasps 130. Fully opening the paddles 120, 122 and the clasps 130 has been found to improve ease of detanglement or detachment from anatomy of the patient, such as the chordae tendineae, during implantation of the device 100.

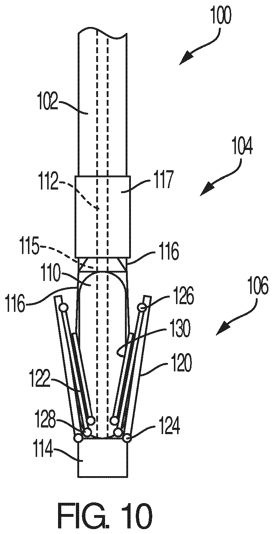

Referring now to Figure 10, the device 100 is shown in a shortened or fully closed condition. The compact size of the device 100 in the shortened condition allows for easier maneuvering and placement within the heart. To move the device 100 from the elongated condition to the shortened condition, the means for actuating or actuation element 112 is retracted to pull the cap 114 towards the means for coapting or coaption element 110. The connection portion(s) 126 (e.g., joint(s), flexible connection(s), etc.) between the outer paddle 120 and inner paddle 122 are constrained in movement such that compression forces acting on the outer paddle 120 from the cap 114 being retracted towards the means for coapting or coaption element 110 cause the paddles or gripping elements to move radially outward. During movement from the open to closed position, the outer paddles 120 maintain an acute angle with the means for actuating or actuation element 112. The outer paddles 120 can optionally be biased toward a closed position. The inner paddles 122 during the same motion move through a considerably larger angle as they are oriented away from the means for coapting or coaption element 110 in the open condition and collapse along the sides of the means for coapting or coaption element 110 in the closed condition. In some embodiments, the inner paddles 122 are thinner and/or narrower than the outer paddles 120, and the connection portions 126, 128 (e.g., joints, flexible connections, etc.) connected to the inner paddles 122 can be thinner and/or more flexible. For example, this increased flexibility can allow more movement than the connection portion 124 connecting the outer paddle 120 to the cap 114. In some embodiments, the outer paddles 120 are narrower than the inner paddles 122. The connection portions 126, 128 connected to the inner paddles 122 can be more flexible, for example, to allow more movement than the connection portion 124 connecting the outer paddle 120 to the cap 114. In some embodiments, the inner paddles 122 can be the same or substantially the same width as the outer paddles

Referring now to Figures 11-13, the device 100 is shown in a partially open, grasp-ready condition. To transition from the fully closed to the partially open condition, the means for actuating or actuation element (e.g., actuation wire, actuation shaft, etc.) is extended to push the cap 114 away from the means for coapting or coaption element 110, thereby pulling on the outer paddles 120, which in turn pull on the inner paddles 122, causing the anchors or anchor portion 106 to partially unfold. The actuation lines 116 are also retracted to open the clasps 130 so that the leaflets can be grasped. In the example illustrated by Figure 11, the pair of inner and outer paddles 122, 120 are moved in unison, rather than independently, by a single means for actuating or single actuation element 112. Also, the positions of the clasps 130 are dependent on the positions of the paddles 122, 120. For example, referring to Figure 10 closing the paddles 122, 120 also closes the clasps. In some embodiments, the paddles 120, 122 can be independently controllable. For example, the device 100 can have two actuation elements and two independent caps (or other attachment portions), such that one independent actuation element (e.g., wire, shaft, etc.) and cap (or other attachment portion) are used to control one paddle, and the other independent actuation element and cap (or other attachment portion) are used to control the other paddle.

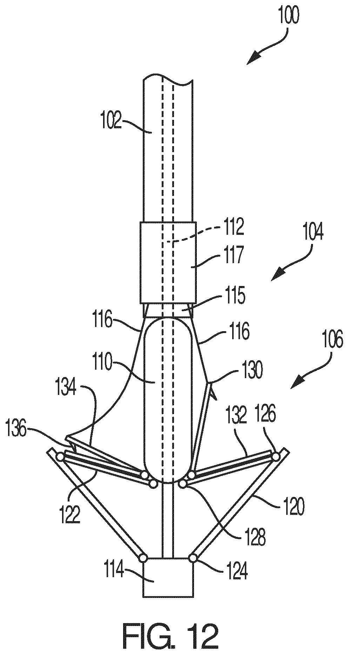

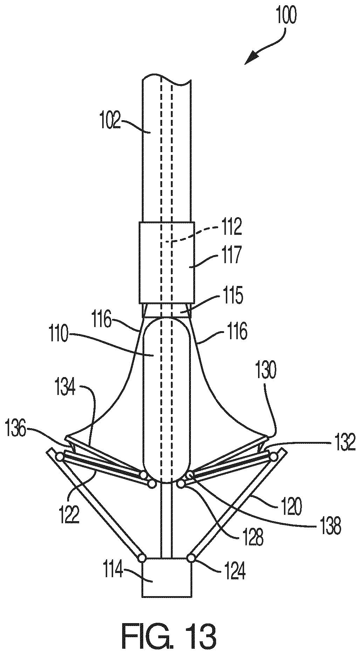

Referring now to Figure 12, one of the actuation lines 116 is extended to allow one of the clasps 130 to close. Referring now to Figure 13, the other actuation line 116 is extended to allow the other clasp 130 to close. Either or both of the actuation lines 116 can be repeatedly actuated to repeatedly open and close the clasps 130.

Referring now to Figures 14A through 14M, the device 100 can be closed or attached, removed, repositioned, and redeployed. As shown in Figure 14A, the device 100 is shown in a fully closed and deployed condition. The paddles 120, 122 and clasps 130 remain in a fully closed position. Once in the deployed condition, the device 100 can be maintained in the fully closed position with a mechanical latch or can be biased to remain closed through the use of spring materials, such as steel, other metals, plastics, composites, etc. or shape-memory alloys such as Nitinol. For example, the connection portions 124, 126, 128, the joint portion(s) 138, and/or the inner and outer paddles 122, 120, and/or an additional biasing component can be formed of metals such as steel or shape-memory alloy, such as Nitinol-produced in a wire, sheet, tubing, or laser sintered powder-and are biased to hold the outer paddles 120 closed around the coaption element 110 and the clasps 130 pinched around native leaflets. Similarly, the fixed and moveable arms 132, 134 of the clasps 130 are biased to pinch the leaflets. In some embodiments, the attachment or connection portions 124, 126, 128, the joint portion(s) 138, and/or the inner and outer paddles 122, and/or an additional biasing component can be formed of any other suitably elastic material, such as a metal or polymer material, to maintain the device in the closed condition after implantation.

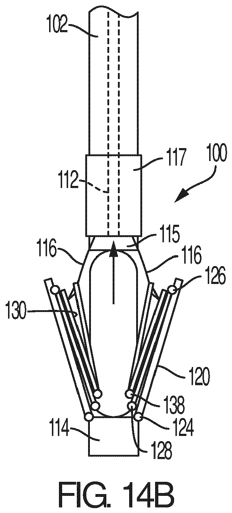

Referring now to Figure 14B, the device 100 can be uncoupled from the actuation element 112. The actuation element 112 can be retracted from the device 100 and the collar 115 and into the coupler 117 or the delivery catheter 102. The actuation element 112 can be retracted such that the end of the actuation element 112 is positioned within the coupler 117 or the delivery catheter 102. In such a position, the device 100 and the collar 115 can move or pivot relatively freely from the coupler 117, the delivery catheter 102, and the actuation element 112.

As shown in Figure 14C, slack can be introduced to the clasp actuation lines 116 such that the delivery catheter 102, the actuation element 112, and the coupler 117 can be retracted from the device 100 and the collar 115. The device 100 is still tied to the delivery catheter 102, the actuation element 112, and/or the coupler 117 via one or more coupling tethers 119. The device 100 can remain tethered in a wide variety of different ways. For example, the collar 115 can be tied to the delivery catheter 102, the actuation element 112, and/or the coupler 117 via one or more coupling tethers 119. However, any component of the device 100 can be tethered. The one or more coupling tethers 119 can be looped around or otherwise secured or attached to the collar 115, the coupler 117, the actuation element 112, and/or the delivery catheter 102. In such a position, the device 100 remains tethered or tied to the delivery catheter 102, the actuation element 112, and/or the coupler 117 but, with sufficient slack in the clasp actuation lines 116 and the tether, that the device 100 is generally free to move relative to the delivery catheter 102, the actuation element 112, and the coupler 117. In such a position, a user, such as a physician, may observe or check how the device 100 actually looks or operates when the device 100 is actually implanted, such as on the native leaflets (e.g., native mitral valve leaflets, etc.) of the heart. That is, the slack in the tether 119 and the actuation lines is selected such that the tether does not influence or substantially does not influence the position of the device 100 and/or the valve leaflets.

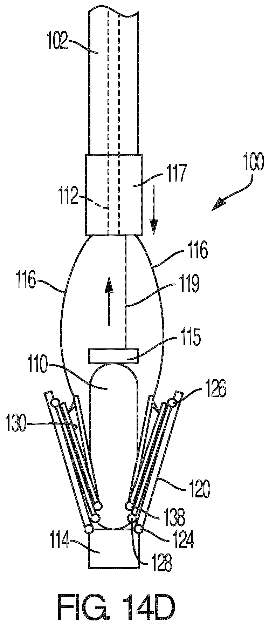

Referring now to Figures 14D through 14F, the device 100 and collar 115 can be recoupled to the delivery catheter 102, the actuation element 112, and the coupler 117. For example, the device 100 and the collar 115 can be recoupled to the delivery catheter 102, the actuation element 112, and the coupler 117 if the user observes that the device 100 is not properly implanted, the desired efficacy has not been obtained (e.g. regurgitation is not reduced or not reduced to the expected extent), the device is not in the proper intended position, and/or the device moves relative to the native valve leaflets from an initial capture position (e.g. one or more of the leaflets slip out or partially slip out of a clasp).

As shown in Figure 14D, the device 100 and the collar 115 can be recoupled to the delivery catheter 102 and the coupler 117. Tension can be applied to the one or more coupling tethers 119 and the delivery catheter 102 and the coupler 117 can be advanced toward the collar 115. When the coupler 117 and collar 115 are brought back together, the device 100 can be recoupled to the delivery catheter 102, the actuation element 112, and the coupler 117. Tension can also be applied to the clasp actuation lines 116 to bring the clasp actuation lines 116 closer to the device 100 and within the coupler 117 and/or the delivery catheter 102 such that the clasp actuation lines 116 do not move around, get tangled, or get pinched between the collar 115 and the coupler 117.

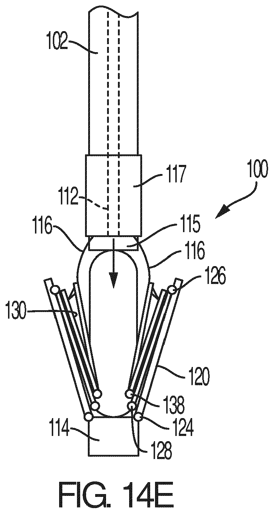

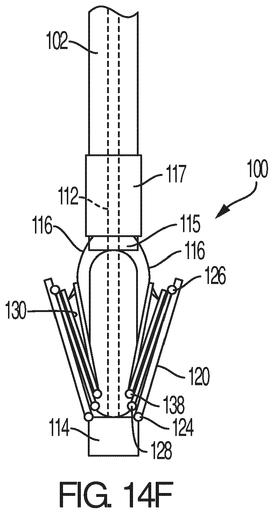

Figure 14E illustrates the coupler 117 brought back into contact with the collar 115. The actuation element 112 can be advanced through the coupler 117 and the collar 115 and into the device 100. As shown in Figure 14F, the actuation element 112 can be advanced through the collar 115 and the coupler 117 and into the device 100 until the actuation element 112 reengages the cap 114 of the device 100.

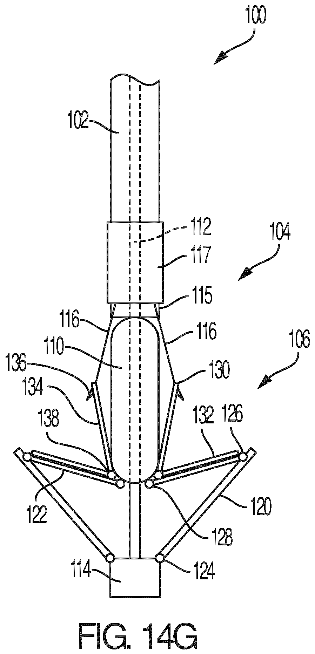

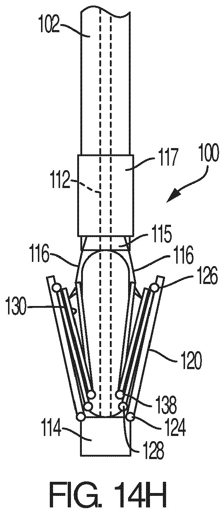

Referring now to Figures 14G and 14H, the device 100 can be reopened or moved back to the partially open, grasp-ready condition. As shown in Figure 14G, to transition from the fully closed condition to the partially open condition, the actuation element 112 is extended to push the cap 114 away from the coaption element 110, thereby pulling on the outer paddles 120, which in turn pulls on the inner paddles 122, causing the anchor portion 106 to partially unfold. The actuation lines 116 are also retracted to open the clasps 130 so that the leaflets can be released. However, the device can be moved to any of the positions described herein to release or fully release the leaflets.

In the example illustrated by Figure 14G, the pair of inner and outer paddles 122, 120 are moved in unison, rather than independently, by a single actuation element 112. Also, the positions of the clasps 130 are dependent on the positions of the paddles 122, 120. For example, referring to Figure 10 closing the paddles 122, 120 also closes the clasps. In some embodiments, the paddles 120, 122 can be independently controllable. For example, the device 100 can have two actuation elements and two independent caps (or other attachment portions), such that one independent actuation element (e.g., wire, shaft, etc.) and cap (or other attachment portion) are used to control one paddle, and the other independent actuation element (e.g., wire, shaft, etc.) and cap (or other attachment portion) are used to control the other paddle. While in the partially open, grasp-ready condition, the device 100 can be moved or repositioned. For example, a user can reposition the device 100 to properly grasp the native leaflets (e.g., the native mitral valve leaflets, etc.).

As shown in Figure 14H, the device 100 can be moved to the fully closed or deployed condition. For example, the device 100 can be moved back to the deployed condition once the device 100 has been positioned or repositioned to the desired position. The actuation lines 116 can be extended to allow the clasps 130 to close. Either or both of the actuation lines 116 can be repeatedly actuated to repeatedly open and close the clasps 130. For example, the clasps 130 can be repeatedly opened and closed to ensure that the device 100 is properly placed.

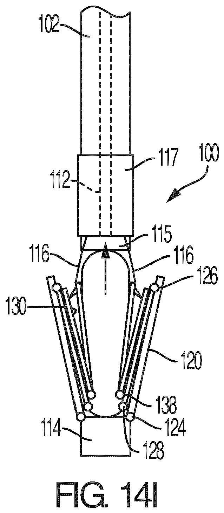

Referring now to Figures 14I through 14M, the device 100 can be deployed from the delivery catheter 102, the coupler 117, and the actuation element 112. For example, the device 100 can be deployed once the device 100 is in the proper place and grasping the native leaflets (e.g., native mitral valve leaflets, etc.).

As shown in Figure 14I, the device 100 can be uncoupled from the actuation element 112. The actuation element 112 can be retracted from the device 100 and the collar 115 and into the coupler 117 or the delivery catheter 102. The actuation element 112 can be retracted such that the end of the actuation element 112 is positioned within the coupler 117 or the delivery catheter 102. In such a position, the device 100 and the collar 115 can move or pivot relatively freely from the coupler 117, the delivery catheter 102, and the actuation element 112.

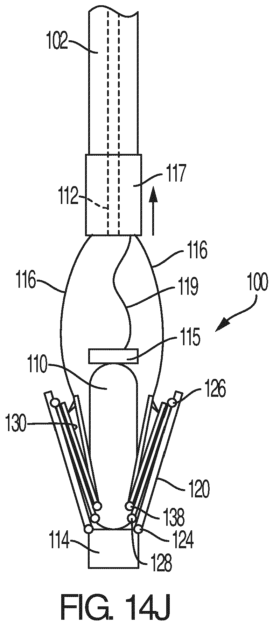

As shown in Figure 14J, slack can be introduced into the clasp actuation lines 116 and the tether 119 such that the delivery catheter 102, the actuation element 112, and the coupler 117 can be retracted away from the device 100 and the collar 115. The collar 115 can still be attached to the delivery catheter 102, the actuation element 112, and/or the coupler 117 via one or more coupling tethers 119. The one or more coupling tethers 119 can be looped around or otherwise secured or attached to the collar 115 and can attach the collar 115 to the coaption portion 104 of the device 100. The one or more coupling tethers 119 can couple the device 100 and the collar 115 to the coupler 117, the actuation element 112, and/or the delivery catheter 102 in a wide variety of ways. For example, the one or more coupling tethers 119 can couple the device 100 and the collar 115 to the coupler 117, the actuation element 112, and/or the delivery catheter 102 as described later herein. In such a position, the device 100 remains tethered or tied to the delivery catheter 102, the actuation element 112, and/or the coupler 117 but, with sufficient slack in the clasp actuation lines 116 and tether 119, the device 100 is generally free to move relative to the delivery catheter 102, the actuation element 112, and the coupler 117. In such a position, a user may observe or check how the device 100 will look or operate when the device 100 is actually deployed, such as on the native leaflets (e.g., the native mitral valve leaflets, etc.).

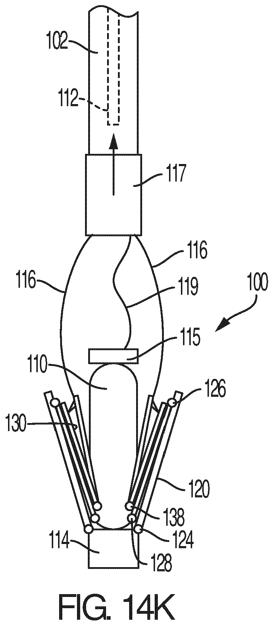

As shown in Figure 14K, the actuation element 112 can optionally be retracted or withdrawn farther into the delivery catheter 102 and away from the coupler 117 to release the one or more coupling tethers 119. The one or more coupling tethers 119 can be released in a wide variety of ways. For example, the one or more coupling tethers 119 can be released as described later herein.

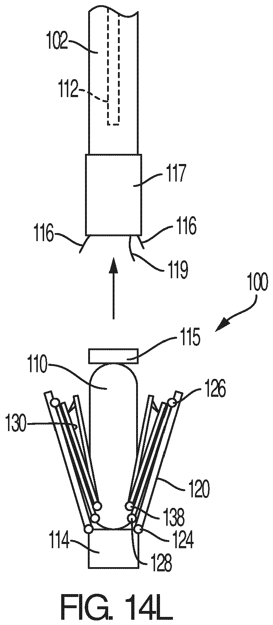

As shown in Figure 14L, the device 100 and collar 115 can be decoupled from the one or more coupling tethers 119 and thereby released from the delivery catheter 102, the actuation element 112, and the coupler 117. The one or more coupling tethers 119 can be retracted away from the device 100 and the collar 115. The clasp actuation lines 116 can also be detached from the moveable arms 134 and pulled or otherwise retracted toward or into the coupler 117 and/or delivery catheter 102. In such an embodiment, the device 100 and collar 115 are completely detached from the delivery catheter 102, the coupler 117, and the actuation element 112.

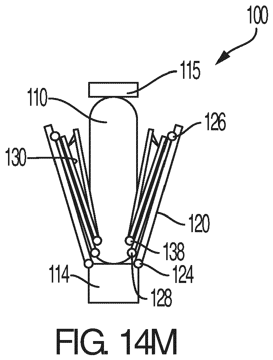

As shown in Figure 14M, the device 100 is shown in a fully closed and deployed condition. The delivery catheter 102, the coupler 117, and the actuation element 112 are retracted and the paddles 120, 122 and clasps 130 remain in a fully closed position. Once deployed, the device 100 can be maintained in the fully closed position with a mechanical latch or can be biased to remain closed through the use of spring materials, such as steel, other metals, plastics, composites, etc. or shape-memory alloys such as Nitinol. For example, the jointed or flexible portions 124, 126, 128, 138, and/or the inner and outer paddles 122, 120 and/or an additional biasing component can be formed of metals such as steel or shape-memory alloy, such as Nitinol-produced in a wire, sheet, tubing, or laser sintered powder-and are biased to hold the outer paddles 120 closed around the coaption element 110 and the clasps 130 pinched around native leaflets. Similarly, the fixed and moveable arms 132, 134 of the clasps 130 are biased to pinch the leaflets. In some embodiments, the joint portions 124, 126, 128, 138, and/or the inner and outer paddles 122, and/or an additional biasing component can be formed of any other suitably elastic material, such as a metal or polymer material, to maintain the device in the closed condition after implantation.

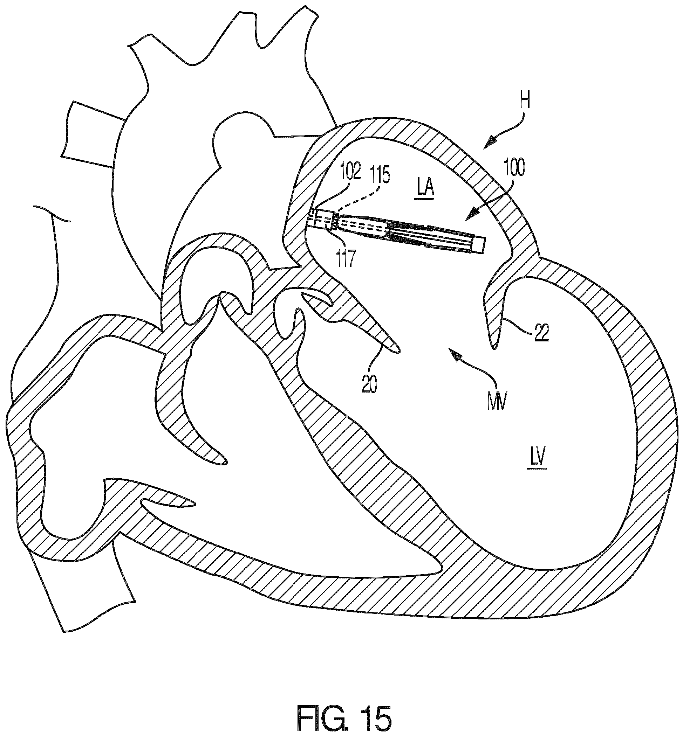

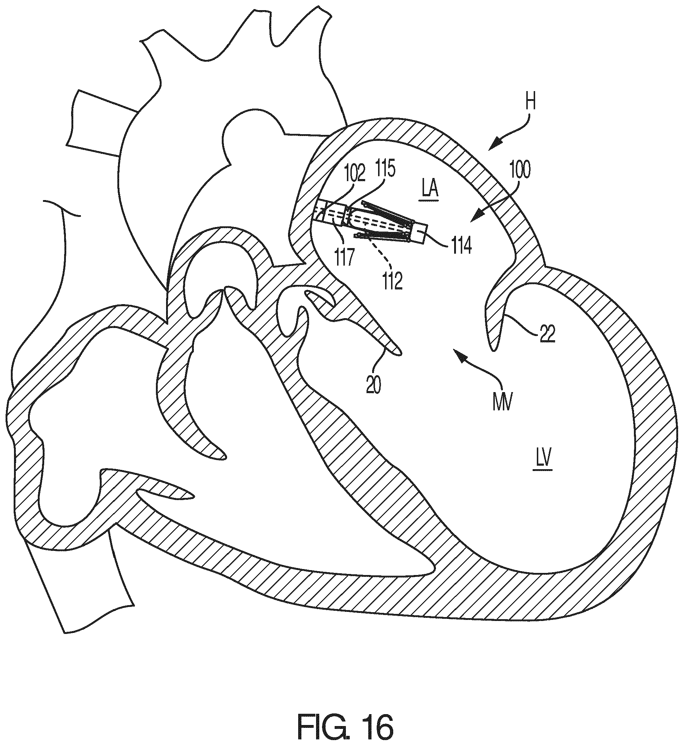

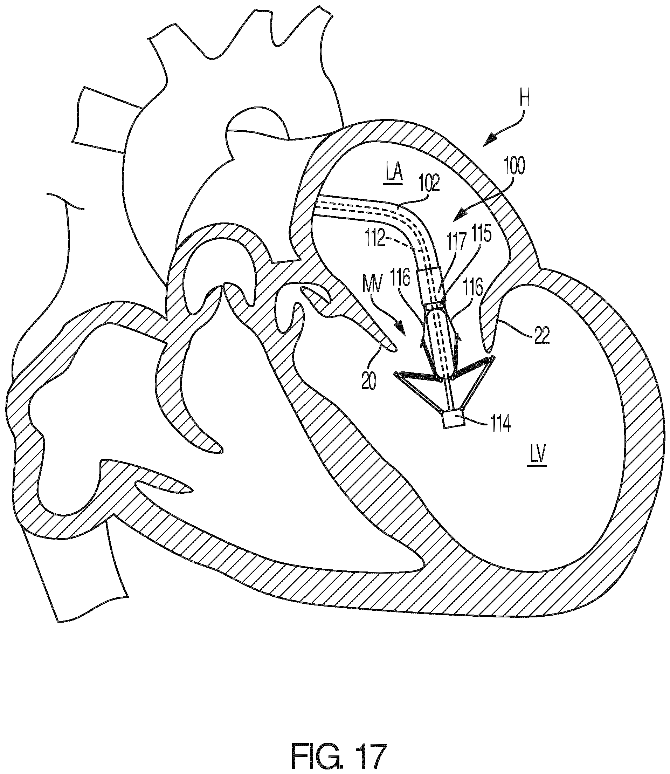

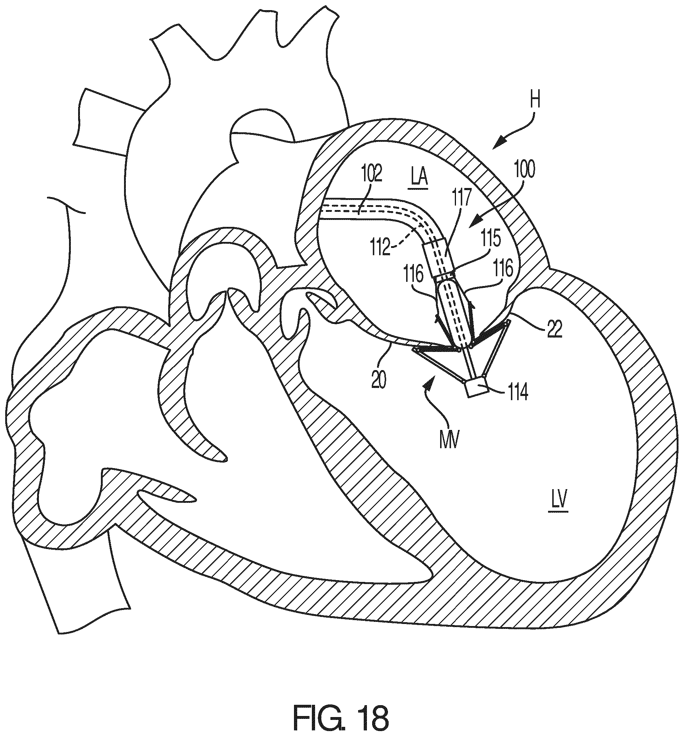

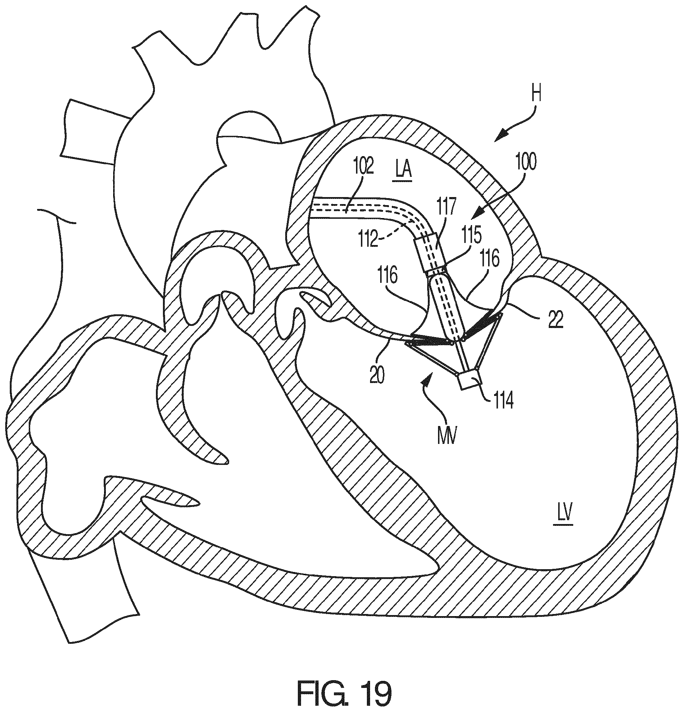

Referring now to Figures 15 through 20M, the implantable device 100 of Figures 8 through 14M is shown being delivered and implanted, for example, within the native mitral valve MV of the heart H. Referring now to Figure 15, the delivery catheter is inserted into the left atrium LA through the septum and the device 100 is deployed from the delivery catheter in the fully open condition. The device 100 is moved into the fully closed condition shown in Figure 16, e.g., this can be done in some implementations by retracting actuation element 112. As can be seen in Figure 17, the device 100 is moved into position within the mitral valve MV into the ventricle LV and partially opened so that the leaflets 20, 22 can be grasped. Referring now to Figure 18, an actuation line 116 is extended to close one of the clasps 130, capturing a leaflet 20. Figure 19 shows the other actuation line 116 being then extended to close the other clasp 130, capturing the remaining leaflet 22.

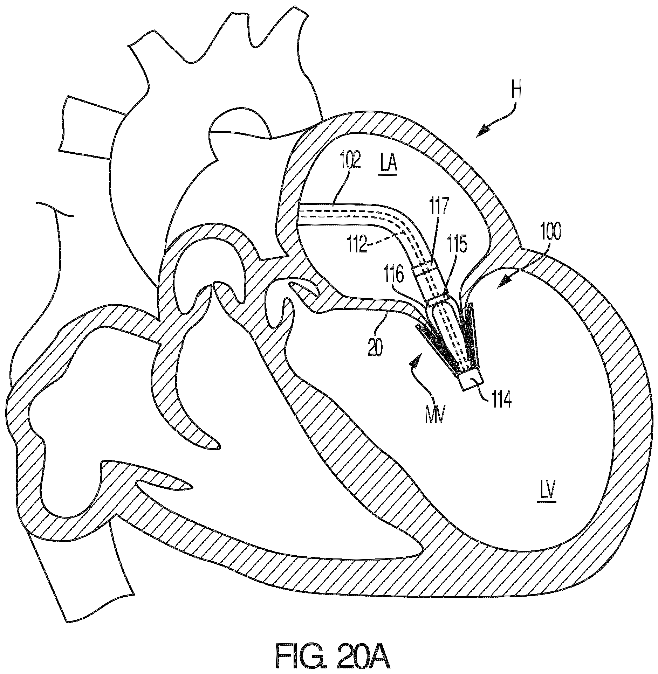

Referring now to Figures 20A through 20M (and corresponding Figures 14A-14M), the implantable device 100, as shown, can be decoupled from the delivery catheter 102 and actuation element 112, recoupled to the delivery catheter 102 and actuation element 112, repositioned, and redeployed within the native mitral valve MV of the heart H. As shown in Figure 20A (See also Fig. 14A), the actuation lines 116 can be released and the device 100 is moved to the fully closed position such that the clasps 130 are secured on the leaflets 20, 22. The device 100 remains coupled to the delivery catheter 102, the actuation element 112, and the coupler 117 in FIG. 20A.

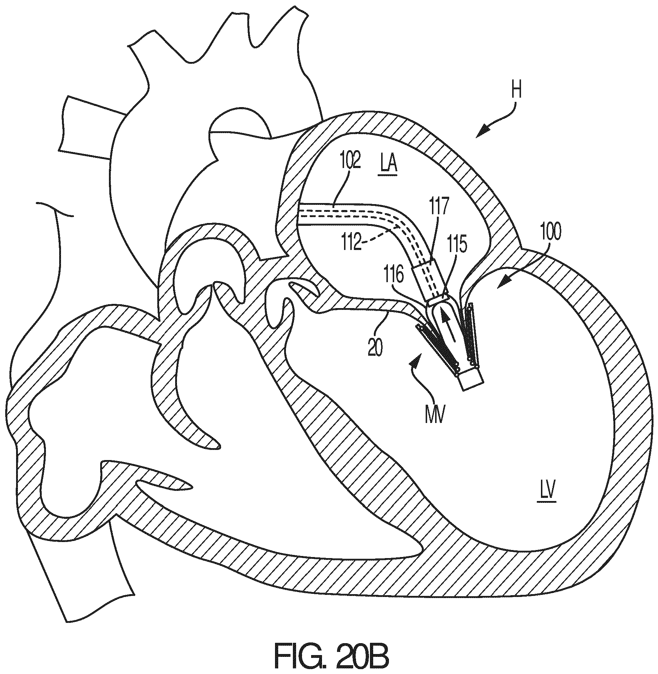

As shown in Figure 20B (See also Figure 14B), in some implementations, the device 100 can be uncoupled from the actuation element 112. The actuation element 112 can be retracted from the device 100 and the collar 115 and into the coupler 117 or the delivery catheter 102. The actuation element 112 can be retracted such that the end of the actuation element 112 is positioned within the coupler 117 or the delivery catheter 102. In such a position, the device 100 and the collar 115 can move or pivot relatively freely from the coupler 117, the delivery catheter 102, and the actuation element 112.

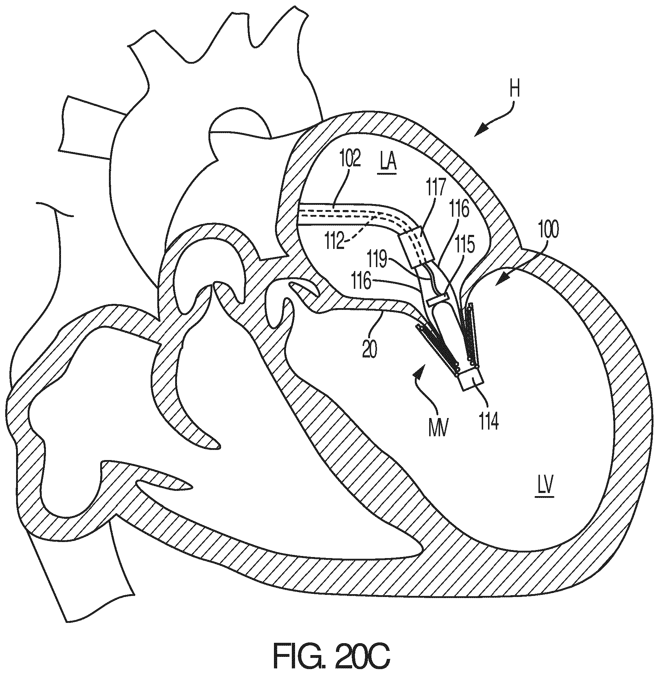

As shown in Figure 20C (See also Figure 14C), slack can be introduced to the clasp actuation lines 116 and the tether 119 such that the delivery catheter 102, the actuation element 112, and the coupler 117 can be retracted from the device 100 and the collar 115. The device 100 is still tied to the delivery catheter 102, the actuation element 112, and/or the coupler 117 via one or more coupling tethers 119. The device 100 can remain tethered in a wide variety of different ways. For example, the collar 115 can be tied to the delivery catheter 102, the actuation element 112, and/or the coupler 117 via one or more coupling tethers 119. However, any component of the device 100 can be tethered. The one or more coupling tethers 119 can be looped around or otherwise secured or attached to the collar 115. The device 100 remains tethered or tied to the delivery catheter 102, the actuation element 112, and/or the coupler 117 but, with sufficient slack in the clasp actuation lines 116 and the tethers 119, the device 100 is generally free to move relative to the delivery catheter 102, the actuation element 112, and the coupler 117. In such a position, a user, such as a physician, may observe or check how the device 100 actually looks or operates when the device 100 is actually implanted, such as on the mitral valve leaflets of the heart. That is, the slack in the tether 119 and the actuation lines is selected such that the tether does not influence or substantially does not influence the position of the device 100 and/or the valve leaflets.

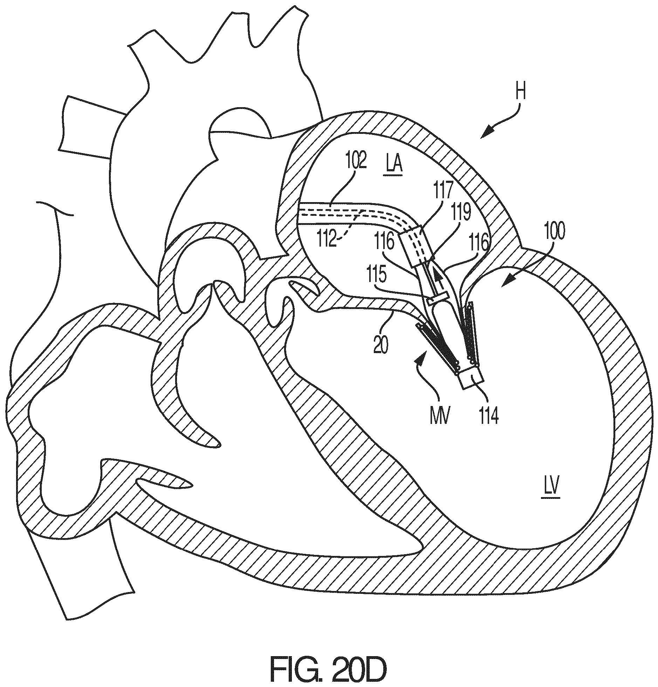

As shown in Figure 20D (See also Figure 14D), the device 100 and the collar 115 can be recoupled to the delivery catheter 102, the actuation element 112, and the coupler 117. For example, the device 100 can be recoupled to the delivery catheter 102, the actuation element 112, and the coupler 117 if the device 100 does not properly grasp the leaflets 20, 22 or the native valve or native mitral valve MV of the heart H is regurgitating or otherwise malfunctioning with the device 100 in place. Tension can be applied to the one or more coupling tethers 119 and the coupler 117 can be advanced along the tether toward the collar 115. When the coupler 117 and collar 115 are brought back together, the device 100 can be recoupled to the delivery catheter 102, the actuation element 112, and the coupler 117. Tension can also be applied to the clasp actuation lines 116 to bring the clasp actuation lines 116 closer to the device 100 and within the coupler 117 and/or the delivery catheter 102 such that the clasp actuation lines 116 do not move around or get tangled or get pinched between the collar 115 and the coupler 117.

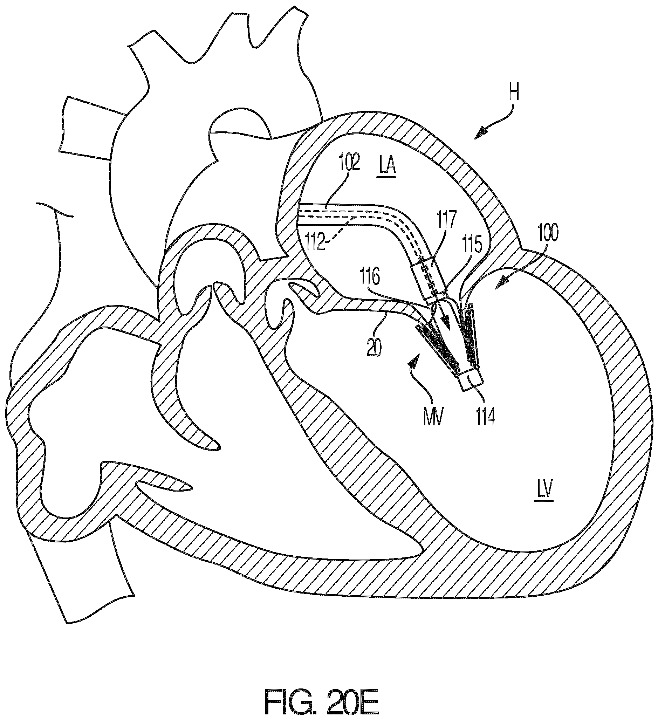

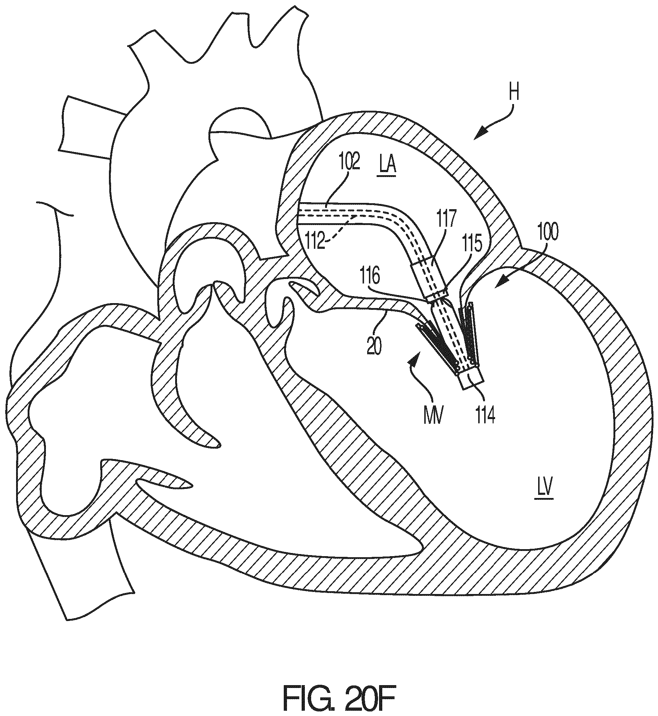

Figure 20E (See also Figure 14E) shows the coupler 117 brought back into contact with the collar 115. The actuation element 112 can be advanced through the coupler 117 and the collar 115 and into the device 100. As shown in Figure 20F (See also Figure 14F), the actuation element 112 can be advanced through the collar 115 and the coupler 117 and into the device 100 until the actuation element 112 reengages the anchor portion 106 and/or cap 114 of the device 100.

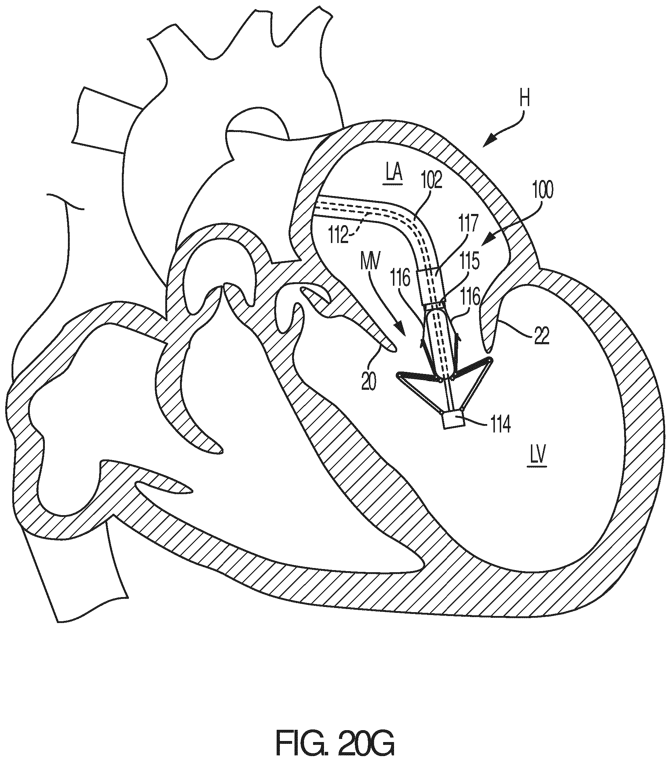

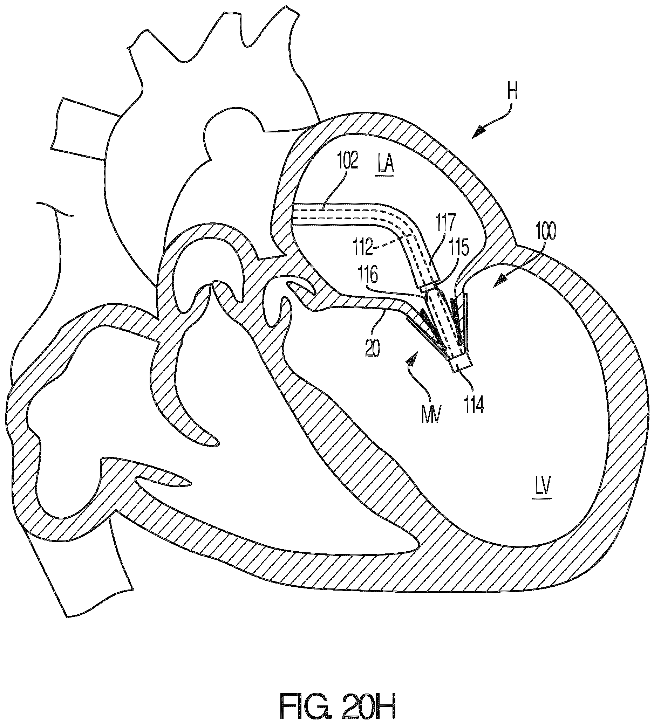

Referring now to Figures 20G and 20H (See Also Figure 20G and 20H), the device 100 can be reopened or moved back to the partially open, grasp-ready condition within the heart H. As shown in Figure 20G, to transition from the fully closed to the partially open condition (or other position), the actuation element 112 is extended to push the cap 114 away from the coaption element 110, thereby pulling on the outer paddles 120, which in turn pulls on the inner paddles 122, causing the anchor portion 106 to partially unfold. The clasp actuation lines 116 are also retracted to open the clasps 130 so that the leaflets can be released, the device repositioned, and the leaflets recaptured. However, the device can be moved to any of the positions described herein to release or fully release the leaflets or to recapture the leaflets after repositioning of the device.

In the example illustrated by Figure 20G, the pair of inner and outer paddles 122, 120 are moved in unison, rather than independently, by a single actuation element 112. Also, the positions of the clasps 130 are dependent on the positions of the paddles 122, 120. For example, referring to Figure 10 closing the paddles 122, 120 also closes the clasps. In some embodiments, the paddles 120, 122 can be independently controllable. For example, the device 100 can have two actuation elements and two independent caps (or other attachment portions), such that one independent actuation element (e.g., wire, shaft, etc.) and cap (or other attachment portion) are used to control one paddle, and the other independent actuation element and cap (or other attachment portion) are used to control the other paddle. While in the partially open, grasp-ready condition, the device 100 can be moved or repositioned. For example, a user may reposition the device 100 to properly grasp one or more native valve leaflets that were not properly captured or grasped on a previous attempt.

As shown in Figure 20H (See also Figure 14H), the device 100 can be moved to the fully closed or deployed condition in the native valve or native mitral valve MV of the heart H. For example, the device 100 can be moved back to the deployed condition once the device 100 has been positioned or repositioned to the desired position, properly grasping the leaflets 20, 22 in the native valve or native mitral valve MV of the heart H. The actuation lines 116 can be extended to allow the clasps 130 to close. Either or both of the actuation lines can be repeatedly actuated to repeatedly open and close the clasps 130. For example, the clasps 130 can be repeatedly opened and closed to ensure that the device 100 properly grasps the leaflets 20, 22 and the device 100 is properly placed in the native valve or native mitral valve MV of the heart H.

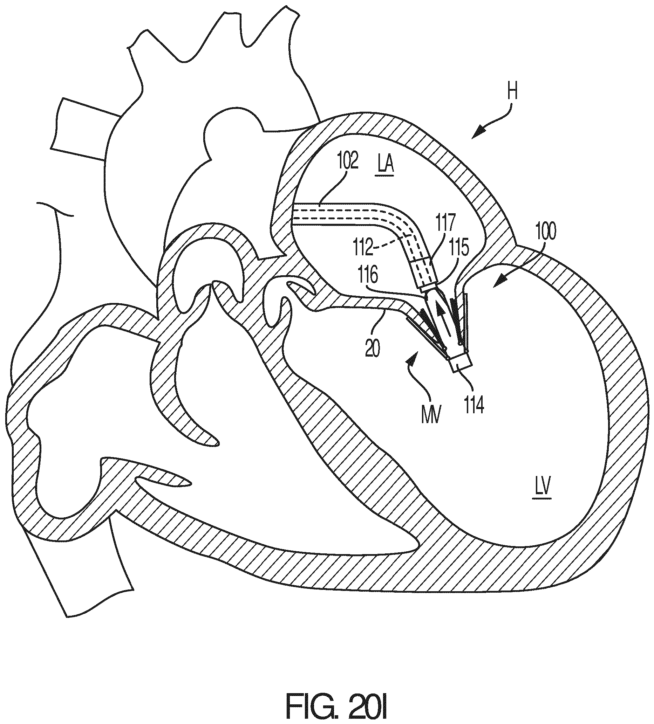

Referring now to Figures 20I through 20M (See also Figures 14I through 14M), the device 100 can be detached from the delivery catheter 102, the coupler 117, and the actuation element 112 and deployed in the native valve or native mitral valve MV of the heart H. For example, the device 100 can be irretrievably released once the device 100 is in the proper place and the device is properly grasping the native valve leaflets 20, 22.

As shown in Figure 20I (See also Figure 14I), the device 100 can be uncoupled from the actuation element 112. The actuation element 112 can be retracted from the device 100 and the collar 115 and into the coupler 117 or the delivery catheter 102. The actuation element 112 can be retracted such that the end of the actuation element 112 is positioned within the coupler 117 or the delivery catheter 102. With the actuation element in such a position, the device 100 and the collar 115 can move or pivot relatively independently with respect to the coupler 117, the delivery catheter 102, and the actuation element 112 within the native valve or mitral valve MV in the heart H.

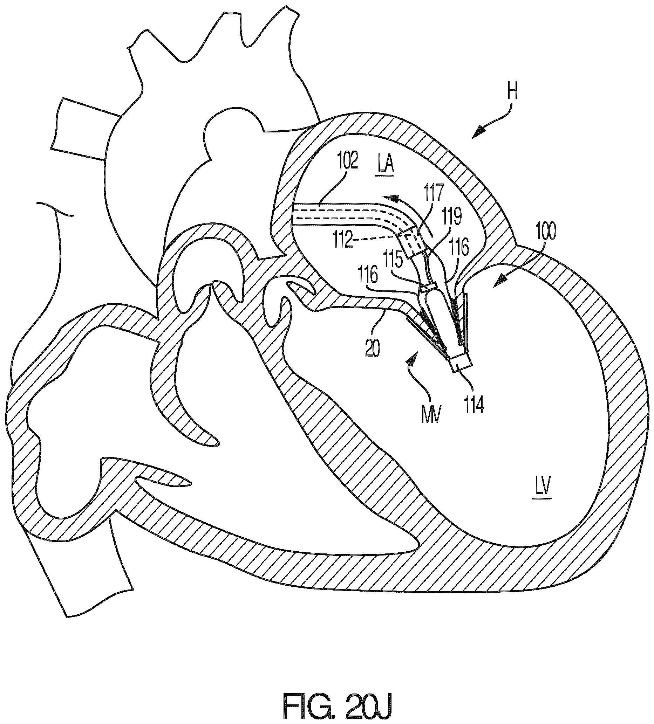

As shown in Figure 20J (See also Figure 14J), slack can be introduced to the clasp actuation lines 116 again such that the delivery catheter 102, the actuation element 112, and the coupler 117 can be retracted from the device 100 and the collar 115. The device 100 can still be reattached to the delivery catheter 102, the actuation element 112, and/or the coupler 117 via one or more coupling tethers 119. The one or more coupling tethers 119 can be looped around or otherwise secured or attached to the collar 115. The one or more coupling tethers 119 can couple the device 100 and the collar 115 to the coupler 117, the actuation element 112, and/or the delivery catheter 102 in a wide variety of ways. For example, the one or more coupling tethers 119 can couple the device 100 and the collar 115 to the coupler 117, the actuation element 112, and/or the delivery catheter 102 as described later herein.

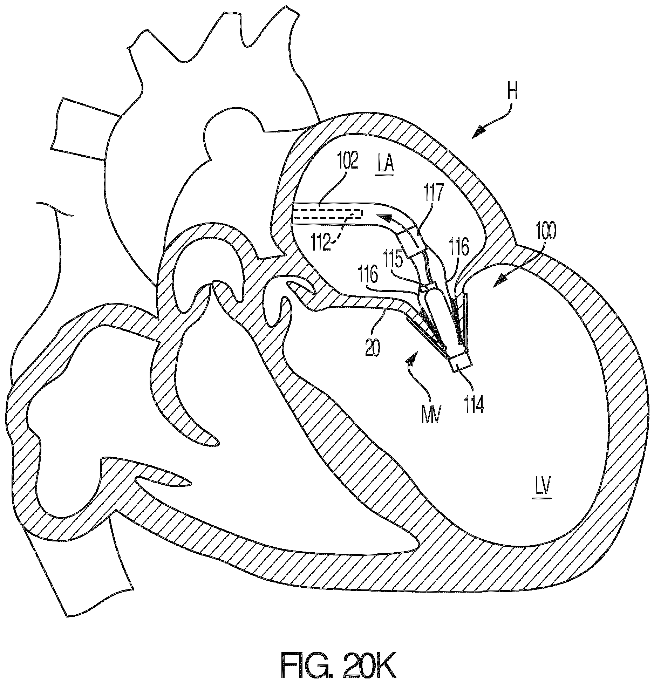

In the position illustrated by Figure 20K (See also Figure 14K), the device 100 remains tethered or attached to the delivery catheter 102, the actuation element 112, and/or the coupler 117 but, with sufficient slack in the clasp actuation lines 116 and the tether 119, the device 100 is again generally free to move relative to the delivery catheter 102, the actuation element 112, and the coupler 117. In such a position, a user may again observe or check how the device 100 will work or otherwise operate when deployed in the native valve or native mitral valve MV of the heart H.

The one or more coupling tethers 119 can be released in a wide variety of ways and/or with a variety of release mechanisms. In one example embodiment, as shown in Figure 20K, the one or more tethers are coupled to the actuation element 112 and the actuation element 112 can be retracted or withdrawn farther into the delivery catheter 102 and away from the coupler 117 to release the one or more coupling tethers 119. In an example embodiment, the tether 119 is simply looped around or through a component of the device 100, such as the cap, and the tether 119 is released from the device 100 by pulling one end of the tether loop into and through the catheter. Additional examples of releasing the one or more coupling tethers 119 are described later herein.

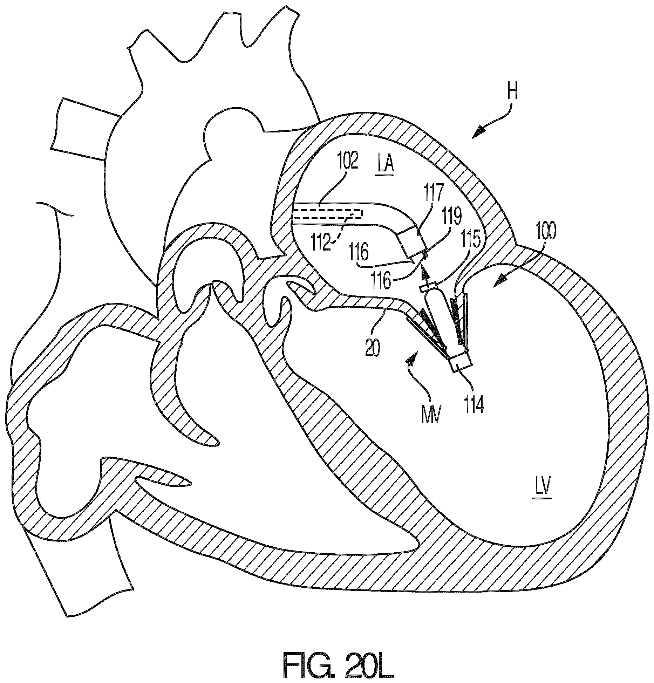

As shown in Figure 20L (See also Figure 14L), the device 100 and collar 115 can be decoupled from the one or more coupling tethers 119 and the one or more clasp actuation lines. The one or more coupling tethers 119 and clasp actuation lines 116 can be retracted away from the device 100 and the collar 115 and pulled or otherwise retracted toward or into the coupler 117 and/or delivery catheter 102. In such an embodiment, the device 100 and collar 115 are completely detached from the delivery catheter 102, the coupler 117, and the actuation element 112 and the device 100 is implanted in the native valve (in this example, in the native mitral valve MV) of the heart H.

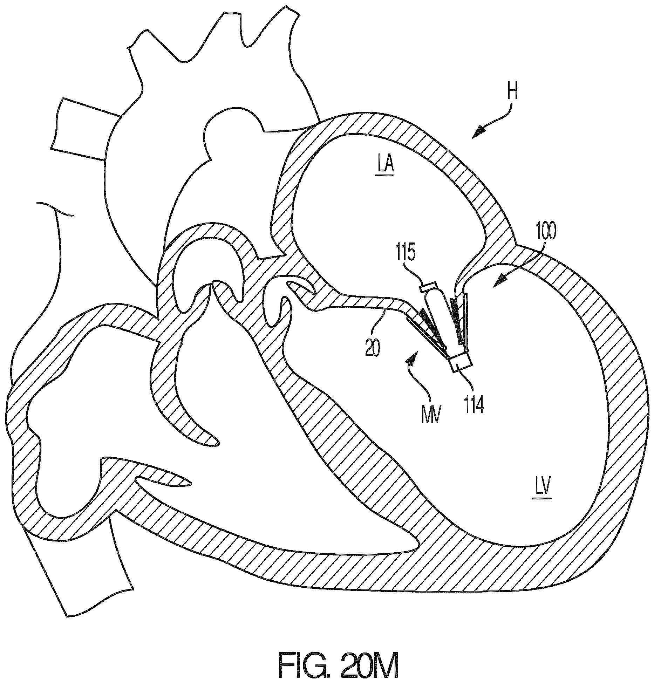

As shown in Figure 20M, the device 100 is shown in a fully closed and deployed condition. The delivery catheter 102, the coupler 117, the actuation element 112, the one or more coupling tethers 119, and the clasp actuation lines 116 are retracted and the paddles 120, 122 and clasps 130 remain in a fully closed position. Once deployed, the device 100 can be maintained in the fully closed position with a mechanical latch or can be biased to remain closed through the use of spring materials, such as steel, other metals, plastics, composites, etc. or shape-memory alloys such as Nitinol. For example, the jointed or flexible portions 124, 126, 128, 138, and/or the inner and outer paddles 122, and/or an additional biasing component can be formed of metals such as steel or shape-memory alloy, such as Nitinol-produced in a wire, sheet, tubing, or laser sintered powder-and are biased to hold the outer paddles 120 closed around the coaption element 110 and the clasps 130 pinched around native leaflets. Similarly, the fixed and moveable arms 132, 134 of the clasps 130 are biased to pinch the native leaflets (e.g., leaflets 20, 22) of the native valve of the heart H. In some embodiments, the joint portions 124, 126, 128, 138, and/or the inner and outer paddles 122, and/or an additional biasing component can be formed of any other suitably elastic material, such as a metal or polymer material, to maintain the device in the closed condition after implantation.

The concepts disclosed in the present patent application can be used with a wide variety of different valve repair devices 100. For example, the concepts disclosed in the present application can be applied to any of the valve repair devices that are disclosed by PCT Patent Publication WO 2020/076898, PCT Patent Publication WO 2019/139904, and US Patent No. 10,136,993 which are incorporated by reference in their entirety. The concepts disclosed by the present application can be used with any implantable prosthetic device, such as any transcatheter mitral valve repair device and any transcatheter tricuspid valve repair device. The implantable devices disclosed herein are just a few examples of the many implantable devices that the concepts disclosed herein can be used with. As such, the concepts disclosed herein can be used with implantable devices that do not include all or any of the features of the implantable devices disclosed herein. As one example, many embodiments show and/or describe a prosthetic device with a coaption or spacer member which provides many important benefits, but otherwise similar devices without a coaption or spacer member could also be used.

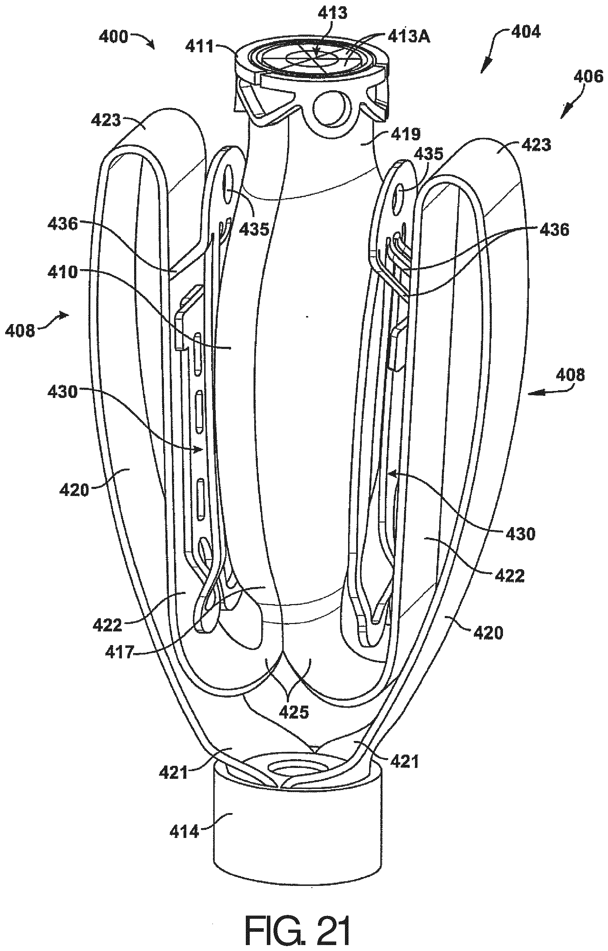

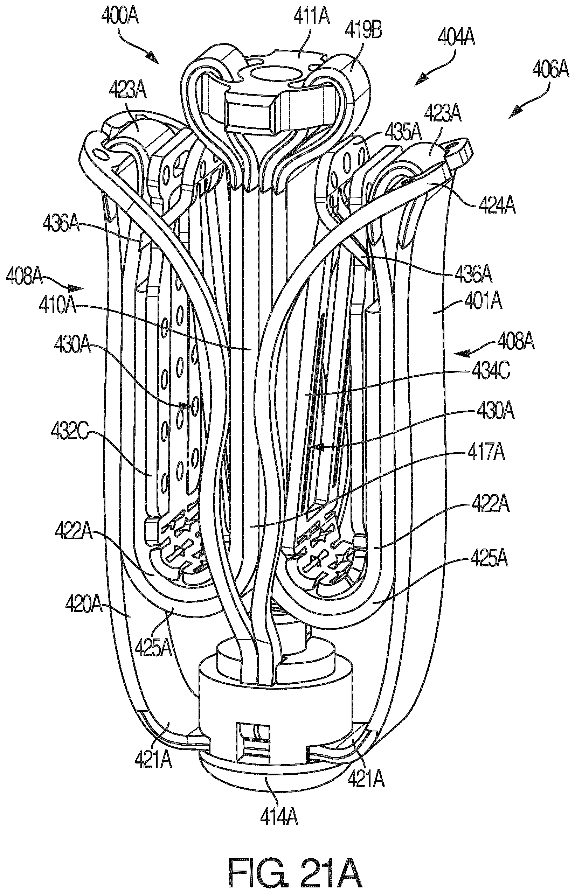

Figures 21 and 21A illustrate two of the many different valve repair devices that the concepts of the present application can be used with. In some examples, the device 400 illustrated by Figure 21 can optionally include paddle frames that are similar to the paddle frames 424a, 424b of the example illustrated by Figure 21A. PCT patent application publication WO2020/076898 disclose details of embodiments of the device of Figure 21 having such paddle frames.

The devices 400, 400A illustrated by Figures 21 and 21A can include any other features for an implantable prosthetic device discussed in the present application, and the devices 400, 400A can be positioned or repositioned to engage valve tissue (e.g., leaflets 20, 22) as part of any suitable valve repair system (e.g., any valve repair system disclosed in the present application).

Referring now to Figure 21, the device 400 (e.g., implantable device, implantable prosthetic device, prosthetic spacer, or coaption device, etc.) can include a coaption portion 404 and an anchor portion 406, the anchor portion 406 including a plurality of anchors 408. The coaption portion 404 includes a coaption or spacer member 410. The anchor portion 406 includes a plurality of paddles 420 (e.g., two in the illustrated embodiment), and a plurality of clasps 430 (e.g., two in the illustrated embodiment). A first or proximal collar 411, and a second collar or cap 414 are used to move the coaption portion 404 and the anchor portion 406 relative to one another. First connection portions 425 of the anchors 408 can be coupled to and extend from a first portion 417 of the coaption or spacer member 410, and second connection portions 421 of the anchors 408 can be coupled to the second collar 414. The proximal collar 411 can be coupled to a second portion 419 of the coaption member 410.

The coaption member 410 and the anchors 408 can be coupled together in various ways. For example, as shown in the illustrated embodiment, the coaption member 410 and the anchors 408 can be coupled together by integrally forming the coaption member 410 and the anchors 408 as a single, unitary component. This can be accomplished, for example, by forming the coaption member 410 and the anchors 408 from a braided or woven material, such as braided or woven nitinol wire. In some embodiments, the coaption member 410 and the anchors 408 can be coupled together by welding, fasteners, adhesive, joint connections, sutures, friction fittings, swaging, and/or other means for coupling.

As illustrated in Figure 21, the anchors 408 can comprise first portions or outer paddles 420 and second portions or inner paddles 422 separated by joint portions 423. In this manner, the anchors 408 are configured similar to legs in that the inner paddles 422 are like upper portions of the legs, the outer paddles 420 are like lower portions of the legs, and the joint portions 423 are like knee portions of the legs. In some embodiments, the inner paddle portion 422, the outer paddle portion 420, and the joint portion 423 are formed from a continuous strip of fabric, such as a metal fabric or other fabric. In some embodiments, the strip of fabric can be a composite strip of fabric.

The anchors 408 can be configured to move between various configurations by axially moving the distal end (e.g., cap 414, etc.) relative to the proximal collar 411 and thus moving the anchors 408 (e.g., moving the anchors 408 relative to a coaption member 410 and/or another portion of the device) along a longitudinal axis extending between the first or distal and second or proximal portions 417, 419 of the coaption member 410. For example, the anchors 408 can be positioned in a straight configuration by moving the distal end or cap 414 away from the coaption member 410 and/or another portion of the device. In the straight configuration, the paddle portions are aligned or straight in the direction of the longitudinal axis of the device and the joint portions 423 of the anchors 408 are adjacent the longitudinal axis of the device and/or a coaption member 410 of the device. From the straight configuration, the anchors 408 can be moved to a fully folded configuration (e.g., Figure 21 (or any position in between) by moving the anchors toward the coaption member 410 and/or another portion of the device. Initially as the distal end or cap 414 moves toward the coaption member 410 and/or another portion of the device, the anchors 408 bend at the joint portions 423 and the joint portions 423 move radially outwardly relative to the longitudinal axis of the device and/or a coaption member 410 of the device and axially toward the first portion of the device and/or coaption member 410. As the distal end or cap 414 continues to move toward the coaption member 410 and/or another portion of the device, the joint portions 423 move radially inwardly relative to the longitudinal axis of the device and/or coaption member 410 and axially toward the proximal portion 419 of the device and/or coaption member 410, as shown in Figure 21.

In some embodiments, an angle between the inner paddles 422 of the anchors 408 and the coaption member 410 and/or a midline of the device can be approximately 180 degrees when the anchors 408 are in the straight configuration, and the angle between the inner paddles 422 of the anchors 408 and the coaption member 410 and/or a midline of the device can be approximately 0 degrees when the anchors 408 are in the fully folded configuration The anchors 408 can be positioned in various partially folded configurations such that the angle between the inner paddles 422 of the anchors 408 and the coaption member 410 and/or a midline of the device can be approximately 10-170 degrees or approximately 45-135 degrees. The midline can be a longitudinal axis of the device.

Referring again to Figure 21 the clasps 430 can comprise attachment or fixed portions and arm or moveable portions. The attachment or fixed portions can be coupled to the inner paddles 422 of the anchors 408 in various ways such as with sutures, adhesive, fasteners, welding, stitching, swaging, friction fit and/or other means for coupling. The moveable portions 434 can flex, articulate, or pivot relative to the fixed portions 432 between an open configuration and a closed configuration (Figure 21). In some embodiments, the clasps 430 can be biased to the closed configuration. In the open configuration, the fixed portions and the moveable portions flex, articulate, or pivot away from each other such that native leaflets can be positioned between the fixed portions and the moveable portions. In the closed configuration, the fixed portions and the moveable portions flex, articulate, or pivot toward each other, thereby clamping the native leaflets between the fixed portions and the moveable portions.

Referring now to Figure 21A, an example embodiment of an implantable prosthetic device 400A is shown. The device 400A can include any other features for an implantable prosthetic device discussed in the present application, and the device 400A can be positioned to engage valve tissue (e.g., leaflets 20, 22) as part of any suitable valve repair system (e.g., any valve repair system disclosed in the present application).

The prosthetic device 400A can include a coaption portion 404A and an anchor portion 406A, the anchor portion 406A including a plurality of anchors 408A. The coaption portion 404A includes a coaption member or spacer 410A. The anchor portion 406A includes a plurality of paddles 420A (e.g., two in the illustrated embodiment), and a plurality of clasps 430A (e.g., two in the illustrated embodiment). A first or proximal collar 411A, and a second collar or cap 414A are used to move the coaption portion 404A and the anchor portion 406A relative to one another.

The coaption member 410A extends from a proximal portion 419B assembled to the collar 411A to a distal portion 417A that connects to the anchors 408A. The coaption member 410A and the anchors 408A can be coupled together in various ways. For example, as shown in the illustrated embodiment, the coaption member 410A and the anchors 408A can be coupled together by integrally forming the coaption member 410A and the anchors 408A as a single, unitary component. This can be accomplished, for example, by forming the coaption member 410A and the anchors 408A from a continuous strip 401A of a braided or woven material, such as braided or woven nitinol wire.

The anchors 408A are attached to the coaption member 410A by portions 425A and to the cap 414Aby portions 421A. The anchors 408A can comprise first portions or outer paddles 420A and second portions or inner paddles 422A separated by joint portions 423A. The joint portions 423A are attached to paddle frames 424A that are attached to the cap 414A. In this manner, the anchors 408A are configured similar to legs in that the inner paddles 422A are like upper portions of the legs, the outer paddles 420A are like lower portions of the legs, and the joint portions 423A are like knee portions of the legs. In the illustrated example, the inner paddle portion 422A, the outer paddle portion 420A, and the joint portion 423A are formed from the continuous strip of fabric 401A, such as a metal fabric.

The anchors 408A can be configured to move between various configurations. For example, the anchors 408A can be moved relative to a coaption member 410A and/or another portion of the device. In some embodiments, the anchors can be moved between configurations by axially moving a distal end of the device (e.g., a cap 414A) relative to a proximal end of the device (e.g., a proximal collar 411A) and thus moving the anchors 408A along a longitudinal axis extending between the distal end (or cap 414A) and the proximal end (or proximal collar 411A). For example, the anchors 408 can be positioned in a straight configuration (see for example, Figures 8, 9) by moving the cap 414A away from the coaption member 410A and/or another portion of the device. In the straight configuration, the paddle portions 420A, 422A are aligned or straight in the direction of the longitudinal axis of the device and the joint portions 423A of the anchors 408A are adjacent the longitudinal axis of the device and/or coaption member 410A of the device. From the straight configuration, the anchors 408 can be moved to a fully folded configuration by moving the distal end of the device (or cap 414A) toward the coaption member 410A and/or another portion of the device. Initially, as the cap 414A moves toward the coaption member 410A and/or another portion of the device, the anchors 408A bend at joint portions 421A, 423A, 425A, and the joint portions 423A move radially outwardly relative to the longitudinal axis of the device 400A and axially toward the distal portion 417A of the device and/or coaption member 410A. As the cap 414A continues to move toward the coaption member 410A and/or another portion of the device, the joint portions 423A move radially inwardly relative to the longitudinal axis of the device 400A and axially toward the proximal portion 419B of the device and/or coaption member 410A.

In some embodiments, an angle between the inner paddles 422A of the anchors 408A and the coaption member 410A and/or a midline of the device can be approximately 180 degrees when the anchors 408A are in the straight configuration and the angle between the inner paddles 422A of the anchors 408A and the coaption member 410A and/or a midline of the device can be approximately 0 degrees when the anchors 408A are in the fully folded configuration (see e.g., Figure 21A). The anchors 408A can be positioned in various partially folded configurations such that the angle between the inner paddles 422A of the anchors 408A and the coaption member 410A and/or a midline of the device can be approximately 10-170 degrees or approximately 45-135 degrees. The midline can be a longitudinal axis of the device.

Configuring the prosthetic device 400A such that the anchors 408A can extend to a straight or approximately straight configuration (e.g. approximately 120-180 degrees relative to the coaption member 410A and/or a midline of the device) can provide several advantages. For example, this can reduce the radial crimp profile of the prosthetic device 400A. It can also make it easier to grasp the native leaflets by providing a larger opening in which to grasp the native leaflets. Additionally, the relatively narrow, straight configuration can prevent or reduce the likelihood that the prosthetic device 400A will become entangled in native anatomy (e.g., chordae tendineae) when positioning and/or retrieving the prosthetic device 400A into the delivery apparatus.

The clasps 430A can comprise attachment or fixed portions 432C and arm or moveable portions 434C. The attachment or fixed portions 432C can be coupled to the inner paddles 422A of the anchors 408A in various ways such as with sutures, adhesive, fasteners, welding, stitching, swaging, friction fit, and/or other means for coupling. The clasps 430A are similar to the clasps 430.

In some embodiments, the moveable portions 434C can articulate, flex, or pivot relative to the fixed portions 432C between an open configuration and a closed configuration. In some embodiments, the clasps 430A can be biased to the closed configuration. In the open configuration, the fixed portions 432C and the moveable portions 434C articulate, pivot, or flex away from each other such that native leaflets can be positioned between the fixed portions 432C and the moveable portions 434C. In the closed configuration, the fixed portions 432C and the moveable portions 434C articulate, pivot, or flex toward each other, thereby clamping the native leaflets between the fixed portions 432C and the moveable portions 434C.

The strip 401A is attached to the collar 411A, cap 414A, paddle frames 424A, clasps 430A to form both the coaption portion 404A and the anchor portion 406A of the device 400A. In the illustrated embodiment, the coaption member 410A, hinge portions 421A, 423A, 425A, outer paddles 420A, and inner paddles 422A are formed from the continuous strip 401A. The continuous strip 401A can be a single layer of material or can include two or more layers. In some embodiments, portions of the device 400A have a single layer of the strip of material 401A and other portions are formed from multiple overlapping or overlying layers of the strip of material 401A. For example, Figure 21A shows the coaption member 410A and inner paddles 422A formed from multiple overlapping layers of the strip of material 401A. The single continuous strip of material 401A can start and end in various locations of the device 400A. The ends of the strip of material 401A can be in the same location or different locations of the device 400A. For example, in the illustrated embodiment of Figure 21A, the strip of material begins and ends in the location of the inner paddles 422A.

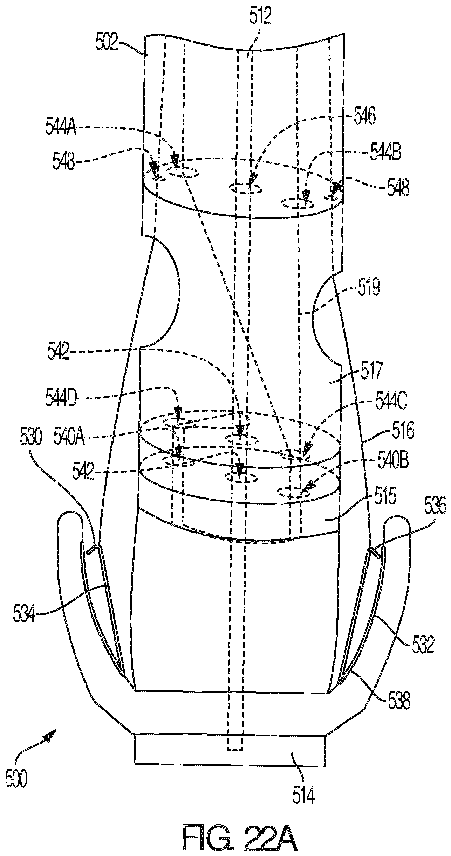

Referring to Figures 22A through 22M, an implantable prosthetic device 500, a delivery catheter 502, an actuation element 512, a collar 515, and a coupler 517 are depicted according to one embodiment. The device 500 can incorporate any of the features of any of the devices 100, 400, 400a described herein, including a cap 514 (or other attachment portion) and clasps 530 that include a base or fixed arm 532, a moveable arm 534, barbs 536, and a joint portion 538. Clasp actuation lines 516 can connect the moveable arms 534 to the coupler 517 and/or the delivery catheter 502. The clasps 530 can be opened by applying tension to the actuation lines 516 attached to the moveable arms 534, thereby causing the moveable arms 534 to flex, articulate, or pivot on the joint portions 538.

The collar 515 includes a first tether passage 540A, a second tether passage 540B, and a wire passage 542 extending through the collar 515. The coupler 517 includes a first tether passage 544A and a second tether passage 544B in the top or upper portion of the collar 515, a third tether passage 544C and a fourth tether passage 544D in the bottom or lower portion of the coupler 517, and a wire passage 546 extending through the coupler 517. The collar 515 can be attached, coupled, or secured to the delivery catheter 502, the actuation element 512, and/or the coupler 517 via a coupling tether 519.

By diametrically opposing the tether passages 544A, 544B and corresponding passages in the catheter 502, tensile forces applied to the tether cancel one another out. This prevents unintended bending of the catheter due to pulling on the tether lines. In some example embodiments, the tether passages 544A, 544B and corresponding catheter passages for the tether are not diametrically opposed.

As shown in Figure 22A, the actuation element 512 can be inserted through the wire passage 546 of the coupler 517 and the wire passage 542 of the collar 515 and into the device 500 such that the actuation element 512 engages the cap 514. The coupling tether 519 can be inserted through the first tether passage 544A of the coupler 517, through the third tether passage 544C of the coupler 517, through the first tether passage 540A of the collar 515, around the bottom of the collar 515 and through the second tether passage 540B of the collar 515, through the fourth tether passage 544D of the coupler 517, around the actuation element 512, back through the fourth tether passage 544D of the coupler 517, through the second tether passage 540B of the collar 515, back around the bottom of the collar 515 and through the first tether passage 540A of the collar 515, through the third tether passage 544C of the coupler 517, and through the second tether passage 544B of the coupler 517.

The coupler 517 can also include one or more actuation passages 548. The one or more clasp actuation lines 516 can extend from the delivery catheter 502, through the actuation passages 548 and connect to the moveable arms 534.

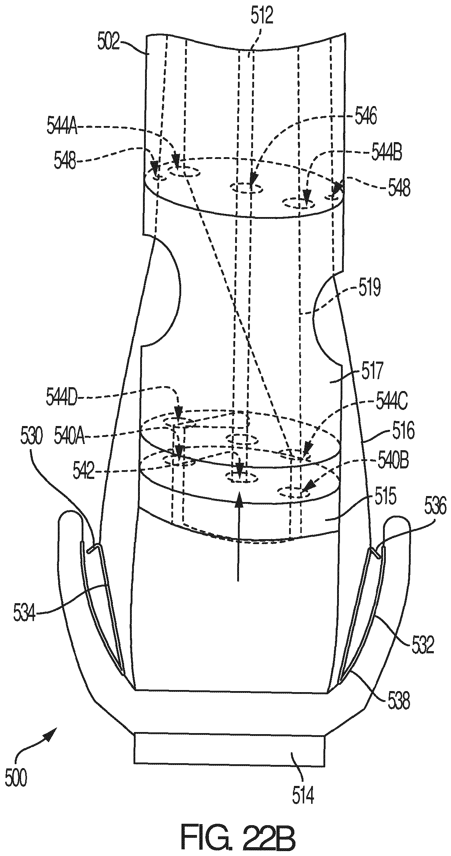

As shown in Figure 22B, the device 500 can be uncoupled from the actuation element 512. For example, the actuation element can be unscrewed or otherwise released from the cap 514. The actuation element 512 can be retracted from the device 500 and the collar 515 and into the coupler 517 such that the coupling tether 519 remains looped around the actuation element 512. In such a position, the device 500 and the collar 515 can move or pivot relatively freely from the coupler 517, the delivery catheter 502, and the actuation element 512 but remain coupled to the coupler 517, the delivery catheter 502, and the actuation element 512 via the coupling tether 519.

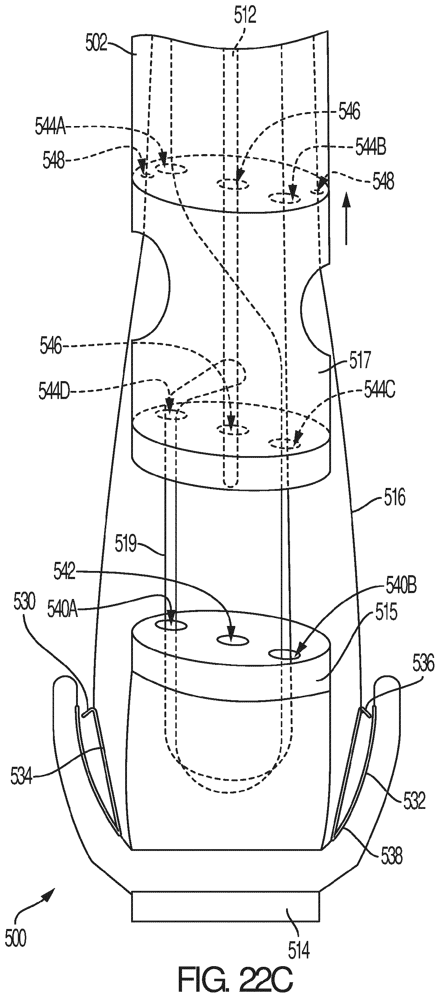

As shown in Figure 22C, slack can be introduced into the coupling tether 519 and clasp actuation lines 516 and the delivery catheter 502, the actuation element 512, and the coupler 517 can be retracted from the device 500 and the collar 515. The collar 515 and the device 500 can still be attached to the delivery catheter 502, the actuation element 512, and/or the coupler 517 via the coupling tether 519 and the actuation lines 516. In such a position, the device 500 remains tethered and/or attached to the delivery catheter 502, the actuation element 512, and/or the coupler 517 but, with sufficient slack in the clasp actuation lines 516 and the coupling tether 519, the device 500 is generally free to move relative to the delivery catheter 502, the actuation element 512, and the coupler 517. In such a position, a user may observe or check how the device 500 looks or operates when the device 500 is actually deployed, such as on the native leaflets or native mitral valve leaflets.

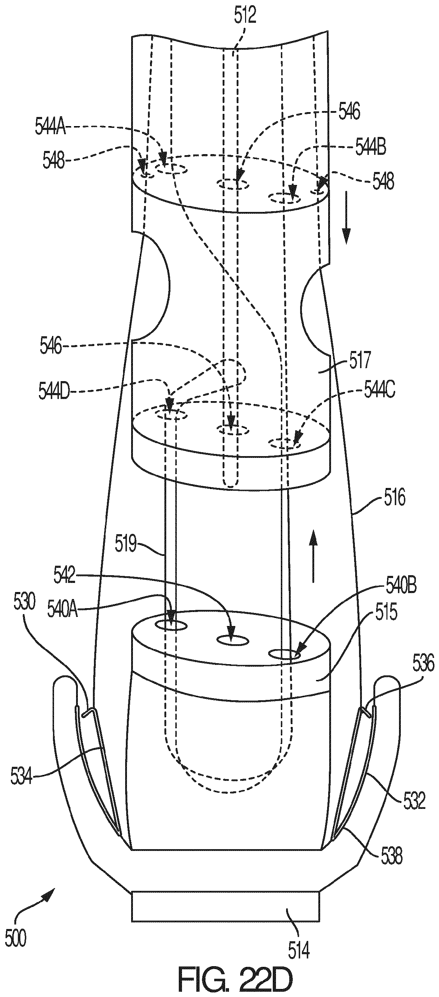

Referring to Figures 22D through 22F, the deice 500 and the collar 515 can be recoupled to the delivery catheter 502, the actuation element 512, and the coupler 517. For example, the device 500 and the collar 515 can be recoupled to the delivery catheter 502, the actuation element 512, and the coupler 517 if the user observes that the device 500 is not properly deployed or properly in place.

As shown in Figure 22D, the device 500 and the collar 515 can be recoupled to the delivery catheter 502, the actuation element 512, and the coupler 517. Tension can be applied to the coupling tether 519 and/or the delivery catheter 502, the actuation element 512, and the coupler 517 can be advanced forward such that the device 500 and the collar 515 can be recoupled to the delivery catheter 502, the actuation element 512, and the coupler 517. Tension can also be applied to the clasp actuation lines 516 to bring the clasp actuation lines 516 closer to the device 500 and within the coupler 517 and/or the delivery catheter 502 such that the clasp actuation lines 516 do not move around, get tangled, or get pinched between the coupler 517 and the cap 514. In one embodiment, the structure of the coupler 517 and the collar 515 operate as a pulley or force multiplier with the coupling tether 519. With the illustrated routing of the tether, retraction of only one end of the coupling tether 519 results in the collar 515 and the coupler 517 being brought together one-fourth of the distance the end coupling tether 519 is retracted. Similarly, wherein retraction of both ends of the coupling tether 519 through the catheter results in the collar 515 and the coupler 517 being brought together one-half the distance the two ends of the coupling tether 519 are retracted.

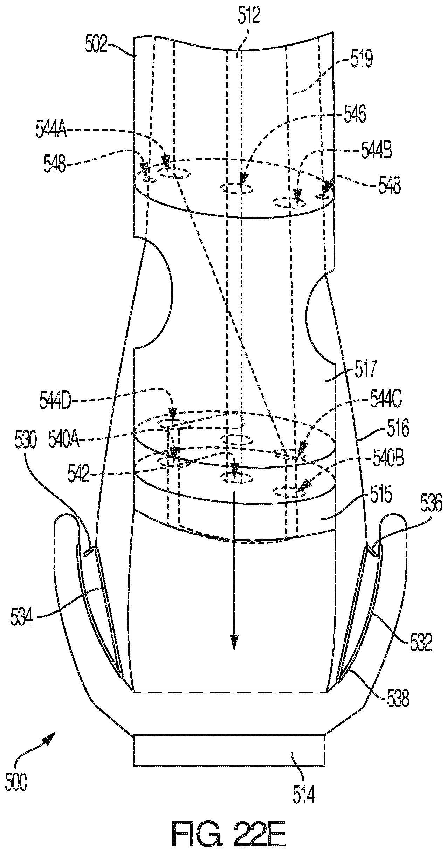

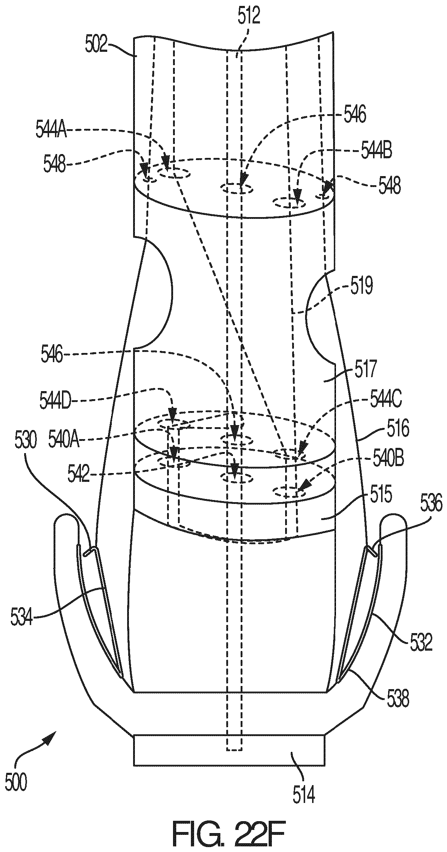

As shown in Figure 22E, the coupler 517 can be brought back into contact with the collar 515 and the actuation element 512 can be advanced through the wire passage 546 of the coupler 517 and the wire passage 542 of the collar 515 and into the device 500. As shown in Figure 22F, the actuation element can be advanced through the collar 515 and the coupler 517 and into the device 500 until the actuation element 512 reengages the anchor portion 506 and/or the cap 514 of the device 500.

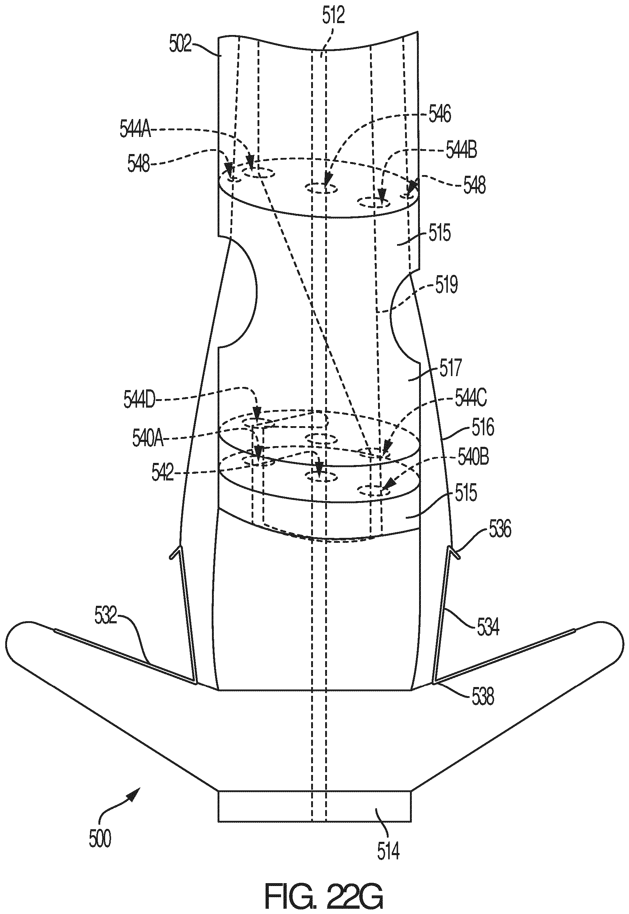

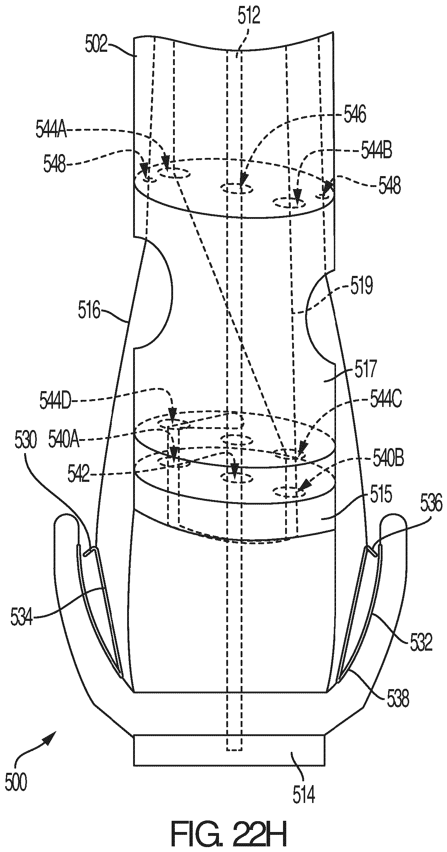

Referring now to Figures 22G and 22H, the device 500 can be reopened or moved back to the partially open, grasp-ready condition. This movement allows removal of the device from the native valve leaflets and allows the device to be repositioned on the native valve leaflets. As shown in Figure 22G, to transition from the fully closed condition to the partially open condition (or another position), the actuation element 512 is extended to push the cap 514 away from the collar 515. The actuation element 512 can move the device 500 to the partially open, grasp-ready condition in a wide variety of ways. For example, the actuation element 512 can move the device 500 to the partially open, grasp-ready condition as described relating to Figure 14G. While in the partially open, grasp-ready condition, the device 500 can be moved or repositioned. For example, a user may reposition the device 500 to properly grasp the native valve leaflets.

As shown in Figure 22H, the device 500 can be moved to the fully closed or deployed condition. For example, the device 500 can be moved back to the deployed condition once the device 500 has been positioned or repositioned to the desired position. The actuation lines 516 can be extended to allow the clasps 530 to close. Either or both of the actuation lines 516 can be repeatedly actuated to repeatedly open and close the clasps 530. For example, the clasps 530 can be repeatedly opened and closed to ensure that the device 500 is properly placed.

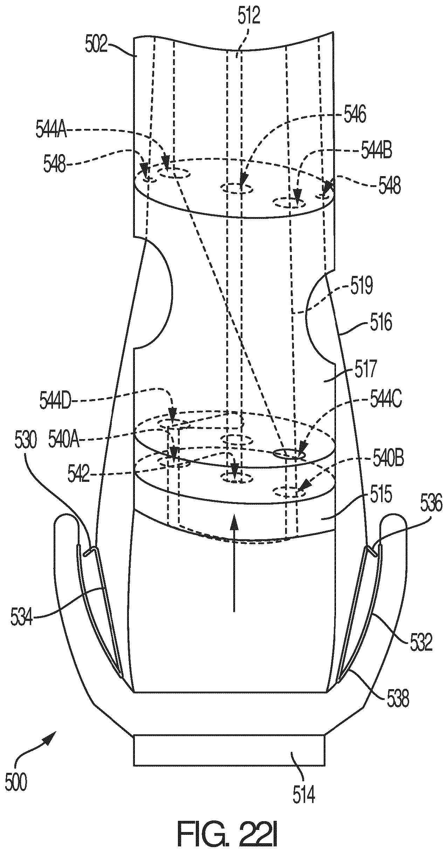

Referring now to Figures 22I through 22M, the device 500 can be deployed from the delivery catheter 502, the coupler 517, and the actuation element 512. For example, the device 500 can be deployed once the device 500 is repositioned in the proper place and grasping the native valve leaflets.

As shown in Figure 22I, the device 500 can be uncoupled from the actuation element 512. The actuation element 512 can be retracted from the device 500 and the collar 115, toward the coupler 517 and the delivery catheter 502. The actuation element 512 can be retracted such that the end of the actuation element 512 is positioned within the wire passage 546 of the coupler 517. In such a position, the device 500 and the collar 515 can move or pivot relatively freely from the coupler 517, the delivery catheter 502, and the actuation element 512.

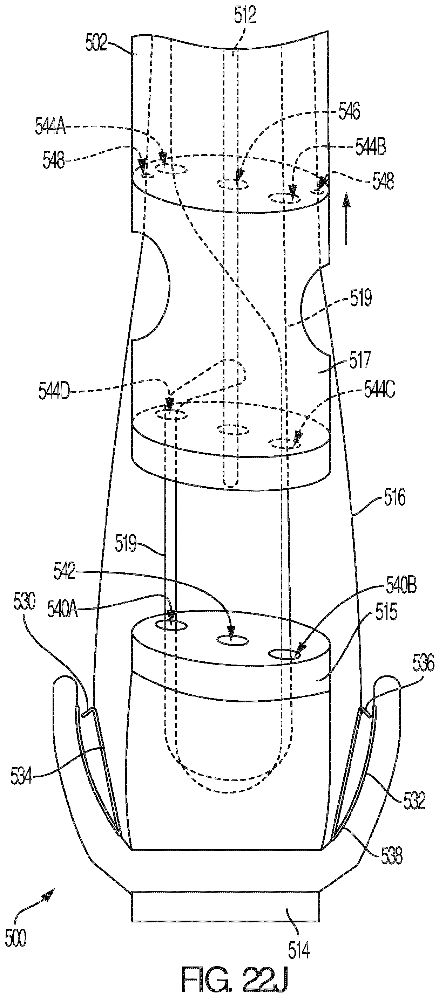

As shown in Figure 22J, slack can be introduced into the clasp actuation lines 516 and the coupling tether 519 such that the delivery catheter 502, the actuation element 512, and the coupler 517 can be retracted from the device 500 and the collar 515. The collar 515 can remain attached to the delivery catheter 502, the actuation element 512, and/or the coupler 517 via the coupling tether 519 and the device 500 can remain attached to the coupler 517 and/or the delivery catheter 502 via the clasp actuation lines 516. The coupling tether 516 can remain looped around or otherwise secured to the actuation element 512 within the wire passage 546 of the coupler 517. In some example embodiments, the coupling tether 516 can be looped around another structure of the coupler 517 and not around the wire. With the wire in the position illustrated by Figure 22J, the device 500 remains tethered or attached to the delivery the actuation element 512 and the coupler 517. With sufficient slack in the clasp actuation lines 516 and the coupling tether 519, the device 500 is generally free to move relative to the delivery catheter 502, the actuation element 512, and the coupler 517. As such, a user may observe or check how the device 500 will look or operate when the device 500 is completely implanted, such as on the native leaflets or native mitral valve leaflets.

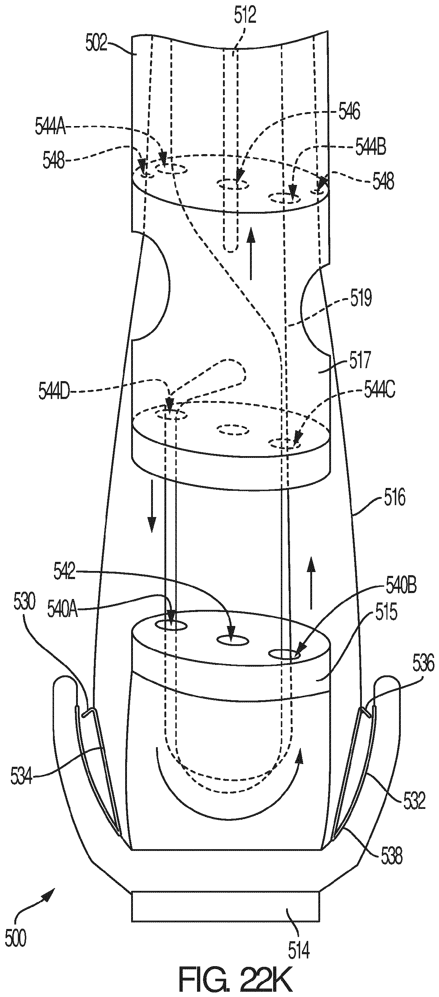

As shown in Figure 22K, the actuation element 512 can be retracted or withdrawn further into the coupler 517 or into the delivery catheter 502, beyond the coupling tether 519. As a result, the coupling tether 519 is no longer secured around the actuation element 512 above the third and fourth tether passageways 544C, 544D of the coupler 517. As such, the coupling tether 519 no longer securely couples the collar 515 and the device 500 to the coupler 517. As mentioned above, in an example embodiment, the coupling tether 519 can be looped around another structure of the coupler 517, instead of around the wire. In either case, one end of the one or more coupling tethers 519 can be pulled or retracted into the delivery catheter 502 to decouple the tether from the collar 515, instead of decoupling the looped end and withdrawing both free ends of the tether. Either end of the coupling tether 519 can be pulled or retracted such that the looped portion of the coupling tether 519 is pulled or retracted through the fourth tether passage 544D of the coupler 517, through the second tether passage 540B of the collar 515, around the bottom of the collar 515, through the first tether passage 540A of the collar 515, through the third tether passage 544C of the coupler, and through either the first or second tether passage 544A, 544B of the coupler 517, depending on which end of the coupling tether 519 is pulled or retracted. However, the coupling tether 519 can be pulled or retracted to decouple the coupler 517 and the collar 515 in a variety of other ways. For example, both ends of the coupling tether 519 might be pulled or retracted such that the looped portion of the coupling tether 519 is pulled or retracted through the fourth tether passage 544D of the coupler 517, through the second tether passage 540B of the collar 515, around the bottom of the collar 515, through the first tether passage 540A of the collar 515, and through the third tether passage 544C of the coupler, the coupling tether 519 can be pulled or retracted to a distance less than through the third tether passage 544C of the coupler 517, the coupler 517 and the delivery catheter 502 can be retracted to pull or retract the looped portion of the coupling tether 519 through the fourth tether passage 544D of the coupler 517, the second tether passage 540B of the collar 515, and the first tether passage 540A of the collar 515, or any combination thereof.

While it has been described that the actuation element 512 is retracted before the coupling tether 519 is pulled to decouple the collar 515 from the coupler 517, other methods of decoupling the collar 515 and the coupler 517 are contemplated. For example, one end of the coupling tether 519 might be pulled such that the opposing end of the coupling tether 519 is pulled through the first or second tether passage 544A, 544B of the coupler 517 (depending on which end of the coupling tether 519 is pulled), through the third tether passage 544C of the coupler, through the first tether passage 540A of the collar 515, through the second tether passage 540B of the collar 515, through the fourth tether passage 544D of the coupler 517, around the actuation element 512, back through the fourth tether passage 544D of the coupler 517, through the second tether passage 540B of the collar 515, through the first tether passage 540A of the collar 515, through the third tether passage 544C of the coupler 517, and, optionally, through the other of the first or second tether passage 544A, 544B of the coupler 517. In some example embodiments, one of the lines of the looped tether can be cut in or near the coupler 517. This reduces the length of tether that needs to be pulled through coupler 517 and/or cap 514 to release the device 500, when the tether is not released by retracting the actuation element 512.

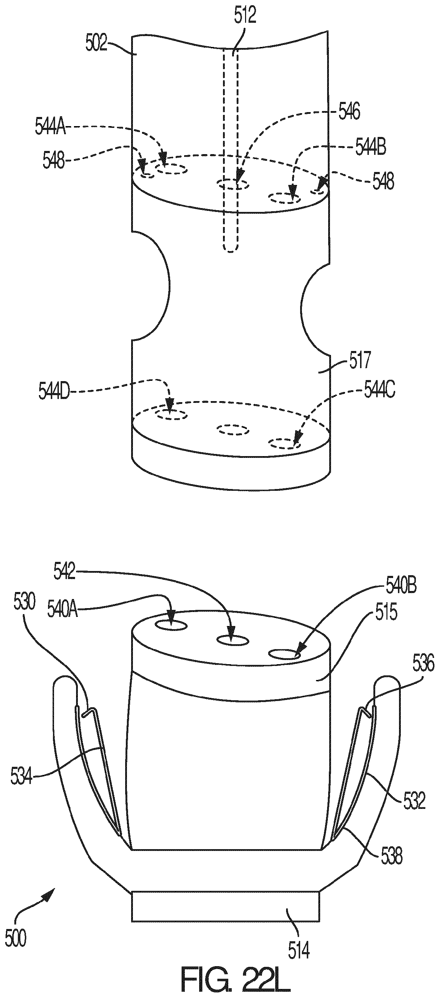

As shown in Figure 22L, once the device 500 and collar 515 are decoupled from the coupling tether 519, and the actuation element 512, the coupler 517 can be retracted away from the device 500. The clasp actuation lines 516 can be detached from the moveable arms 534 and pulled or otherwise retracted toward or into the coupler 517 and/or delivery catheter 502. In such an embodiment, the device 500 is completely detached from the delivery catheter 502, the coupler 517, and the actuation element 512

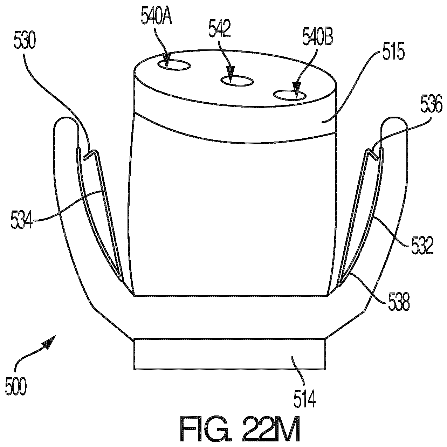

As shown in Figure 22M, the device 500 is shown in the fully closed and deployed condition. The delivery catheter 502, the coupler 517, and the actuation element 512 have been retracted and the clasps 530 remain in a fully closed position. Once deployed, the device 500 can be maintained in the fully closed position in a variety of ways. For example, the device 500 can be maintained in the fully closed position in any of the ways described relating to Figure 14M. In an example embodiment, the paddles of the device are configured to open and close with the beating of the heart, while the clasps remain in their closed configuration.

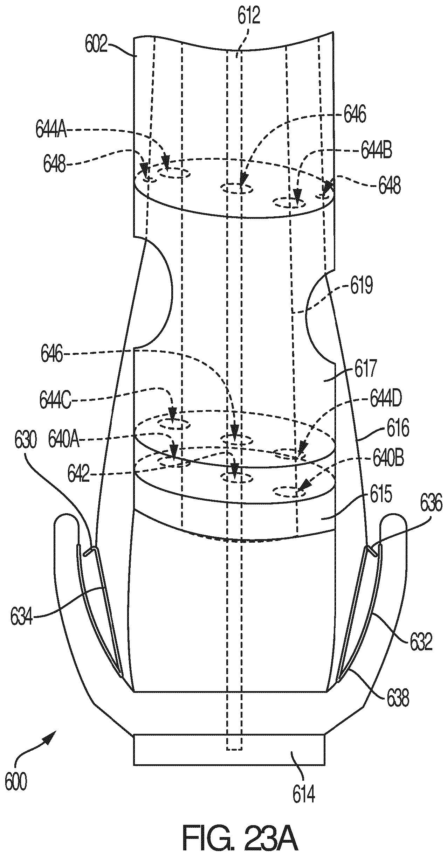

Referring to Figures 23A through 23M, an implantable prosthetic device 600, a delivery catheter 602, an actuation element 612, a collar 615, and a coupler 617 are depicted according to one example embodiment. The device 600 can incorporate any of the features of any of the devices 100, 400, 500 described herein, including a cap 614 and clasps 630 that include a base or fixed arm 632, a moveable arm 634, barbs 636, and a joint portion 638. Actuation lines 616 can connect the moveable arms 634 to the coupler 617 and/or the delivery catheter 602. The clasps 630 can be opened by applying tension to the actuation lines 616 attached to the moveable arms 634, thereby causing the moveable arms 634 to flex, articulate, or pivot on the joint portions 638.

The collar 615 includes a first tether passage 640A, a second tether passage 640B, and a wire passage 642 extending through the collar 615. The coupler 617 includes a first tether passage 644A and a second tether passage 644B in the top or upper portion of the collar 615, a third tether passage 644C and a fourth tether passage 644D in the bottom or lower portion of the coupler 617, and a wire passage 646 extending through the coupler 617. The collar 615 can be attached, coupled, or secured to the delivery catheter 602, the actuation element 612, and/or the coupler 617 via a coupling tether 619.

As shown in Figure 23A, the actuation element 612 can be inserted through the wire passage 646 of the coupler 617 and the wire passage 642 of the collar 615 and into the device 600 such that the actuation element 612 engages the cap 614. The coupling tether 619 can be inserted through the first tether passage 644A of the coupler 617, through the third tether passage 644C of the coupler 617, through the second tether passage 640B of the collar 615, around the bottom of the collar 615 and through the first tether passage 640A of the collar 615, through the fourth tether passage 644D of the coupler 617, and through the second tether passage 544B of the coupler 617.

The coupler 617 can also include one or more actuation passages 648. The one or more clasp actuation lines 516 can extend from the delivery catheter 602, through the actuation passages 648 and connect to the moveable arms 634.

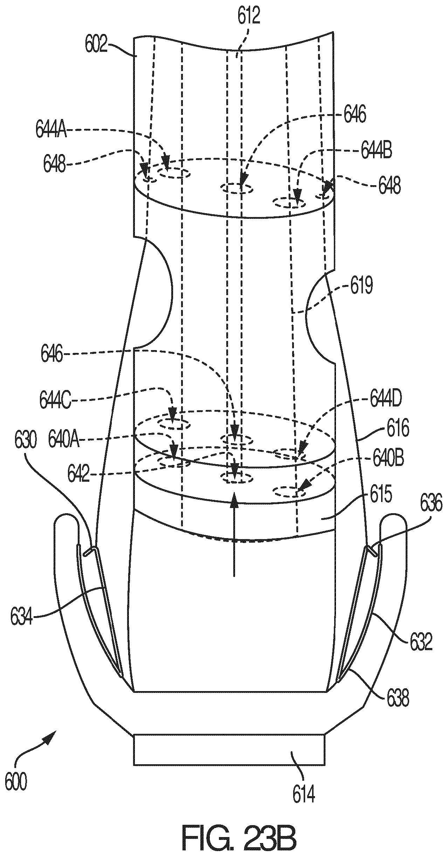

As shown in Figure 23B, the device 600 can be uncoupled from the actuation element 612. The actuation element 612 can be retracted from the device 600 and the collar 615 and into the coupler 617. In such a position, the device 600 and the collar 615 can move or pivot relatively freely from the coupler 617, the delivery catheter 602, and the actuation element 612, but remain tied to the coupler 617, the delivery catheter 602, and the actuation element 612 via the coupling tether 619.

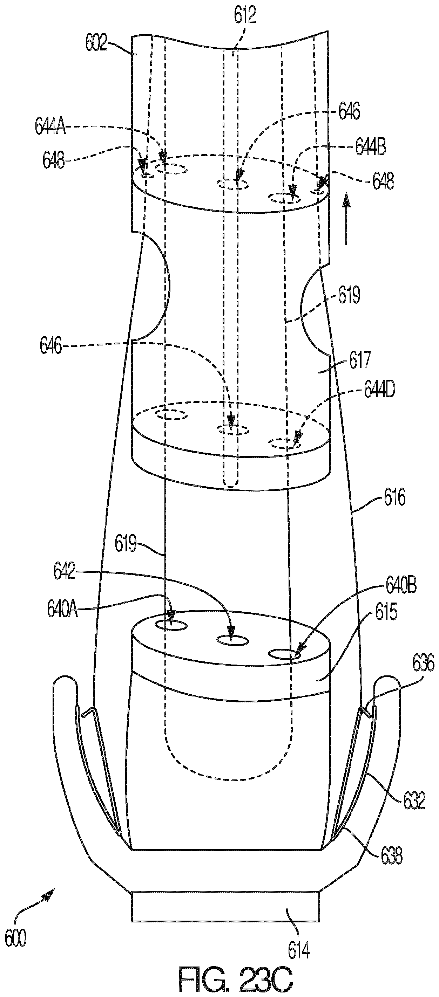

As shown in Figure 23C, slack can be introduced into the coupling tether 619 and clasp actuation lines 616 and the delivery catheter 602, the actuation element 612, and the coupler 617 can be retracted from the device 600 and the collar 615. The collar 615 and the device 600 can still be attached to the delivery catheter 602, the actuation element 612, and/or the coupler 617 via the coupling tether 619 and the actuation lines 616. In such a position, the device 600 remains tethered to the delivery catheter 602, the actuation element 612, and/or the coupler 617 but, with sufficient slack in the clasp actuation lines 616 and the coupling tether 619, the device 600 is generally free to move relative to the delivery catheter 602, the actuation element 612, and the coupler 617. In such a position, a user may observe or check how the device 600 will look or operate when the device 600 is completely implanted and released, such as on the native valve leaflets.

Referring to Figures 22D through 22F, the deice 600 and the collar 615 can be recoupled to the delivery catheter 602, the actuation element 612, and the coupler 617. For example, the device 600 and the collar 615 can be recoupled to the delivery catheter 602, the actuation element 612, and the coupler 617 if the user observes that the device 600 is not properly deployed or properly in place.

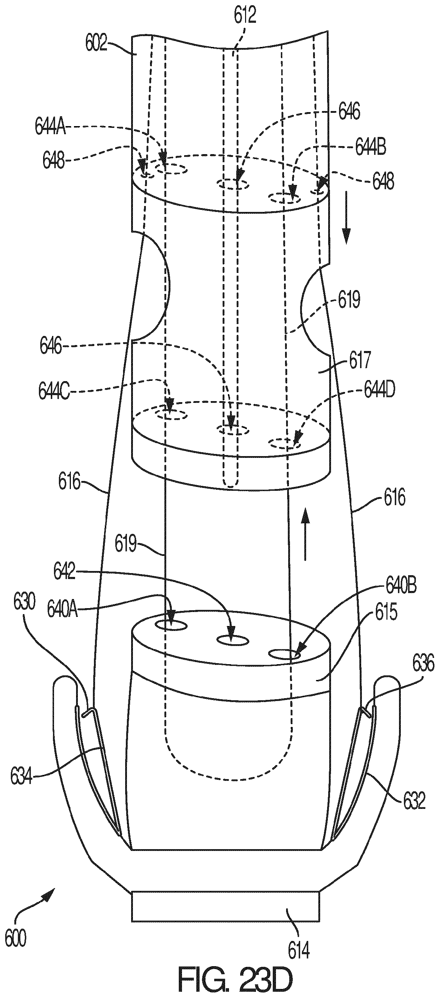

As shown in Figure 23D, the device 600 and the collar 615 can be recoupled to the delivery catheter 602, the actuation element 612, and the coupler 617. Tension can be applied to the coupling tether 619 and the delivery catheter 602, the actuation element 612, and the coupler 617 can be advanced over the tether 619 such that the device 600 can be recoupled to the delivery catheter 602, the actuation element 612, and the coupler 617. Tension can also be applied to the clasp actuation lines 616 to bring the clasp actuation lines 616 closer to the device 600 and within the coupler 617 and/or the delivery catheter 602 such that the clasp actuation lines 516 do not move around, get tangled or get pinched between the collar 615 and the coupler 617. In such an embodiment, the structure of the coupler 617 and the collar 615 operate as a pulley or force multiplier with the coupling tether 619 wherein retraction of one end of the coupling tether 619 results in the collar 615 and the coupler 617 being brought together one-half of the distance the end coupling tether 619 is retracted and wherein retraction of both ends of one of the coupling tether 619 results in the collar 615 and the coupler 617 being brought together the same distance the ends of the coupling tether 619 are retracted.

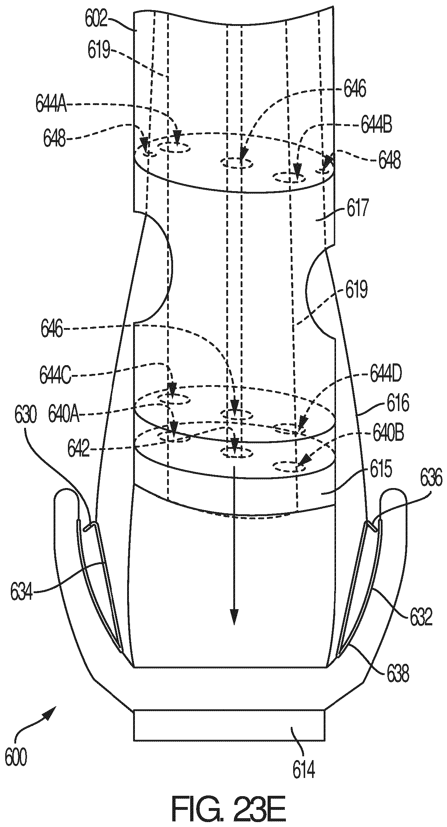

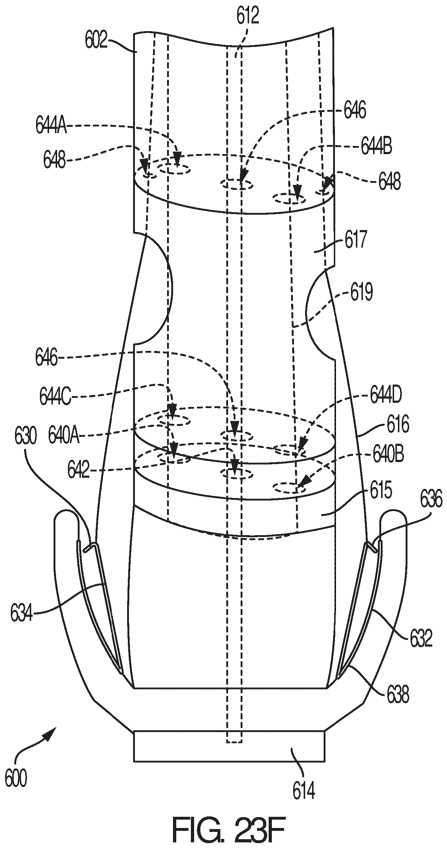

As shown in Figure 23E, the coupler 617 can be brought back into contact with the collar 615 and the actuation element 612 can be advanced through the wire passage 646 of the coupler 617 and the wire passage 642 of the collar 615 and into the device 600. As shown in Figure 23F, the actuation element can be advanced through the collar 615 and the coupler 617 and into the device 600 until the actuation element 612 reengages the cap 614 of the device 600.

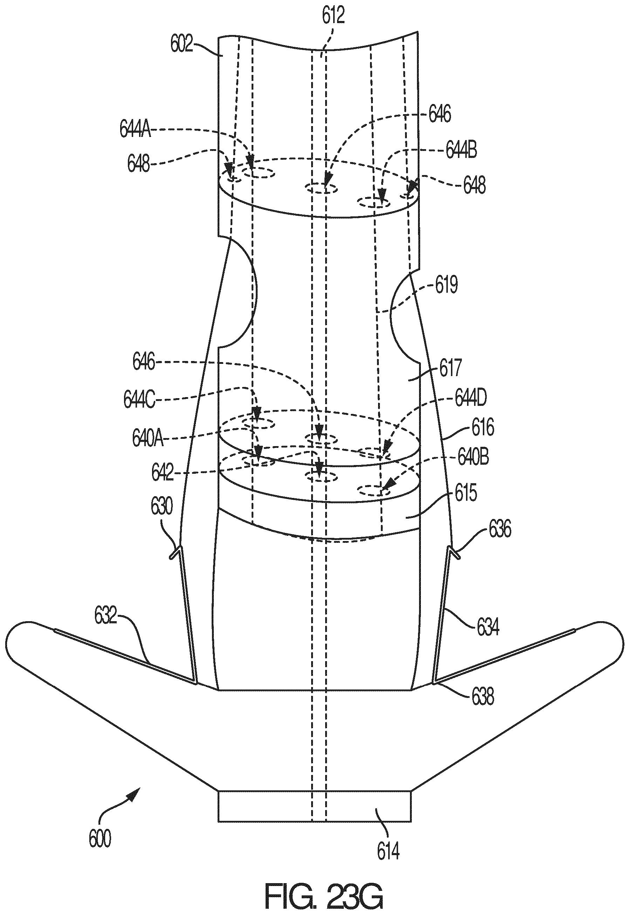

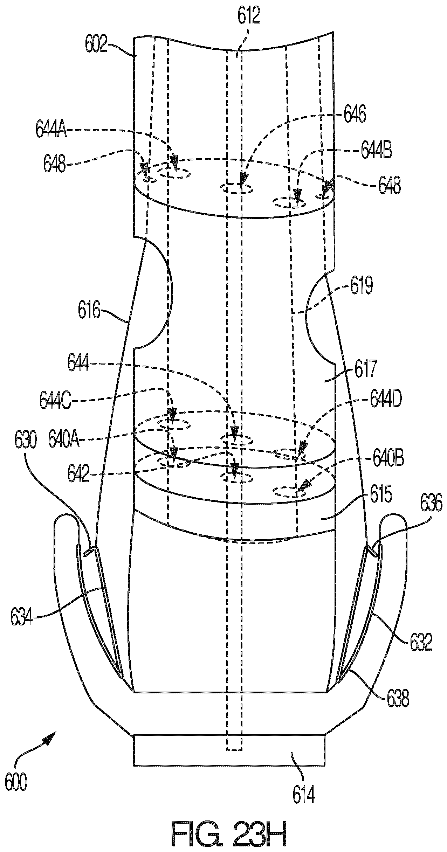

Referring now to Figures 23G and 23H, the device 600 can be reopened or moved back to the partially open position to release the previously captured leaflet(s), move to a new position, and recapture the leaflets. As shown in Figure 23G, to transition from the fully closed condition to the partially open condition, the actuation element 612 is extended to push the cap 614 away from the collar 615. The actuation element 612 can move the device 600 to the partially open condition in a wide variety of ways. For example, the actuation element 612 can move the device 600 to the partially open condition as described relating to Figure 14G. While in the partially open, grasp-ready condition, the device 600 can be moved or repositioned. For example, a user may reposition the device 600 to properly grasp the native valve leaflets.

As shown in Figure 23H, the device 600 can be moved to the fully closed or deployed condition. For example, the device 600 can be moved back to the deployed condition once the device 600 has been positioned or repositioned to the desired position on the native valve leaflets. The actuation lines 616 can be extended to allow the clasps 630 to close. Either or both of the actuation lines 516 can be repeatedly actuated to repeatedly open and close the clasps 630. For example, the clasps 630 can be repeatedly opened and closed to ensure that the device 600 is properly placed.

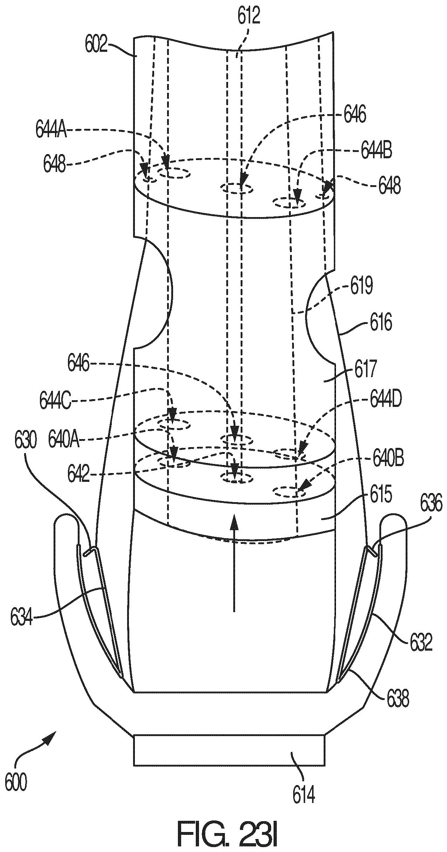

Referring now to Figures 23I through 23M, the device 600 can be deployed from the delivery catheter 602, the coupler 617, and the actuation element 612. For example, the device 600 can be deployed once the device 600 is in the proper place and grasping the native valve leaflets.

As shown in Figure 23I, the device 600 can be uncoupled from the actuation element 612. The actuation element 612 can be retracted from the device 600 and the collar 115, toward the coupler 617 and the delivery catheter 602. The actuation element 612 can be retracted such that an end of the actuation element 612 is positioned within the wire passage 646 of the coupler 617 or within the delivery catheter 602. In such a position, the device 600 and the collar 615 can move or pivot relatively freely from the coupler 617, the delivery catheter 602, and the actuation element 612.

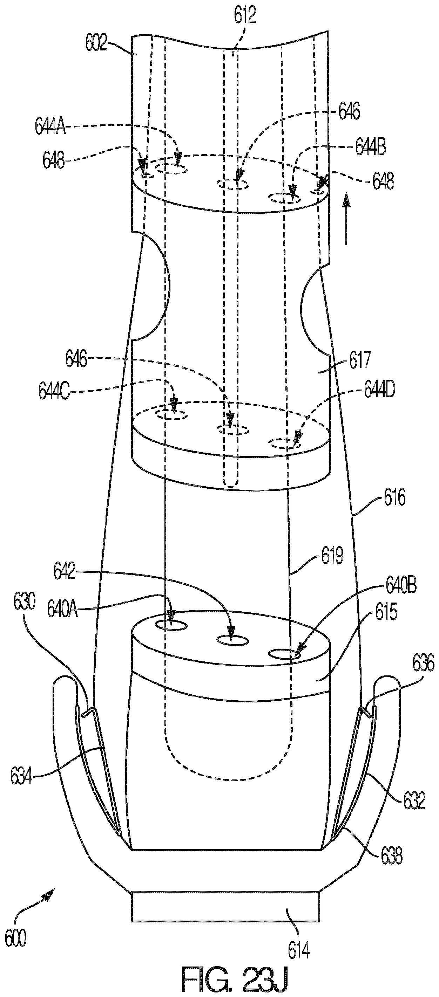

As shown in Figure 23J, slack can be introduced into the clasp actuation lines 616 and the coupling tether 619 such that the delivery catheter 602, the actuation element 612, and the coupler 617 can be retracted from the device 600 and the collar 615. The collar 615 can remain attached to the delivery catheter 602, the actuation element 612, and/or the coupler 617 via the coupling tether 619 and the device 600 can remain attached to the coupler 617 and/or the delivery catheter 602 via the clasp actuation lines 616. The coupling tethers 619 can remain looped around or otherwise secured to the collar 615. In such a position, the device 600 remains tethered or attached to the delivery catheter 602 and the coupler 617 but, with sufficient slack in the clasp actuation lines 616 and the coupling tether 619, the device 600 is generally free to move relative to the delivery catheter 602, the actuation element 612, and the coupler 617. As such, a user may observe or check how the device 600 will look or operate when the device 600 is finally deployed, such as on the native valve leaflets.

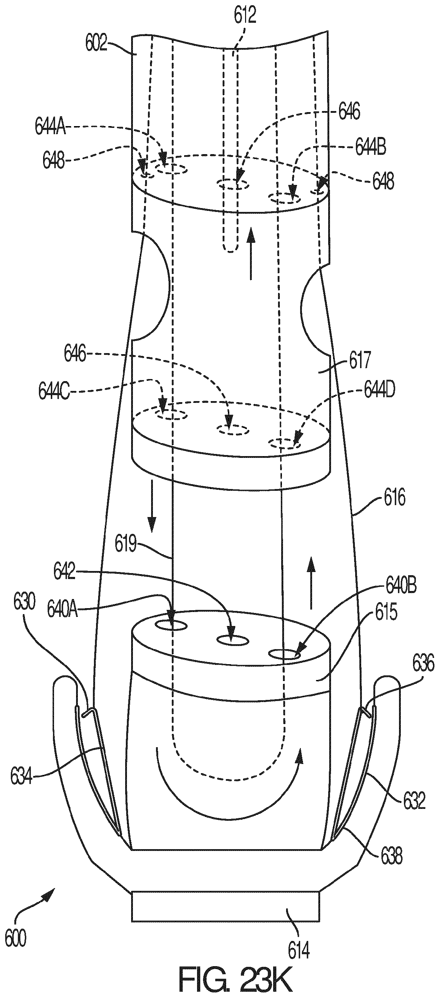

As shown in Figure 23K, the actuation element 612 can optionally be retracted or withdrawn further into the coupler 617 or into the delivery catheter 602. The coupling tether 619 can be retracted or decoupled from the collar 615. One end of the coupling tether 619 can be pulled such that the other end is pulled through the first tether passage 644A of the coupler 617, through the third tether passage 644C of the coupler 617, through the first tether passage 640A of the collar 615, and through the second tether passage 640B of the collar 615. Optionally, such end can also be pulled through the fourth tether passage 644D of the coupler and, further still, can be pulled through the second tether passage 644B. Conversely, the other end of the coupling tether 619 can be pulled such that the other end is pulled through the second tether passage 644B of the coupler 617, through the fourth tether passage 644D of the coupler 617, through the second tether passage 640B of the collar 615, and through the first tether passage 640A of the collar 615. Optionally, such end can also be pulled through the third tether passage 644C of the coupler and, further still, can be pulled through the first tether passage 644A.

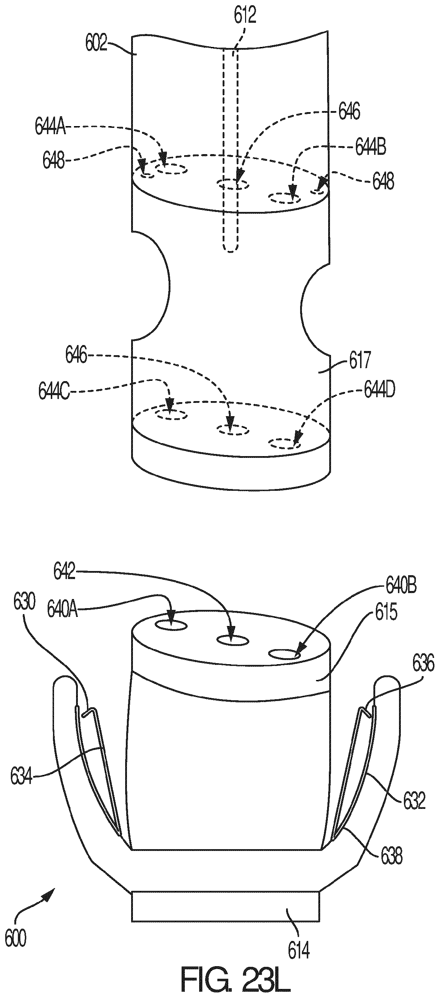

As shown in Figure 23L, after the device 600 is decoupled from the coupling tether 619, the delivery catheter 602, the actuation element 612, the coupler 617, and the coupling tether 619 can be retracted away from the device 600. The clasp actuation lines 616 are also detached from the moveable arms 634 and pulled or otherwise retracted toward or into the coupler 617 and/or delivery catheter 602. In such an embodiment, the device 600 and collar 615 are completely detached from the delivery catheter 602, the coupler 617, and the actuation element 612

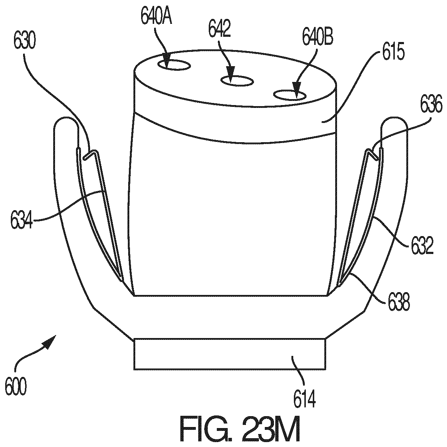

As shown in Figure 23M, the device 600 is shown in the fully closed and deployed condition. The delivery catheter 602, the coupler 617, and the actuation element 612 are retracted and the clasps 630 remain in a fully closed position. Once deployed, the device 600 can be maintained in the fully closed position in a variety of ways. For example, the device 600 can be maintained in the fully closed position in any of the ways described relating to Figure 14M. In one example embodiment, the paddles of the device are configured to open and close with the beating of the heart, while the clasps remain in their closed configuration.

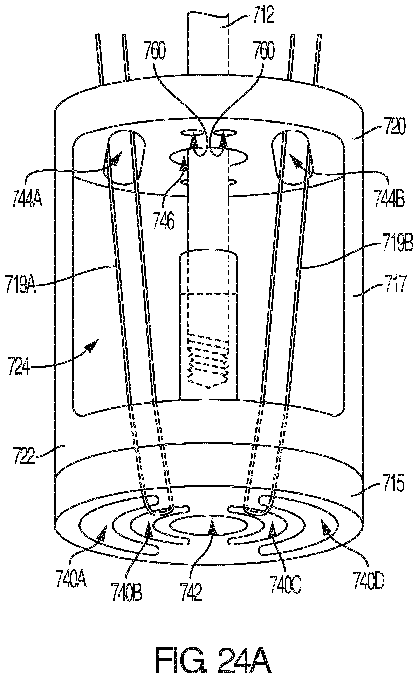

Referring now to Figures 24A through 24E, an actuation element 712, a collar 715, a coupler 717, and two coupling tethers 719 are depicted according to one embodiment. The collar 715 can be connected to any of the implantable prosthetic devices 100, 400, 500, 600 previously described herein or can be any other implantable prosthetic device.

The collar 715 includes a first tether passage 740A, a second tether passage 740B, a third tether passage 740C, and a fourth tether passage 740D, and a wire passage 742 extending through the collar 715. In the illustrated embodiment, the first, second, third, and fourth tether passages 740A, 740B, 740C, 740D are curved with the second tether passage 740B radially between the first tether passage 740A and the wire passage 742 and the third tether passage 740C radially between the wire passage 742 and the fourth tether passage 740D, however the first, second, third, and fourth tether passages 740A, 740B, 740C, 740D can have any shape or location. For example, the first, second, third, and fourth tether passages 740A, 740B, 740C, 740D can be circular and can be equidistant from the wire passage 742.

The coupler 717 is generally cylindrical with a top portion 720 and a bottom portion 722 which define a cutout or window 724 therebetween. The coupler 717 also includes a first tether passage 744A, a second tether passage 744B, and a wire passage 746 extending through the top portion 720 with the first and second tether passages 744A, 744B located on either side of the wire passage 746. The coupler also includes a third tether passage 744C, a fourth tether passage 744D, a fifth tether passage 744E, a sixth tether passage 744F, and a wire passage 746 extending through the bottom portion 722. The wire passage 746 of the bottom portion 722 corresponds to the wire passage 746 of the top portion 720. The third and the fourth tether passages 744C, 744D are radially opposite the wire passage 746 from the fifth and the sixth tether passages 744E, 744F, with the fourth tether passage 744D radially between the third tether passage 744C and the wire passage 746 and the fifth tether passage 744E radially between the wire passage 746 and the sixth tether passage 744F.

In the illustrated embodiment, the third, fourth, fifth, and sixth tether passages 744C, 744D, 744E, 744F of the coupler 717 correspond to the first, second, third, and fourth tether passages 740A, 740B, 740C, 740D of the collar 715, respectively. While the coupler 717 has been described as having third, fourth, fifth, and sixth tether passages 744C, 744D, 744E, 744F in the bottom portion 722, it will be appreciated that the coupler 717 can have a variety of configurations. For example, the third and fourth tether passages 744C, 744D and/or the fifth and sixth tether passages 744E, 744F can be combined to a single tether passage, respectively.

In one example embodiment, one or more of the passages 742, 746, 740A, 740B, 740C, 740D, 740E, 740F and/or other portions of the coupler and/or collar can include a keying feature that aligns passages of the coupler 717 with the passages of the collar 715. For example, a lip or protrusion around one or more of the passages 742, 746, 740A, 740B, 740C, 740D, 740E, 740F can fit within a corresponding passage to key the coupler 717 and collar 715 (i.e. align the passages).

Optionally, the coupler 717 can have two actuation passages 760 in the top or upper portion 720 which can receive one or more clasp actuation lines which can be coupled to moveable arms of an implantable device and extend through a delivery device as previously described herein. The clasp actuation lines extend out of the coupler 717 through the cutout or window 724.

As shown in Figure 24A, the collar 715 and the coupler 717 can be coupled together via a first coupling tether 719A and a second coupling tether 719B with the actuation element 712 disposed within the wire passages 746 of the coupler 717. The actuation element 712 can extend through the wire passage 742 of the collar 715. The actuation element 712 can extend into the implantable prosthetic device and engage a cap to open the device as previously described herein. The first coupling tether 719A passes through the first tether passage 744A of the coupler 717, through the third tether passage 744C of the coupler 717, through the first tether passage 740A of the collar 715, up through the second tether passage 740B of the collar 715, through the fourth tether passage 740D of the coupler 717, and back through the first tether passage 744A of the coupler 717. The second coupling tether 719B passes through the second tether passage 744B of the coupler 717, through the sixth tether passage 744F of the coupler 717, through the fourth tether passage 740D of the collar 715, up through the third tether passage 740C of the collar 715, through the fifth tether passage 744E of the coupler 717, and back through the second tether passage 744B of the coupler 717. The first and/or the second coupling tethers 719A, 719B can be threaded through the coupler 717 and the collar 715 in reverse order.

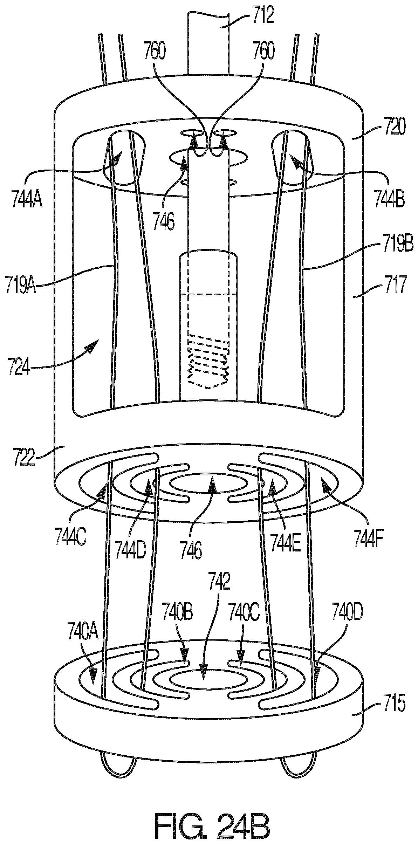

The actuation element 712, the collar 715, the coupler 717, the two coupling tethers 719 can be used to position and reposition an implantable prosthetic device in the native valve (e.g., the native mitral valve MV, native tricuspid valve, etc.) of the heart H, as previously described herein. Referring now to Figures 24B though 24E, the collar 715 can be decoupled from the coupler 717, the actuation element 712, and the coupling tethers 719. For example, the collar 715 can be decoupled to deploy an implantable prosthetic device in the closed position in the native valve of the heart H.

As shown in Figure 24B, slack can be introduced into the first and second coupling tethers 719A, 719B such that the collar 715 can be separated from the coupler 717 and the actuation element 712. In such a position, the collar 715 can move relatively freely from the coupler 717 and the actuation element 712. The collar 715 can be coupled with an implantable prosthetic device and, in such a position, the user may check the placement and functionality of the device as previously described herein.

In such an embodiment, the coupling tethers 719A, 719B, the third, fourth, fifth, and sixth tether passages 744C, 744D, 744E, 744F of the coupler 717, and the first, second, third, and fourth tether passages 740A, 740B, 740C, 740D of the collar 715 operate as two pulleys or two force multipliers. For example, a pull on one end of the first and second coupling tethers 719A, 719B results in a change in position of the collar 715 equivalent to one-half the distance of the pull and a pull on both ends of each of the first and second coupling tethers 719A, 719B results in a change in position equivalent to the distance of the push or pull.

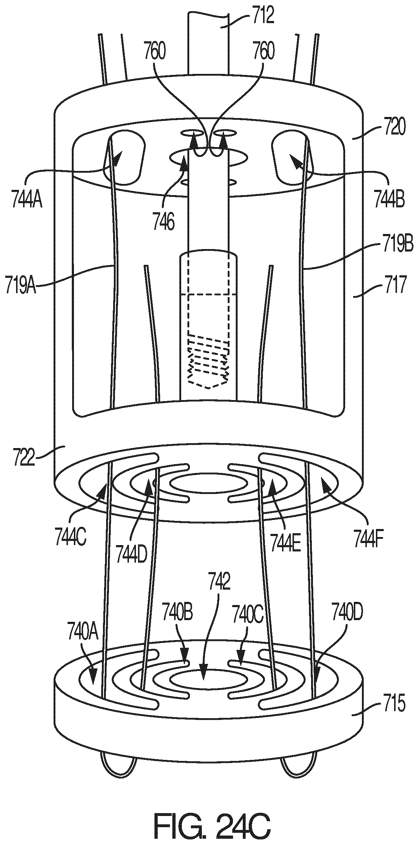

As shown in Figure 24C, one end of each of the coupling tethers 719A, 719B can be pulled or otherwise retracted to begin to release the collar 715 from the coupler 717. In the illustrated embodiment, the first coupling tether 719A is retracted to pull one end of the first coupling tether 719A through the first tether passage 744A of the coupler 717 toward the fourth tether passage 744D of the coupler 717 and the second coupling tether 719B is retracted to pull one end of the second coupling tether 719B through the second tether passage 744B of the coupler 717 toward the fifth tether passage 744E of the coupler 717.

Optionally, the first and second coupling tethers 719A, 719B can be retracted to pull one end of the first coupling tether 719A through the first tether passage 744A of the coupler 717 toward the third tether passage 744C of the coupler 717 and/or to pull one end of the second coupling tether 719B through the second tether passage 744B of the coupler 717 toward the sixth tether passage 744F of the coupler 717. For example, a user can begin to retract or otherwise release the coupling tethers 719A, 719B once the user has observed that the device is properly in place and functioning correctly.

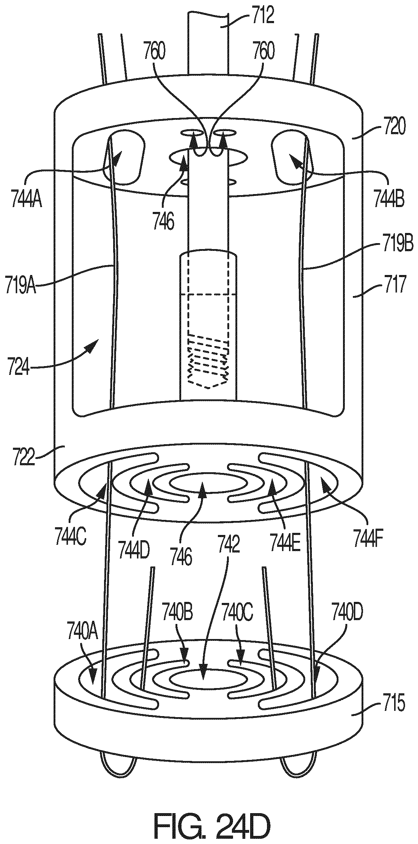

As shown in Figure 24D, the coupling tethers 719A, 719B can be further retracted to further release the collar 715 from the coupler 717. In the illustrated embodiment, the first coupling tether 719A is further retracted to pull one end of the first coupling tether 719A through the fourth tether passage 744D of the coupler 717 and toward the second tether passage 740B of the collar 715.The second coupling tether 719B is further retracted to pull one end of the second coupling tether 719B through the fifth tether passage 744E of the coupler 717 toward the third tether passage 740C of the collar 715. Optionally, the first and second coupling tethers 719A, 719B can be retracted to pull one end of the first coupling tether 719A through the third tether passage 744C of the coupler 717 toward the first tether passage 740A of the collar 715 and/or to pull one end of the second coupling tether 719B through the sixth tether passage 744F of the coupler 717 toward the fourth tether passage 740D of the collar 715.

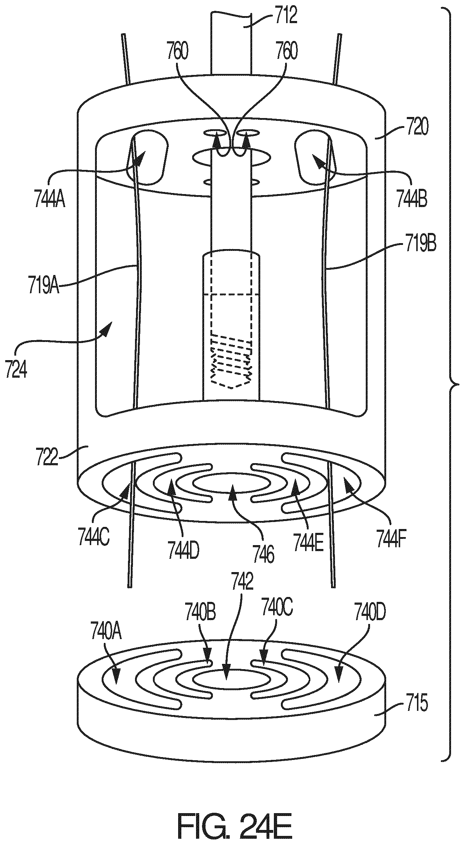

As shown in Figure 24E, the coupling tethers 719A, 719B are further retracted to completely release the collar 715 from the coupler 717, the actuation element 712, and the coupling tethers 719A, 719B. In the illustrated embodiment, the first coupling tether 719A is further retracted to pull one end of the first coupling tether 719A down through the second tether passage 740B of the collar 715, up through the first tether passage 740A of the collar 715, and toward the third tether passage 744C of the coupler and the second coupling tether 719B is further retracted to pull one end of the second coupling tether 719B down through the third tether passage 740C of the collar 715, up through the fourth tether passage 740D of the collar 715, and toward the sixth tether passage 744F of the coupler 717. Optionally, the first and second coupling tethers 719A, 719B can be retracted to pull one end of the first coupling tether 719A down through the first tether passage 740A of the collar 715, up through the second tether passage 740B of the collar 715, and toward the fourth tether passage 744D of the coupler 717 and/or to pull one end of the second coupling tether 719B down through the fourth tether passage 740D of the collar 715, up through the third tether passage 740C of the collar 715, and toward the fifth tether passage 744E of the coupler 717. In such a position, the collar 715 is completely decoupled from coupler 717, actuation element 712, and the coupling tethers 719A, 719B and the implantable prosthetic device would be deployed, such as in the native valve or native mitral valve MV of the heart H.

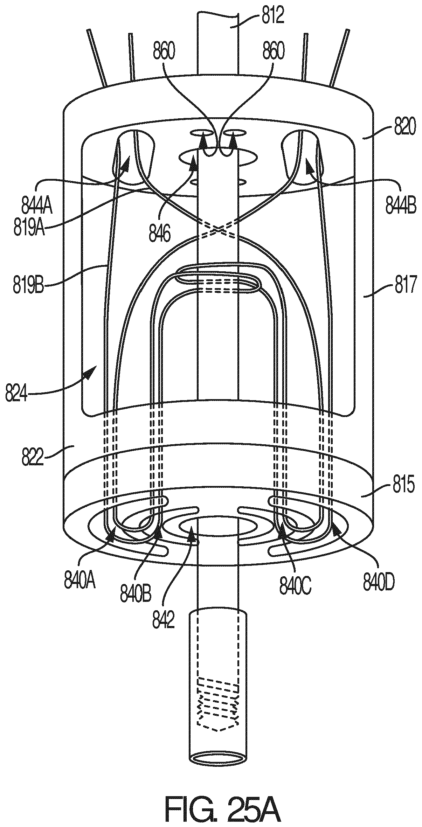

Referring now to Figures 25A through 25G, an actuation element 812, a collar 815, a coupler 817 and two coupling tethers 819 are depicted according to one embodiment. The embodiment is similar to that depicted in Figures 24A through 24E, however, the coupling of the coupler 817, the collar 815, and the actuation element 812 via the two coupling tethers 819 results in a double pulley. In the example illustrated by Figures 25A through 25G, the routing of the tethers 819 through the delivery catheter can be symmetrical to equalize the force applied to opposing sides of the catheter by applying tension to the tethers. This routing prevents unintended flexing of the catheter due to tension applied by the tethers.

The collar 815 includes a first tether passage 840A, a second tether passage 840B, a third tether passage 840C, and a fourth tether passage 840D, and a wire passage 842 extending through the collar 815. In the illustrated embodiment, the first, second, third, and fourth tether passages 840A, 840B, 840C, 840D are curved with the second tether passage 840B radially between the first tether passage 840A and the wire passage 842 and the third tether passage 840C radially between the wire passage 842 and the fourth tether passage 840D, however the first, second, third, and fourth tether passages 840A, 840B, 840C, 840D can have any shape or location. For example, the first, second, third, and fourth tether passages 840A, 840B, 840C, 840D can be circular and they can be equidistant from the wire passage 842.

The coupler 817 is generally cylindrical with a top portion 820 and a bottom portion 822 which define a cutout or window 824 therebetween. The coupler 817 also includes a first tether passage 844A, a second tether passage 844B, and a wire passage 846 extending through the top portion 820 with the first and second tether passages 844A, 844B located on either side of the wire passage 846. The coupler also includes a third tether passage 844C, a fourth tether passage 844D, a fifth tether passage 844E, a sixth tether passage 844F, and a wire passage 846 extending through the bottom portion 822. The wire passage 846 of the bottom portion 822 corresponds to the wire passage 846 of the top portion 820. The third and the fourth tether passages 844C, 844D are radially opposite the wire passage 846 from the fifth and the sixth tether passages 844E, 844F, with the fourth tether passage 844D radially between the third tether passage 844C and the wire passage 846 and the fifth tether passage 844E radially between the wire passage 846 and the sixth tether passage 844F.

In the illustrated embodiment, the third, fourth, fifth, and sixth tether passages 844C, 844D, 844E, 844F of the coupler 817 correspond to the first, second, third, and fourth tether passages 840A, 840B, 840C, 840D of the collar 815, respectively. While the coupler 817 has been described as having third, fourth, fifth, and sixth tether passages 844C, 844D, 844E, 844F in the lower portion 822, it will be appreciated that the coupler 817 can have a variety of configurations. For example, the third and fourth tether passages 844C, 844D and/or the fifth and sixth tether passages 844E, 844F can be combined to a single tether passage, respectively.

Optionally, the coupler 817 can have two actuation passages 860 in the top or upper portion 820 which can receive one or more clasp actuation lines which can be coupled to moveable arms of an implantable device and extend through a delivery device as previously described herein. The clasp actuation lines extend out of the windows 424.

As shown in Figure 25A, the collar 815 and the coupler 817 can be coupled together via a first coupling tether 819A and a second coupling tether 819B with the actuation element 812 extending through the wire passages 846 of the coupler 817 and the wire passage 842 of the collar 815. The actuation element 812 can extend into the implantable prosthetic device and engage a cap to open the device as previously described herein. The first coupling tether 819A passes through the first tether passage 844A of the coupler 817, behind the actuation element 812, through the sixth tether passage 844F of the coupler 817, through the fourth tether passage 840D of the collar 815, around the bottom of the collar 815, up through the third tether passage 840C of the collar 815, through the fifth tether passage 844E of the coupler 817, up and around the actuation element 812, back down through the fifth tether passage 844E of the coupler 817, through the third tether passage 840C of the collar 815, around the bottom of the collar 815, up through the fourth tether passage 840D of the collar 815, through the sixth tether passage 844F of the coupler 817, and through the second tether passage 844B of the coupler 817. The second coupling tether 819B passes through the second tether passage 844B of the coupler 817, behind the actuation element 812, through the third tether passage 844C of the coupler 817, through the first tether passage 840A of the collar 815, around the bottom of the collar 815, up through the second tether passage 840B of the collar 815, through the fourth tether passage 844D of the coupler 817, up and around the actuation element 812, through the fourth tether passage 844D of the coupler 817, through the second tether passage 840B of the collar 815, around the bottom of the collar 815, up through the first tether passage 840A of the collar 815, through the third tether passage 844C of the coupler 817, and through the first tether passage 844A of the coupler 817.

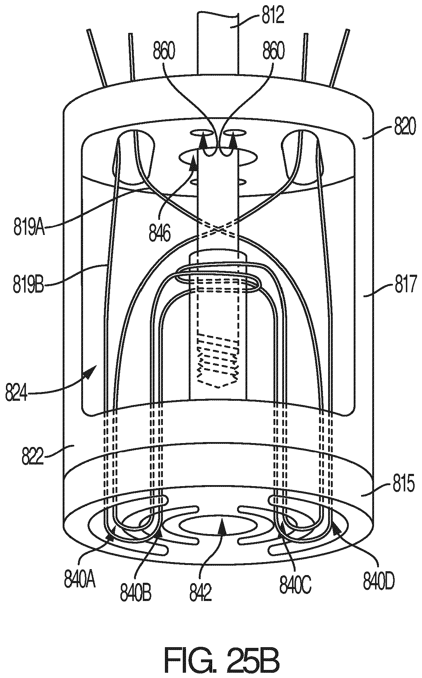

The actuation element 812, the collar 815, the coupler 817, the two coupling tethers 819 can be used to position and reposition an implantable prosthetic device in the native valve (e.g., the native mitral valve MV, native tricuspid valve, etc.) of the heart H, as previously described herein. Referring now to Figures 25B though 25E, the collar 815 can be decoupled from the coupler 817, the actuation element 812, and the coupling tethers 819. For example, the collar 815 can be decoupled to deploy an implantable prosthetic device in the closed position in the native valve, such as the native mitral valve MV, of the heart H.

As shown in Figure 25B, the collar 815 can be uncoupled from the actuation element 812. The actuation element 812 can be retracted through the wire passage 842 of the collar 815 and into the coupler 817. The collar 815 can remain tied to the coupler 817 via the coupling tethers 819A, 819B which remain looped around the actuation element 812.

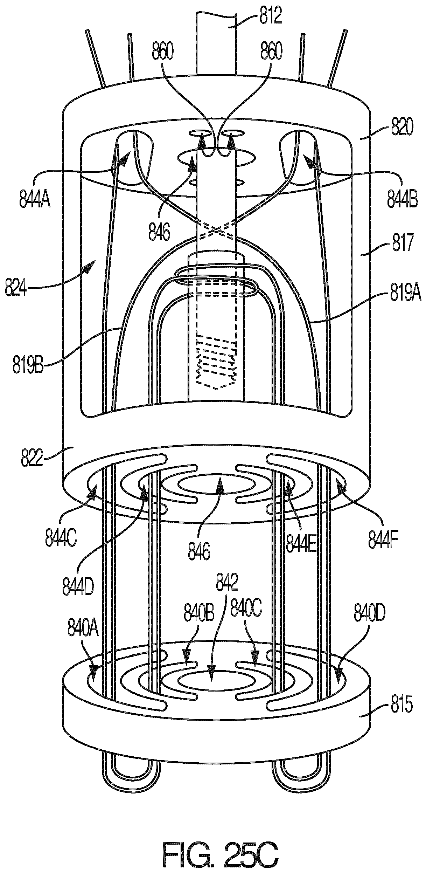

As shown in Figure 25C, slack can be introduced into the first and second coupling tethers 819A, 819B such that the collar 815 can be separated from the coupler 817 and the actuation element 812. In such a position, the collar 815 can move relatively freely from the coupler 817 and the actuation element 812. The collar 815 can be coupled with an implantable prosthetic device and, in such position, the user can check the placement and functionality of the device as previously described herein.

In such an embodiment, the coupling tethers 819A, 819B, the third, fourth, fifth, and sixth tether passages 844C, 844D, 844E, 844F of the coupler 817, and the first, second, third, and fourth tether passages 840A, 840B, 840C, 840D of the collar 815 operate as two double pulleys or force multipliers. For example, a pull on one end of the first and second coupling tethers 819A, 819B results in a change in position of the collar 815 equivalent to one-fourth the distance of the pull and a pull one both ends of each of the first and second coupling tethers 819A, 819B results in a change in position equivalent to one-half the distance of the pull.

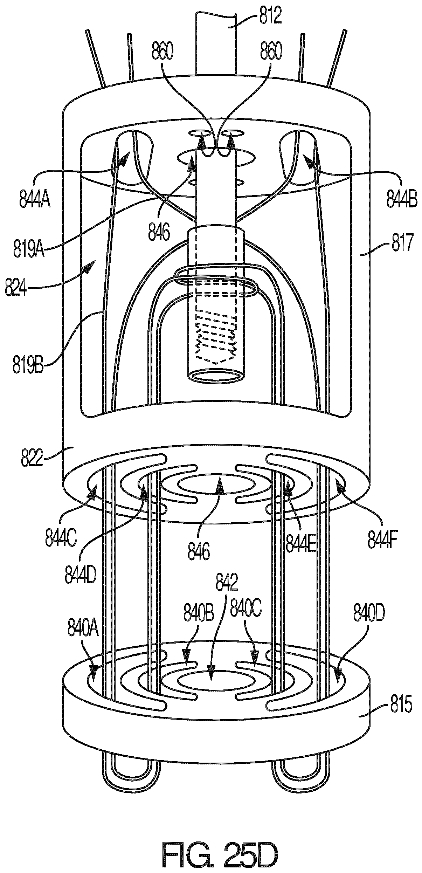

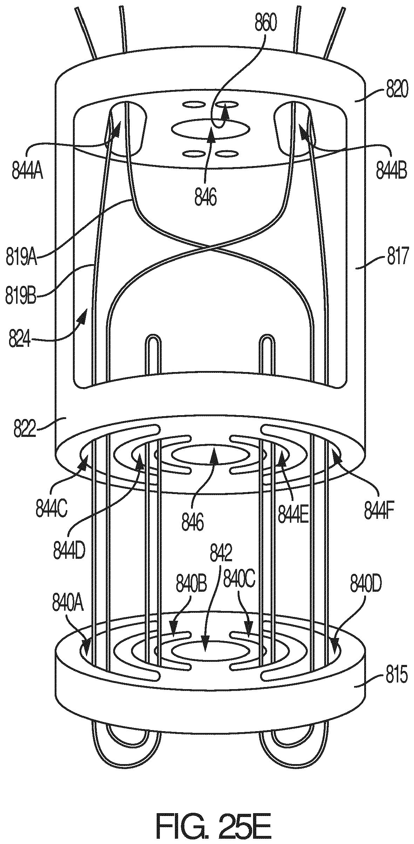

Referring now to Figures 25D and 25E, the collar 815 can be detached from the actuation element 812. As shown in Figure 25D, the actuation element 812 can be further retracted away from the collar 815. For example, the actuation element 812 can be retracted into a delivery catheter as previously described. As shown in Figure 25E, the actuation element 812 can be retracted from the coupler 817 such that the coupling tethers 819A, 819B are no longer looped around the actuation element 812.

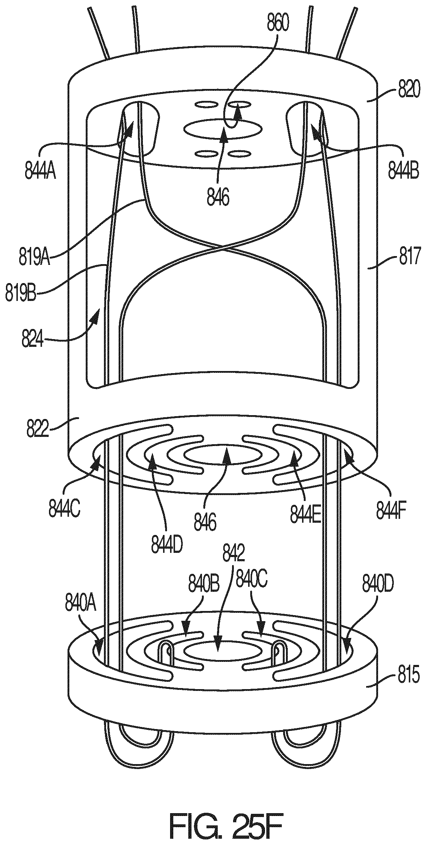

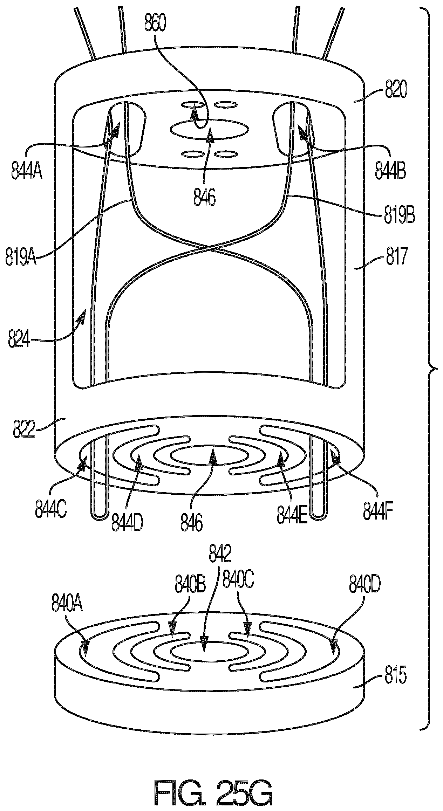

Referring now to Figures 25F and 25G, the collar 815 can be released from the coupler 817. As shown in Figure 25F, the first and second coupling tethers 819A, 819B can be pulled or otherwise retracted such that the first and second coupling tethers 819A, 819B are retracted through the fourth tether passage 844D of the coupler 817 and the second tether passage 840B of the collar 815 and through the fifth tether passage 844E of the coupler 817 and the third tether passage 840C of the collar 815, respectively. As shown in Figure 25G, the first and second coupling tethers 819A, 819B can be further pulled or retracted such that the first and second coupling tethers 819A, 819B are retracted through the first tether passage 840A of the collar 815 and through the fourth tether passage 840D of the collar 815, respectively. In such a position, the collar 815 is completely decoupled from the coupler 817, actuation element 812, and the coupling tethers 819A, 819B, and the implantable prosthetic device would be deployed, such as in the native valve or native mitral valve MV of the heart H.

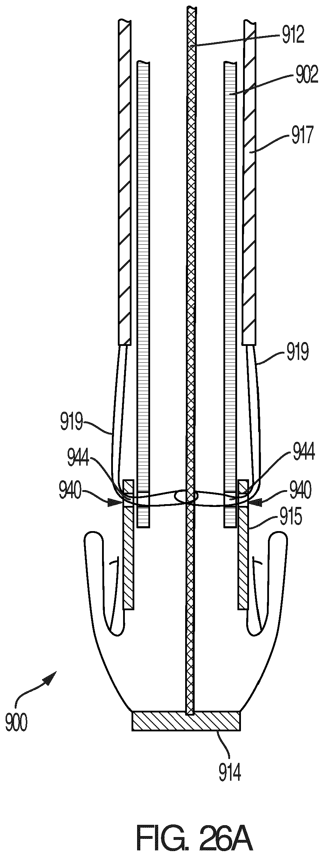

Referring now to Figures 26A through 26E, an implantable prosthetic device 900 is depicted according to one embodiment. The device 900 includes a cap 914 and a collar 915 and the device 900 can incorporate any of the features of any of the implantable prosthetic devices 100, 400, 400A described herein or any other implantable prosthetic device. For example, the device 900 can include clasps or barbed clasps that include a base or fixed arm, a moveable arm, barbs, and a joint portion which can be coupled to actuation lines which can open the clasps as previously described.

The device 900 can be deployed, recoupled, repositioned, and redeployed by a delivery catheter 902, an actuation element 912, an outer shaft 917, and one or more coupling tethers 918. The delivery catheter 902 can be sized such that the distal portion of the delivery catheter 902 fits or can be otherwise disposed within the collar 915 of the device 900. The outer shaft 917 can be disposed around the delivery catheter 902 and the coupling tethers 919 can be attached or otherwise secured to the distal portion of the outer shaft 917.

The collar 915 extends generally upward from the device 900 and includes two tether passages 940 oriented laterally in the collar 915. The delivery catheter 902 also includes two tether passages 944 laterally oriented in a distal portion of the delivery catheter 902. The tether passages 940 of the collar 915 and the tether passages 944 of the delivery catheter 902 can correspond to the circumferential position of the coupling tethers 918 attached to the outer shaft 917. In the illustrated embodiment, the coupling tethers 919, the tether passages 940 of the collar 915, and the tether passages 944 of the delivery catheter 902 are located opposite from each other, respectively.

As shown in Figure 26A, device 900 is depicted in the fully closed position. The device 900 can be deployed in the fully open position and moved to the closed position by any other method previously described herein. The actuation element 912 extends through the device 900 and engages the cap 914. In such a position, the tether passages 940 of the collar 915 and the tether passages 944 of the delivery catheter 902 are aligned and the coupling tethers 919 extend through the tether passages 940 of the collar 915 and the tether passages 944 of the delivery catheter 902 and are each looped around the actuation element 912. The coupling tethers 919 can be inserted through the tether passages 940 of the collar 915 and the tether passages 944 of the delivery catheter 902 and looped around the actuation element 912 before the procedure has begun.

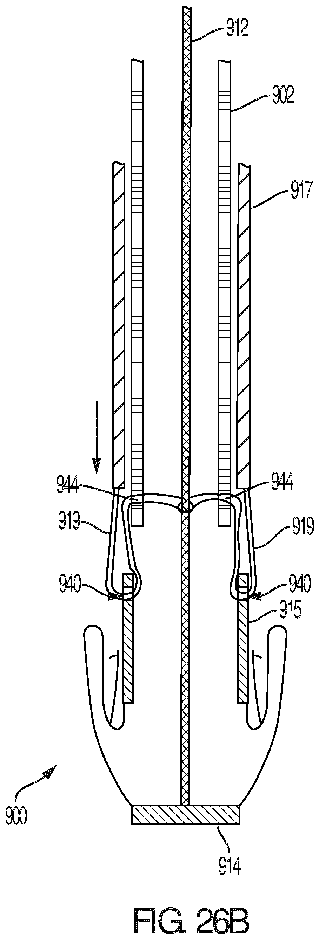

Referring to Figure 26B, the device 900 can be relatively decoupled from the delivery catheter 902. As shown in Figure 26B, the slack can be introduced into the coupling tethers 919 such that the delivery catheter 902 can be retracted from the device 900. Slack can be introduced into the coupling tethers 919 by pushing or otherwise advancing the outer shaft 917 forward, thus decreasing the distance between the distal end of the outer shaft 917 and the tether passages 940 of the collar 915. The actuation element 912 can be retracted to a point that is not beyond the looped portions of the coupling tethers 919, such that the actuation element is decoupled from the device, but the tethers remain coupled to the device. In such a position, the device 900 and the collar 915 are decoupled from the actuation element 912 and can move relatively freely from the delivery catheter 902. In such a position, a user can observe or check how the device 100 will work or otherwise operate when deployed, such as in the native valve (e.g., native mitral valve MV, etc.) of the heart H.

It will be appreciated that the device 900 can be uncoupled or otherwise allowed to move in a variety of ways. For example, the actuation element 912 can be flexible enough that the actuation element 912 can remain engaged to the cap of the device 900 when sufficient slack is introduced into the coupling tethers 919 such that a user can observe or check how the device 900 will work or otherwise operate when deployed, such as in the native valve (e.g., native mitral valve MV, etc.) of the heart H. The method of using a flexible actuation element 912, which is not decoupled when the device is sufficiently decoupled from the delivery catheter and allows a user to observe or check how the device will work or otherwise operate when deployed, can also be used in any of the other embodiments presented herein.

If the user observes that the device 900 is not properly placed, the user can recouple the device 900, the delivery catheter 902, and the actuation element 912. For example, the actuation element 912 can be advanced back through the loops of the coupling tethers 919 and into the device 900 such that the actuation element 912 engages the cap 914 (This step can be omitted if the actuation element is not decoupled). The outer shaft 917 can be retracted such that the device 900 is recoupled to the delivery catheter 902 as shown in Figure 26A. The device 900 can then be reopened, repositioned, and redeployed by any other method disclosed herein.

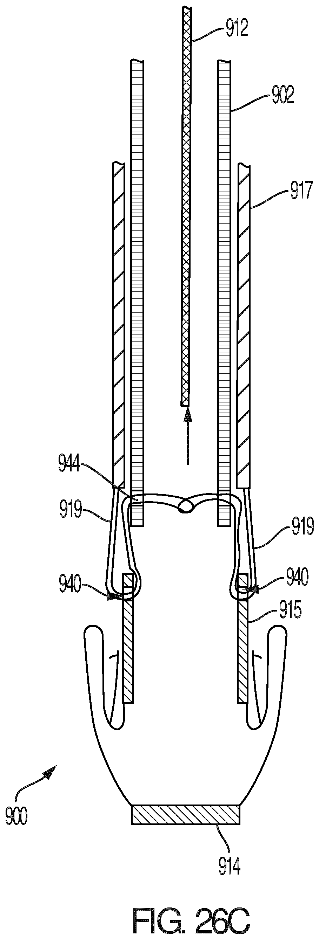

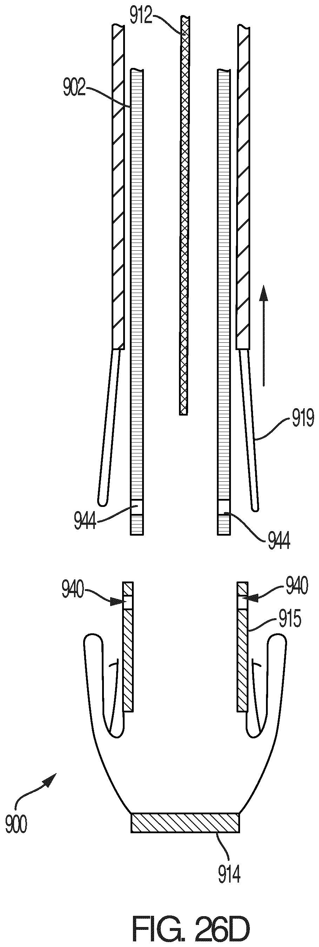

Referring now to Figures 26C through 26E, the device 900 can be completely deployed from the delivery catheter 902, the actuation element 912, and the outer shaft 917. For example, the device 900 can be deployed after the user has observed that the device 900 is properly in place, such as in the native valve (e.g., native mitral valve, etc.) of the heart H. The control wire 1012 is retracted out of the tether loops as illustrated by Figure 26C. The outer shaft 917 is retracted, thus retracting the coupling tethers 919 from the tether passages 944 of the delivery catheter 902 and the tether passages 940 of the collar 940 as illustrated by Figure 26D. The device 900 is then completely decoupled from the delivery catheter 902, the outer shaft 917, the actuation element 912, and the coupling tethers 919.

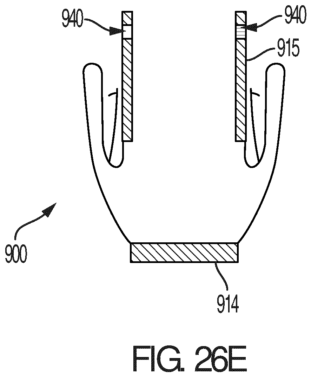

As shown in Figure 26E, the device 900 is shown in the fully closed and deployed condition. The delivery catheter 902, the outer shaft 917, and the actuation element 912 are retracted and the clasps of the device 900 remain in a fully closed position. Once deployed, the device 900 can be maintained in the fully closed position in a variety of ways. For example, the device 900 can be maintained in the fully closed position in any of the ways described relating to Figure 14M.

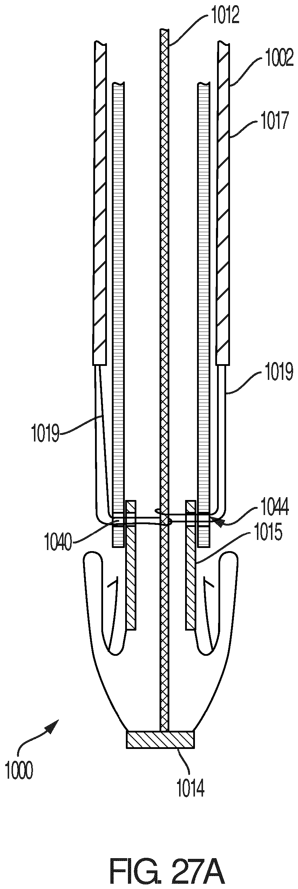

Referring now to Figures 27A through 27E, an implantable prosthetic device 1000 is depicted according to one embodiment. The device 1000 includes a cap 1014 and a collar 1015 and the device 1000 can incorporate any of the features of any of the implantable prosthetic devices 100, 400, 400A described herein. For example, the device 1000 can include clasps that include a base or fixed arm, a moveable arm, barbs, and a joint portion which can be coupled to actuation lines which can open the clasps as previously described.

The device 1000 can be deployed, recoupled, repositioned, and redeployed by a delivery catheter 1002, an actuation element 1012, an outer shaft 1017, and one or more coupling tethers 1019. The device 1000 is substantially similar to the device 1000 described in Figures 26A through 26E, however, the device 1000 is designed and shaped such that the collar 1015 or a portion of the collar of the device 1000 fits or can be otherwise disposed within the delivery catheter 1002. The outer shaft 1017 can be disposed around the delivery catheter 1002 and the coupling tethers 1019 can be attached or otherwise secured to the distal portion of the outer shaft 1017.

The collar 1015 extends generally upward from the device 1000 and includes two tether passages 1040 oriented laterally in the collar 1015. The delivery catheter 1002 also includes two tether passages 1044 laterally oriented in a distal portion of the delivery catheter 1002. The tether passages 1040 of the collar 1015 and the tether passages 1044 of the delivery catheter 1002 can correspond to the circumferential position of the coupling tethers 1018 attached to the outer shaft 1017. In the illustrated embodiment, the coupling tethers 1019, the tether passages 1040 of the collar 1015, and the tether passages 1044 of the delivery catheter 1002 are located opposite from each other, respectively.

As shown in Figure 27A, device 1000 is depicted in the fully closed position. The device 1000 can be deployed in the fully open position and moved to the closed position by any other method previously described herein. The actuation element 1002 extends through the device 1000 and engages the cap 1014. In such a position, the tether passages 1040 of the collar 1015 and the tether passages 1044 of the delivery catheter 1002 are aligned and the coupling tethers 1019 extend through the tether passages 1044 of the delivery catheter 1002 and the tether passages 1040 of the collar 1015 and are each looped around the actuation element 1012. The coupling tethers 1019 can be inserted through the tether passages 1044 of the delivery catheter 1002 and the tether passages 1040 of the collar 1015 and looped around the actuation element 1012 before the procedure has begun.

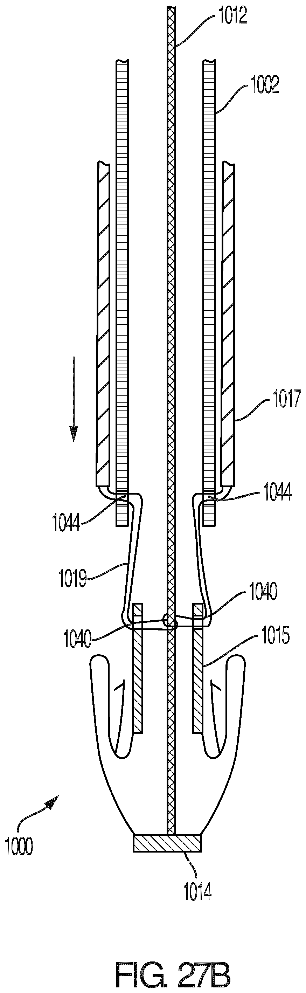

Referring to Figure 27B, the device 1000 can be relatively decoupled from the delivery catheter 1002. As shown in Figure 27B, the slack can be introduced into the coupling tethers 1019 such that the delivery catheter 1002 can be retracted from the device 1000. Slack can be introduced into the coupling tethers 1000 by pushing or otherwise advancing the outer shaft 1017 forward, thus decreasing the distance between the distal end of the outer shaft 1017 and the tether passages 1044 of the delivery catheter 1002. The actuation element 1012 can be retracted to a point that is disengaged from the cap but is not beyond the looped portions of the coupling tethers 1019. In such a position, the device 1000 and the collar 1015 are decoupled from the actuation element 1012 and can move relatively freely from the delivery catheter 1002. In such a position, a user can observe or check how the device 100 will work or otherwise operate when deployed, such as in the native valve (e.g., native mitral valve MV, etc.) of the heart H. However, it will be appreciated that the device 1000 can be uncoupled or otherwise allowed to move in a variety of ways. For example, the actuation element 1012 can be flexible enough that the actuation element 1012 can remain engaged to the cap of the device 1000 when sufficient slack is introduced into the coupling tethers 1019 such that a user can observe or check how the device 1000 will work or otherwise operate when deployed, such as in the native valve (e.g., native mitral valve MV, etc.) of the heart H. The method of using a flexible actuation element 1012, which is not decoupled when the device is sufficiently decoupled from the delivery catheter and allows a user to observe or check how the device will operate when deployed, can also be used in any of the other embodiments presented herein.

If the user observes that the device 1000 is not properly placed, the user can recouple the device 1000, the delivery catheter 1002, and the actuation element 1002. For example, the actuation element 1012 can be advanced through the loops of the coupling tethers 1019 and into the device 1000 such that the actuation element 1002 engages the cap 1014 and the outer shaft 1017 can be retracted such that the device 1000 is recoupled to the delivery catheter 1002 as shown in Figure 26A. The device 1000 can then be reopened, repositioned, and redeployed by any other method disclosed herein.

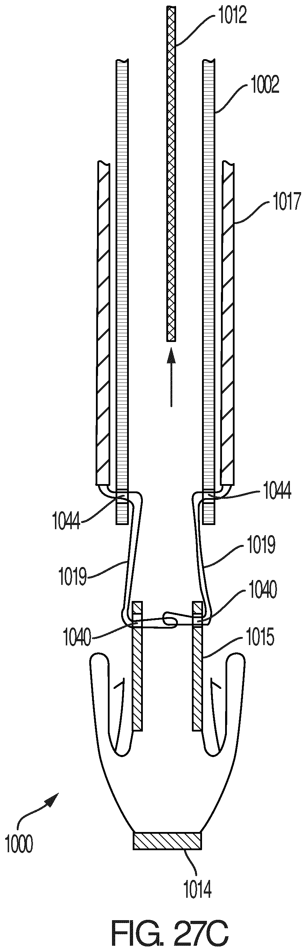

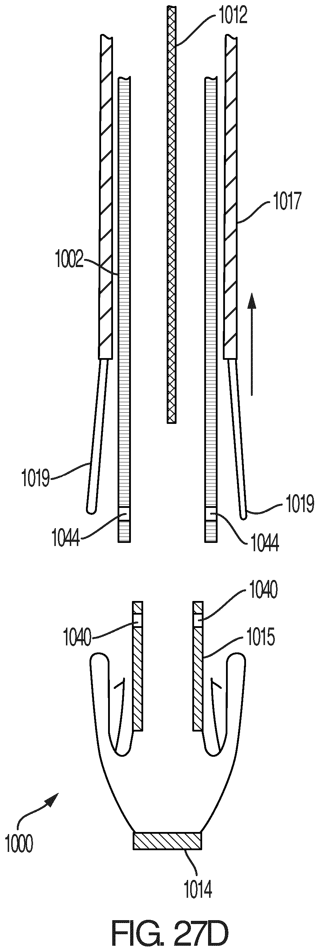

Referring now to Figures 27C through 27E, the device 1000 can be completely deployed from the delivery catheter 1002, the actuation element 1012, and the outer shaft 1017. For example, the device 1000 can be deployed after the user has observed that the device 1000 is properly in place, such as in the native valve (e.g., native mitral valve, etc.) of the heart H. The control wire 1012 is retracted out of the tether loops as illustrated by Figure 27C. The outer shaft 1017 is then retracted, thus retracting the coupling tethers 1019 from the tether passages 1040 of the collar 1040 and the tether passages 1044 of the delivery catheter 1002 as illustrated by Figure 27D. The device 1000 is then completely decoupled from the delivery catheter 1002, the outer shaft 1017, the actuation element 1012, and the coupling tethers 1019.

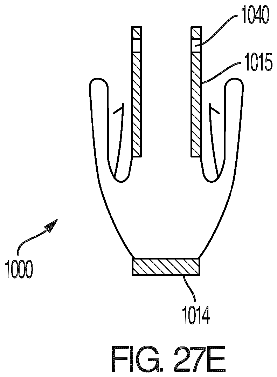

As shown in Figure 27E, the device 1000 is shown in the fully closed and deployed condition. The delivery catheter 1002, the outer shaft 1017, and the actuation element 1012 are retracted and the clasps of the device 1000 remain in a fully closed position. Once deployed, the device 1000 can be maintained in the fully closed position in a variety of ways. For example, the device 1000 can be maintained in the fully closed position in any of the ways described relating to Figure 14M or the device can be configured such that the paddles open and close with the beating of the heart, while the clasps remain closed on the native valve leaflets.

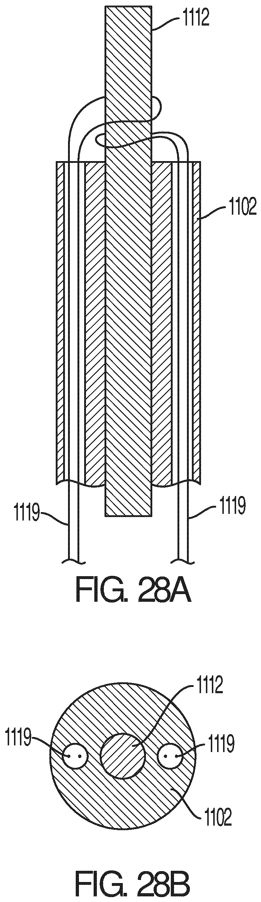

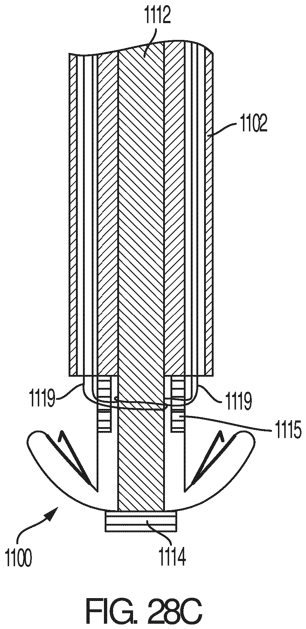

Referring now to Figures 28A through 28F, an implantable prosthetic device 1100 (see e.g., Figure 28C) and delivery system is depicted according to one embodiment. The device 1100 includes a cap 1114 and a collar 1115 and the device 1100 can incorporate any of the features of any of the implantable prosthetic devices 100, 400, 400A described herein. For example, the device 1100 can include clasps that include a base or fixed arm, a moveable arm, barbs, and a joint portion which can be coupled to actuation lines which can open the clasps as previously described.

The device 1100 can be deployed, recoupled, repositioned, and redeployed by a delivery catheter 1102, an actuation element 1112, and one or more coupling tethers 1119. The delivery catheter 1102 has a number of lumens 1104 corresponding to the number of coupling tethers 1119. The lumens 1104 extend longitudinally through the delivery catheter 1102 and a looped coupling tether 1119 can extend through each lumen 1104.

As shown in Figures 28A and 28B, the actuation element 1112 extends longitudinally through the delivery catheter 1102. The coupling tethers 1119 are each looped around a proximal portion of the actuation element 1112 extending proximally beyond the delivery catheter 1102 and each coupling tether 1119 extends longitudinally through one of the lumens 1104 of the delivery catheter 1102.

Referring now to Figures 28C through 28G, the device 1100 can be coupled to and deployed from the delivery catheter 1102 and actuation element 1112. The collar 1115 extends generally upward from the device 1100 and includes two tether passages 1140 oriented laterally through the collar 1015. The tether passages 1140 of the collar 1115 can correspond to the circumferential position of the lumens 1104 and/or the coupling tethers 1018 extending through the lumens 1104 of the delivery catheter 1102. In the illustrated embodiment, the coupling tethers 1119, the tether passages 1140 of the collar 1115, and the lumens 1104 of the delivery catheter 1102 are located opposite from each other, respectively.

As shown in Figure 28C, device 1000 is coupled to the delivery catheter 1102, the actuation element 1112, and the coupling tethers 1119. The device 1100 can be deployed in the fully open position and moved to the partially open, grasp-ready condition and the closed position by any method previously described herein. The actuation element 1112 extends through the device 1100 and engages the cap 1114. In such a position, the coupling tethers 1119 extend through the tether passages 1140 of the collar 1115 and are each looped around the actuation element 1112. The coupling tethers 1119 can be inserted through the tether passages 1140 of the collar 1115 and looped around the actuation element 1112 before the procedure has begun.

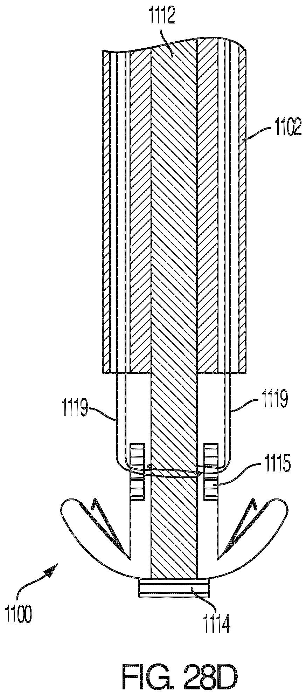

As shown in Figure 28D, slack can be introduced into the coupling tethers 1119 such that the delivery catheter 1102 can be retracted from the device 1100 and/or the device 1100 can be advanced away from the delivery catheter 1102. Slack can be introduced into the coupling tethers 1119 by advancing the actuation element 1112 through the delivery catheter 1102 or by any other means. In such a position, remains tethered to the delivery catheter 1102 via the coupling tethers and directly attached to the actuation element 1112. In some example embodiments, the actuation element 1112 is flexible and a user can observe or check how the device 1100 will work or otherwise operate when deployed, such as in the native valve (e.g., native mitral valve MV, etc.) of the heart H. The tethers 1119 can be used to bring the catheter 1102 back to the device and the actuation element 1112 can reopen the device 1100 as previously described herein. For example, a user can recouple the device 1100 to the catheter 1102 and reopen the device 1100 if the user observes that the device 1100 is not properly positioned, such as in the native valve (e.g., native mitral valve MV, etc.) of the heart H.

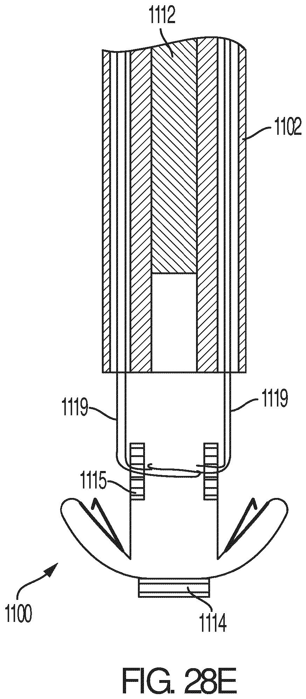

As shown in Figure 28E, the device 1100 can be decoupled from the actuation element 1112 and the tethers. For example, the actuation element 1112 can be retracted from the device 1110 and into the delivery catheter 1102 to disconnect the tethers from the device.

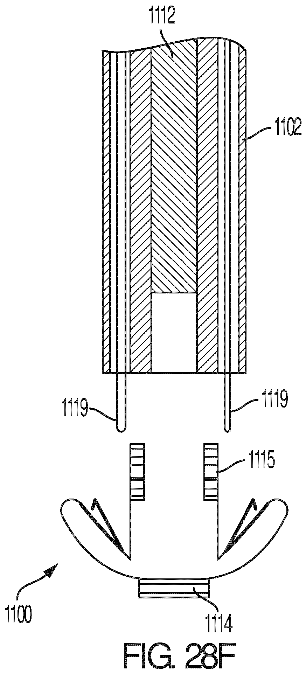

As shown in Figure 28F, the device 1100 can be deployed from the delivery catheter 1102 and the coupling tethers 1119. The coupling tethers 1119 can be retracted through the tether passages 1140 of the collar 1115 to detach the device 1100 from the delivery catheter 1102. The coupling tethers 1119 can be retracted by pulling the ends of the coupling tethers 1119, by retracting the actuation element 1112 thereby retracting the coupling tethers 1119, or by any other means. In such a position, the device 1100 is completely deployed, such as in the native valve (e.g., native mitral valve MV, etc.) of the heart H. The delivery catheter 1102, the actuation element 1112, and the coupling tethers 1119 can be further retracted and the device 1100 can be left in place.

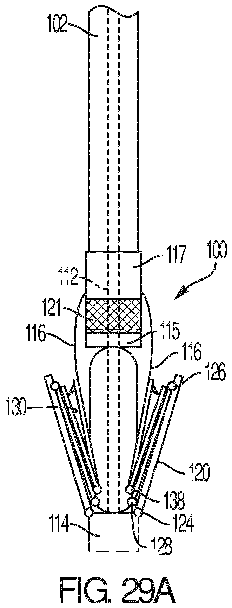

Referring now to Figures 29A through 29O, a system for implanting an implantable prosthetic device 100 is depicted according to one embodiment. The device 100 can be any of the devices described herein, any of the devices that are described in a patent or patent application that is referenced herein, or any other known device for repairing a native heart valve, such as a mitral valve or a tricuspid valve.

The device 100 illustrated by Figures 29A through 29O is deployed from a delivery catheter 102 and includes a coaption portion 104 and an anchor portion 106. The coaption portion 104 of the device 100 includes a coaption element 110 that is adapted to be implanted between the leaflets of the native valve and is slidably attached to an actuation element or shaft 112. The anchor portion 106 is actuatable between open and closed conditions and can take a wide variety of forms, such as, for example, paddles, gripping elements, or the like. Actuation of the actuation element 112 opens and closes the anchor portion 106 of the device 100 to grasp the native valve leaflets during implantation. The actuation element or shaft 112 can take a wide variety of different forms. For example, the actuation element or shaft can be threaded such that rotation of the actuation element or shaft moves the anchor portion 106 relative to the coaption portion 104. Or, the actuation element or shaft can be unthreaded, such that pushing or pulling the actuation element or shaft 112 moves the anchor portion 106 relative to the coaption portion 104.

The anchor portion 106 of the device 100 includes outer paddles 120 and inner paddles 122 that are connected between a cap 114 and the coaption element 110 by portions 124, 126, 128. The portions 124, 126, 128 can be jointed and/or flexible to move between all of the positions described below. The interconnection of the outer paddles 120, the inner paddles 122, the coaption element 110, and the cap 114 by the portions 124, 126, and 128 can constrain the device to the positions and movements illustrated herein.

The actuation element 112 extends through the delivery catheter 102 and the coaption element 110 to the cap 114 at the distal connection of the anchor portion 106. Extending and retracting the actuation element 112 increases and decreases the spacing between the coaption element 110 and the cap 114, respectively. A collar 115 removably attaches the coaption element 110 to the delivery catheter 102, either directly or indirectly, so that the actuation element 112 slides through the collar 115 and coaption element 110 during actuation to open and close the paddles 120, 122 of the anchor portion 106.

The device 100 can also include a coupler 117 which removably attaches the collar 115 to the delivery catheter 102. The coupler 117 can be removably attached to the collar 115 and can be fixedly or removably attached to the delivery catheter 102. The coupler 117 can attach the delivery catheter 102 to the collar 115 in a wide variety of ways. For example, the coupler 117 can attach the collar 115 to the delivery catheter 102 in any of the ways described in PCT patent application publication WO2020/076898 or in any of the ways previously described herein. In such an embodiment, the actuation element 112 slides through the coupler 117, the collar 115, and the coaption element 110 during actuation to open and close the paddles 120, 122 of the anchor portion 106. As detailed below, the coupler 117 can facilitate the placement, repositioning, and/or replacement of the device 100.

The device 100 also includes a compressible sleeve 121 disposed between the collar 115 and the coupler 117. The sleeve 121 can be a wire mesh, a compressible rubber or plastic, a spiral spring material, or any other material which is compressible and expandable, particularly in a longitudinal direction. The sleeve 121 is about the same width as the coupler 117 and the collar 115. The sleeve 121 encases one or more coupling tethers 119 which couple the collar 115 to the coupler 117, delivery catheter 102, and/or actuation element 112. The sleeve 121 can encase the one or more coupling tethers 119 such that the one or more coupling tethers 119 do not detach, get tangled, and or become caught when the device 100 is decoupled, recoupled, repositioned, or redeployed, as detailed below. The sleeve 121 can extend from the coupler 117 and/or the delivery catheter 102 and can extend to or substantially to the collar 115. As detailed below, in the expanded position, the sleeve 121 can prevent the clasp actuation lines 116 from getting pinched between the coupler 117 and the collar 115 and/or from getting tangled with the one or more coupling tethers 119 and/or the actuation element 112. For example, when the tether 119 and/or clasp control lines 116 are slackened to check the efficacy of the device, there is a substantial gap between the coupler 117 and the collar 115 (see e.g., Figure 14C). The slack control lines 116 can potentially get tangled with the slack tether 119. Also, when tension is applied to the tether 119 and the coupler 117 is moved back to the collar 115, slack control lines 116 could get pinched between the coupler 117 and the collar 115. This tangling or pinching of the clasp control lines 116 can temporarily prevent the clasp control lines from opening the clasps. The sleeve 121 prevents both the pinching and the tangling. The sleeve 121 can also be used in any of the other embodiments described herein.

The device 100 can be inserted and deployed by any method or procedure previously described herein. For example, the device 100 can be inserted and deployed as described and illustrated in Figures 8-14A and 15-20A.

As shown in Figure 29A, the device 100 is shown in a fully closed and deployed condition. The paddles 120, 122 and clasps 130 remain in a fully closed position. Once in the deployed condition, the device 100 can be maintained in the fully closed position with a mechanical latch or can be biased to remain closed through the use of spring materials, such as steel, other metals, plastics, composites, etc. or shape-memory alloys such as Nitinol. For example, the jointed or flexible portions 124, 126, 128, 138, and/or the inner and outer paddles 122, and/or an additional biasing component can be formed of metals such as steel or shape-memory alloy, such as Nitinol-produced in a wire, sheet, tubing, or laser sintered powder-and are biased to hold the outer paddles 120 closed around the coaption element 110 and the clasps 130 pinched around native leaflets. Similarly, the fixed and moveable arms 132, 134 of the clasps 130 are biased to pinch the leaflets. In some embodiments, the joint portions 124, 126, 128, 138, and/or the inner and outer paddles 122, and/or an additional biasing component can be formed of any other suitably elastic material, such as a metal or polymer material, to maintain the device in the closed condition after implantation.

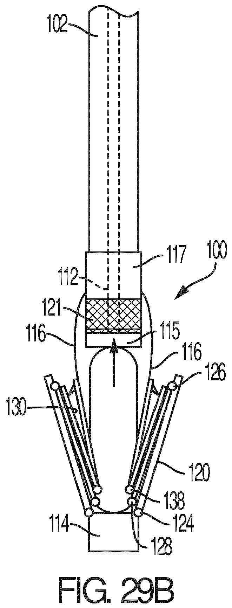

Referring now to Figure 29B, the device 100 can be uncoupled from the actuation element 112. The actuation element 112 can be retracted from the device 100 and the collar 115 and into the sleeve 121, the coupler 117, and/or the delivery catheter 102. The actuation element 112 can be retracted such that the end of the actuation element 112 is positioned within the sleeve 121, the coupler 117, and/or the delivery catheter 102. In such a position, the device 100 and the collar 115 can move or pivot relatively freely from the sleeve 121, the coupler 117, the delivery catheter 102, and the actuation element 112.

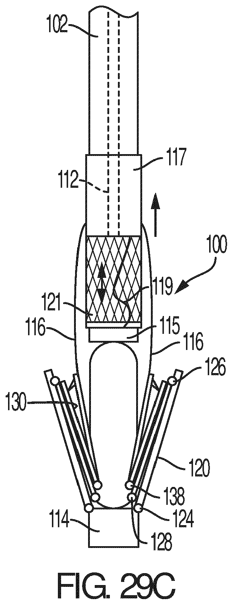

As shown in Figure 29C, slack can be introduced to the clasp actuation lines 116 such that the delivery catheter 102, the actuation element 112, and the coupler 117 can be retracted from the device 100 and the collar 115. The collar 115 can still be tied to the delivery catheter 102, the actuation element 112, and/or the coupler 117 via one or more coupling tethers 119. The one or more coupling tethers 119 can be looped around or otherwise be secured or attached to the collar. The retraction of the delivery catheter 102 and the coupler 117 causes the compressible sleeve 121 to expand longitudinally between the collar 115 and the retracted position of the coupler 117. The sleeve 121 encases the one or more coupling tethers 119 and may expand to a maximum distance where the sleeve 121 blocks or substantially blocks the gap between the collar 115 and the coupler 117. In such a position, the device 100 remains tethered or attached to the delivery catheter 102, the actuation element 112, and/or the coupler 117 but, with sufficient slack in the clasp actuation lines 116, the device 100 is generally free to move relative to the delivery catheter 102, the actuation element 112, and the coupler 117. In such a position, a user may observe or check how the device 100 will look or operate when the device 100 is actually deployed, such as on the native valve leaflets.

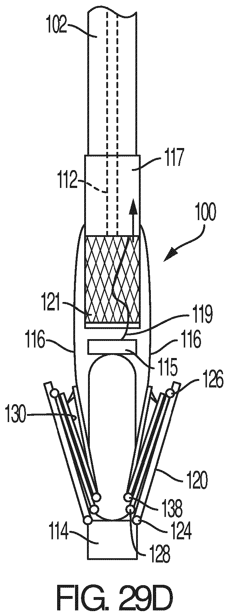

As shown in Figure 29D, the delivery catheter 102, the actuation element 112, the coupler 117, and the sleeve 121 can be retracted farther away from the collar 115. The sleeve 121 can optionally be separated from the collar 115. However, the sleeve 121 still blocks a portion, and optionally a majority, of the gap between the coupler 117 and the cap 114. In such a position, the device 100 remains tethered or attached to the delivery catheter 102, the actuation element 112, and/or the coupler 117 but, with sufficient slack in the clasp actuation lines 116, the device 100 is generally free to move relative to the delivery catheter 102, the actuation element 112, and the coupler 117. Further, the expanded compressible sleeve 121 allows the one or more coupling tethers 119 to keep the collar 115 coupled to the coupler 117 and the sleeve 121 can prevent the one or more coupling tethers 119 from getting tangled with the clasp actuation lines 116 and can prevent the clasp actuation lines 116 from getting pinched between the collar 115 and the coupler 117. In such a position, a user may observe or check how the device 100 will look or operate when the device 100 is actually deployed, such as on the native valve leaflets.

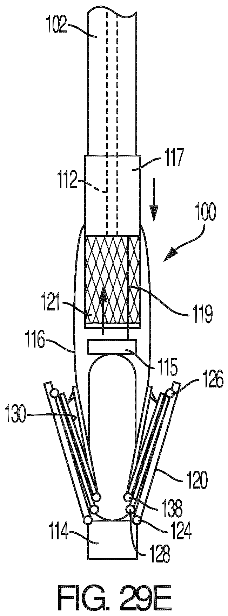

Referring now to Figures 29E through 29H, the device 100 and collar 115 can be recoupled to the delivery catheter 102, the actuation element 112, and the coupler 117. For example, the device 100 and the collar 115 can be recoupled to the delivery catheter 102, the actuation element 112, and the coupler 117 if the user observes that the device 100 is not properly deployed or properly in place.

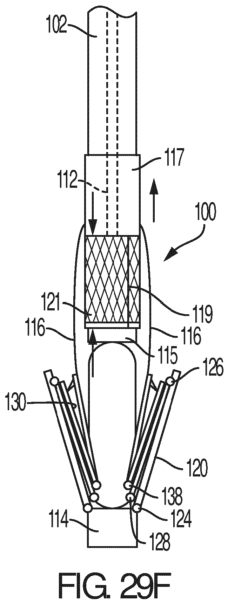

As shown in Figures 29E through 29G, the device 100 and the collar 115 can be recoupled to the delivery catheter 102, the actuation element 112, the coupler 117, and the sleeve 121. Tension can be applied to the one or more coupling tethers 119 and the coupler 117 can be advanced along the one or more coupling tethers such that the device 100 and the collar 115 can be recoupled to the delivery catheter 102, the actuation element 112, the coupler 117, and the sleeve 121. Tension can also be applied to the clasp actuation lines 116 to bring the clasp actuation lines 116 closer to the device 100 and within the coupler 117 and/or the delivery catheter 102 such that the clasp actuation lines 116 do not move around or get tangled. As shown in Figures 29E and 29F, the delivery catheter 102, the coupler 117, and the sleeve 121 can be advanced such that the sleeve 121 can be brought back into contact with the collar 115.

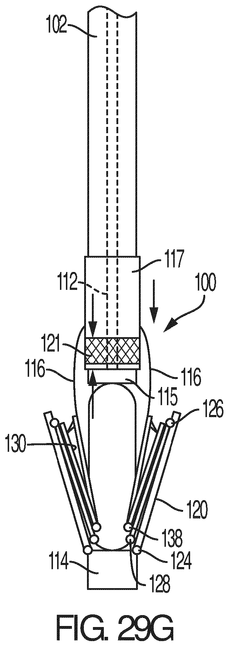

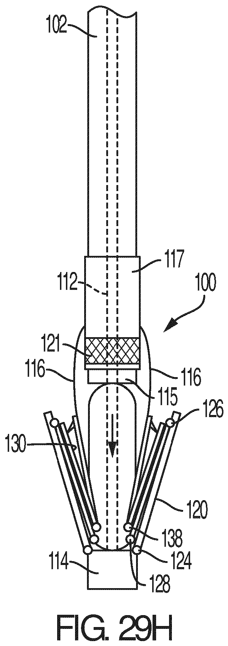

As shown in Figures 29G and 29H, the device 100 can be recoupled to the actuation element 112. As shown in Figure 29G, the delivery catheter 102 and the coupler 117 can be advanced farther such that the sleeve 121 is compressed between the collar 115 and the coupler 117. As shown in Figure 29H, the actuation element 112 can be advanced through the coupler 117 and the collar 115 and into the device 100. The actuation element 112 can be advanced through the collar 115 and the coupler 117 and into the device 100 until the actuation element 112 reengages the anchor portion 106 and/or the cap 114 of the device 100.

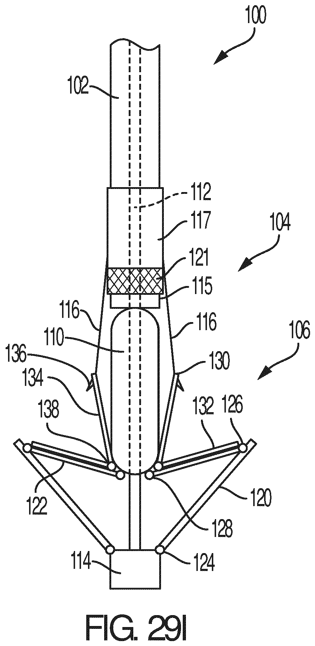

Referring now to Figure 29I, the device 100 can be reopened or moved back to the partially open condition to release the valve leaflets, reposition the device, and recapture the leaflets. To transition from the fully closed condition to the partially open condition, the actuation element 112 is extended to push the cap 114 away from the coaption element 110, thereby pulling on the outer paddles 120, which in turn pulls on the inner paddles 122, causing the anchor portion 106 to partially unfold. The actuation lines 116 are also retracted to open the clasps 130 so that the leaflets can be grasped. While in the partially open condition, the device 100 can be moved or repositioned. For example, a user can reposition the device 100 to properly grasp the native valve leaflets.

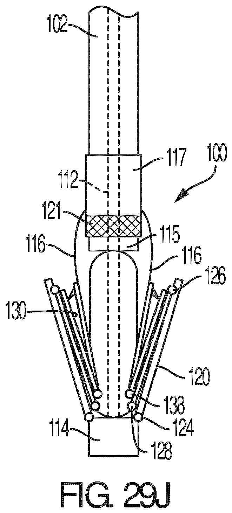

As shown in Figure 29J, the device 100 can be moved to the fully closed or deployed condition. For example, the device 100 can be moved back to the deployed condition once the device 100 has been positioned or repositioned to the desired position. The actuation lines 116 can be extended to allow the clasps 130 to close. Either or both of the actuation lines 116 can be repeatedly actuated to repeatedly open and close the clasps 130. For example, the clasps 130 can be repeatedly opened and closed to ensure that the device 100 is properly placed.

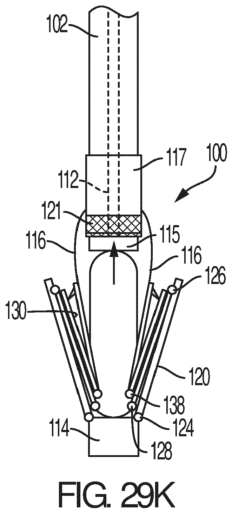

Referring now to Figures 29K through 29O, the device 100 can be deployed from the delivery catheter 102, the coupler 117, the actuation element 112, and the sleeve 121. For example, the device 100 can be deployed once the device 100 is in the proper place and grasping the native valve leaflets.

As shown in Figure 29K, the device 100 can be uncoupled from the actuation element 112. The actuation element 112 can be retracted from the device 100 and the collar 115 and into the coupler 117, the sleeve 121, or the delivery catheter 102. The actuation element 112 can be retracted such that the end of the actuation element 112 is positioned within the coupler 117, the sleeve 121, or the delivery catheter 102. In such a position, the device 100 and the collar 115 can move or pivot relatively freely from the coupler 117, the delivery catheter 102, and the actuation element 112.

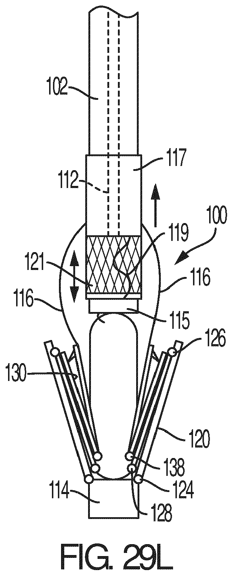

As shown in Figure 29L, slack can be introduced to the clasp actuation lines 116 such that the delivery catheter 102, the actuation element 112, and the coupler 117 can be retracted from the device 100 and the collar 115. The collar 115 can still be tied to the delivery catheter 102, the actuation element 112, and/or the coupler 117 via one or more coupling tethers 119. The one or more coupling tethers 119 can be looped around or otherwise secured or attached to the collar 115 and can attach the collar 115 to the coaption portion 104 of the device 100. The retraction of the delivery catheter 102 and the coupler 117 causes the compressible sleeve 121 to expand longitudinally between the collar 115 and the retracted position of the coupler 117. The sleeve 121 encases the one or more coupling tethers 119 and can expand to a maximum distance. In such a position, the device 100 remains tethered to the delivery catheter 102, the actuation element 112, and/or the coupler 117 but, with sufficient slack in the clasp actuation lines 116 and the tether 119, the device 100 is generally free to move relative to the delivery catheter 102, the actuation element 112, and the coupler 117. In such a position, a user can observe or check how the device 100 will look or operate when the device 100 is actually deployed, such as on the native valve leaflets.

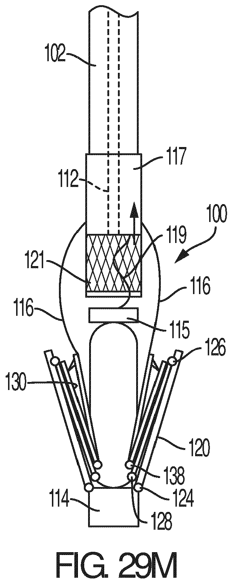

As shown in Figure 29M, the delivery catheter 102, the actuation element 112, the coupler 117, and the sleeve 121 can be retracted farther away from the collar 115. The sleeve 121 can be separated from the collar 115. In such a position, the device 100 remains tethered or attached to the delivery catheter 102, the actuation element 112, and/or the coupler 117 but, with sufficient slack in the clasp actuation lines 116, the device 100 is generally free to move relative to the delivery catheter 102, the actuation element 112, and the coupler 117. Further, the expanded compressible sleeve 121 allows the one or more coupling tethers 119 to keep the collar 115 coupled to the coupler 117 and the sleeve 121 can prevent the one or more coupling tethers 119 from getting tangled with the clasp actuation lines 116. In such a position, a user may observe or check how the device 100 will look or operate when the device is actually deployed, such as on the native valve leaflets.

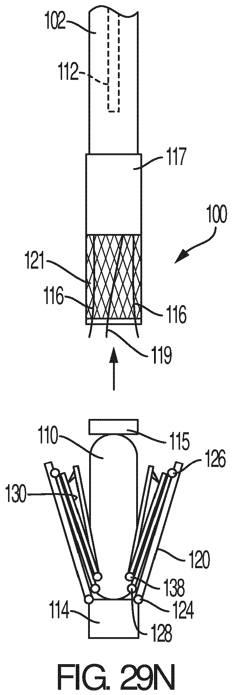

As shown in Figure 29N, the device 100 and collar 115 can be decoupled from the one or more coupling tethers 119 and the delivery catheter 102, the actuation element 112, the coupler 117, the sleeve 121 and the one or more coupling tethers 119 can be retracted away from the device 100 and the collar 115. The one or more coupling tethers 119 can be decoupled in a variety of ways. For example, the one or more coupling tethers can be decoupled by any way previously described herein. The clasp actuation lines 116 can be detached from the moveable arms 134 and pulled or otherwise retracted toward or into the coupler 117 and/or delivery catheter 102. In such an embodiment, the device 100 and collar 115 are completely detached from the delivery catheter 102, the coupler 117, and the actuation element 112.

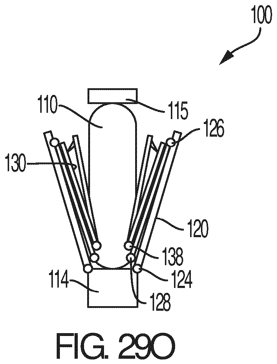

As shown in Figure 29O, the device 100 is shown in the fully closed and deployed condition. The delivery catheter 102, the coupler 117, the actuation element 112, and the sleeve 121 are retracted and the clasps 130 remain in a fully closed position. Once deployed, the device 100 can be maintained in the fully closed position in a variety of ways. For example, the device 100 can be maintained in the fully closed position in any of the ways described relating to Figure 14M.

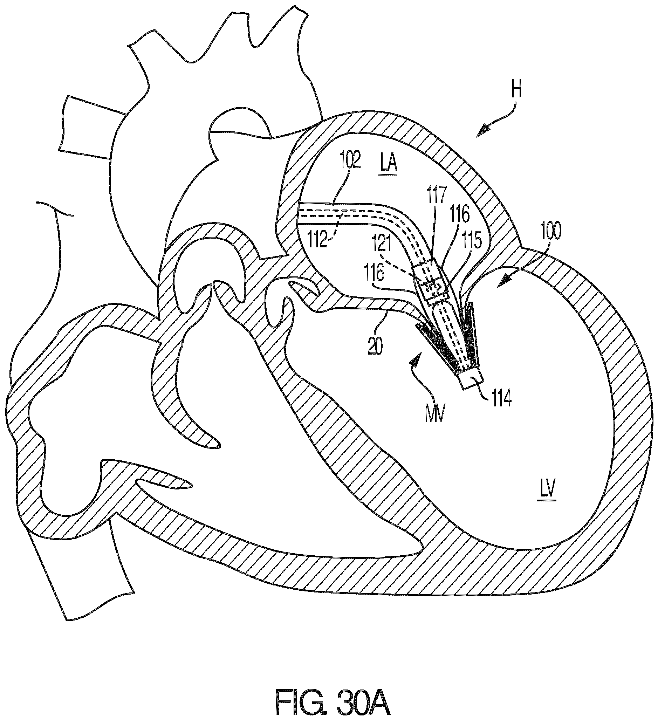

Referring now to Figures 30A through 30L, the implantable device 100 of Figures 28A through 28O is shown implanted and then being reopened, repositioned, and redeployed within the native valve or native mitral valve MV of the heart H. As shown in Figure 30A, the device 100 is shown implanted within the native mitral valve MV of the heart H. The device 100 can be implanted within the native mitral valve MV of the heart H in a variety of ways. For example, the device 100 can be implanted within the native valve or native mitral valve MV of the heart H, as described and illustrated in Figures 8-14H and 15-20H. The device 100 can also be implanted in other native valves, such as the tricuspid valve in a similar manner.

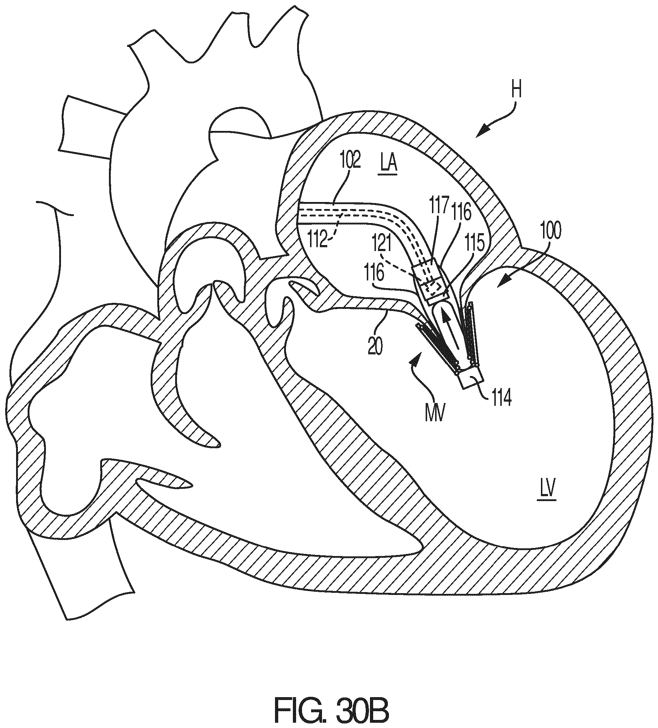

Referring now to Figure 30B, the device 100 can be uncoupled from the actuation element 112. The actuation element 112 can be retracted from the device 100 and the collar 115 and into the sleeve 121, the coupler 117, or the delivery catheter 102. The actuation element 112 can be retracted such that the end of the actuation element 112 is positioned within the sleeve 121, the coupler 117, or the delivery catheter 102. In such a position, the device 100 and the collar 115 can move or pivot relatively freely from the sleeve 121, the coupler 117, the delivery catheter 102, and the actuation element 112.

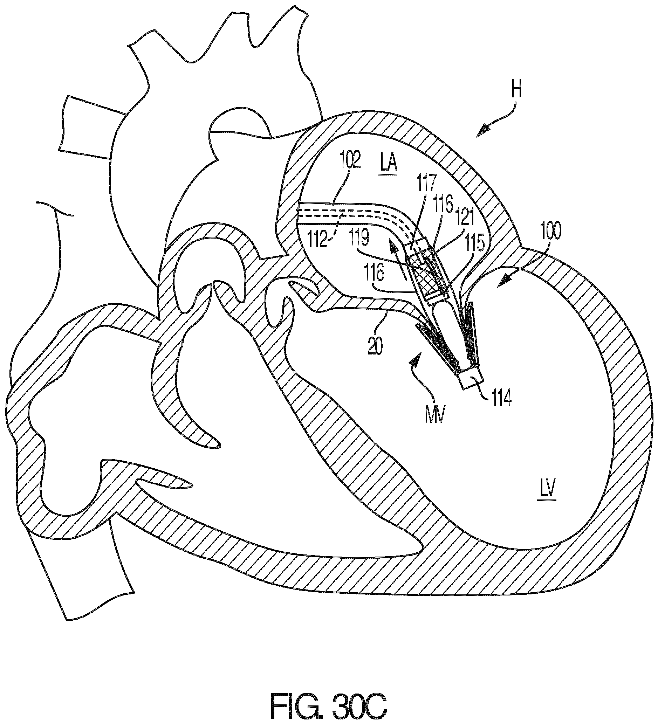

Referring now to Figure 30C, slack can be introduced to the clasp actuation lines 116 and the tether 119 such that the delivery catheter 102, the actuation element 112, and the coupler 117 can be retracted from the device 100 and the collar 115. The collar 115 can still be tied to the delivery catheter 102, the actuation element 112, and/or the coupler 117 via one or more coupling tethers 119. The one or more coupling tethers 119 can be looped around or otherwise secured or attached to the collar 115 and can attach the collar 115 to the coaption portion 104 of the device 100. The retraction of the delivery catheter 102 and the coupler 117 causes the compressible sleeve 121 to expand longitudinally between the collar 115 and the retracted position of the coupler 117. The sleeve 121 encases the one or more coupling tethers 119 and may expand to a maximum distance. In such a position, the device 100 remains tethered or attached to the delivery catheter 102, the actuation element 112, and/or the coupler 117 but, with sufficient slack in the clasp actuation lines 116, the device 100 is generally free to move relative to the delivery catheter 102, the actuation element 112, and the coupler 117. In such a position, a user may observe or check how the device 100 will look or operate when the device 100 is actually deployed, such as on the native valve leaflets.

As shown in Figure 30C, slack can be introduced to the one or more coupling tethers 119 such that the delivery catheter 102, the actuation element 112, and the coupler 117 can be retracted from the device 100 and the collar 115. The sleeve 121 may remain in the expanded or extended condition and may span or substantially span the distance between the collar 115 and the coupler 117. The collar 115 can still be tied to the delivery catheter 102, the actuation element 112, and/or the coupler 117 via the one or more coupling tethers 119. The one or more coupling tethers 119 can be looped around or otherwise secure or attach the collar 115 to the coupler 117 in a variety of ways. For example, the one or more coupling tethers 119 can be looped around or otherwise secure or attach the collar 115 to the coupler 117 in any of the ways previously described herein.

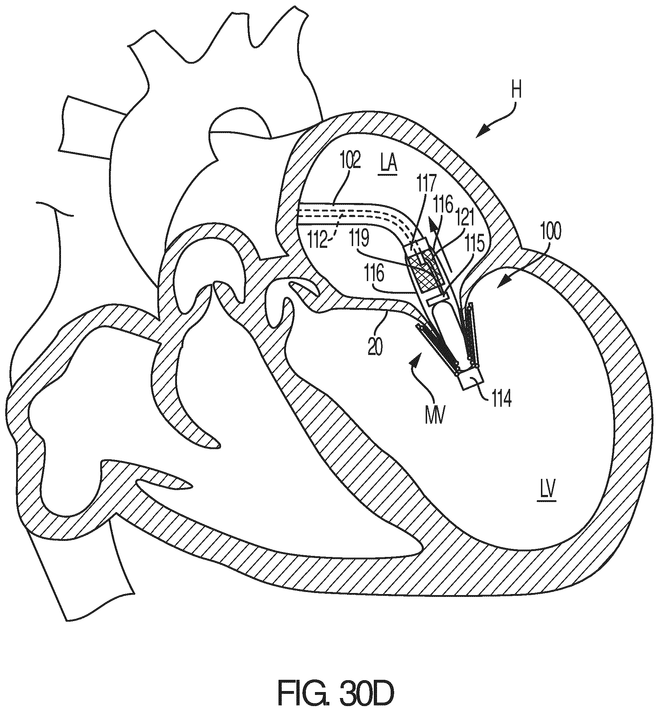

As shown in Figure 30D, the delivery catheter 102, the actuation element 112, the coupler 117, and the sleeve 121 can be retracted farther away from the collar 115. The sleeve 121 can be separated from the collar 115. In such a position, the device 100 remains tethered or attached to the delivery catheter 102, the actuation element 112, and/or the coupler 117 but, with sufficient slack in the clasp actuation lines 116, the device 100 is generally free to move relative to the delivery catheter 102, the actuation element 112, and the coupler 117. Further, the expanded compressible sleeve 121 allows the one or more coupling tethers 119 to keep the collar 115 tied to the coupler 117 and the sleeve 121 can prevent the one or more coupling tethers 119 from getting tangled with the clasp actuation lines and the sleeve 121 can prevent the clasp actuation lines 116 from getting pinched between the collar 115 and the coupler 117. In such a position, a user may observe or check how the device 100 will look or operate when the device 100 is actually implanted, such as on the native valve leaflets.

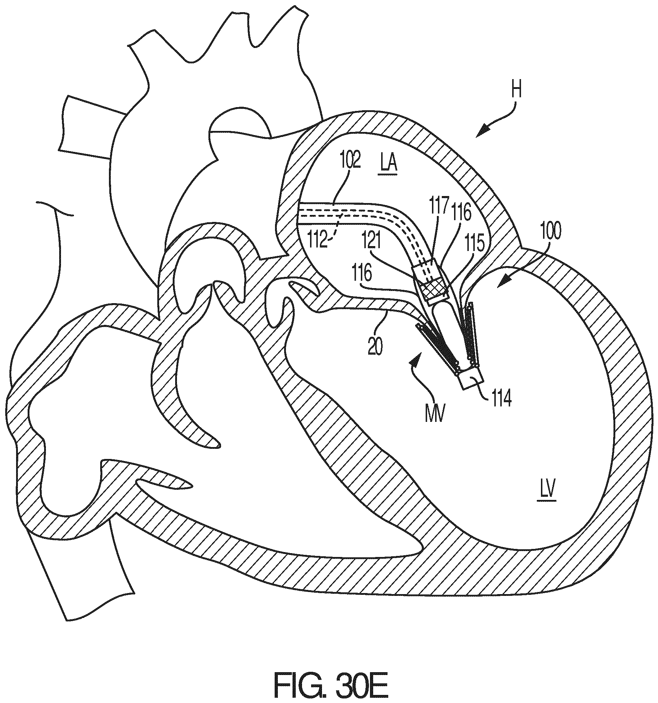

Referring now to Figures 30D through 30F, the device 100 and the collar 115 can be recoupled to the delivery catheter 102 and the actuation element 112. Tension can be applied to the one or more coupling tethers 119 and the coupler 117 can be advanced forward such that the device 100 and the collar 115 can be recoupled to the delivery catheter 102, the actuation element 112, the coupler 117, and the sleeve 121. Tension can also be applied to the clasp actuation lines 116 to bring the clasp actuation lines 116 closer to the device 100 and within the coupler 117 and/or the delivery catheter 102 such that the clasp actuation lines 116 do not move around or get tangled. As shown in Figures 30D and 30E, the delivery catheter 102, the coupler 117, and the sleeve 121 can be advanced such that the sleeve 121 can be brought back into contact with the collar 115. Additionally, as shown in Figure 30E, the delivery catheter and the coupler 117 can be advanced farther such that the sleeve 121 compresses between the collar 115 and the coupler 117.

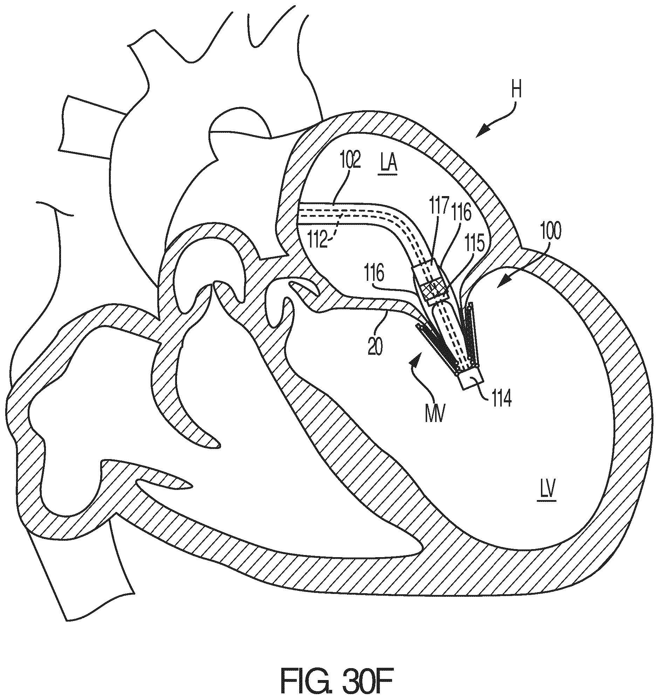

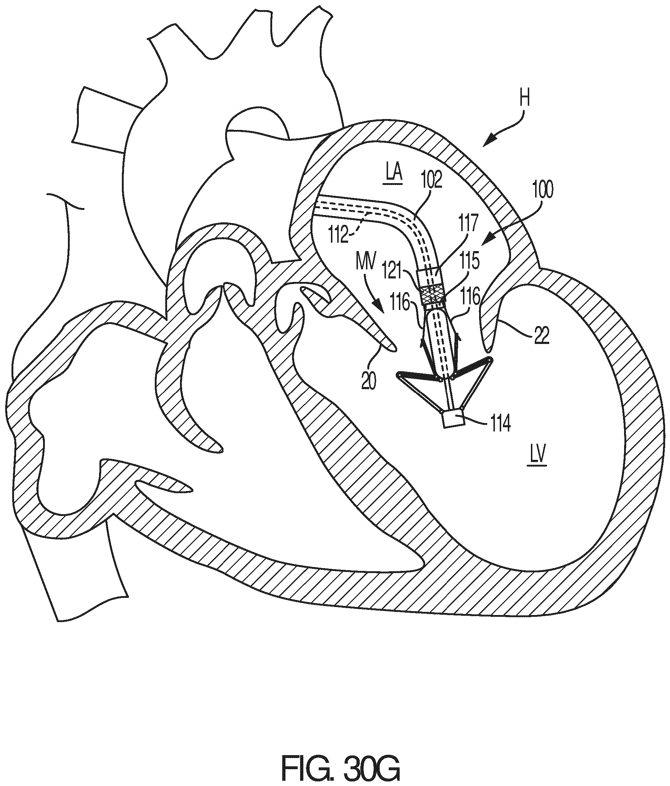

Referring now to Figures 30F through 30H, the device 100 can be recoupled to the actuation element 112, reopened, repositioned, and redeployed in a native valve of the heart. As shown in Figure 30F, the actuation element 112 can be advanced through the collar 115 and into the device 100 such that the actuation element 112 reengages the cap 114 of the device 100. As shown in Figure 30G, the actuation element 112 can be advanced to reopen the device 100 into the partially open, grasp-ready condition. The actuation element 112 can reopen the device 100 in a variety of ways. For example, the actuation element 112 can reopen the device 100 via any of the ways previously described herein. While in the partially open condition, the device 100 can be released from the native valve leaflets, moved or repositioned, and recapture the native valve leaflets. For example, a user may reposition the device 100 to properly grasp the valve leaflets 20, 22.

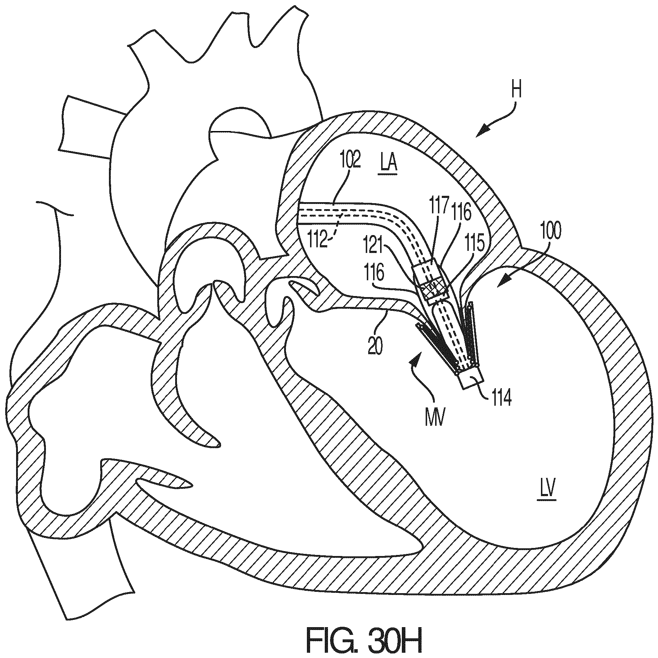

As shown in Figure 30H, the device 100 can be moved to the fully closed or deployed condition. For example, the device 100 can be moved back to the deployed condition once the device 100 has been positioned or repositioned to the desired position. The actuation lines 116 can be extended to allow the clasps 130 to close. Either or both of the actuation lines 116 can be repeatedly actuated to repeatedly open and close the clasps 130. For example, the clasps 130 can be repeatedly opened and closed to ensure that the device 100 is properly placed.

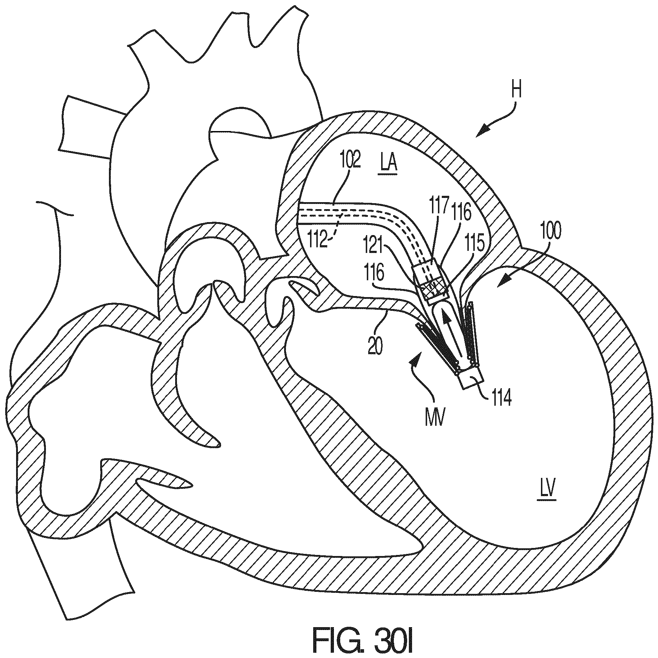

Referring to Figures 30I through 30L, the device 100 can be decoupled from the delivery catheter 102, the coupler 117, and the actuation element 112 and the device 100 can be deployed, such as in the native valve, (e.g., native mitral valve MV, etc.) of the heart H. As shown in Figure 30I, the device can be uncoupled from the actuation element 112. The actuation element 112 can be retracted from the device and the collar 115 and into the sleeve 121, the coupler 117, or the delivery catheter 102. The actuation element 112 can be retracted such that the end of the wire 112 is positioned within the sleeve 121, the coupler 117, or the delivery catheter 102. In such a position, the device 100 and the collar 115 can move or pivot relatively freely from the sleeve 121, the coupler 117, the delivery catheter 102, and the actuation element 112.

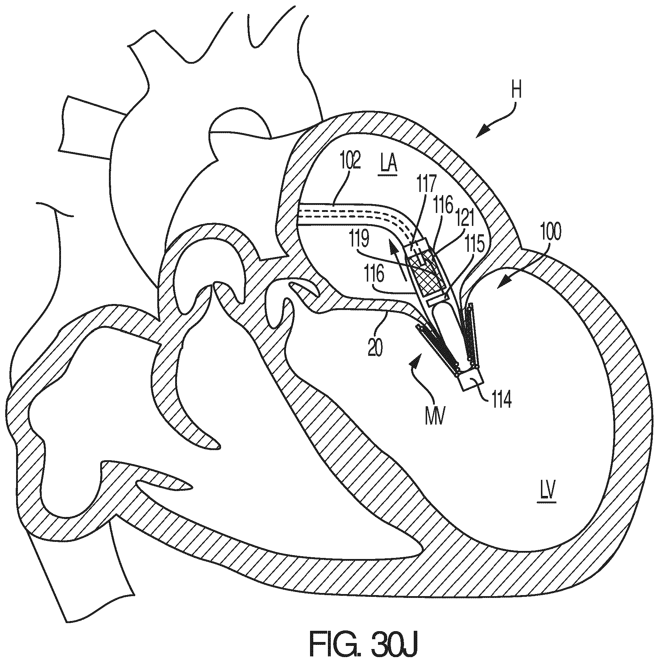

As shown in Figure 30J, slack can be introduced to the one or more coupling tethers 119 such that the delivery catheter 102, the actuation element 112, and the coupler 117 can be retracted from the device 100 and the collar 115. The sleeve 121 can remain in the expanded or extended condition and may span or substantially span the distance between the collar 115 and the coupler 117. The collar 115 can still be tied to the delivery catheter 102, the actuation element 112, and/or the coupler 117 via the one or more coupling tethers 119. The one or more coupling tethers 119 can be looped around or otherwise tie the collar 115 to the coupler 117 in a variety of ways. For example, the one or more coupling tethers 119 can be looped around or otherwise secure or attach the collar 115 to the coupler 117 in any of the ways previously described herein.

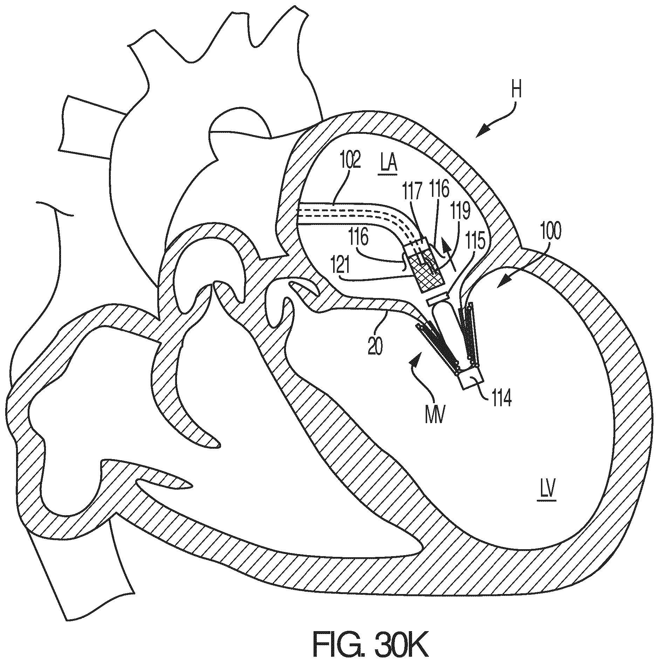

As shown in Figure 30K, the device 100 and collar 115 can be decoupled from the one or more coupling tethers 119, the delivery catheter 102, the actuation element 112, and the coupler 117, and the one or more coupling tethers 119 can be retracted away from the device 100 and the collar 115. The clasp actuation lines 116 can be detached from the moveable arms 134 and pulled or otherwise retracted toward or into the coupler 117 and/or delivery catheter 102. In such an embodiment, the device 100 and collar 115 are completely detached from the delivery catheter 102, the coupler 117, and the actuation element 112 and the device 100 is deployed in the native valve or native mitral valve MV of the heart H. The one or more coupling tethers 119 can be decoupled from the collar 115 in a variety of ways. For example, the one or more coupling tethers 119 can be decoupled from the collar 115 in any of the ways previously described herein.

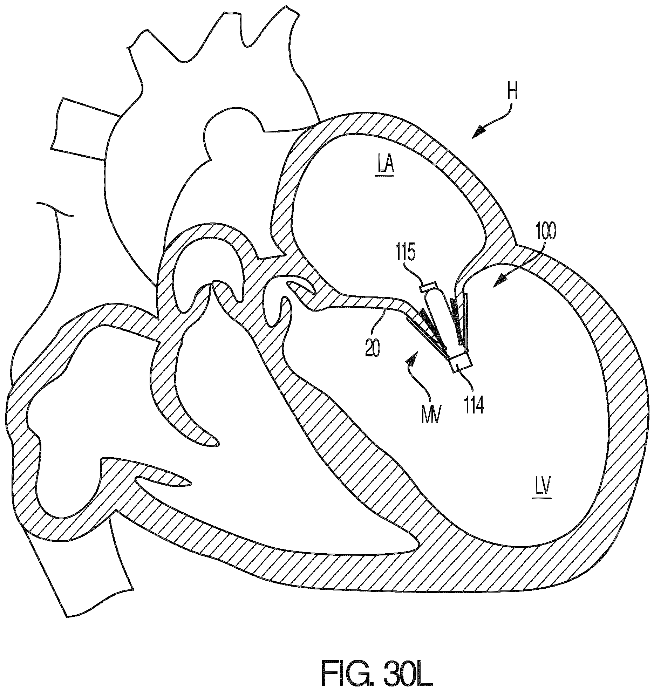

As shown in Figure 30L, the device 100 can be detached from the delivery catheter 102, the coupler 117, and the actuation element 112 and deployed in the native valve or native mitral valve MV of the heart H. For example, the device 100 can be deployed once the device 100 is in the proper place and grasping the native valve leaflets 20, 22.

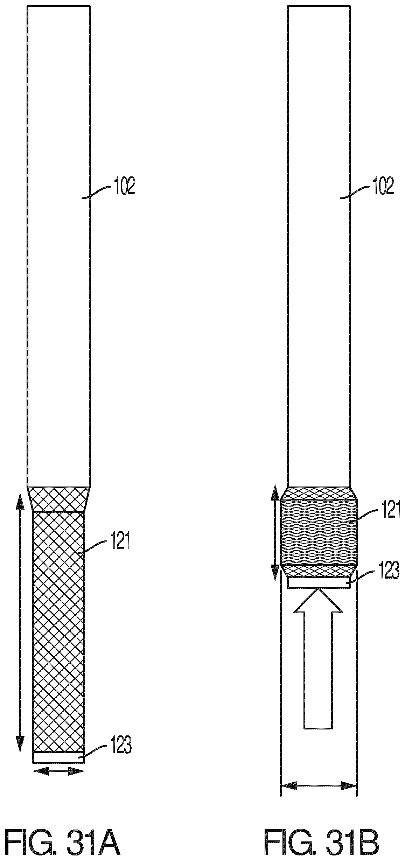

The compressible sleeve 121 can take a wide variety of different forms. Any sleeve configuration that can expand and compress between a collar 115 and a coupler 117 can be used. Examples of compressible sleeves 121 are illustrated by Figures 31A - 31I. In the example illustrated by Figures 31A and 31B, a side view of the delivery catheter 102 and the compressible sleeve 121 is shown with the compressible sleeve 121 being moved from an expanded or elongated position to a retracted or compressed position. The sleeve 121 can be any expandable and compressible material or composite of materials. The sleeve 121 can be a wire mesh, a compressible rubber or plastic, a spiral spring material, or any other material which is compressible and expandable, particularly in a longitudinal direction. The compressible sleeve 121 can also have an end portion 123 at the distal portion of the sleeve 121.

As shown in Figure 31A, the sleeve 121 can be moved into the expanded or elongated position. The sleeve 121 can expand such that the end portion 123 of the sleeve 121 is a maximum distance away from the delivery catheter 102. In the elongated position, the sleeve 121 can contract radially. The sleeve 121 can be spring-loaded or otherwise configured such that the sleeve 121 is biased to the expanded or elongated position.

As shown in Figure 31B, the sleeve 121 can be moved into the retracted or compressed position. The sleeve 121 can be compressed in a variety of ways. For example, the sleeve 121 can be compressed by advancing the delivery catheter 102 toward the implantable prosthetic device such that the end portion 123 of the sleeve 121 contacts the device. After this contact, the further the delivery catheter 102 is advanced, the further the sleeve 121 is compressed. Compression of the sleeve 121 into the retracted position can cause the sleeve 121 to expand radially. However, in some example embodiments the longitudinal compression is greater than the radial expansion.

In some exemplary embodiments, the sleeve is configured such that there is no radial expansion when the sleeve is compressed from the elongated position illustrated by Figure 31A to the position illustrated by Figure 31B or there us a small amount of radially expansion, but the sleeve does not extend radially beyond the catheter or the sleeve does not extend radially or substantially radially beyond the catheter.

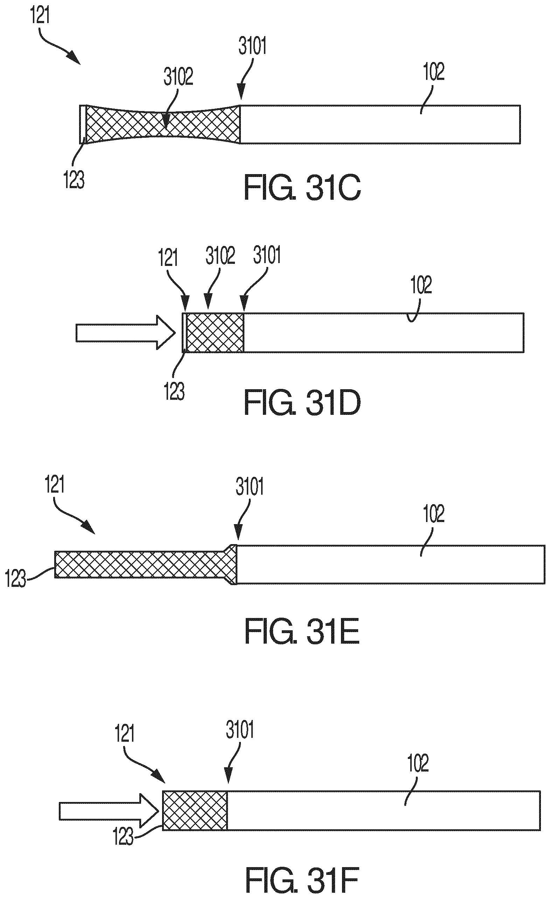

In the example embodiments shown in Figures 31C-31I, the sleeves 121 are tapered radially inward such that the compressed sleeve does not extend radially beyond the catheter or the compressed sleeve does not extend radially beyond or not substantially radially beyond the catheter. The sleeves 121 can be tapered radially inward in a variety of different ways. For example, the sleeve 121 can taper gradually radially inward from a first end and then taper gradually outward toward the second end, the sleeve can taper radially inward only from one end to a cylindrical portion, the sleeve can taper radially inward form each end to a central cylindrical portion, the sleeve can have an undulating outer surface, etc.

One example of a compressible sleeve 121 is illustrated by Figures 31C and 31D. In this example, a side view of the delivery catheter 102 and the compressible sleeve 121 is shown with the compressible sleeve 121 being moved from an expanded or elongated position (Figure 31C) to a retracted or compressed position (31D). The compressible sleeve 121 can also have an end portion 3101 at the proximal portion of the sleeve 121 and a central portion 3102. In this example, the sleeve is tapered so that the end portions 123, 3101 of the sleeve 121 have a greater outer diameter than the outer diameter of the central portion 3102 of the sleeve 121. The diameter of the central portion 3102 of the sleeve 121 is less than the outer diameter of the delivery catheter 102. When the sleeve 121 is compressed, the inner diameter of the central portion 3102 of the sleeve 121 grows allowing the sleeve 121 to neatly stack over the outer diameter of the delivery catheter 102 while not exceeding the overall diameter of the delivery catheter 102 or not substantially exceeding the overall diameter of the delivery catheter 102.

Another example of a compressible sleeve 121 is illustrated by Figures 31E and 31F. In this example, a side view of the delivery catheter 102 and the compressible sleeve 121 is shown with the compressible sleeve 121 being moved from an expanded or elongated position (Figure 31 E) to a retracted or compressed position (Figure 31F). In this example, the sleeve is tapered so that the end portion 3101 at the proximal end of the sleeve 121 has a greater outer diameter than the remainder of the sleeve 121. The diameter of the sleeve 121 is less than the outer diameter of the delivery catheter 102. When the sleeve 121 is compressed the smaller diameter of the sleeve 121 grows, allowing the sleeve 121 to neatly stack over the outer diameter of the delivery catheter 102. The compressed sleeve does not exceed the overall diameter of the delivery catheter 102 or does not substantially exceed the overall diameter of the delivery catheter 102.

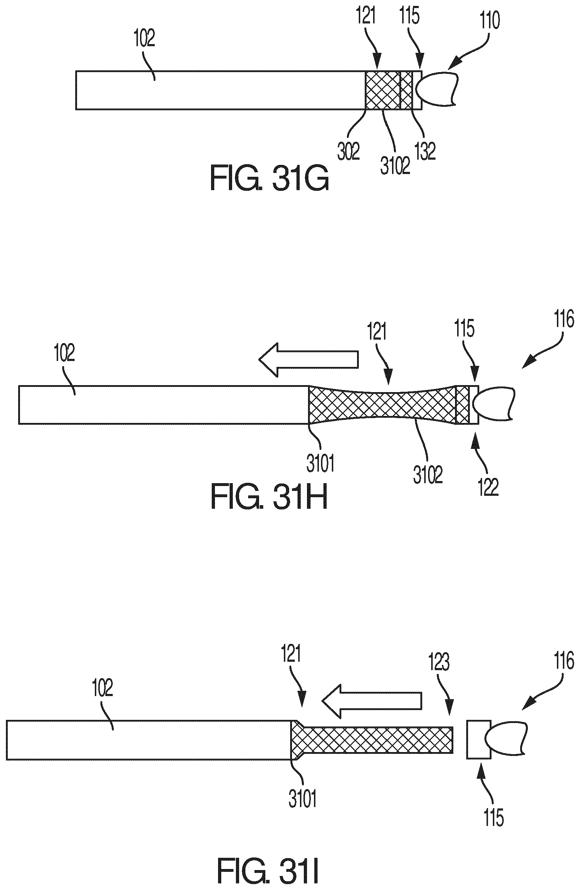

As shown in Figures 31G-31I, in one exemplary embodiment the sleeve 121 can be connected to the collar 115 of the device 100. The sleeve 121 can be connected to the collar 115 in a wide variety of different ways. In one exemplary embodiment, the sleeve 121 can be detached from the collar 115 by pulling on the catheter. In the exemplary embodiment illustrated by Figures 31G-31I, the sleeve 121 stretches and/or friction fits over the collar 115 of the device 100. In Figure 31G, the collar 115 is secured within the distal end 123 of the sleeve 121 due to the distal end 123 being stretched over the collar. Still referring to Figure 31G, the compressed sleeve does not exceed the overall diameter of the delivery catheter 102 or does not substantially exceed the overall diameter of the delivery catheter 102. Referring to Figures 31H and 31I, when the sleeve 121 is extended, the inner diameter of the sleeve 121 decreases such that tension is applied to the portion of the sleeve holding the collar 115 and the sleeve 121 pulls off of the collar. As such, the sleeve passively deploys when the device 100 separates from the catheter 102 and then releases from the device.

While the compressible sleeve 121 is depicted as being directly attached to the delivery catheter 102, it will be appreciated that other embodiments are contemplated. For example, the sleeve 121 can be attached to a coupler which is disposed between the sleeve 121 and the delivery catheter 102. The sleeve 121 can be attached or otherwise secured to a wide variety of couplers. For example, the sleeve can be attached or otherwise secured to any of the couplers 117, 517, 617, 717, 817, 917, 1017 described herein. Additionally, the compressible sleeve 121 can be used with any of the devices 100, 400, 500, 600, 900, 1000, 1100 described herein or can be used with any other implantable prosthetic device.

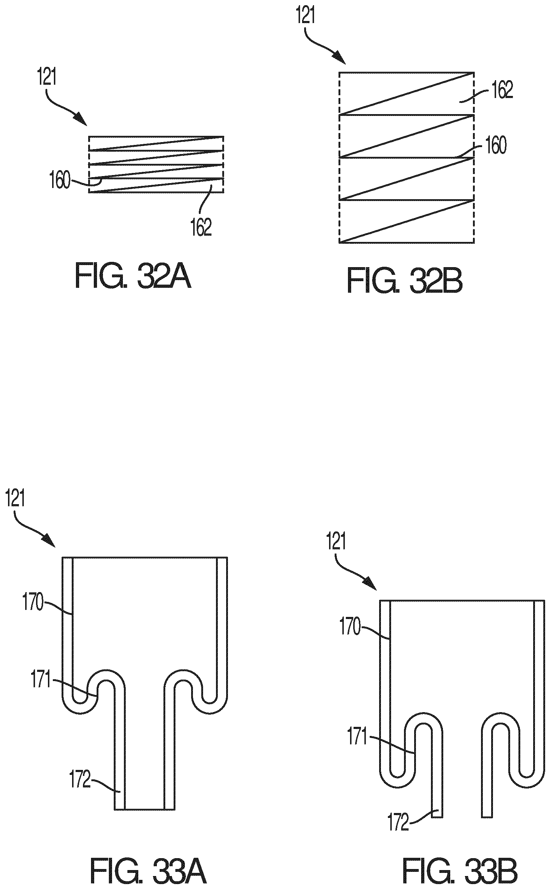

Referring now to Figures 32A and 32B, a cross-section of a compressible sleeve 121 is shown according to one embodiment. The compressible sleeve 121 is generally cylindrical with a frame member 160 extending through the sleeve 121 and a cover 162 attached to the frame member 160. The frame member 160 can be a semi-rigid or rigid material which is moveable between a compressed position and an expanded position, such as a wire spring material, and the cover 162 can be a fabric, mesh, or other suitable covering. In the illustrated embodiment, the frame member 160 is a spiral or otherwise compressible wire or spring and the cover 162 is a fabric or mesh which spans the length of the sleeve 121 and covers or substantially covers the frame member 160. However, the frame member 160 and the cover 162 can take a variety of other forms. For example, the frame member 160 can be a wire or mesh frame or other suitable design.

As shown in Figure 32A, the sleeve 121 is in the retracted or compressed position. The frame member 160 can be compressed to decrease the longitudinal length of the frame member 160. The cover 162 extends between the spiral portions of the frame member 160.

As shown in Figure 32B, the sleeve 121 is in the expanded or elongated position. The frame member 160 can be expanded or elongated to increase the longitudinal length of the frame member 160. The cover 162 extends between the spiral portions of the frame member 160. The frame member 160 can be biased to the elongated or expanded position by a variety of ways, such as by a biasing spring force.

Referring now to Figures 33A and 33B, a cross-section of a compressible sleeve 121 is shown according to one embodiment. The compressible sleeve 121 can be a single piece of compressible or otherwise adjustable material having an outer portion 170 with a first diameter, an inner portion 170 with a second diameter, and an intermediate portion 171 having a diameter between the first diameter and the second diameter and connecting the outer portion 170 and the inner portion 172. The sleeve 121 can be configured such that the inner portion 172 can be moved or otherwise slid within the outer portion 170 of the sleeve 121 such that the length of the intermediate portion 171 is increased and the overall length of the sleeve 121 is decreased. The sleeve 121 can also be configured such that the inner portion 172 can be moved or otherwise slid outside or away from the outer portion 170 of the sleeve such that the length of the intermediate portion 171 is decreased and the overall length of the sleeve 121 is increased. The sleeve 121 can be a compressible, semi-flexible, or otherwise moveable material, such as rubber, plastic, polymer, or other similar material. The outer portion 170, the intermediate portion 171, and the inner portion 172 can be sized and shaped such that the sleeve 121 can cover the coupler 117, the collar 115, and/or the one or more coupling tethers 119, as previously described.

As shown in Figure 33A, the sleeve 121 is in the expanded or elongated position. The inner portion 172 is moved or otherwise slid outside of or away from the outer portion 170 of the sleeve 121 such that the length of the intermediate portion 171 is decreased and the overall length of the sleeve 121 is increased. The inner portion 172 may extend beyond outer portion 170 such that the sleeve 121 is elongated to a maximum length position.

As shown in Figure 33B, the sleeve 121 is in the retracted or compressed position. The inner portion 172 is moved or otherwise slid within or into the outer portion 170 of the sleeve 121 such that the length of the intermediate portion 171 is increased and the overall length of the sleeve is decreased. The inner portion 172 can extend into the outer portion 170 such that the sleeve is compressed to a minimum length position.

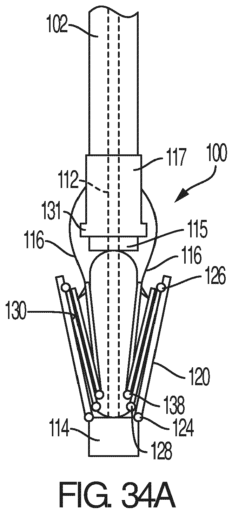

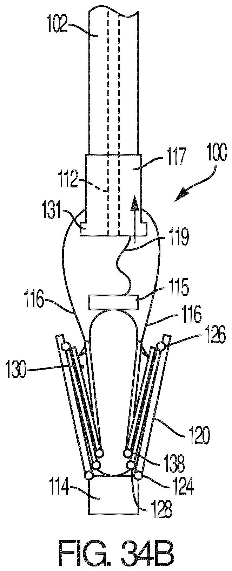

Referring now to Figures 34A and 34B, an implantable prosthetic device 100 is depicted according to one embodiment. The device 100 is deployed from a delivery catheter 102 and includes a coaption portion 104 and an anchor portion 106. In some embodiments, the coaption portion 104 of the device 100 can optionally include a coaption element 110 (e.g., spacer, plug, membrane, sheet, etc.) that is adapted to be implanted between native leaflets of a native valve and is slidably attached to an actuation element or shaft 112. The anchor portion 106 is actuatable between open and closed conditions and can take a wide variety of forms, such as, for example, one or more of paddles, gripping elements, and/or the like. Actuation of the actuation element 112 opens and closes the anchor portion 106 of the device 100 to grasp the native valve leaflets during implantation. The actuation element or shaft 112 can take a wide variety of different forms. For example, the actuation element or shaft can be threaded such that rotation of the actuation element or shaft moves the anchor portion 106 relative to the coaption portion 104. Or, the actuation element or shaft can be unthreaded, such that pushing or pulling the actuation element or shaft 112 moves the anchor portion 106 relative to the coaption portion 104.

In some embodiments, the anchor portion 106 of the device 100 includes outer paddles 120 and inner paddles 122 that are connected between a cap 114 and the coaption element 110 by portions 124, 126, 128. The portions 124, 126, 128 can be jointed and/or flexible to move between all of the positions described herein. The interconnection of the outer paddles 120, the inner paddles 122, the coaption element 110, and the cap 114 by the portions 124, 126, and 128 can constrain the device to the positions and movements illustrated herein.

In some embodiments, the actuation element 112 extends through the delivery catheter 102 and a center of the device (e.g., though a coaption element 110, etc.) to the distal end (e.g., a cap 114 at the distal connection of the anchor portion 106). Extending and retracting the actuation element 112 increases and decreases the spacing between the proximal end of the device and the distal end of the device (e.g., in some embodiments it increases and decreases the spacing between a coaption element 110 and a cap 114), respectively. A collar 115 or other attachment portion removably attaches the coaption element 110 to the delivery catheter 102, either directly or indirectly, so that the actuation element 112 slides through the collar 115 and coaption element 110 during actuation to open and close the paddles 120, 122 of the anchor portion 106.

In some embodiments, the device 100 can also include a coupler 117 which removably attaches the collar 115 to the delivery catheter 102. The coupler 117 can be removably attached to the collar 115 and can be fixedly or removably attached to the delivery catheter 102. The coupler 117 can attach the delivery catheter 102 to the collar 115 in a wide variety of ways. For example, the coupler 117 can attach the collar 115 to the delivery catheter 102 in any of the ways described in PCT patent application publication WO2020/076898 or in any of the ways previously described herein. In such an embodiment, the actuation element 112 slides through the coupler 117, the collar 115, and the coaption element 110 during actuation to open and close the paddles 120, 122 of the anchor portion 106. As detailed herein, the coupler 117 can facilitate the placement, repositioning, and/or replacement of the device 100.

In some embodiments, he coupler 117 includes a flange 131 at a distal portion of the coupler 117 which extends radially beyond the remainder of the coupler 117. The flange 131 can extend radially beyond the diameter of the delivery catheter 102 and/or the collar 115. The flange 131 can take a wide variety of different forms. The flange can be flexible, such that the flange can compress or flex radially inward when disposed inside another catheter, such as a guide sheath and/or a positioning catheter. For example, the flange 131 can be compressed or flexed radially inward such that the flange does not extend past the radially outer surface of the remainder of the coupler.

The flange 131 can prevent the clasp actuation lines 116 from getting tangled with the tether or pinched between the coupler 117 and the collar 115, such as when device 100 is moved toward and away from the collar. For example, the flange 131 guides the clasp control lines 116 radially outward away from the gap between the collar 115 and the coupler 117. The flange 131 can be used in any of the other embodiments described herein.

The device 100 can be inserted and deployed by any method or procedure previously described herein or simulations of those methods or procedures. For example, the device 100 can be inserted and deployed as described and illustrated in Figures 8-14M and 15-20M.

As shown in Figure 34A, the device 100 can be coupled to the actuation element 112 and the coupler 117. The coupler 117 can be in contact with the collar 115 and the coupler 117 and the collar 115 can be coupled via the one or more coupling tethers 116. As shown in Figure 34B, the device 100 can be decoupled from the actuation element 112 and the coupler 117, the actuation element 112, and the delivery catheter 102 can be retracted from the device 100 and the collar 115. The collar 115 remains coupled to the coupler 117 via the one or more coupling tethers 119. The flange 131 extends radially outward and pushes the clasp actuation lines 116 outward, thereby preventing the clasp actuation lines 116 from getting tangled with the one or more coupling tethers 119 and from getting pinched between the collar 115 and the coupler.

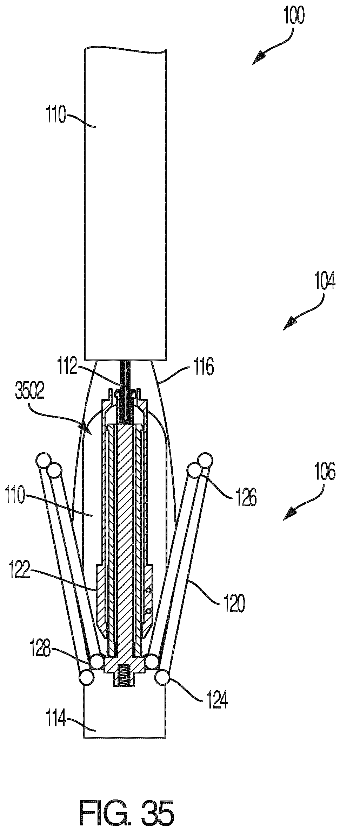

Referring to Figure 35, in one example embodiment the actuation element 112 is attached to the device 100 at the proximal end instead of directly to the cap 114 at the distal end. This can be accomplished in a wide variety of different ways using a variety of different couplers that couple the actuation element 112 to the distal end or to a distally positioned cap 114. This proximal reconnection point makes the reattachment of the shaft 112 to the device easier in all of the tethering embodiments described above. As such, the following embodiments of attaching the actuation element at the proximal end of the device can be applied to any of the tethering embodiments disclosed herein and can be used in applications that do not include a tether.

As is illustrated in Figure 35, during installation the device 100 can be positioned into the native valve or mitral valve and affixed to the anterior leaflet 20 and the posterior leaflet 22 as described above. The device 100 may require repositioning to achieve the desired results. This involves attaching the device 100 and then observing the native valve to determine if the valve is functioning correctly. In order to determine if the device 100 has modified the native valve as desired, the device 100 can be released from the actuation element 112 so as to allow the device 100 and leaflets 20 and 22 to move about without influence from the actuation element 112. However, if the device 100 is required to be repositioned, it may need to be reattached to the actuation element 112 as described above. In other example embodiments illustrated herein, the actuation element 112 can be passed through a proximal end of the device 100 and threaded into or otherwise coupled to the cap 114 located at the distal end of the device 100. As illustrated in the example embodiment of Figure 35, a telescoping coupler 3502 is positioned within the coaption element 110 and is affixed to the cap 114 and adapted to receive the threaded end 3504 (or other coupling structure) of the actuation element 112.

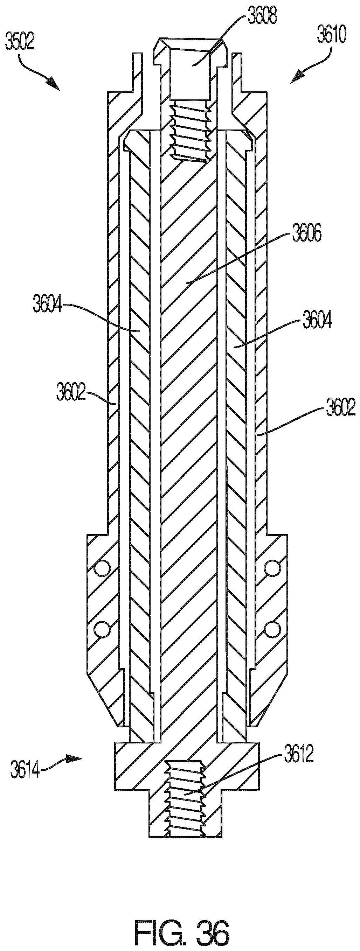

Figure 36 illustrates a cross-section of the coupler 3502 separately from the device 100. In the example embodiment illustrated, the coupler 3502 comprises three members. However, the coupler 3502 can comprise any number of members. In the illustrated embodiment, the coupler 3502 can be formed from an outer sleeve 3602, an inner sleeve 3604, and an inner shaft 3606. Each of the parts can be annular, have annular portions, or have other closed, tubular shapes. The inner shaft 3606 can include a threaded portion 3608 or other coupling structure for attachable/releasable connection with the actuation element. The illustrated inner shaft 3606 also includes a threaded receptacle 3612 or other coupling structure for attachment to the cap 114 at the distal end of the inner shaft. In an example embodiment, the distal end of the inner shaft 3606 can be permanently attached to the cap 114.

In some example embodiments, the components of the coupler 3502 can optionally be formed in shapes that resist rotation between the outer sleeve 3602, inner sleeve 3604, and /or the inner shaft 3606. For example, the components can be, without limitation, oval, rectangular, formed with an alignment structure such as a spline, or another shape that resists rotation among the components. Such shapes can prevent threaded chambers 3608, 3612 from rotating relative to the other components of the coupler 3502. However, in some example embodiments the outer sleeve 3602, inner sleeve 3604, and/or inner shaft 3606 are configured to rotate relative to one another.

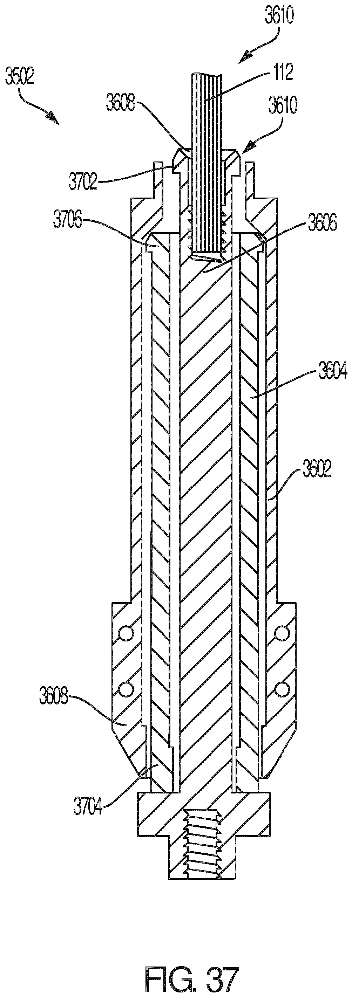

Figure 37 illustrates a cross section view of the coupler 3502 of Figure 36 with the actuation element 112 threaded into the threaded chamber 3608 of the proximal end 3610 of the coupler 3502 (or otherwise connected to the coupler). A first radially outwardly extending step 3702 is shown at the proximal end of the inner shaft 3606. A first radially inwardly extending step 3704 is shown at the distal end of the inner sleeve. A second radially outwardly extending step 3706 is shown at the proximal end of the inner sleeve 3604, and a second radially inwardly extending step 3708 is shown at the distal end of the outer sleeve 3602.

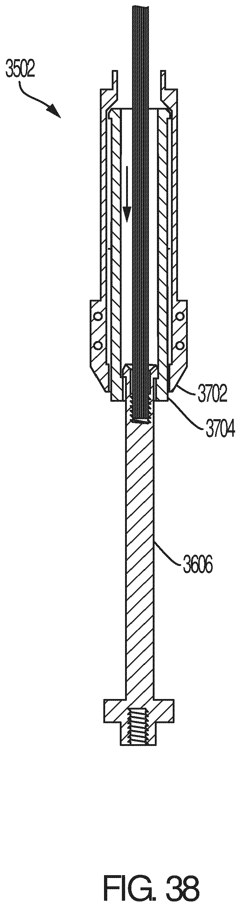

As noted earlier herein, components of the device 100 are actuated via the extension of the actuation element 112. Figure 38 illustrates the coupler 3502 as the actuation element 112 is extended from the catheter (not shown in Figure 38). The inner shaft 3606 is caused to extend by the actuation element 112 such that the inner shaft 3606 extends outward from the inner sleeve 3604. At a certain amount of extension, the first radially inwardly extending step 3702 meets the first radially outwardly extending step 3704. However, in some embodiments, depending on the friction between the outer sleeve 3602, inner sleeve 3604, and inner shaft 3606, the inner sleeve 3604 can begin to extend out of the outer sleeve 3602 before the first radially inwardly extending step 3702 meets the first radially outwardly extending step 3704.

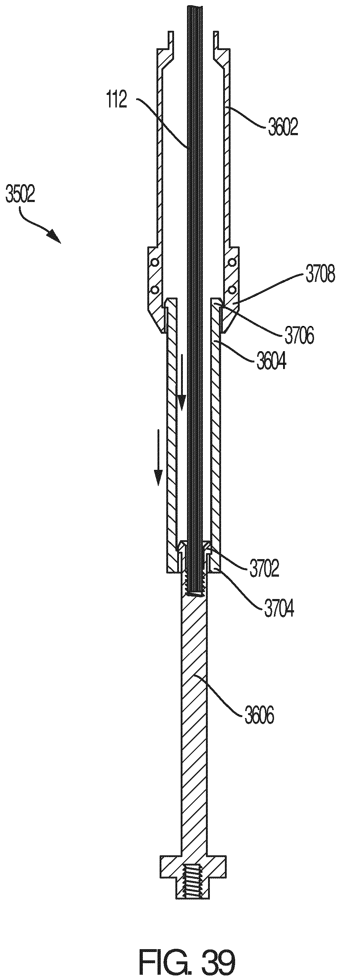

As illustrated in Figure 39, as the actuation element 112 continues to extend, the engagement of the first radially outwardly extending step 3702 against the first radially inwardly extending step 3704 causes the inner shaft 3606 to extend the inner sleeve 3604 outward from the outer sleeve 3602. As the actuation element 112 continues to extend into the coupler 3502, the inner sleeve 3504 is extended out of the outer sleeve 3602 until the second radially outwardly extending step 3706 contacts the second radially inwardly extending step 3708.

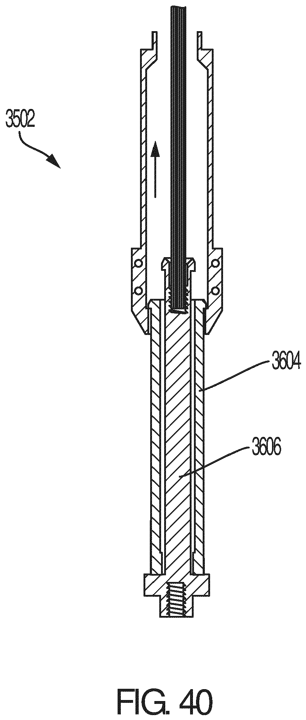

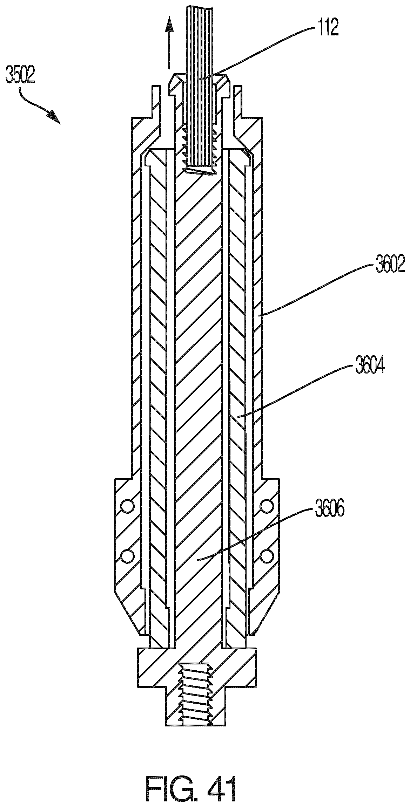

Referring to Figures 40 and 41, the process is reversed to draw the inner shaft 3606 back into the outer sleeve 3602. As the actuation element 112 is retracted, the inner shaft 3606 is drawn into the inner sleeve 3604 as illustrated in Figure 40. Again, depending on the amount of friction between the outer sleeve 3602, inner sleeve 3604, and inner shaft 3606, the inner sleeve 3604 can begin to retract into the outer sleeve 3602 before the inner shaft 3606 fully retracts into the inner shaft 3604. As the retraction of the actuation element 112 continues, the inner shaft 3606 and inner sleeve 3604 are drawn into the outer sleeve 3602 as illustrated in Figure 41.

The process of Figures 38-39 is illustrated within an example device 100 in Figures 35 and 42-43. As illustrated in Figure 35, the coupler 3502 is located within the coaption element 110 with the threaded inner chamber 3608 (or other coupling structure) positioned such that it is adjacent to the proximal end of the device 100. The distal end 3614 of the coupler 3502 is affixed to the cap 114 via the threaded receptacle 3612 (or other coupling structure). Some example embodiments can use other fixing methods, such as, but not limited to, welding or an interference fit.

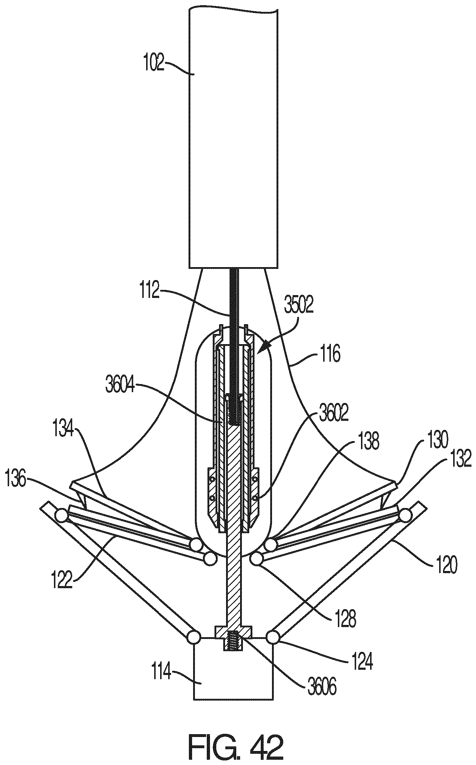

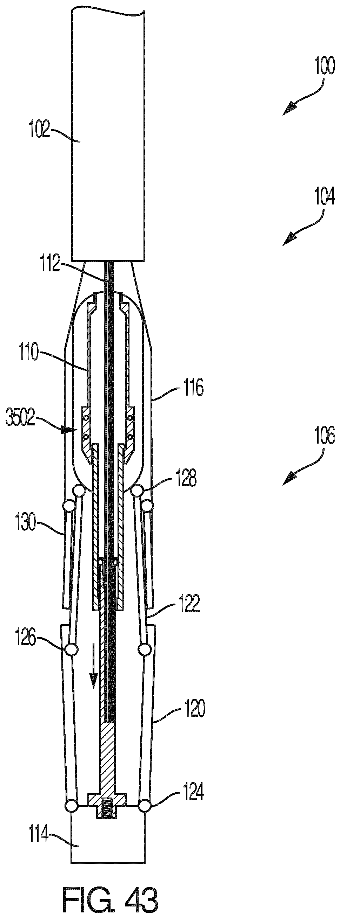

Figure 42 illustrates the inner shaft 3606 being extended from the inner sleeve 3604 by the actuation element 112. As the inner shaft 3606 extends, it pushes on the distal end (e.g., on cap 114, another attachment portion, etc.), causing the outer paddle 120 and the inner paddle 122 to straighten with respect to each other. As the actuation element 112 continues to extend from the delivery catheter 102, the sections of the coupler 3502 extend outward as illustrated in Figures 38 and 39 until the coupler is fully extended as is illustrated in Figure 43. As shown, the device 100 is fully extended such that the anchor portion 106 is also in its fully extended configuration.

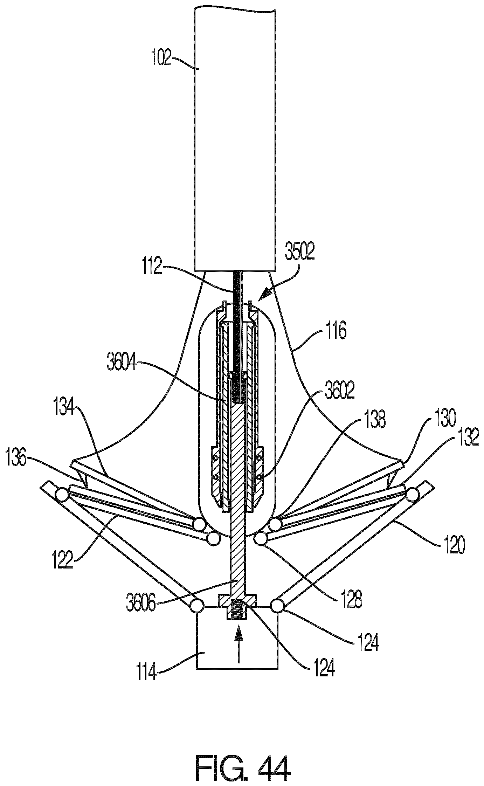

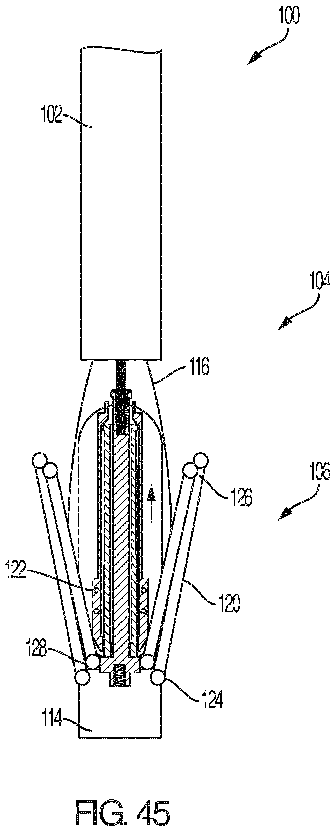

As the actuation element 112 is retracted, the device moves the inner paddles 122 and the outer paddles 120 outward as illustrated in Figure 44. The device 100 can be positioned in the native valve (e.g., in the native mitral valve MV and affixed to the anterior leaflet 20 and the posterior leaflet 22) and closed as illustrated in Figure 45. The installing physician can decouple the actuation element 112 from the coupler 3502 such that the device is not prevented from moving as the result of attachment to the actuation element. For example, the installing physician can rotate the actuation element 112 such that it unscrews from and becomes detached from the coupler 3502.

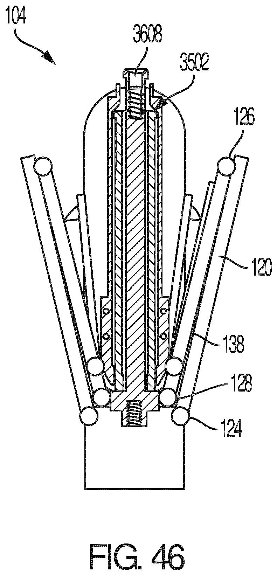

However, if after releasing the device, it is determined that the position of the device is not desirable, the actuation element 112 can be reattached to the device 100 in order to allow the device 100 to be repositioned in any of the manners described herein. The threaded or other coupling portion 3608 of the coupler 3502 remains at the proximal portion of the coaption portion 104 of the device 100, as is illustrated in Figure 46.

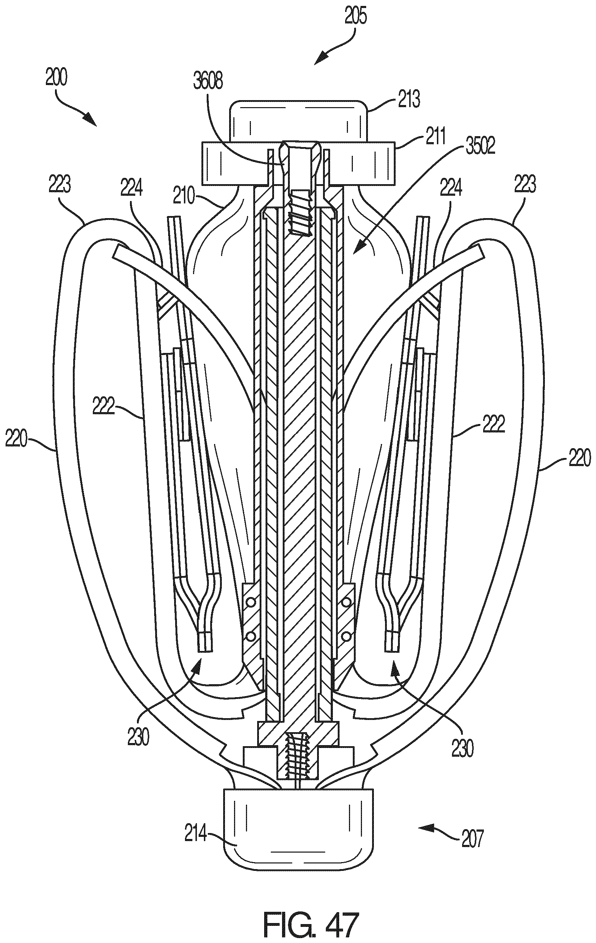

An example embodiment is illustrated in Figure 47. As illustrated, the coupler 3502 is positioned between the cap 214 and the proximal collar 211. In one example embodiment, the collar is used to connect and/or tether the device 200 to the delivery sheath in any of the manners described herein. Figure 47 illustrates the coaption element 210, the connection portion 223, the paddle frames 224, inner paddles 222, paddles 220, and clasps 230 of one of the devices shown and described in PCT patent application publication WO2020/076898, which is incorporated herein by reference.

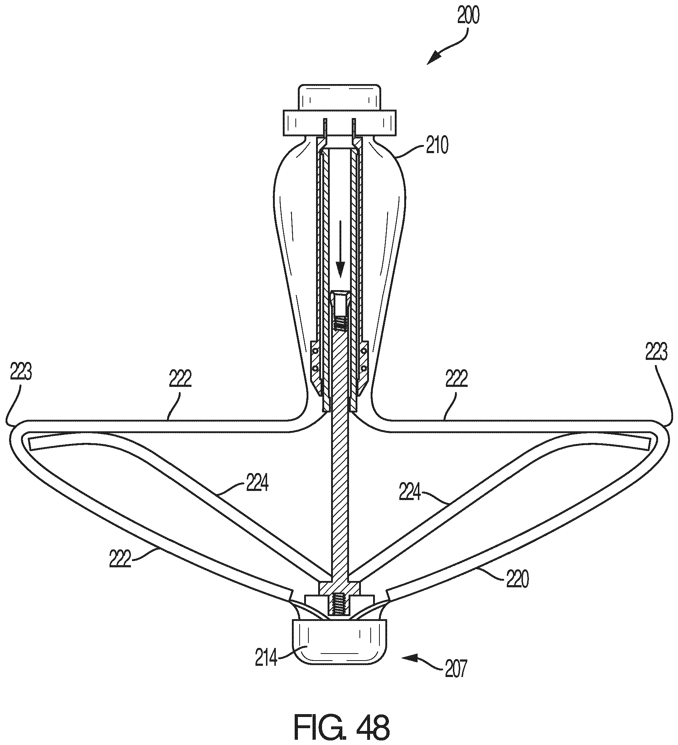

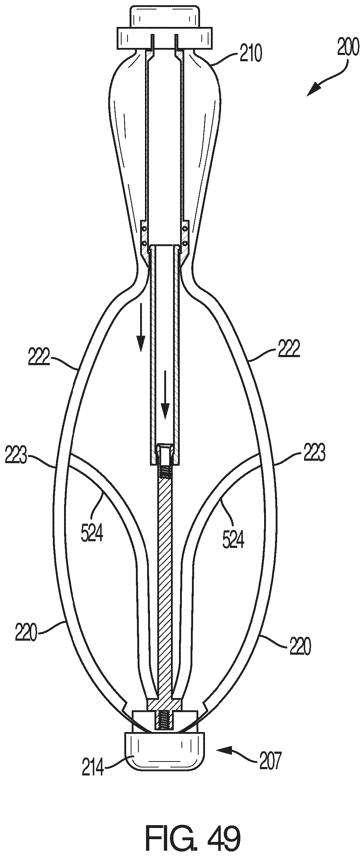

As the actuation element 112 (not shown in Figures 47-51) is extended, the coupler 3502 extends is described with respect to Figures 38 and 39. Figure 48 shows the example embodiment of Figure 47 where the coupler is partially extended by the actuation element 112 (not shown in Figures 47-51). The clasps are not illustrated to simplify the drawing. The inner paddles 222 and the paddles 220 are shown in an open position. Also illustrated are the connection portions 223, the paddle frames 224, and the cap 214 located at the distal portion 207 of the device 200. Figure 49 shows the device 200 of Figures 47 and 48 with the coupler 3502 fully extended.

Figure 47 shows the device 200 in an installed condition. After the actuation element is removed from the device 100 to test its placement, an installer can decide to reposition the device. In such a case, the actuation element 112 is reinserted into the threaded chamber or other coupling structure 3608 at the proximate end of the device 100 and the paddles extended as shown in Figure 48. The device can also be fully extended as shown in Figure 49 or to any other position needed to reposition the device in the native valve.

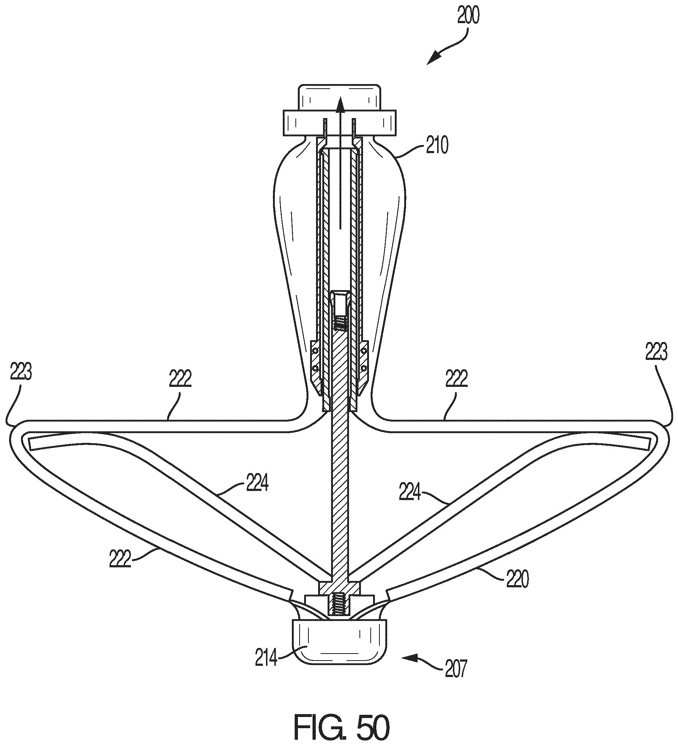

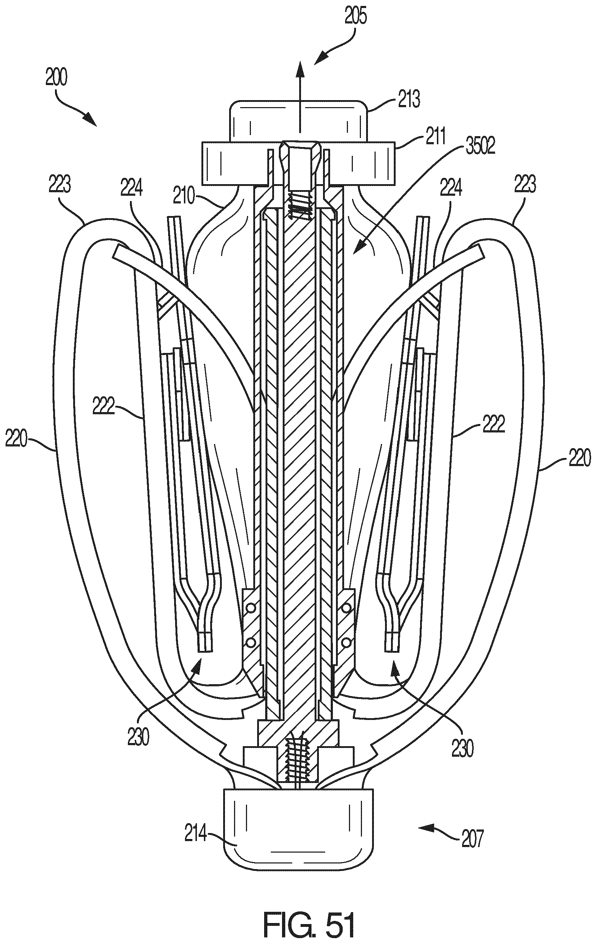

As illustrated in Figure 50 and 51, the actuation element 112 is drawn back into the delivery sheath (not shown) to close the device 200 with the coupler 3502. The repositioning and closing of the device 100 recapture and affix the device to a native valve, such as to the anterior leaflet 20 and the posterior leaflet 22 of the native valve.

An optional seal 213 is illustrated in the Figure 47 example. In one example embodiment, the seal 213 is omitted and the distal end of the coaption element 210 and the outer sleeve 3602 are sealingly connected together. Also, a blood tight seal can be provided between the outer sleeve 3602, the inner sleeve 3604, and inner shaft 3606. The sealed connection between the coaption element 210 and the outer sleeve 3602 and the blood tight coupling between the outer sleeve 3602, the inner sleeve 3604, and inner shaft 3606 prevent or inhibit blood from flowing into the distal end of the coaption element, no matter what position the device is in.

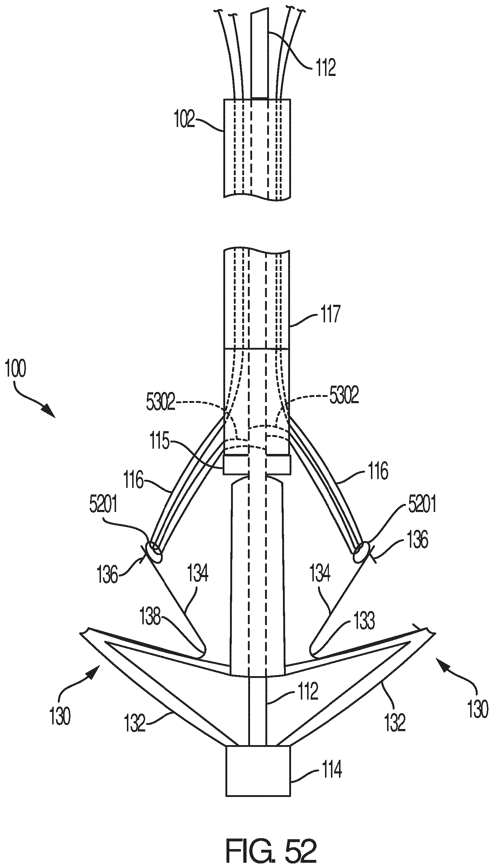

Clasp control lines 116 (see e.g., Figure 42) and/or tethers 119 can be connected to a prosthetic device in a wide variety of different ways. Figures 52-60 illustrate an exemplary embodiment of a releasable connection between the clasp control lines 116 and the device 100. However, the releasable connection illustrated by Figures 52-60 can also be used to connect the tether(s) to the device. The device 100 illustrated by Figures 52-60 can be any of the devices described herein, any of the devices that are described in a patent or patent application that is referenced herein, or any other known device for repairing a native heart valve, such as a mitral valve or a tricuspid valve.

Referring now to Figure 52, an implantable prosthetic device 100, a delivery catheter 102, an actuation element 112, a collar 115, and a coupler 117 are depicted according to one embodiment. The device 100 can also comprise a cap 114 (or other attachment portion) and/or clasps 130. In some embodiments, the clasps include a base or fixed arm 132, a moveable arm 134, barbs 136, and a joint portion 138. Optionally, the device can include a coaption element (e.g., a spacer, etc.). In one embodiment, clasp actuation lines 116 are looped to connect to the moveable arms 134 of the clasps. In the example illustrated by Figure 52, the clasp actuation lines extend through the delivery catheter 102 and the coupler 117, through a ring, loop, or other opening 5201 of the movable arm 134, around the actuation element 112, back through the ring 5201, back through the coupler 117, and back through the delivery catheter 102 towards the proximal end. The clasps 130 can be opened by applying tension to the actuation lines 116 attached to the moveable arms 134, thereby causing the moveable arms 134 to flex, articulate, or pivot on the joint portions 138.

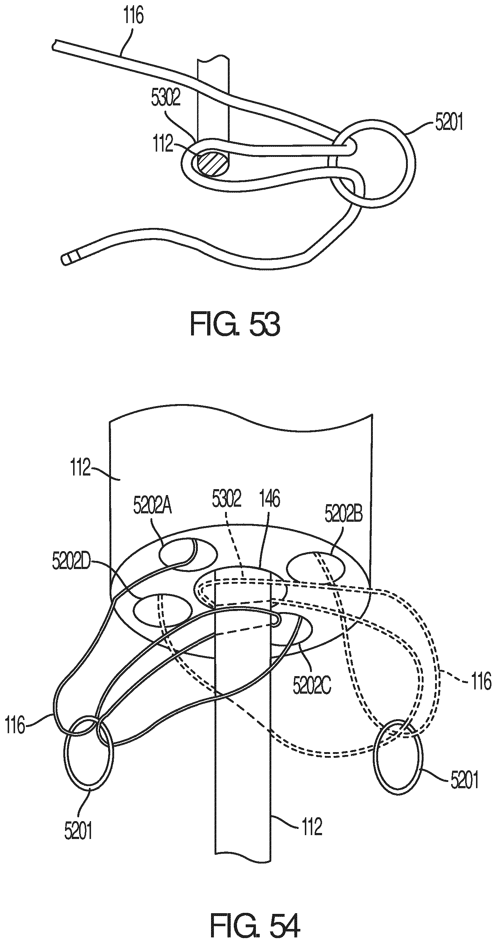

As shown in Figure 53, a looped end 5302 of the clasp actuation line 116 extends through the ring, loop, or other opening 5201 on the movable clasp arm 134 of the device 100 (not shown in Figure 53). The ends 5304 of the clasp actuation line 116 extend through the ring, loop, or other opening 5201 of the clasp. As such, removing the wire 112 from the looped end 5302 will decouple the clasp actuation line 116 from the clasp.

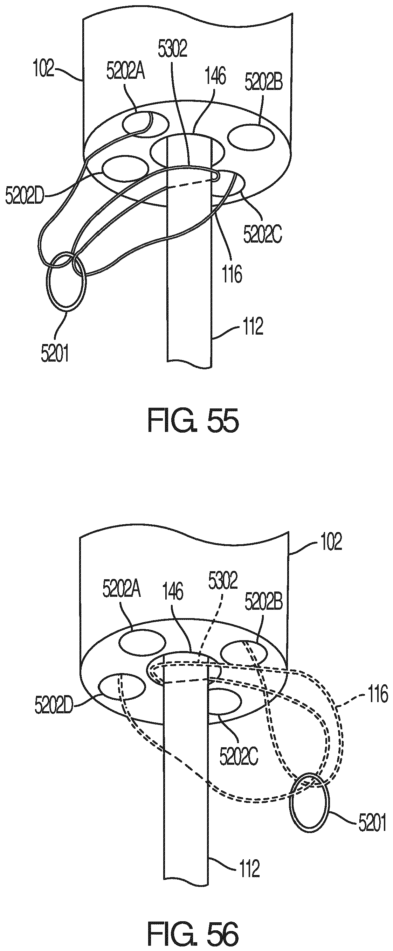

The catheter 102 can be designed in a variety of ways and have one or more lumens/passages. In the exemplary embodiment shown in Figures 54-56, the catheter 102 includes a first line passage 5202A, a second line passage 5202B, a third line passage 5202C, and a fourth line passage 5202D. A wire passage 146 also extends through the catheter. Referring to Figures 54 and 55, a first clasp actuation line 116 extends through the first line passage 5202A of the catheter 102, through the ring, loop, or other opening 5201 of the clasp, around the actuation element 112, back through the ring, loop, or other opening 5201 of the clasp, and through the third line passage 5202C of the catheter 102 (Figure 55 is included to more clearly show the actuation line routing). Referring to Figures 54 and 56, a second clasp actuation line 116 extends through the second line passage 5202B of the catheter 102, through the ring, loop, or other opening 5201 of the clasp, around the actuation element 112, back through the ring, loop, or other opening 5201 of the clasp, and through the fourth line passage 5202D of the catheter (Figure 55 is included to more clearly show the actuation line routing).

By diametrically opposing line passages 5202A from 5202C, and line passages 5202B from 5202D in the catheter, the tensile forces applied to the catheter by the lines cancel one another out. This prevents unintended bending of the catheter 102 due to pulling on the clasp actuation lines 116. In some embodiments, the line passages 5202A, 5202C, 5202B and 5202D are not diametrically opposed.

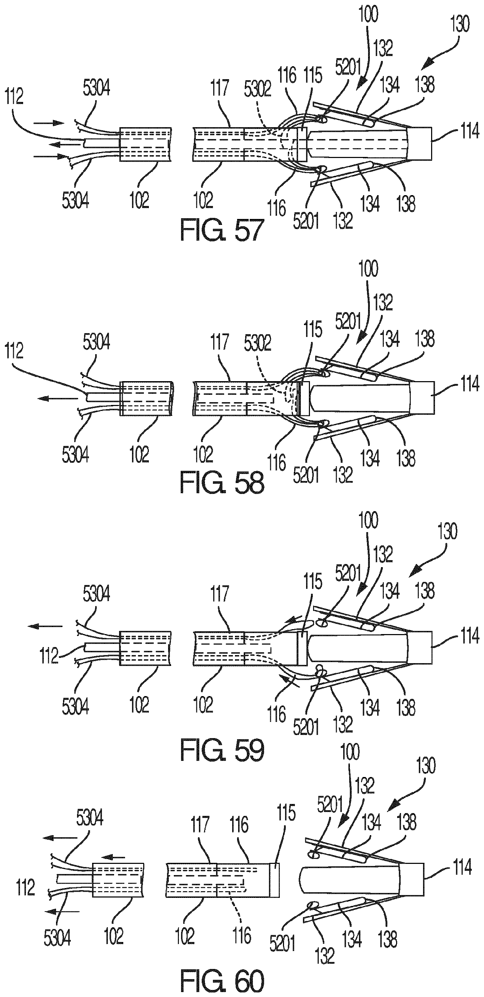

Referring now to Figures 57-60, the device 100 can be deployed from the delivery catheter 102, the coupler 117, the actuation element 112, and the clasp actuation lines 116. Referring to Figure 57, the barb clasps 130 can be closed by lessening the tension to the actuation lines 116 attached to the moveable arms 134, thereby causing the moveable arms 134 to close towards the fixed arms 132 of the device 100. As shown by comparing Figures 57 and 58, the device can be uncoupled from the actuation element 112. As shown in Figure 58, the actuation element 112 can be retracted from the device 100 and the collar 115, toward the coupler 117 and the delivery catheter 102. The actuation element 112 can be retracted such that the end of the actuation element 112 is positioned within the wire passage 146 of the coupler 117 (or further retracted). In such a position, the clasp actuation lines 116 are no longer secured around the actuation element 112, such that the delivery catheter 102 and the coupler 117 can be retracted from the device 100.

As shown in Figure 59, once the device 100 and collar 115 are decoupled from the actuation element 112, the coupler 117 can be retracted away from the device 100. The clasp actuation lines 116 are pulled through the rings or other openings 5201 on the movable arms 134 of the device 100 and retracted into the coupler 117. As such, the device 100 is completely detached from the delivery catheter 102, the coupler 117, and the actuation element 112.

As shown in Figure 60, the device 100 is shown in the fully closed and deployed condition. The delivery catheter 102, the coupler 117, and the actuation element 112 have been retracted and the clasps 130 remain in the fully closed position. Once deployed, the device 100 can be maintained in the fully closed position in a variety of ways. For example, the device 100 can be maintained in the fully closed position in any of the ways described relating to Figure 14M. In another example embodiment, the paddles of the device can optionally be configured to partially open and close with the beating of the heart, while the clasps remain in their closed configuration.

When the clasp actuation lines 116 are routed in the manner illustrated by Figures 52-60, pulling both proximal ends 5304 a first distance results in movement of the ring or other opening 5201 a second distance that is one-half of the first distance. This may be advantageous in some other applications. For example, the slower opening movement of the clasp can make the clasp easier to control and the force applied to the ring or other opening 5201 would be twice or about twice the pulling force applied to the proximal ends 5304. However, in some applications it can be beneficial to provide a one-to-one correlation between the movement of the proximal ends 5304 of the clasp actuation lines 116 and the clasp ring or other opening 5201 and/or to provide a one-to-one correlation between the force applied to the proximal ends 5304 of the clasp actuation lines and the clasp ring or other opening 5201.

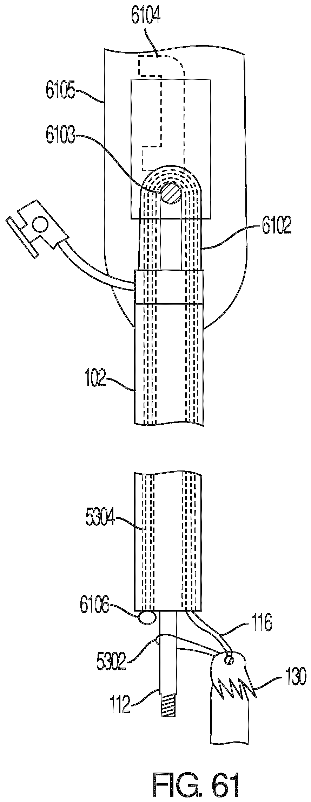

A one-to-one correlation between the movement of the proximal ends 5304 of the clasp actuation lines and the clasp ring or other opening 5201 can be provided in a variety of different ways. Figure 61 shows an exemplary embodiment of a clasp actuation control 6100 that can be used with the clasp actuation arrangement illustrated by Figures 52-60 to provide a one-to-one correlation between the movement of the proximal ends 5304 of the clasp actuation lines and the clasp ring or other opening 5201. In this embodiment, both ends of a clasp actuation line 116 are routed through a delivery catheter 102. The loop 5302 of the clasp actuation line 116 is disposed around the actuation element 112. Both ends 5304 of the clasp actuation line 116 enter the delivery catheter 102 and exit into a lumen 6102 at the proximal end of the delivery catheter 102. The lumen 6102 or a portion of the lumen is moveable relative to the end of the catheter 102. In some exemplary embodiments, the lumen 6102 is made of an elastic material, such as an elastic polymer, but can be made of a wide variety of different materials. The lumen 6102 passes around a pin 6103 on a slider 6104. The slider is on the handle 6105 of the delivery catheter 102.

The control line 116 exits the lumen 6102 and the ends 5304 are connected to the delivery catheter 102 on the opposite side of the pin 6103. Pulling on the pin 6103 pulls the lumen 6102 away from the catheter or stretches the lumen and pulls the doubled-back control line that is in the lumen 6102. Pulling on the pin 6103 a first distance results in movement of the ring or other opening 5201 the same, first distance. This equal movement is caused by the two ends 5304 being loop both through the ring or other opening 5201 and around the pin 6103. At the distal end of the catheter, the control line is constrained by being looped around the control wire 112 and at the proximal end of the catheter, the control line 116 is secured to the catheter.

The ends 5304 of the control line 116 can be connected to the delivery catheter in a wide variety of different ways. For example, the ends 5304 can be connected to the proximal end of the delivery catheter 102, connected to the distal end of the catheter, or tied to an intermediate pull wire that is connected to the catheter and that provides tension equalization. The illustrated example, the clasp actuation line 116 passes through the delivery catheter 102 and terminates at the distal end of the delivery catheter 102 with a knot 6106 or a stop to provide tension equalization to the catheter. As such, the embodiment shown in Figure 61, provides 1-to-1 actuation of the clasp 130 by movement of the slider 6104.

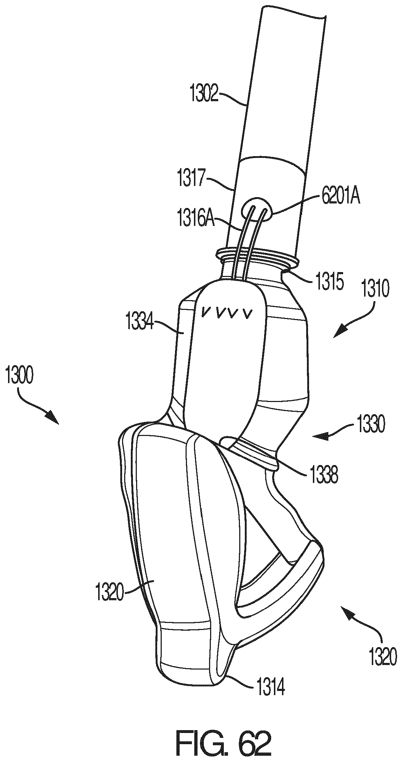

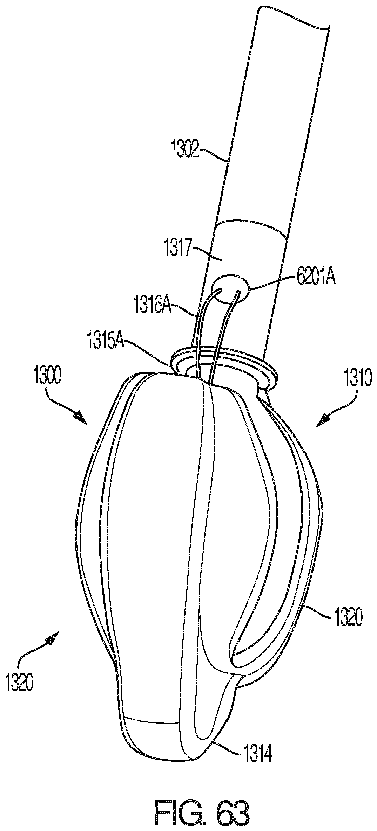

Referring now to Figures 62-64, an exemplary embodiment of a delivery system and a valve repair device 1300 wherein a portion of the device 1300 fits within a coupler 1317 at the distal end of a delivery catheter 1302. The device 1300 can take a wide variety of different forms. For example, the device 1300 can be any of the devices described herein, any of the devices that are described in a patent or patent application that is referenced herein, or any other known device for repairing a native heart valve, such as a mitral valve or a tricuspid valve. A portion of the device 1300 can fit within a coupler 1317 in a variety of different ways. In one embodiment, a portion of the coupler 1317 can fit within a portion of the device.

Referring to Figures 62-64, the illustrated example coupler 1317 has at least one line opening 6201A, 6201B. Clasp actuation lines 1316A, 1316B extend from the proximal end of the delivery catheter 1302, through the coupler 1317, and outward through the line openings 6201A, 6201B. At which point, the clasp actuation lines 1316A, 1316B can be secured to the clasps 1330, such as on the movable arm 1334 of the clasps 1330.

Referring to Figure 62, the clasps 1330 can be closed by reducing the tension to the actuation lines 1336A, 1336B attached to the moveable arms 1334. As a result, the moveable arms 1334 to flex, articulate, or pivot on the joint portions 1338 towards the fixed arms 1332 of the device 100. The paddles 1320 can be closed by retracting the control wire 112 (not shown in Figures 62-64) in any of the manners described herein.

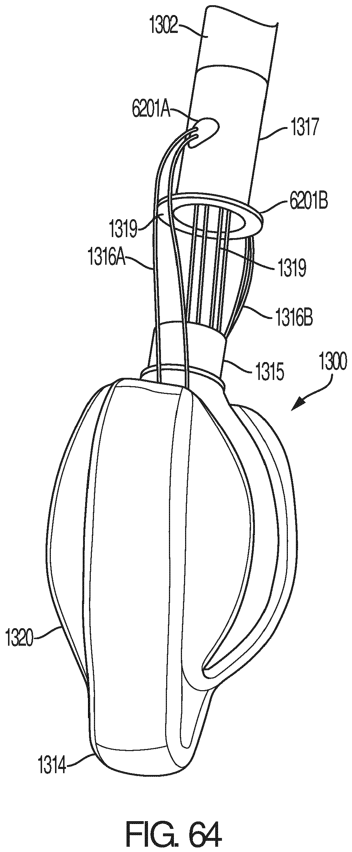

As shown in Figure 64, the device 1300 can be released from the coupler 1317 such as by retracting an activation wire (not shown in figures 62-64) into the coupler 1317 from within the device 1300. The device may remain attached to the delivery catheter 1302 by a plurality of coupling tethers 1319 which extend from the distal end of the delivery catheter 1302 through the coupler 1317 and attach to the device 1300, such as to the collar 1315. The device 1300 may remain tethered to the delivery catheter 1302 as a means of testing the positioning of the device 1300 prior to final detachment. In some embodiments, the coupling tethers 1319 can be inserted through a plurality of holes within the top or upper portion of the coupler 1317. In some embodiments, the coupling tethers 1319 can be secured to the device 1300 by inserting the tethers 1319 through a plurality of holes within a surface of the collar 1315.

The tethers 1319 can have a wide variety of different configurations. For example, the four lines illustrated by Figure 64 can be two tethers 1319. Each tether 1319 extends through the catheter 1302, loops through the device 1300 to couple the tether to the device and extends back through the catheter. However, the tethers can have a wide variety of different routing configurations.

In one exemplary embodiment, the coupler 1317 and the collar 1315 can be keyed to prevent relative rotation between the coupler 1317 (and attached catheter 1305) and the collar 1315 (and attached device 1300). The coupler 1317 and collar 1315 can be keyed in a wide variety of different ways. For example, the coupler 1317 and the collar 1315 can have complementary shapes that cannot rotate relative to one another when the collar is inserted into the coupler or vice versa.

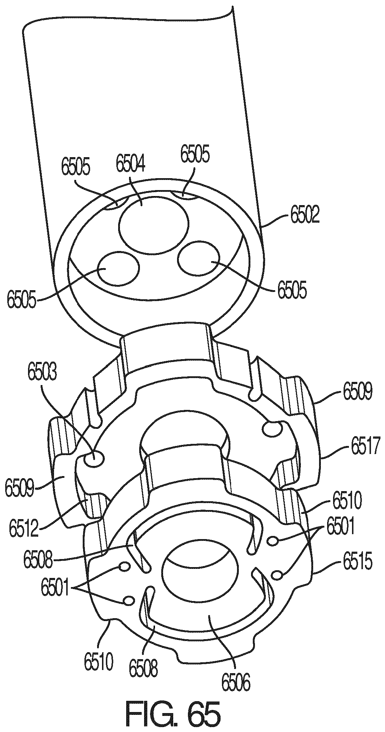

Referring now to Figure 65, an exemplary keyed coupler 6517 and collar 6515 is illustrated. In this Figure, a catheter 6502 and the coupler 6517 are depicted in an exploded view but would typically be attached. The distal end of the catheter 6502 comprises a plurality of tether passages 6505 and an actuation element passage 6504. The coupler 6157 comprises a wire passage 6507 aligned with the actuation element passage 6504 of the catheter 6502, a plurality of tether passages 6503, and a plurality of keying features 6509. The coupler 6157 has an inset portion 6512 with a similar or substantially similar profile as the shape of the collar 6515. The collar 6515 fits within the inset portion 6512 of the collar 6517. The collar 6515 comprises a wire passage 6505 which aligns with the wire passages 6504, 6507 of the catheter 6502 and the coupler 6517, respectively. The collar also includes a plurality of tether passages 6501, and at least one orifice 6508 for the termination and connection of the coaption element (not shown). In exemplary embodiments, the orifices 6508 are C-shaped, but they can also be circular, S-shaped, or any other configuration.

In the illustrated exemplary embodiment of Figure 65, there are four tether passages 6501 within the collar 6515. The collar 6515 has a plurality of keying features 6510. The keying features 6510 of the collar 6515 fit within the inset portion 6512 of the coupler 6517 such that the collar 6515 is prevented from rotating within the coupler 6517. The catheter 6502, coupler 6517, and a collar 6515 are designed to receive an actuation element (not shown) through the wire passages 6504, 6507, 6506, respectively, in order to attach, transport, and deploy a device (not shown). In exemplary embodiments, a plurality of tethers (not shown) can be inserted through the tether passages 6505, 6503, 6501 of the catheter 6502, coupler 6517 and collar 6515, respectively. The illustrated embodiment prevents rotation of the device (not shown) and the catheter 6502 in relation to one another as a result of the keyed fit of the collar 6515 within the coupler 6517.

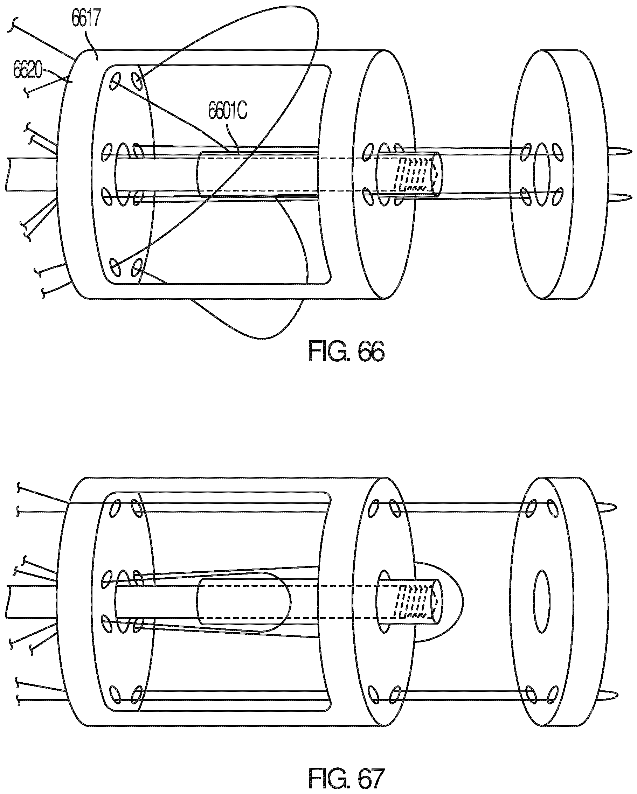

The couplers and collars shown and described in the present application can be tethered together in a variety of different ways. Referring now to Figure 66, an actuation element 6612, a collar 6615, a coupler 6617, two clasp actuation lines 6616, and two coupling tethers 6619 are depicted according to one embodiment. The routing of the coupling tethers 6619 and clasp actuation lines 6616, as depicted in Figure 66, prevents unintended flexing of the catheter due to tension applied by the tethers by placing the tether passages close to the center of the coupler 6617 and the collar 6615.

The collar 6615 includes a first tether passage 6604A, a second tether passage 6604B, a third tether passage 6604C, and a fourth tether passage 6604D, and a wire passage 6650 extending through the collar 6615. In the illustrated embodiment, the first, second, third, and fourth tether passages 6604A, 6604B, 6604C, 6604D are circular, and located equidistant or substantially equidistant from one another and equidistant to the wire passage 6650 in the center of the collar 6615. However, the first, second, third, and fourth tether passages 6604A, 6604B, 6604C, 6604D can have any shape or location.

The coupler 6617 is generally cylindrical with a top portion 6620 and a bottom portion 6621 which define two cutout windows 6605A, 6605B therebetween. The coupler 6617 includes a first tether passage 6602A, a second tether passage 6602B, a third tether passage 6602C, and a fourth tether passage 6602D, a wire passage 6646, and a first line passage 6601A, second line passage 6601B, third line passage 6601C, and fourth line passage 6601D extending through the top portion 6620. The first, second, third, and fourth tether passages 6602A, 6602B, 6602C, 6602D are located equidistant or substantially equidistant from one another and equidistant to the wire passage 6646 in the center of the top portion 6620 of the coupler 6617. The first, second, third, and fourth line passages 6601A, 6601B, 6601C, 6601D are located equidistant or substantially equidistant from one another and equidistant to the wire passage 6646, such that the first, second, third, and fourth line passages 6601A, 6601B, 6601C, 6601D are radially farther from the wire passage 6646 than the first, second, third, and fourth tether passages 6602A, 6602B, 6602C, 6602D, nearer the outer edge of the top portion 6620 of the coupler 6617.

The coupler 6617 also includes a fifth tether passage 6603A, a sixth tether passage 6603B, a seventh tether passage 6603C, an eighth tether passage 6603D, and a wire passage 6648 extending through the bottom portion 6621. The fifth, sixth, seventh, and eighth tether passages 6603A, 6603B, 6603C, 6603D located equidistant or substantially equidistant from one another and equidistant to the wire passage 6648 in the center of the bottom portion 6621 of the coupler 6617. The wire passage 6646 of the top portion 6620 corresponds to the wire passage 6648 of the bottom portion 6621. The first and the third tether passages 6602A, 6602C are radially opposite the wire passage 6646 from the second and the fourth tether passages 6602B, 6602D on the top portion 6620 of the coupler 6617, and the fifth and the seventh tether passages 6603A, 6603C are radially opposite the wire passage 6648 from the sixth and the eighth tether passages 6603B, 6603D on the bottom portion 6621 of the coupler 6617.

In the illustrated embodiment, the first, second, third, and fourth tether passages 6602A, 6602B, 6602C, 6602D of the top portion 6620 of the coupler 6617 correspond to the fifth, sixth, seventh, and eighth tether passages 6603A, 6603B, 6603C, 6603D of the bottom portion 6621 of the coupler 6617, respectively, and the first, second, third, and fourth tether passages 6604A, 6604B, 6604C, 6604D of the collar 6615, respectively.

As shown in Figure 66, the collar 6615 and the coupler 6617 can be coupled together via a first coupling tether 6619A and a second coupling tether 6619B with the actuation element 6612 extending through the wire passages 6646, 6648 of the coupler 6617 and the wire passage 6650 of the collar 6615. The actuation element 6612 can extend into the implantable prosthetic device and engage a cap to open the device as previously described herein. The first coupling tether 6619A passes through the first tether passage 6602A of the coupler 6617, through the fifth tether passage 6603A of the coupler 6617, through the first tether passage 6604A of the collar 6615, around the bottom of the collar 6615, up through the second tether passage 6604B of the collar 6615, through the sixth tether passage 6603B of the coupler 6617, and through the second tether passage 6602B of the coupler 6617. The second coupling tether 6619B passes through the third tether passage 6602C of the coupler 6617, through the seventh tether passage 6603C of the coupler 6617, through the third tether passage 6604C of the collar 6615, around the bottom of the collar 6615, up through the fourth tether passage 6604D of the collar 6615, through the eighth tether passage 6603D of the coupler 6617, and through the fourth tether passage 6602D of the coupler 6617.

As shown in Figure 66, a first clasp actuation line 6616A passes through the first line passage 6601A, around the coupling tethers 6619A, 6619B and the actuation element 6612, through a second cutout window 6605B in the coupler 6617, affixes to the clasps of an implantable prosthetic device (not shown), passes back through the second cutout window 6605B, and through the third line passage 6601C. A second clasp actuation line 6616B passes through the second line passage 6601B, around the coupling tethers 6619A, 6619B and the actuation element 6612, through a first cutout window 6605A in the coupler 6617, affixes to the clasps of an implantable prosthetic device (not shown), passes back through the first cutout window 6605A, and through the fourth line passage 6601D. In the illustrated embodiment, the first cutout window 6605A is radially opposite the wire passages 6646, 6648 from the first and the third line passages 6601A, 6601C, and the second cutout window 6605B is radially opposite the wire passages 6646, 6648 from the second and fourth line passages 6601B, 6601D.

The actuation element 6612, the collar 6615, the coupler 6617, the two coupling tethers 6619, and the two clasp actuation lines 6616 can be used to position and reposition an implantable prosthetic device in the native valve (e.g., the native mitral valve MV, native tricuspid valve, etc.) of the heart H, as previously described herein.

Referring now to Figure 67, an actuation element 6712, a collar 6715, a coupler 6717, two clasp actuation lines 6716, and two coupling tethers 6719 are depicted according to one embodiment. The routing of the coupling tethers 6719 and clasp actuation lines 6716, as depicted in Figure 67, prevent flexing of the catheter due to tension applied by the clasp actuation lines by placing the clasp actuation line passages close to the center of the coupler 6617 and the collar 6615.

The collar 6715 includes a first tether passage 6704A, a second tether passage 6704B, a third tether passage 6704C, and a fourth tether passage 6704D, and a wire passage 6750 extending through the collar 6715. In the illustrated embodiment, the first, second, third, and fourth tether passages 6704A, 6704B, 6704C, 6704D are circular, and located equidistant or substantially equidistant from one another, and equidistant to the wire passage 6650, along the outer edge of the collar 6715. However, the first, second, third, and fourth tether passages 6704A, 6704B, 6704C, 6704D can have any shape or location.

The coupler 6717 is generally cylindrical with a top portion 6720 and a bottom portion 6671 which define two cutout windows 6705A, 6705B therebetween. The coupler 6717 includes a first tether passage 6702A, a second tether passage 6702B, a third tether passage 6702C, and a fourth tether passage 6702D, a wire passage 6746. and the coupler also includes a first line passage 6701A, second line passage 6701B, third line passage 6701C, and fourth line passage 6701D extending through the top portion 6720. The first, second, third, and fourth tether passages 6702A, 6702B, 6702C, 6702D are located equidistant or substantially equidistant from one another and equidistant to the wire passage 6746 in the top portion 6720 of the coupler 6717. The first, second, third, and fourth line passages 6701A, 6701B, 6701C, 6701D are located equidistant or substantially equidistant from one another and equidistant to the wire passage 6746. The first, second, third, and fourth line passages 6701A, 6701B, 6701C, 6701D are radially nearest the wire passage 6746 compared to the first, second, third, and fourth tether passages 6702A, 6702B, 6702C, 6702D, which are located nearer the outer edge of the top portion 6720 of the coupler 6717.

The coupler 6717 also includes a fifth tether passage 6703A, a sixth tether passage 6703B, a seventh tether passage 6703C, an eighth tether passage 6703D, and a wire passage 6748 extending through the bottom portion 6721. The fifth, sixth, seventh, and eighth tether passages 6703A, 6703B, 6703C, 6703D located equidistant or substantially equidistant from one another and equidistant to the wire passage 6748 and near the outer edge of the bottom portion 6721 of the coupler 6717. The wire passage 6746 of the top portion 6720 corresponds to the wire passage 6748 of the bottom portion 6721. The first and second tether passages 6702A, 6702B are radially opposite the wire passage 6746 from the third and the fourth tether passages 6702C, 6702D on the top portion 6720 of the coupler 6717. The fifth and the sixth tether passages 6703A, 6703B are radially opposite the wire passage 6748 from the seventh and the eighth tether passages 6703C, 6703D on the bottom potion 6721 of the coupler 6717.

In the illustrated embodiment, the first, second, third, and fourth tether passages 6702A, 6702B, 6702C, 6702D of the top portion 6720 of the coupler 6717 correspond to the fifth, sixth, seventh, and eighth tether passages 6703A, 6703B, 6703C, 6703D of the bottom portion 6621 of the coupler 6717, respectively, and the first, second, third, and fourth tether passages 6704A, 6704B, 6704C, 6704D of the collar 6715, respectively.

As shown in Figure 67, the collar 6715 and the coupler 6717 can be coupled together via a first coupling tether 6719A and a second coupling tether 6719B with the actuation element 6712 extending through the wire passages 6746, 6748 of the coupler 6717 and the wire passage 6750 of the collar 6715. The actuation element 6712 can extend into the implantable prosthetic device and engage a cap to open the device as previously described herein. The first coupling tether 6719A passes through the first tether passage 6702A of the coupler 6617, through the fifth tether passage 6703A of the coupler 6717, through the first tether passage 6704A of the collar 6715, around the bottom of the collar 6715, up through the second tether passage 6704B of the collar 6715, through the sixth tether passage 6703B of the coupler 6717, and through the second tether passage 6702B of the coupler 6717. The second coupling tether 6719B passes through the third tether passage 6702C of the coupler 6717, through the seventh tether passage 6703C of the coupler 6717, through the third tether passage 6704C of the collar 6715, around the bottom of the collar 6715, up through the fourth tether passage 6704D of the collar 6715, through the eighth tether passage 6703D of the coupler 6717, and through the fourth tether passage 6702D of the coupler 6717.

As shown in Figure 67, a first clasp actuation line 6716A passes through the first line passage 6701A, through a first cutout window 6705A in the coupler 6717, affixes to the clasps of an implantable prosthetic device (not shown), passes back through the first cutout window 6705A, and through the fourth line passage 6701D. A second clasp actuation line 6716B passes through the second line passage 6701B, through a second cutout window 6705B in the coupler 6717, affixes to the clasps of an implantable prosthetic device (not shown), passes back through the second cutout window 6705B, and into the passage 6701D. In the illustrated embodiment, the first cutout window 6705A is radially opposite the wire passages 6746, 6748 from the second and the fourth line passages 6701B, 6701D, and the second cutout window 6705B is radially opposite the wire passages 6746, 6748 from the first- and third-line passages 6701A, 7601C.

The actuation element 6712, the collar 6715, the coupler 6717, the two coupling tethers 6719, and the two clasp actuation lines 6716 can be used to position and reposition an implantable prosthetic device in the native valve (e.g., the native mitral valve MV, native tricuspid valve, etc.) of the heart H, as previously described herein.

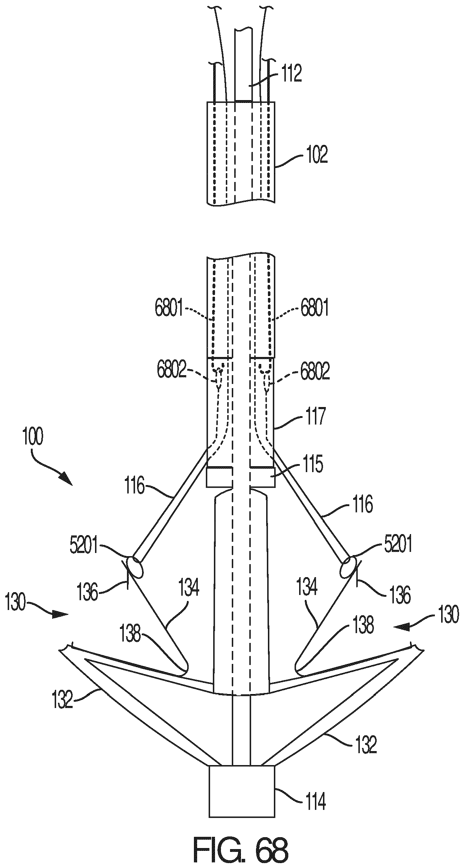

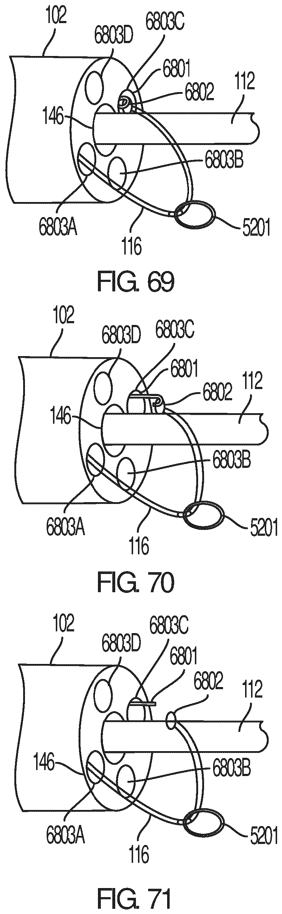

Figures 68-77 illustrate an exemplary embodiment of a releasable connection between the clasp control lines 116 and a device 100. However, the releasable connection illustrated by Figures 69-77 can also be used to connect the tether(s) to the device. The device 100 can be any of the devices described herein, any of the devices that are described in a patent or patent application that is referenced herein, or any other known device for repairing a native heart valve, such as a mitral valve or a tricuspid valve.

Figure 68 illustrates an example implantable prosthetic device 100, a delivery catheter 102, an actuation element 112, a collar 115, and a coupler 117. The device 100 can incorporate any of the features of any of the devices 100, 400, 400a, 500, 600, 700, 800, 900, 1000, and 1100 described herein. The illustrated device 100 comprises a cap 114 (but could be another type of attachment portion) and clasps 130 (which can optionally include one or more of a base or fixed arm 132, a moveable arm 134, barbs or means for securing 136, and a joint portion 138. In one embodiment, the clasp actuation lines 116 are connected to the moveable arms 134. In the illustrated example, the lines 116 extend through the delivery catheter 102 and the coupler 117, through a ring, loop, or other opening 5201 of the arm 134, back through the coupler 117, and secured (directly or indirectly; e.g., via a loop 6802, ring, hook, latch, and/or other similar means) to a clasp actuation element 6801 (e.g., a clasp actuation wire, clasp actuation shaft, clasp actuation rod, etc.). In some embodiments, the clasp actuation element is a hooked rod or wire. The clasps 130 can be opened by applying tension to the actuation lines 116 attached to the moveable arms 134, thereby causing the moveable arms 134 to pivot on the joint portions 138. In some embodiments, tension is applied directly to the actuation lines 116. In some embodiments, tension is applied to the actuation lines 116 by pulling on the clasp actuation elements 6801.

As shown in Figures 69-71, the catheter 102 can include a first line passage 6803A, a second line passage 6803B, a third line passage 6803C, and a fourth line passage 6803D in the top or upper portion of the collar 115, and a wire passage 146 extending through the catheter 102. The actuation element 112 can be inserted through the wire passage 146 of the catheter 102. A first clasp actuation line 116 can be inserted through the first line passage 6803A of the catheter 102, through the ring 5201, and secured to a clasp actuation element 6801 (e.g., a shaft, a rod, a wire, a hooked rod, hooked wire, etc.) directly or indirectly (e.g., , by a loop 6802, a ring, a hook, latch, or similar means). This can be within the third line passage 6803C of the catheter 102. A second clasp actuation line 116 can be inserted through the second line passage 6803B of the catheter 102, through the ring 5201, and secured to a clasp actuation element 6801 (e.g., a hooked rod, hooked wire, etc.) directly or indirectly (e.g., by a loop 6802, ring, hook, latch, or similar means. This can be within the fourth line passage 6803D of the catheter 102.

Referring to Figure 69, the clasp actuation element 6801 is shown configured as a hooked rod/wire 6801 comprising a straight rod or substantially straight rod made of such a material that the distal end can be temporarily configured into a hook by bending the distal end back into the line passage from which the clasp actuation element 6801 originates. The clasp actuation element or hooked rod 6801 secures the clasp actuation line 116 until such time as the user advances the clasp actuation element or hooked rod 6801 past the distal end of the catheter 102. As shown in Figure 70 and 71, once the clasp actuation element or hooked rod 6801 exits the line passage 6803C, the rod 6801 is free to return to a straightened or substantially straight configuration, and the loop 6802 at the end of the clasp actuation line 116 is released from the end of the clasp actuation element or hooked rod 6801. At which point, the clasp actuation line 116 can be removed from the ring 5201 on the clasp 130.

By diametrically opposing line passages 6803A from 6803C, and line passages 6803B from 6803D, the tensile forces applied to the lines cancel one another out. This prevents unintended bending of the catheter 102 due to pulling on the clasp actuation lines 116. In some example embodiments, the line passages 6803A, 6803C, 6803B and 6803D are not diametrically opposed.

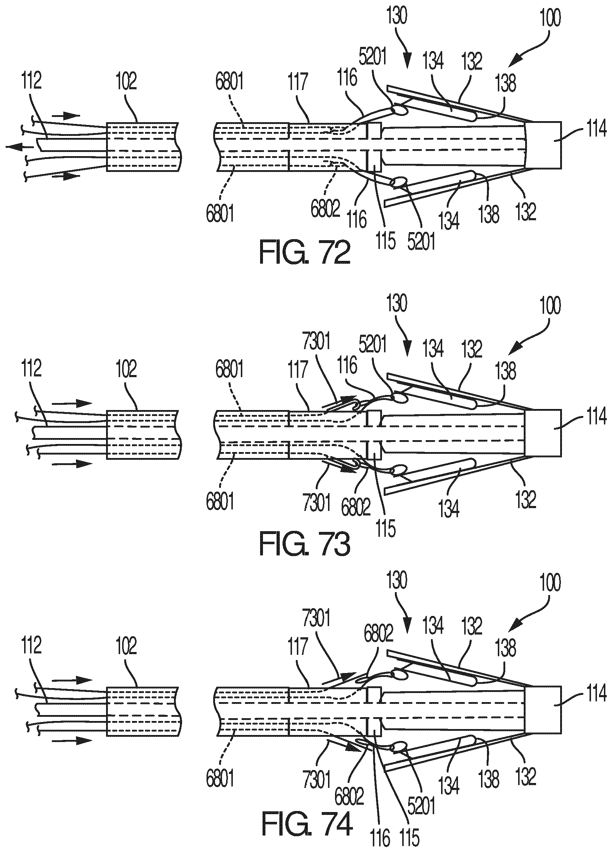

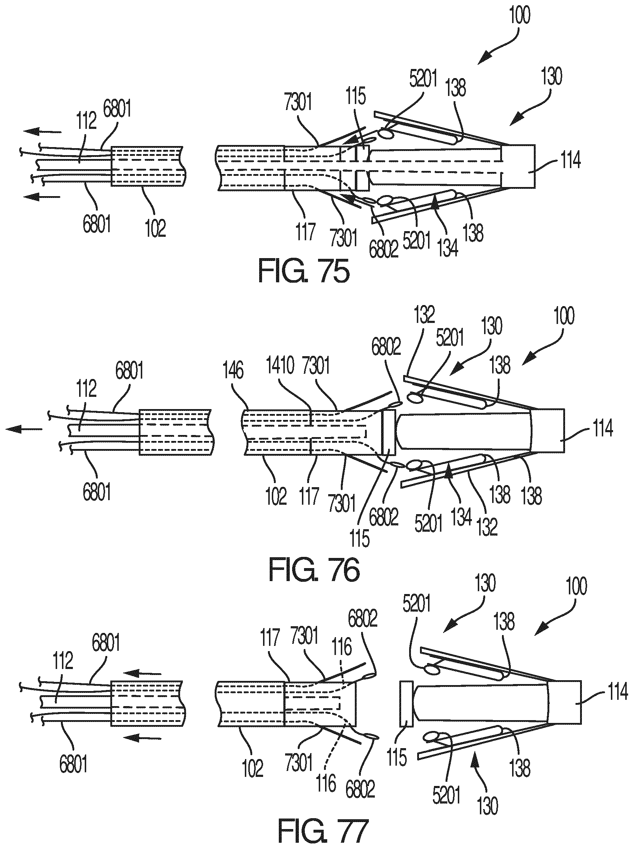

Referring now to Figures 72-77, the device 100 can be deployed from the delivery catheter 102, the coupler 117, the actuation element 112, and the clasp actuation lines 116. The barb clasps 130 can be closed by lessening the tension to the actuation lines 116 attached to the moveable arms 134 by advancing the clasp actuation elements or hooked rods 6801 towards the distal end of the coupler 117, thereby causing the moveable arms 134 to flex, articulate, or pivot on the joint portions 138 towards the fixed arms 132 of the device 100. As shown by comparing Figure 72 and Figure 76, the device can be uncoupled from the actuation element 112.

As shown in Figure 73, the clasp actuation elements or hooked rods 6801 can be advanced through the coupler 117 until it reaches one of a plurality of orifices 7301 on either side of the coupler 117, at which point the distal ends of the clasp actuation elements or hooked rods 6801 will exit the coupler 117 through one of the orifices. The clasp actuation elements or hooked rods 6801 are held within the catheter 102 and/or the coupler 117 in a hooked configuration with spring tension with the inner wall of the catheter 102 and/or the coupler 117. Once the clasp actuation elements or hooked rods 6801 exit the catheter 102 and/or the coupler, such as through the orifice 7301 or other position where the hooked rod becomes unconstrained, the spring tension is released and the clasp actuation elements or rods 6801 can expand outward.

As shown in Figure 74, once the clasp actuation elements or hooked rods 6801 expand outward, such as from the orifices 7301, the elements or rods 6801 return to a straight or substantially straight configuration. The straightening of the elements or rods 6801 releases the looped ends 6802 of the clasp actuation lines 116.

As shown in Figure 75, the user can pull the clasp actuation lines 116 towards the proximal end of the catheter. The clasp actuation lines 116 can exit through the rings or other openings 5201 on the clasps 130 and can travel through the orifices 7301 in the coupler 117. Referring to Figure 76, the actuation element 112 can be retracted from the device 100 and the collar 115 and moved toward the coupler 117 and the delivery catheter 102. The actuation element 112 can be retracted such that the end of the actuation element 112 is positioned within the wire passage 146 of the coupler 117. In such a position, the delivery catheter 102 and the coupler 117 can be moved away from the device 100.

As shown in Figure 77, the device 100 is shown in the fully closed and deployed condition. The delivery catheter 102, the coupler 117, and the actuation element 112 have been retracted and the clasps 130 remain in a fully closed position. Once deployed, the device 100 can be maintained in the fully closed position in a variety of ways. For example, the device 100 can be maintained in the fully closed position in any of the ways described relating to Figure 14M. In another example embodiment, the paddles of the device can be configured to partially open and close with the beating of the heart, while the clasps remain in their closed configuration.

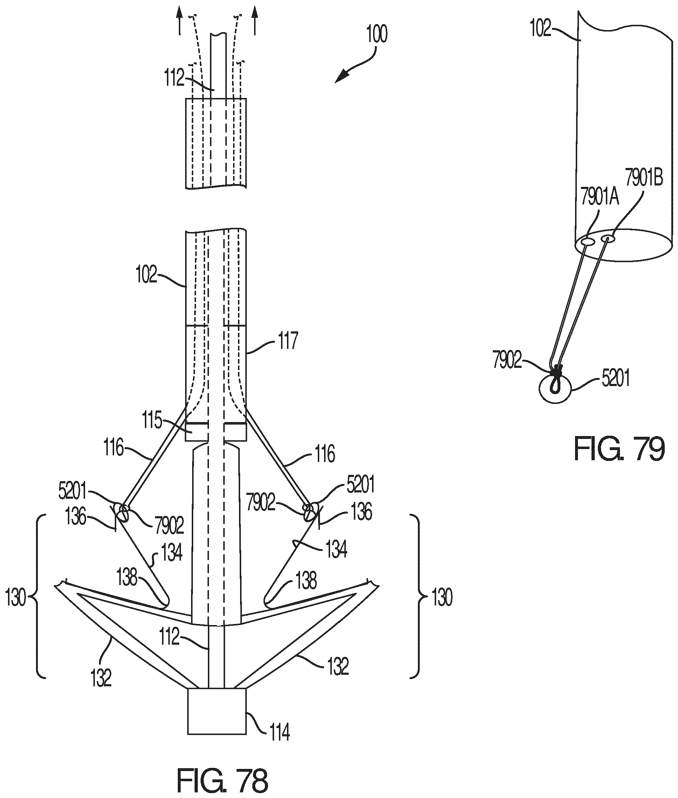

Figures 78-89 illustrate an exemplary embodiment of a releasable connection between the clasp control lines 116 and a device 100. However, the releasable connection illustrated by Figures 78-89 can also be used to connect the tether(s) to the device. The device 100 can be any of the devices described herein, any of the devices that are described in a patent or patent application that is referenced herein, or any other known device for repairing a native heart valve, such as a mitral valve or a tricuspid valve.

Referring to Figure 78, an example implantable prosthetic device 100, a delivery catheter 102, an actuation element 112, a collar 115, and a coupler 117 are illustrated. The device 100 can incorporate any of the features of any of the devices 100, 400, 400a, 500, 600, 700, 800, 900, 1000, and 1100 described herein. In some embodiments, the device 100 comprises a cap 114 (or other attachment portion) and clasps 130 (which can include one or more of a base or fixed arm 132, a moveable arm 134, barbs 136, and/or a joint portion 138). In one embodiment, the clasp actuation lines 116 are routed through the catheter 102 and coupler 117 towards the device 100, and then back through the coupler 117 and catheter 102 towards the proximal end of the catheter, forming a loop at the end of the clasp actuation lines 116. The loop can be secured to the rings 5201 on the movable arms 134 of the clasps 130. The looped clasp actuation line 116 can be secured to the ring 5201 by tying it into a knot 7902, such that the knot 7902 can be tightened by pulling on a first end 7803A of the clasp actuation line 116 and loosened by pulling on a second end 7803B of the clasp actuation line 116. In some embodiments, the knot 7902 is a unidirectional knot, such as a highwayman's hitch, tumble hitch, or equivalent. In some embodiments, the knot 7902 can be secured to the actuation element 112 instead of the clasp (See e.g. Figure 52). The clasps 130 can be opened by applying tension to the actuation lines 116 attached to the moveable arms 134, thereby causing the moveable arms 134 to flex, articulate, or pivot on the joint portions 138.

As shown in Figure 79, the catheter 102 can include a first line passage 7901A and a second line passage 7901B. A first clasp actuation line 116 can be routed through the first line passage 7901A of the catheter 102 towards the device 100 and back through the second line passage 7901B of the catheter 102. Sufficient slack is allowed in the line to create a loop such that a knot 7902 can be formed around the ring, loop, or other clasp opening 5201. The knot secures the clasp actuation line 116 to the ring, loop, or other clasp opening 5201.

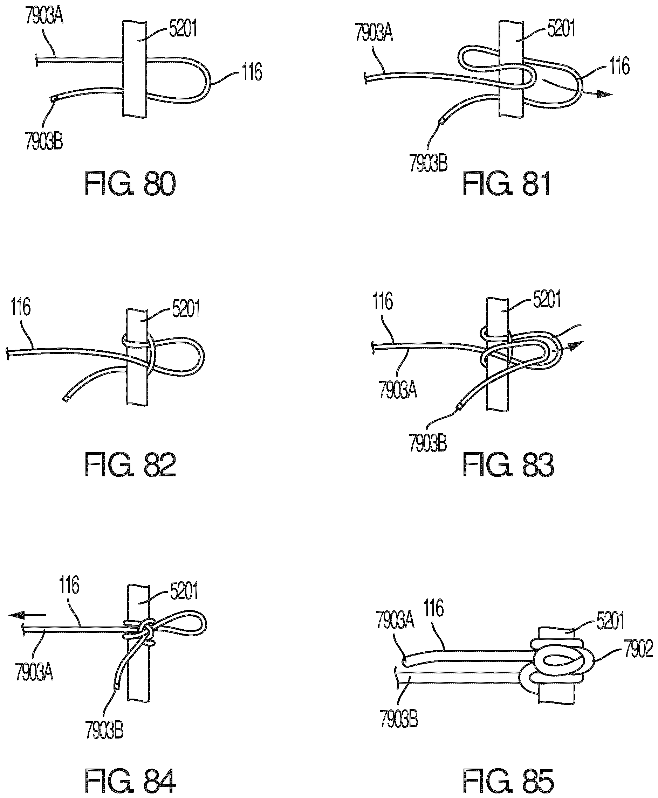

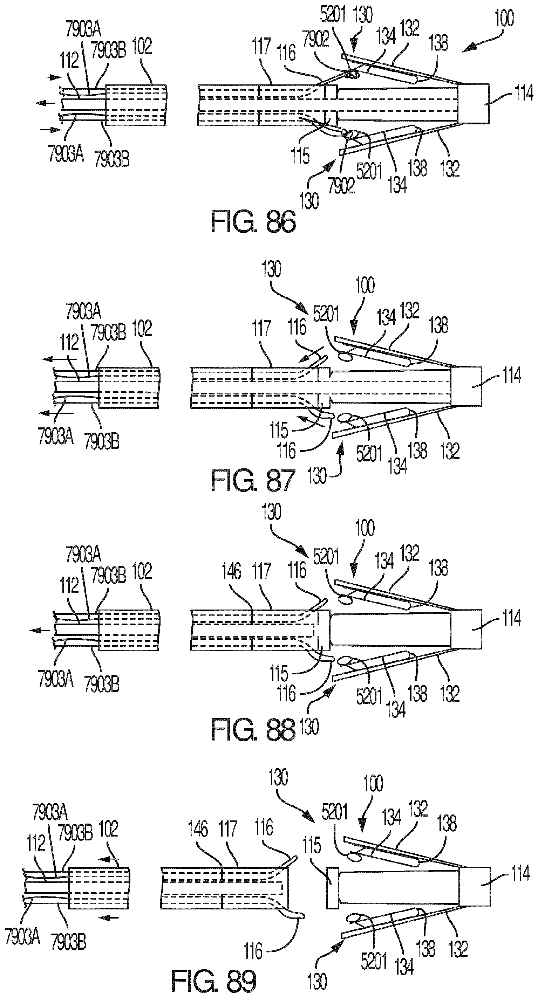

The unidirectional knot can be formed in a variety of different ways. Any knot that tightens when a first end is pulled and loosens when a second end is pulled can be used. Figures 80-84 show the steps of forming a highwayman's hitch knot on the ring, loop, or other attachment portion 5201 of the clasp. Figure 86 illustrates a tumble hitch knot on the ring, loop, or other attachment portion 5201 of the clasp.

Referring now to Figures 86-89, the device 100 can be deployed from the delivery catheter 102, the coupler 117, the actuation element 112, and the clasp actuation lines 116. The barb clasps 130 can be closed by lessening the tension to the actuation lines 116 attached to the moveable arms 134, thereby causing the moveable arms 134 to flex, articulate, or pivot on the joint portions 138 towards the fixed arms 132 of the device 100. As can be seen by comparing Figure 86 and Figure 89, the device can be uncoupled from the actuation element 112.

As shown in Figure 87, the user can pull on the second end 7903B of each clasp actuation line 116 loosening and subsequently untying the knot 7902 from the ring 5201 on the clasps 130. Once untied, the claps actuation lines 116 can be pulled through the rings or other attachment portions 5201 of the clasp. Pulling on the first end 7903A of each clasp actuation line 116 pulls on the moveable arm of the clasp, without untying the knot. When the paddles are in the open position (Figure 78), the first end 7903A of the clasp's actuation lines are used to open the clasps.

As shown in Figure 88, the actuation element 112 can be retracted from the device 100 and the collar 115, toward the coupler 117 and the delivery catheter 102. The actuation element 112 can be retracted such that the end of the actuation element 112 is positioned within the wire passage 146 of the coupler 117. In such a position, the delivery catheter 102 and the coupler 117 can be retracted from the device 100.

As shown in Figure 89, the device 100 is shown in the fully closed and deployed condition. The delivery catheter 102, the coupler 117, and the actuation element 112 have been retracted and the clasps 130 remain in a fully closed position. Once deployed, the device 100 can be maintained in the fully closed position in a variety of ways. For example, the device 100 can be maintained in the fully closed position in any of the ways described relating to Figure 14M. In another example embodiment, the paddles of the device can be configured to partially open and close with the beating of the heart, while the clasps remain in their closed configuration.

The collars and/or caps of the devices disclosed herein can be attached to a coaption element and/or paddles or other anchors in a wide variety of different ways. In some exemplary embodiments, the coaption element and/or the paddles/anchors are made from wire strands. The wire strands can be welded or otherwise adhered to the collar and/or cap to attach the coaption element and/or paddles to the collar and/or cap. The wire strands can be welded or otherwise adhered to the collar and/or cap in a wide variety of different ways. For example, individual wires can be welded or otherwise adhered to the collar and/or cap or the wires can be bunched up and welded or adhered to the collar and/or cap.

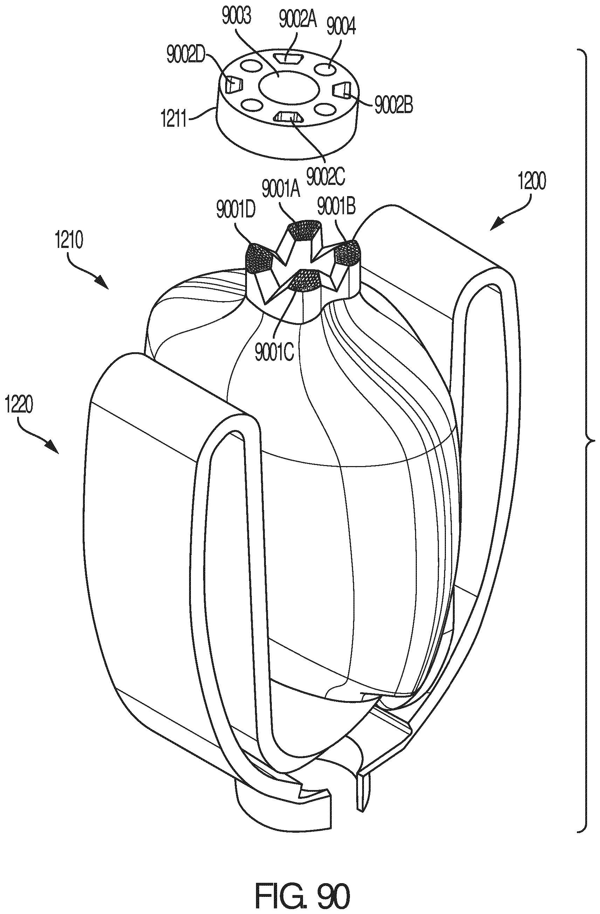

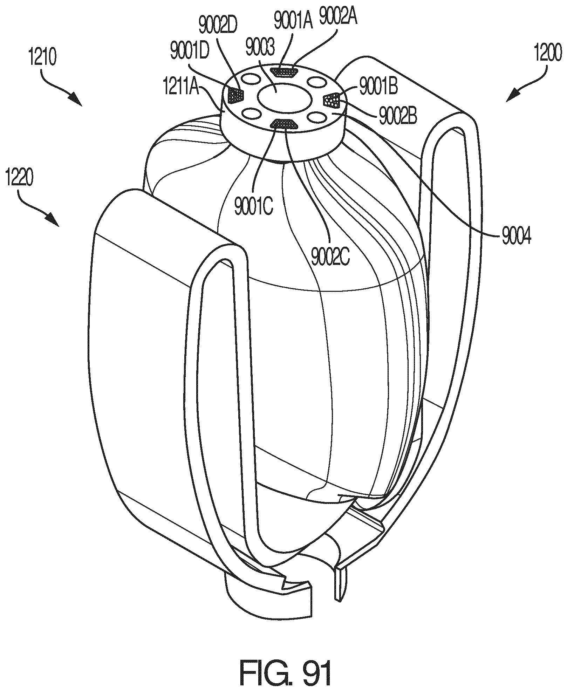

Figures 90 and 91 illustrate an exemplary embodiment where wires of a coaption element are bunched, inserted into openings of a cap, and welded or otherwise adhered to the cap to attach the cap to the coaption portion. Figure90 shows a coaption element 1210 and paddles 1220 of an exemplary device 1200. The coaption element 1210 and the paddles 1220 can be made from a wide variety of different materials. The coaption element 1210 and paddles 1220 may be formed from a material that may be a metal fabric, such as a mesh, woven, braided, electro spun or formed in any other suitable way or a laser cut or otherwise cut flexible material. The material may be cloth, shape-memory alloy wire - such as Nitinol- to provide shape setting capability, or any other flexible material suitable for implantation in the human body.

Referring to Figure 90, in one such exemplary embodiment the coaption element 1210 is made from a braided mesh of metal wires, such as a braided mesh of nitinol wires. The use of shape memory material, such as braided Nitinol wire mesh, for the construction of the coaption element 1210 results in a coaption element 1210 that is self-expandable, flexible in all directions, and/or results in low strains when the coaption element 1210 is crimped and/or bent. The material can be a single piece, two halves joined together, or a plurality of sections or pieces that are fastened or joined together in any suitable manner, such as by welding, with adhesives, or the like.

As shown in Figure 90, ends of the material making up the coaption element 1210 can be joined together, such as by crimping, in a plurality of bundles, for example, a first bundle 9001A, a second bundle 9001B, a third bundle 9001C, and a fourth bundle 9001D. A proximal collar 1211 has a plurality of corresponding orifices, for example, a first orifice 9002A, a second orifice 9002B, a third orifice 9002C, and a fourth orifice 9002D. Referring to Figure 91, the proximal collar 1211can be secured to the coaption element 1210 by inserting each of the gathered bundles 9001A, 9001B, 9001C, and 9001D into the corresponding orifice 9002A, 9002B, 9002C, and 9002D, and joining the collar 1211 and the gathered bundles together in any suitable manner, such as by welding, with adhesives, or the like. The first proximal collar 1211 can also comprise an actuation element opening 9203 through which an actuation element can be inserted, and a plurality of tether holes 9004. In some exemplary embodiments, a chamfer along a top surface of the first proximal plate 1211 guides the actuation element into the top of the device 1200 during reattachment.

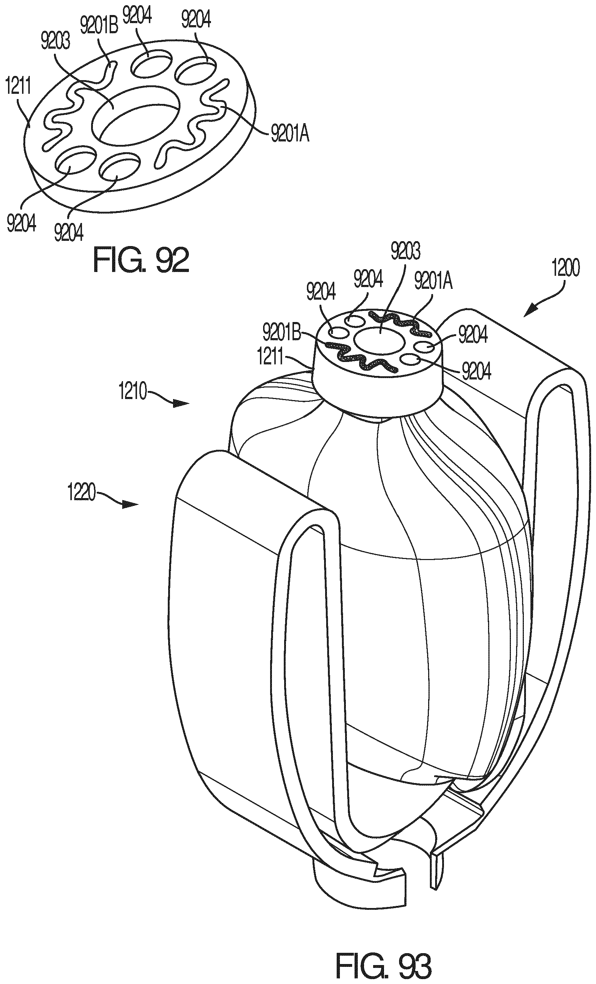

In some exemplary embodiments, wires are attached to a cap in a manner that makes room on the cap for tether passages. For example, a length and/or size of a wire end cutout on a portion of the cap can be increased to make room on the cap for tether passages. That is, increasing the length and/or size of a wire end cutout in a portion of the cap outside the tether passage area makes room for the tether passages. The length and/or size of the wire cutout in the cap can be increased in a variety of different ways. Figures 92 and 93 show an embodiment where a length of a wire end cutout on a portion of the cap is increased to make room on the cap for tether passages.

In the example illustrated by Figure 93, a proximal collar 1211 has two serpentine orifices 9201A, 9201B, an actuation element opening 9203, through which an actuation element can be inserted, and a plurality of tether holes 9204. The wire strands making up the coaption element 1210 can be individually inserted into the serpentine orifices 9201A, 9201B or the wire strands can be fastened or joined together, such as by crimping into bundles or rows and then inserting the bundles or rows into the serpentine orifices 9201A, 9201B. The wires of the coaption element 1210 within the serpentine orifices 9201A, 9201B of the proximal collar 1211 can be joined together by any suitable manner, such as by welding, with adhesives, or the like. In some exemplary embodiments, a chamfer along a top surface of the first proximal plate 1211 guides the actuation element into the top of the device 1200 during reattachment.

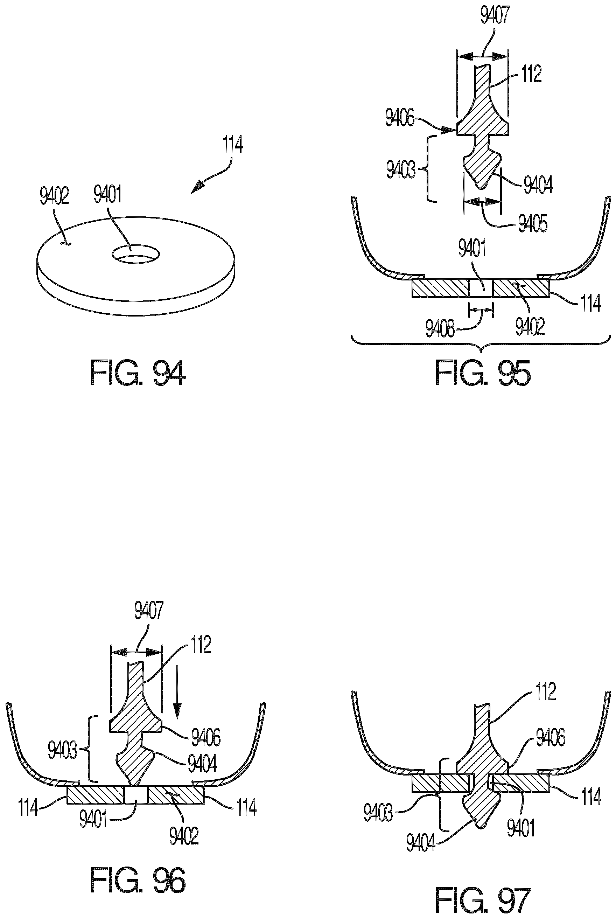

Figures 94-118 show various configurations for reattaching the actuation element 112 to the cap 114 or collar 115 of the device 100. A wide variety of different reattachment configurations can be used. As shown in Figures 95-97, the actuation element 112 can engage with the cap 114 of the device 100 and be held in place by a recapturing feature 9403 at the end of the actuation element 112. The recapturing feature 9403 can take a wide variety of different forms. The recapturing feature can be tapered, have one or more features that can flex inward and spring back outward, have cutting or impaling surfaces or features, and/or have guide surfaces. The recapturing features can be provided on the cap 114, the collar 115, the actuation element 112, and/or another recapturing component. The recapturing feature(s) can be made from a variety of different materials. In exemplary embodiments, the surface 9402 of the cap 114 and/or the recapturing features 9403 are made of durable material for mechanical locking such as a metal, a stiff polymer-based material, etc. Figure 94 shows an exemplary embodiment of a cap 114 with a single orifice 9401 through the surface 9402 of the cap 114. This embodiment can also be used as a collar 115.Figure 95 shows an exemplary embodiment of the proximate end of the actuation element 112 with a recapturing feature 9403. The recapturing feature 9403 comprises a tapered tip 9404 having a maximum diameter 9405. The recapturing feature has a shelf 9406 having a diameter 9407 that is as large or larger than the diameter 9405 of the tip 9404. The surface 9402 of the cap 114 has the orifice 9401. The orifice has a diameter 9408 that is smaller than the maximum diameter 9405 of the tip 9404 and the diameter 9407 of the shelf 9406.

As shown in Figure 96, the tip 9403 of the actuation element 112 can be advanced through the orifice 9401 in the surface 9402 of the cap 114. This advancement flexes or compresses the tip 9404 to allow the tip to pass through the orifice 9401. Advancement of the actuation element 112 is continued until the shelf 9406 is proximate to the surface 9402 of the cap 114.

As shown in Figure 97, the actuation element 112 is prevented from advancing further through the cap 114 because the shelf 9406 is wider than the orifice 9401. The actuation element 112 is not easily removed from the cap 114 due to the tapered tip 9404 having a maximum diameter 9405 slightly larger than the diameter 9408 of the orifice 9401. As such, the device is recaptured by the wire 112. In one exemplary embodiment, the recaptured device can be operated by the wire 112, catheter, coupler, and control lines in the same manner as any of the embodiments described herein.

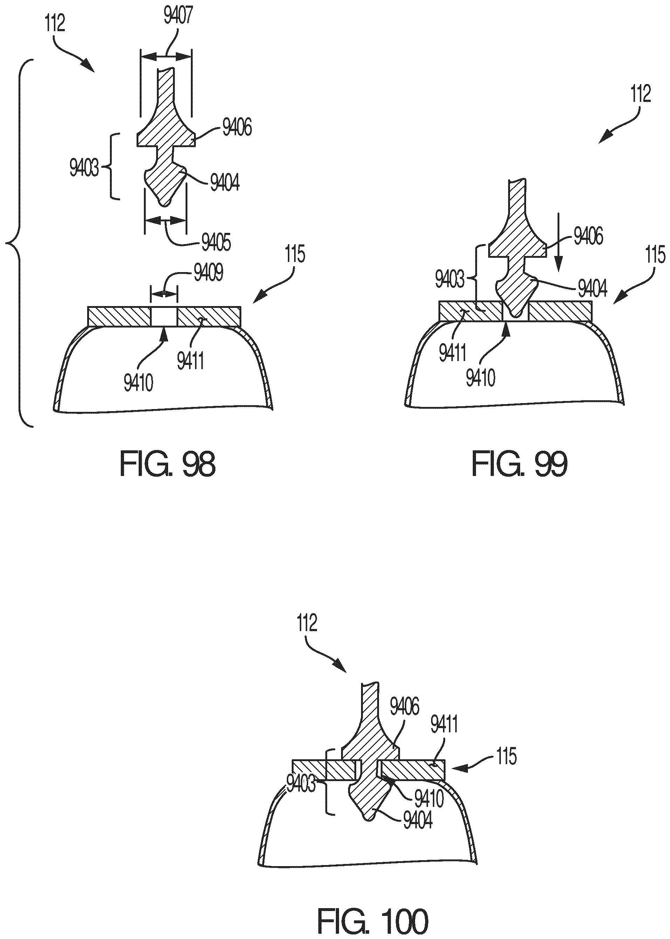

Figures 98-100 show an exemplary embodiment of the actuation element 112 reengaging with the collar 115 of the device 100. Figure 98 shows an exemplary embodiment of the proximate end of the actuation element 112 with a recapturing feature 9403. The recapturing feature 9403 comprises a tapered tip 9404 having a maximum diameter 9405. The wire also includes a shelf 9406 having a diameter 9407 that is larger than the diameter 9405 of the tip 9404. The surface 911 of the collar 115 has an orifice 9410 having a diameter 9409 that is smaller than the maximum diameter 9405 of the tip 9404 and that is smaller than the diameter 9407 of the shelf 9406.

As shown in Figure 99, the tip 9404 of the actuation element 112 can be advanced through the orifice 9410 in the surface 9411 of the collar 115 until the shelf 9406 is proximate to the surface 9411 of the collar 115. As shown in Figure 100, the actuation element 112 is prevented from advancing further through the collar 115, because the shelf 9406 is wider than the orifice 9410. The actuation element 112 is not easily removed from the collar 115 due to the tapered tip 9404 having a maximum diameter 9405 slightly larger than the diameter 9409 of the orifice 9410. As such, the device is recaptured by the wire 112.

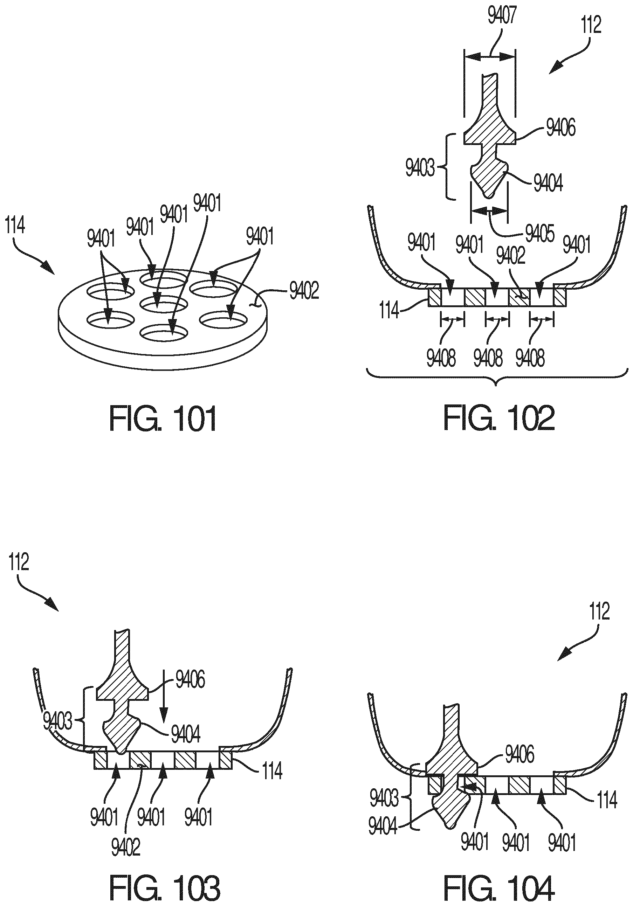

Figures 101-104 show an exemplary embodiment of an actuation element 112 engaging with an end cap 114 wherein the cap 114 has a plurality of orifices 9401. Figure 101 shows an exemplary embodiment of a cap 114 with a plurality of orifices 9401 through the surface 9402 of the cap 114. This embodiment can also be used as a collar 115. As shown in Figures 102-104, the actuation element 112 can engage with the cap 114 of the device 100 and be held in place by a recapturing feature 9403 at the proximal end of the actuation element 112.

Figure 102 shows an exemplary embodiment of the proximate end of the actuation element 112 with a recapturing feature 9403. The recapturing feature 9403 comprises a tapered tip 9404 having a maximum diameter 9405. The wire 112 also includes a shelf 9406 having a diameter 9407 that is larger than the diameter 9405 of the tip 9404. The surface 9402 of the cap 114 has a plurality of orifices 9401 having a diameter 9408 that is smaller than the maximum diameter 9405 of the tip 9404 and the diameter 9407 of the shelf 9406.

As shown in Figure 103, the tip 9403 of the actuation element 112 can be advanced through one of the orifices 9401 in the surface 9402 of the cap 114 until the shelf 9406 is proximate to the surface 9402 of the cap 114. As shown in Figure 104, the actuation element 112 is prevented from advancing further through the cap 114, because the shelf 9406 is wider than the orifice 9401, and the actuation element 112 is not easily removed from the cap 114 due to the tapered tip 9404 having a maximum diameter 9405 slightly larger than the diameter 9408 of the orifice 9401. As such, the device is recaptured by the wire 112. In one exemplary embodiment, the recaptured device can be operated by the wire 112, catheter, coupler, and control lines in the same manner as any of the embodiments described herein.

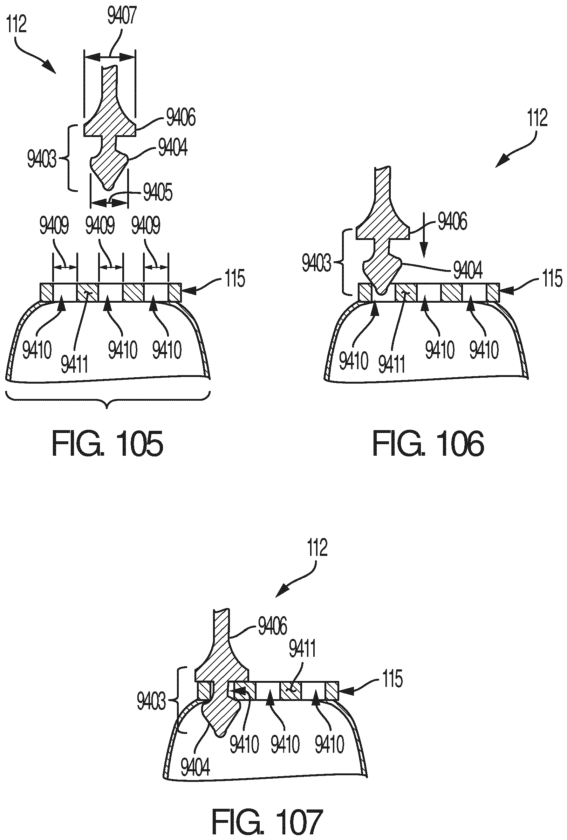

Figures 105-107 show an exemplary embodiment of a reattachment mechanism, comprising the actuation element 112 engaging with the collar 115 of the device 100. Figure 105 shows an exemplary embodiment of the proximate end of the actuation element 112 with a recapturing feature 9403. The recapturing feature 9403 comprises a tapered tip 9404 having a maximum diameter 9405. The wire has a shelf 9406 having a diameter 9407 larger than the diameter 9405 of the tip 9404. The surface 9811 of the collar 115 has a plurality of orifices 9410 each having a diameter 9409 smaller than the maximum diameter 9405 of the tip 9404 and the diameter 9407 of the shelf 9406.

As shown in Figure 106, the tip 9404 of the actuation element 112 can be advanced through one of the orifices 9410 in the surface 9411 of the collar 115 until the shelf 9406 is proximate to the surface 9411 of the collar 115. As shown in Figure 107, the actuation element 112 is prevented from advancing further through the collar 115, because the shelf 9406 is wider than the orifice 9410, and the actuation element 112 is not easily removed from the collar 115 due to the tapered tip 9404 having a maximum diameter 9405 slightly larger than the diameter 9409 of the orifice 9410. As such, the device is recaptured by the wire 112.

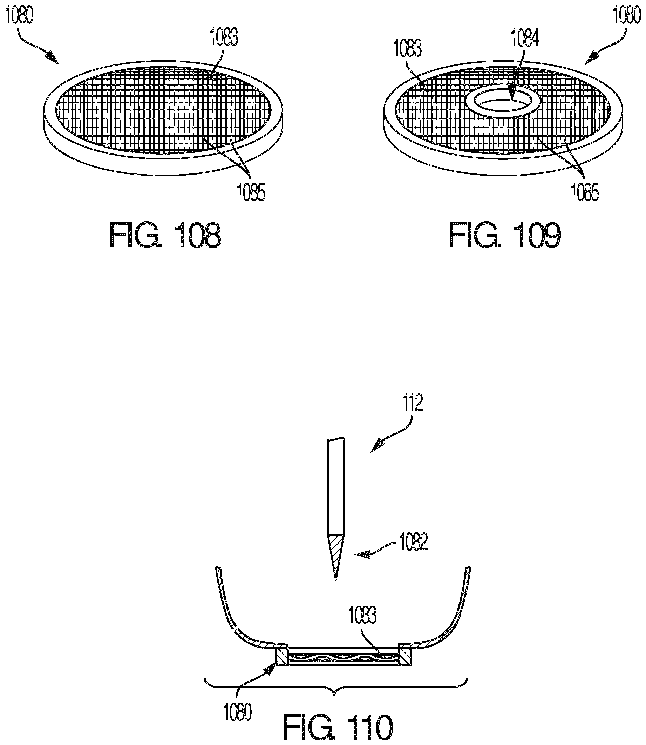

Figures 108 and 109 show exemplary embodiments of a cap 1080 having puncturable or penetrable surface 1083 that facilitates recapture of the device. For example, the puncturable or penetrable surface 1083 can be punctured or penetrated by an actuation element 112 or a modified version of the actuation element. The puncturable or penetrable surface 1083 can take a wide variety of different forms. For example, the puncturable or penetrable surface 1083 can be a grid or mesh, a cloth, a polymer sheet, etc. In the examples illustrated by Figures the puncturable or penetrable surface 1083 is a mesh surface 1083 to facilitate engagement of an actuation element 112. As shown in Figure 109, the cap 1080 can further comprise at least one orifice 1084. The design shown in Figures 108 and 109 can also be an exemplary embodiment of a collar 1088.

Figures 110-112 show an exemplary embodiment of an actuation element 112 engaging with an end cap 1080 wherein the cap 1080 has a puncturable or penetrable surface 1083. Figure 110 shows an exemplary embodiment where the cap 1080 has a mesh puncturable or penetrable surface 1083 and an actuation element 112 has a puncturing tip 1082 at its distal end. The puncturing tip 1082 can take a wide variety of different forms. The puncturing tip 1082 can be any structure that can puncture the puncturable or penetrable surface 1083 and become connected to the puncturable or penetrable surface 1083. In some exemplary embodiments, the tip 1082 is threaded. In some exemplary embodiments, the tip 1082 is tapered to a point.

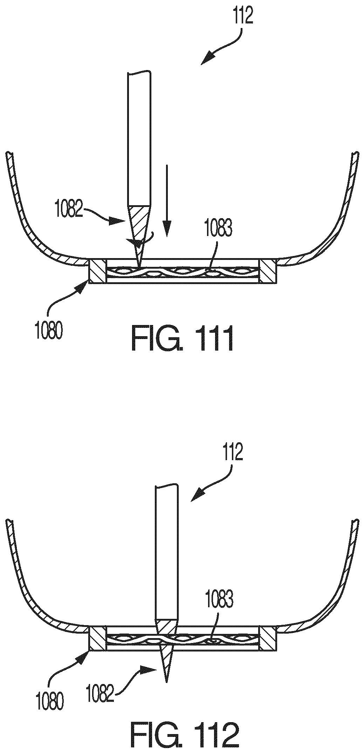

As shown in Figure 111, the distal end of the actuation element 112 reengages with the cap 1080 by rotating and/or moving downward toward the puncturable or penetrable surface 1083 of the cap 1080. In the illustrated example, the tip 1082 of the actuation element 112 can enter any one of a plurality of voids 1085 in the mesh of puncturable or penetrable surface 1083 of the cap 1080. The illustrated mesh puncturable or penetrable surface 1083 is flexible enough to allow the tip 1082 to push through any one of the voids 1085 in the surface 1083.

In the example shown in Figure 112, the tension between the threads of the threaded tip 1082 and the mesh puncturable or penetrable surface 1083 of the cap 1080 allows the cap 1080 to retain the actuation element 112. This embodiment advantageously facilitates engagement of the actuation element 112 with the cap 1080 by providing a plurality of potential engagement points without requiring the precision of mating a single male-female connection. As such, the device is recaptured by the wire 112. In one exemplary embodiment, the recaptured device can be operated by the wire 112, catheter, coupler, and control lines in the same manner as any of the embodiments described herein.

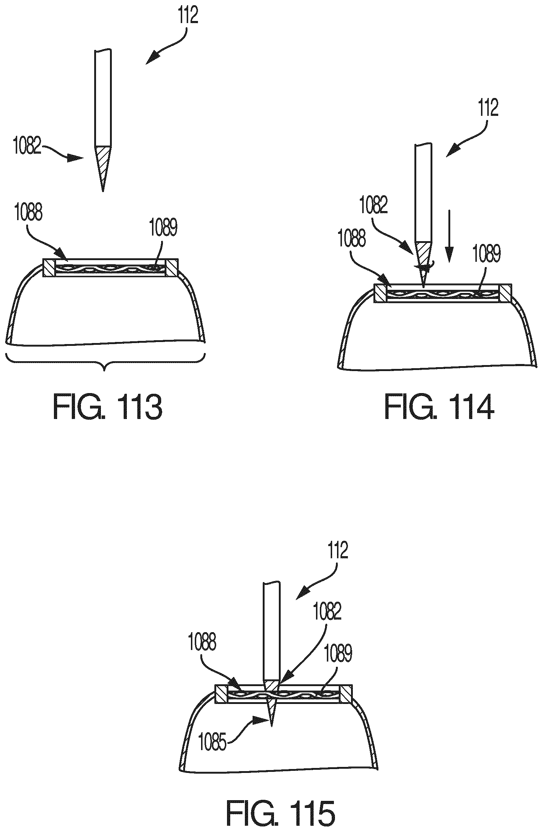

Figures 113-115 show an exemplary embodiment of an actuation element 112 reengaging with a collar 1088 wherein the collar 1088 has a puncturable or penetrable surface 1089. Figure 113 shows an exemplary embodiment of the collar 1088 having a mesh puncturable or penetrable surface 1089. The actuation element 112 has a penetrating tip 1082 at its proximal end. In some exemplary embodiments, the tip 1082 is threaded. In some exemplary embodiments, the tip 1082 is tapered to a point.

As shown in Figure 114, the proximal end of the actuation element 112 engages with the collar 1088 by rotating and/or moving downward toward the mesh puncturable or penetrable surface 1089 of the collar 1088. The mesh puncturable or penetrable surface 1089 is flexible enough to allow the threaded tip 1082 to push through any one of the voids 1085 in the surface 1089 of the collar 1088.

In some embodiments, as shown in Figure 115, the tension between the threads of the threaded tip 1082 and the puncturable or penetrable surface 1089 of the collar 1088 allows the collar 1088 to retain the actuation element 112. This embodiment advantageously facilitates engagement of the actuation element 112 with the collar 1088 by providing a plurality of potential engagement points without requiring the precision of mating a single male-female connection. As such, the device is recaptured by the wire 112.

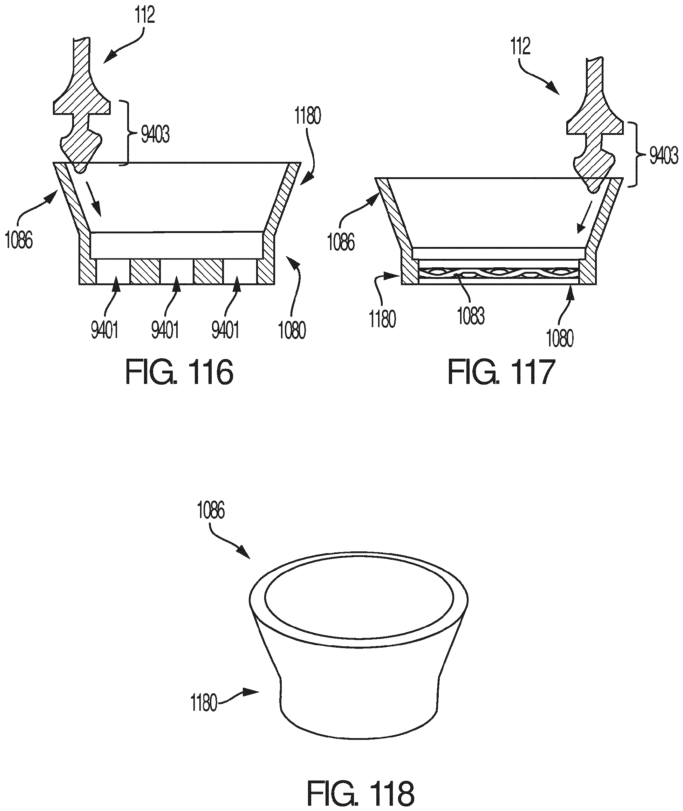

Figures 116-118 show a funnel 1180 capable of facilitating engagement and retention of the actuation element 112 within an attachment area, such as a cap 114, 1080 or collar 115, 1088. As shown in Figure 116, the funnel 1180 has a wide opening 1086 that guides the proximal end of the actuation element 112 towards the plurality of orifices 9401 or other engagement structure of the cap 1080. In some embodiments, the actuation element 112 has a recapturing feature 9403 such that the proximal end of the actuation element 112 can engage with one of the orifices 9401 or other engagement structure of the cap 1080.

In some embodiments, as shown in Figure 117, the funnel 1180 can be used along with a cap 114, 1080 or collar 115, 1088 having a puncturable or penetrable surface 1883, 1089, such as a mesh. The actuation element 112, has a recapturing feature 9403 at its proximal end that is guided towards the puncturable or penetrable surface 1089 of the cap 1080. Figure 118 is a perspective view of the funnel of Figures 116 or 117. The funnel has a wide opening 1086 to capture the actuation element 112 and guide it through the funnel 1180 towards an attachment area, such as a cap 114, 1080 or collar 115, 1088.

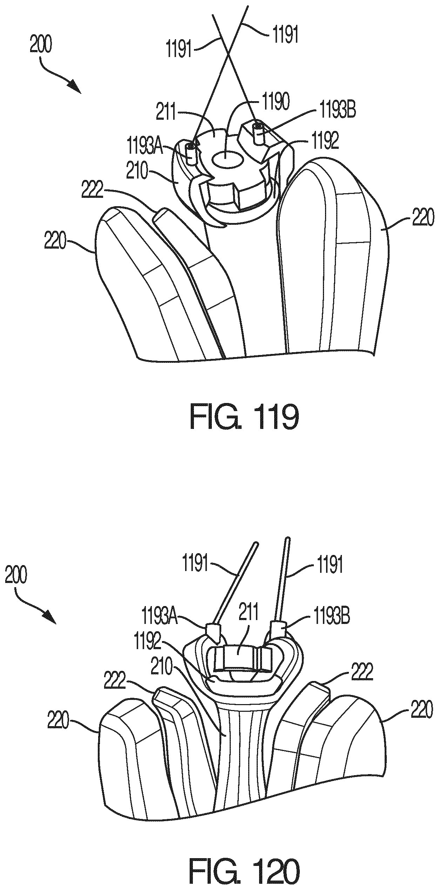

The devices, couplers, catheters, tethers, and clasp actuation lines can be configured to prevent tangling of the tethers and clasp actuation lines in a variety of different ways. Referring now to Figures 119-120, an exemplary embodiment of a device that includes a tether routing tube 1192 is illustrated. This example is shown applied to the device 400A illustrated by Figure 21A, but can be applied to any of the devices described herein, any of the devices that are described in a patent or patent application that is referenced herein, or any other known device for repairing a native heart valve, such as a mitral valve or a tricuspid valve.

Referring to Figure 119, the example device 400A has a collar 411A is shown comprising a wire passage 1190 and a tether routing tube 1192 extending below the collar 411A.Ends of the tube 1192 form first and second tether openings 1193A, 1193B. In the illustrated example, the tether routing tube 1192 extends through the collar 411A. In some embodiments, the ends of the tube extend proximally beyond the surface of the collar 411A as shown. In other exemplary embodiments, the ends of the tether routing tube 1192 are flush with or recessed from the proximal end of the collar.

As shown in Figure 120, the tube 1192 can extend around an external surface of the coaption element 410A of the device 400A. The tube 1192 can also be within the coaption element 410A directly underneath the collar 411A. A tether 1191 can be inserted into the first tether opening 1193A, through the tube 1192, and exit the second tether opening 1193B. The tube 1192 of the illustrated embodiment protects the tether 1191 and prevents entanglement of the tether 1191 with itself or any portion of the device 400A.

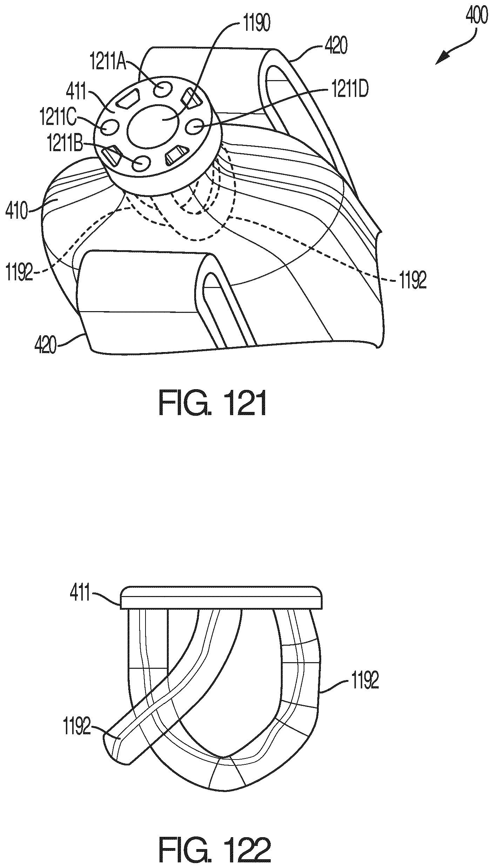

Referring now to Figures 121-122, an exemplary embodiment of a device that includes two tether routing tubes 1192 is illustrated. This example is shown applied to the device 400 illustrated by Figure 21, but can be applied to any of the devices described herein, any of the devices that are described in a patent or patent application that is referenced herein, or any other known device for repairing a native heart valve, such as a mitral valve or a tricuspid valve. The device illustrated by Figure 121includes a collar 411 with a wire passage 1190, first 1211A, second 1211B, third 1211C, and fourth 1211D tether passages, and two tubes 1192. The two tubes 1192 extend below the collar 1210 and are bent to pass one-another and fit within the coaption element 210. The ends of the first tube 1192 connect the first tether passage 1211A to the second tether passage 1211B. The second tube 1192 connects the third tether passage 1211C to the fourth tether passage 1211D. A first tether (not shown) can be inserted through the first tether passage 1211A of the collar 1210, through the first tube 1192, and exit through the second tether passage 1211B of the collar 1210. A second tether (not shown) can be inserted through the third tether passage 1211C of the collar 1210, through the second tube 1192, and exit through the fourth tether passage 1211D.

In some embodiments, the tubes 1192 are welded or otherwise affixed to the bottom of the collar 411. In some embodiments, the ends of the tubes 1192 extend through the tether passages 1211A, 1211B, 1211C, 1211D of the collar 411. In some embodiments, the tubes 1192 are welded or otherwise affixed to a first plate which is itself welded or otherwise affixed to another plate or plates to form the collar 411 of the device 400. The tubes 1192 of the illustrated embodiment of Figures 121-120 protect the tethers and prevent the entanglement of the tethers with themselves or any portion of the device 400.

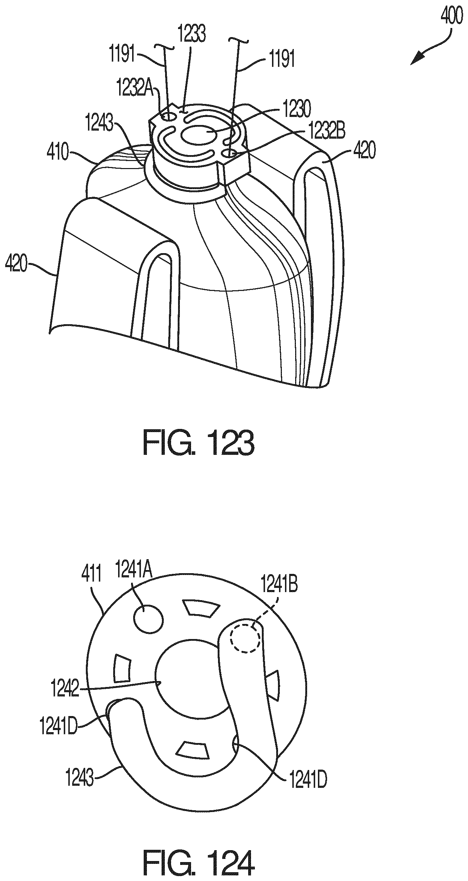

Figure 123 shows an embodiment of a device 400 with a collar 411 having a wire passage 1230, a plurality of orifices 1233, first 1232A and second 1232B tether passages, and a tube 1243. The tube extends below the collar 411 proximate to the external surface of the coaption element 410 of the device 400. The tube 1243 connects the first 1232A tether passage to the second 1232B tether passage. A tether 1191 can be inserted through the first tether passage 1232A, through the tube 1243, and back through the second tether passage 1232B. In some embodiments, the orifices 1233 are C-shaped, S-shaped, trapezoidal, circular. In some embodiments, the tube 1243 is welded or otherwise affixed to a first plate which is itself welded or otherwise affixed to another plate or plates to form the collar of the device 400. Figure 124 illustrates the collar 411 separated from the rest of the device 400.

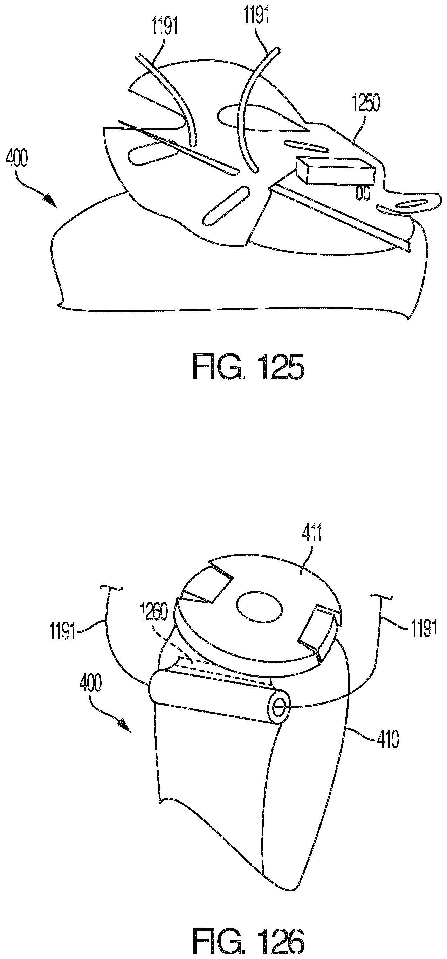

Referring to Figures 125 and 126, in one exemplary embodiment a cover 1250 of the device 400 is used to create a passage or passages for a tether or tethers 1191. This example is shown applied to the device 400 illustrated by Figure 21, but can be applied to any of the devices described herein, any of the devices that are described in a patent or patent application that is referenced herein, or any other known device for repairing a native heart valve, such as a mitral valve or a tricuspid valve. As shown in Figure 125, a cover 1250 comprising a plurality of orifices, can be placed on the proximal end of the device 400 and folded over the collar 411 and the coaption element 410. The cover 1250 in the folded configuration forms a tube through which tethers 1191 can be inserted into the tube formed therewith.

Referring to Figure 126, a tube 1260 could alternatively or additionally be applied separately to any part of the device 400 including to the coaption element 410, collar 411, or both. In one exemplary embodiment, the tube 1260 can be attached to the device 400 adjacent to the coaption element 410 proximate to the collar 411A tether 1191 can be inserted through the tube 1260.

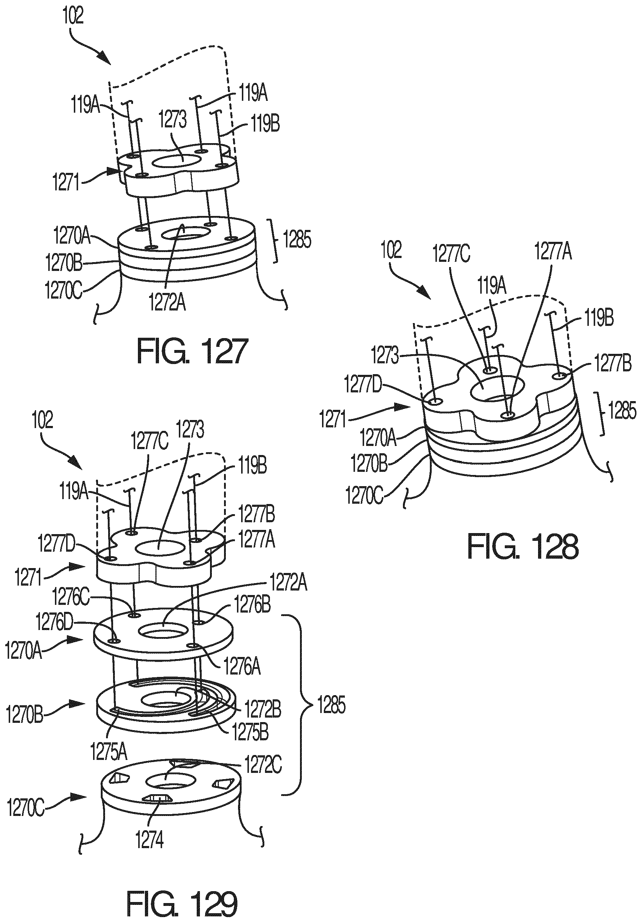

In some exemplary embodiments, the function of the tether tubes illustrated by Figures 119-126 can be incorporated into the structure of a collar 1285. For example, the collar 1285 can include one or more internal tether passages inside the body of the collar itself. The internal collar passages can be formed in a wide variety of different ways. For example, the collar 1285 can be three-dimensionally printed with the internal tether passages, the collar can be assembled from horizontally and/or stacked plates that define the internal tether passages, the tether passages can be bored into the collar 1285, etc. Figures 127 through 131 show various embodiments of a collar 1285 and a coupler 1271, wherein the collar 1285 includes one or more internal passages.

In the example illustrated by Figures 127-129, the collar 1285 comprises a plurality of plates, such as a first plate 1270A, a second plate 1270B, and a third plate 1270C. The first, second, and third plates, 1270A, 1270B, 170C can be joined together in any suitable manner, such as by welding, with adhesives, or the like. In the example illustrated by Figures 127-129, the center collar plate 1270B includes a first and second tether routing channels 1275A, 1275B. First and second tethers 119A, 119B can be routed through the tether routing channels 1275A, 1275Bto tether the coupler 1271 to the collar 1285. When the plates 1270A, 1270B, 1270C are joined together, the plate 1270A closes the tops of the channels 1275A, 1275B and the plate 1270C closes the bottoms of the channels 1275A, 1275B. As such, the joined plates 1270A, 1270B, 1270C form a collar with internal passages that protect the tethers 119A, 119B and prevent the tethers 119A, 119B from entanglement.

As shown in Figures 127-129, a coupler 1271 joined to the end of a catheter 102 (illustrated by dashed lines) can have a wire passage 1273 and a first tether passage 1277A, a second tether passage 1277B, a third tether passage 1277C, and a fourth tether passage 1277D. In this exemplary embodiment, at least one tether passage, such as 1277B, is located radially inward from the other tether passages. This radial offset facilitates the illustrated tether routing. The tether passages 1277A, 1277B, 1277C, 1277D are radially outward from the wire passage 1273 of the coupler 1271.

As mentioned above, the collar 1285 comprises a first plate 1270A, a second plate 1270B, and a third plate 1270C that are joined together. Each of the first, second, and third plates 1270A, 1270B, 1270C comprise a control wire passage 1272A, 1272B, 1272C (see e.g., Figure 129) aligned with one another and with the wire passage 1273 of the coupler 1271. The first plate 1270A can have a wire passage 1272A, a first tether passage 1276A, a second tether passage 12768, a third tether passage 1276C, and a fourth tether passage 1276D. In this example, at least one tether passage, such as 12768, is located radially inward from the other tether passages. The tether passages 1276A, 1276B, 1276C, 1276D are radially outward from the wire passage 1272A of the first plate 1270A.

Figure 129 is an exploded view of the first, second, and third plates 1270A, 1270B, 1270C of the collar 1285. The second plate 1270B can have a wire passage 1272B, a first channel 1275A, and a second channel 1275B. A second end of the first channel 1275A is radially inward from a first end of the first channel 1275A. In exemplary embodiments, the channels 1275A, 1275B can be arcuate, curved, or semi-circular. The third plate 1270C can have a wire passage 1272C and orifices 1274 that are used to join the collar 1285 to a coaption element (not shown). The wire passages 1272A, 1272B, 1272C of the first, second, and third plates 1270A, 1270B, 1270C align vertically with one another. The first passage 1276A and third passage 1276C of the first plate 1270A vertically align with the ends of the second channel 1275B. The second passage 1276B and fourth passage 1276D of the first plate 1270A vertically align with the ends of the first channel 1275A.

When the first, second and third plates 1270A, 1270B, 1270C are joined together, such as by welding, they form the collar 1285 illustrated to Figures 127 and 128. The first and third plates 1270A, 1270C form top and bottom surfaces, respectively, to the channels 1275A, 1275B of the second plate 1270B. The channels 1275A, 1275B form protective tubes or passages inside the collar through which tethers 119A, 119B can be inserted. These protective tubes or passages are completely enclosed.

A first tether 119A can be inserted from the catheter 102 into the first tether passage 1277A of the coupler 1271, through the first tether passage 1276A of the first plate 1270A, into a first end of the second channel 1275B of the second plate 1270B, along the second channel 1275B to a second end, through the third tether passage 1276C of the first plate 1270A, through the third tether passage 1277C of the coupler 1271 and back into the catheter 102. A second tether 119B can be inserted from the catheter 102 into the second tether passage 1277B of the coupler 1271, through the second tether passage 1276B of the first plate 1270A, into the first end of the first channel 1275A of the second plate 1270B, along the first channel 1275A to the second end, through the fourth tether passage 1276D of the first plate 1270A, through the fourth tether passage 1277D of the coupler 1271 and back into the catheter 102.

Tension in the tethers 119A, 119B can be decreased allowing the collar 1285 to separate from the coupler 1271 in order to test the placement of a device. Figure 28 shows the coupler 1271 proximate to the collar 1285. Increasing the tension in the tethers 119A, 119B can seat the collar 1285 against the coupler 1271 in order to recapture the device.

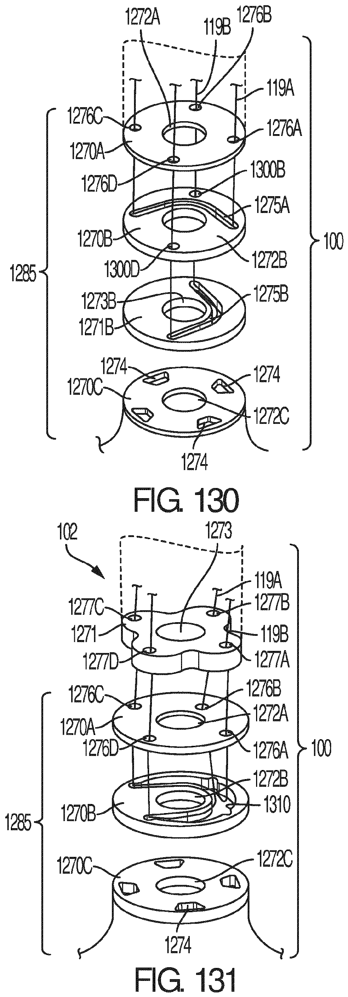

Figures 130 and 131 show various configuration of the collar 1285 shown in Figures 127-129. In the example illustrated by Figure 130, the second or center plate 1270B is replaced by two center plates 1270B, 1271B. The use of the two plates 1270B, 1271B eliminate the need to offset one or more of the passages 1276A, 1276B, 1276C, 1276D.

In the example illustrated by Figure 130, the collar 1285 comprises a plurality of plates, such as a top plate 1270A, a first middle plate 1270B, a second middle plate 1271B, and a bottom plate 1270C. The top plate 1270A, first middle plate 1270B, second middle plate 1271B, and bottom plate 1270C can be joined together in any suitable manner, such as by welding, with adhesives, or the like. In the example illustrated by Figure 130, the first middle collar plate 1270B includes a first tether routing channel 1275A. The second middle collar plate 1270B includes a second tether routing channel 1275B. First and second tethers 119A, 119B can be routed through the tether routing channels 1275A, 1275B to tether the coupler 1271 to the collar 1285. When the plates 1270A, 1270B, 1271B, 1270C are joined together, the plate 1270A closes the top of the channel 1275A, the plate 1271B closes the bottom of the channel 1275A, the plate 1270B closes the top of the channel 1275B, and the plate 1270C closes the bottom of the channel 1275B. As such, the joined plates 1270A, 1270B, 1271B, 1270C form a collar with internal passages that protect the tethers 119A, 119B and prevent the tethers 119A, 119B from entanglement.

A coupler can be joined to collar of Figure 130 in the same manner as shown in Figures 127-129, except one of the coupler passages is not located radially inward from the other tether passages.

Still referring to Figure 130, each of the joined plates 1270A, 1270B, 1271B, 1270C comprise a control wire passage 1272A, 1272B, 1273B, 1272C (see e.g., Figure 129) aligned with one another. The first plate 1270A can have the wire passage 1272A, a first tether passage 1276A, a second tether passage 12768, a third tether passage 1276C, and a fourth tether passage 1276D. The tether passages 1276A, 1276B, 1276C, 1276D are radially outward from the wire passage 1272A of the first plate 1270A.

The first middle plate 1270B can have a wire passage 1272B. and the first channel 1275A. The first middle plate 1270 also includes passages 1300B and 1300D. The second middle plate has a wire passage 1273B and a second channel 1275B. In exemplary embodiments, the channels 1275A, 1275B can be arcuate, curved, or semi-circular. The third plate 1270C can have a wire passage 1272C and orifices 1274 that are used to join the collar 1285 to a coaption element (not shown). The wire passages 1272A, 1272B, 1273B, 1272C align vertically with one another. The first passage 1276A and third passage 1276C of the first plate 1270A vertically align with the ends of the first channel 1275A. The second passage 1276B and fourth passage 1276D of the first plate 1270A vertically align with the passages 1300B, 1300D of the first middle plate 1270B and with the ends of the second channel 1275B.

When the top plate 1270A, first middle plate 1270B, second middle plate 1271B, and bottom plate 1270C are joined together, such as by welding, they form a collar 1285. The channels 1275A, 1275B form protective tubes or passages inside the collar through which tethers 119A, 119B can be inserted. These protective tubes or passages are completely enclosed.

A first tether 119A can be inserted through the first tether passage 1276A of the first plate 1270A, into a first end of the first channel 1275A of the first middle plate 1270B, along the first channel 1275A to a second end, and through the third tether passage 1276C of the first plate 1270A. A second tether 119B can be inserted through the second tether passage 1276B of the first plate 1270A, through the tether passage 1300B of the first middle plate 1270B, into the first end of the second channel 1275B of the second middle plate 1271B, along the second channel 1275B to the second end, through the tether passage 1300D of the first middle plate 1270B, and through the fourth tether passage 1276D of the first plate 1270A.

Figure 131 shows one configuration of the collar 1285 of Figures 127-129. The example illustrated by Figure 130, the second or center plate 1270B is replaced by two center plates 1270B, 1271B. The use of the two plates 1270B, 1271B eliminate the need to offset one or more of the passages 1276A, 1276B, 1276C, 1276D.

In the example illustrated by Figure 131, the second or center plate 1270B is modified to replace the two passages with a single channel 1310. In this example, the tethers 119A and 119B can cross at a single point inside the collar 1285.

As shown in Figure 131, the collar 1285 can comprise a first plate 1270A, a second plate 1270B, and a third plate 1270C, wherein the second plate 1270B has one channel 1310. The first plate 1270A can have a wire passage 1272A, a first tether passage 1276A, a second tether passage 12768, a third tether passage 1276C, and a fourth tether passage 1276D. The tether passages 1276A, 1276B, 1276C, 1276D are radially outward from the wire passage 1272A of the first plate 1270A.

The second plate 1270B can have a wire passage 1272B and the channel 1310. In exemplary embodiments, the channel 1310 can be arcuate, curved, or semi-circular. The third plate 1270C can have a wire passage 1272C and at least one orifice 1274 used to join the collar 1285 to a coaption element (not shown).

The wire passages 1272A, 1272B, 1272C of the first, second, and third plates 1270A, 1270B, 1270C align vertically with one another. The third and fourth passages 1276C, 1276D of the first plate 1270A vertically align with the ends of the channel 1310, and the first and second passages 1276A, 1276B of the first plate 1270A vertically align above another portion of the channel 1310. When the first, second and third plates 1270A, 1270B, 1270C are joined together, such as by welding, they form a collar 1285. The first and third plates 1270A, 1270C form top and bottom surfaces, respectively, to the channel 1310 of the second plate 1270B such that the channel 1310 forms a protective space through which tethers 119A, 119B can be inserted. This space is completely enclosed.

A first tether 119A can be inserted from the catheter 102 into the first tether passage 1277A of the coupler 1271, through the first tether passage 1276A of the first plate 1270A, into the channel 1310 of the second plate 1270B, along the channel 1310 to a second end, through the third tether passage 1276C of the first plate 1270A, through the third tether passage 1277C of the coupler 1271 and back into the catheter 102. A second tether 119B can be inserted from the catheter 102 into the second tether passage 1277B of the coupler 1271, through the second tether passage 1276B of the first plate 1270A, into the channel 1310 of the second plate 1270B, along the channel 1310 to the second end, through the fourth tether passage 1276D of the first plate 1270A, through the fourth tether passage 1277D of the coupler 1271 and back into the catheter 102.

The features including structure, material, and connectivity to each other to form the device and system, e.g., of the clasps, paddles, coaption elements, delivery devices, etc., and methods can vary among the example embodiments, and combinations of the different embodiments can be combined to form additional embodiments within the scope of the disclosure. The devices can incorporate features such as those in U.S. provisional application no. 62/744,031, filed on October 10, 2018, PCT patent application publication WO2020/076898, filed October 9, 2019, and U.S. application no. 15/865,890, filed January 9, 2018, each of which is incorporated by reference herein in their entireties.

The devices 100, 400, 400A 500, 600, 900, 1000, 1100 described herein can be positioned to engage valve tissue 20, 22 as part of any suitable valve repair system (e.g., any valve repair system disclosed in the present application). In one example embodiment, any of these devices 100, 400, 500, 600, 900, 1000, 1100 can be configured to open and close with the opening and closing of the native valve. In one example embodiment, any of these devices 100, 400, 500, 600, 900, 1000, 1100 can be configured to be implanted in a partially open condition and can optionally be configured to further open and return to the partially open, implanted position with the opening and closing of the native valve.

While various inventive aspects, concepts and features of the disclosures can be described and illustrated herein as embodied in combination in the example embodiments, these various aspects, concepts, and features can be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures-such as alternative materials, structures, configurations, methods, devices, and components, alternatives as to form, fit, and function, and so on-can be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein.

Additionally, even though some features, concepts, or aspects of the disclosures can be described herein as being a preferred arrangement or method, such description is not intended to suggest that such feature is required or necessary unless expressly so stated. Still further, example or representative values and ranges can be included to assist in understanding the present application, however, such values and ranges are not to be construed in a limiting sense and are intended to be critical values or ranges only if so expressly stated.

Moreover, while various aspects, features and concepts can be expressly identified herein as being inventive or forming part of a disclosure, such identification is not intended to be exclusive, but rather there can be inventive aspects, concepts, and features that are fully described herein without being expressly identified as such or as part of a specific disclosure, the disclosures instead being set forth in the appended claims. Descriptions of example methods or processes are not limited to inclusion of all steps as being required in all cases, nor is the order that the steps are presented to be construed as required or necessary unless expressly so stated. The words used in the claims have their full ordinary meanings and are not limited in any way by the description of the embodiments in the specification.

The invention further comprises the following embodiments:
1. A system comprising:
   an implantable device having a pair of anchors that are movable between an open position and a closed position to secure the implantable device to a native valve;
   a delivery catheter;
   a coupler disposed at a distal end of the delivery catheter;
      a collar attached to the device;
      an actuation element which extends through the delivery catheter and into the device; and
   wherein the coupler and the collar are tied by a coupling tether which can recouple the device to the coupler after the actuation element has been removed from the device.
2. The system of embodiment 1 wherein the coupling tether is looped around the actuation element.
3. The system of any of embodiments 1-2 wherein a looped end of the coupling tether extends from the coupler through the collar and extends back through the collar into the coupler.
4. The system of any of embodiments 1-3 wherein the coupling tether extends from the coupler through the collar and extends back through the collar into the coupler.
5. The system of any of embodiments 1-4 wherein the coupling tether is a first coupling tether, and the coupler and the collar are tied by a second coupling tether.
6. The system any of embodiments 1-5 wherein a first passage for the coupling tether in the delivery catheter is offset by about 180 degrees from a second passage for the coupling tether in the delivery catheter.
7. The system of embodiment 5 wherein a first passage for the first coupling tether in the delivery catheter is offset by about 180 degrees from a second passage for the coupling tether in the delivery catheter and wherein a third passage for the second coupling tether in the delivery catheter is offset by about 180 degrees from a fourth passage for the second coupling tether in the delivery catheter.
8. The system of embodiment 7 wherein the first passage and the third passage are offset by about 90 degrees.
9. The system of any of embodiments 1-8 wherein the collar extends into the coupler.
10. The system of any of embodiments 1-8 wherein the coupler extends into the collar.
11. The system of any of embodiments 1-10 wherein the coupling tether extends from an end of the coupler.
12. The system of any of embodiments 1-10 wherein the coupling tether extends from a side of the coupler.
13. The system of any of embodiments 1-12 wherein the coupling tether extends into an end of the collar.
14. The system of any of embodiments 1-12 wherein the coupling tether extends into a side of the collar.
15. The system of any of embodiments 1-14 further comprising a compressible sleeve around the coupling tether between the coupler and the collar.
16. The system of embodiment 15 wherein the compressible sleeve is tapered.
17. The system of embodiment any of embodiments 15-16 wherein an outer diameter of the compressible sleeve is less than or equal to an outer diameter of the coupler when the compressible sleeve is compressed between the coupler and the collar.
18. The system of any of embodiments 1-17 further comprising a telescoping actuator coupled to the pair of anchors.
19. The system of any of embodiments 1-18 wherein the coupling tether is connected to the implantable device by a releasable knot.
20. The system of embodiment 19 wherein the releasable knot is configured such that pulling a first end of the coupling tether tightens the releasable knot and pulling a second end of the coupling tether unties the releasable knot.
21. The system of any of embodiments 1-20 wherein the actuation element includes a tapered recapturing feature.
22. The system of any of embodiments 1-21 wherein the collar includes a plurality of recapturing holes.
23. The system of any of embodiments 1-21 wherein the collar includes a penetrable recapturing surface that is configured to be penetrated by the actuation element.
24. The system of embodiment 23 wherein the penetrable recapturing surface comprises a wire mesh.
25. The system of any of embodiments 1-21 wherein the collar includes a coupling tether routing passage.
26. The system of embodiment 25 wherein the tether routing passage comprises a tube.
27. The system of embodiment 25 wherein the tether routing passage is defined inside the collar.
28. The system of embodiment 25 wherein the collar comprises a plurality of stacked plates.
29. The system of any of embodiments 1-28 wherein the coupler, the collar, and coupling tether are configured such that pulling two ends of the coupling tether results in a 2:1 ratio of movement of the two ends to a longitudinal change in position of the collar.
30. A system comprising:
   an implantable device having at least one clasp;
   a delivery catheter;
   a clasp actuation line having two portions that both extend from the delivery catheter and that both pass through a loop of the clasp;
   wherein a looped end of the first clasp actuation line is releasably coupled to at least one of the delivery catheter and the implantable device;
   wherein the at least one clasp is movable from a closed position to an open position by pulling the clasp actuation line;
   wherein the at least one clasp is configured to secure the implantable device to a native valve by moving the clasp from the open position to the closed position.
31. The system of embodiment 30 wherein the clasp actuation line is looped around the actuation element.
32. The system any of embodiments 30-31 wherein a first passage for the clasp actuation line in the delivery catheter is offset by about 180 degrees from a second passage for the clasp actuation line in the delivery catheter.
33. The system of any of embodiments 30-32 wherein pulling the two portions of the clasp actuation line results in a 2:1 ratio of movement of the two portions to a longitudinal change in position of the clasp.
34. A system comprising:
   an implantable device having at least one clasp;
   a delivery catheter;
   a clasp actuation line having a looped end;
   wherein the clasp actuation line extends from the delivery catheter and passes through a loop of the clasp;
   a clasp actuation wire having an end portion that is moveable from a hooked configuration to a straight configuration;
   wherein a looped end of the clasp actuation line is releasably coupled to the end portion of the clasp actuation wire;
   wherein the at least one clasp is movable from a closed position to an open position by pulling at least one of the clasp actuation line and/or the clasp actuation wire; and
   wherein the at least one clasp is configured to secure the implantable device to a native valve by moving the clasp from the open position to the closed position.
35. The system of embodiment 34 wherein the clasp actuation line is released from the clasp actuation wire by moving the end portion of the clasp actuation wire from the hooked configuration to the straight configuration.
36. The system of any of embodiments 34-35 wherein the end portion of the clasp actuation wire is moved from the hooked configuration to the straight configuration by extending the end portion of the clasp actuation wire from the delivery catheter.
37. The system of any of embodiments 34-36 wherein the end portion of the clasp actuation wire is constrained in the hooked configuration in the delivery catheter.
38. The system any of embodiments 34-37 wherein a first passage for the clasp actuation line in the delivery catheter is offset by about 180 degrees from a second passage for the clasp actuation wire in the delivery catheter.
39. The system of any of embodiments 34-38 wherein pulling the clasp actuation line results in a 2:1 ratio of movement of the clasp actuation line to a longitudinal change in position of the clasp.
40. A system comprising:
   an implantable device having at least one clasp;
   a delivery catheter;
   a clasp actuation line having a first portion and a second portion that both extend from the delivery catheter and that both pass through a loop of the clasp;
   wherein a looped end of the first clasp actuation line is tied to at least one of the delivery catheter and the implantable device by a releasable knot;
   wherein the at least one clasp is movable from a closed position to an open position by pulling the first portion of the clasp actuation line;
   wherein the releasable knot is untied by pulling the second portion of the clasp actuation line to release the at least one clasp from the clasp actuation line;
   wherein the at least one clasp is configured to secure the implantable device to a native valve by moving the clasp from the open position to the closed position.
41. The system of embodiment 40 wherein the releasable knot is tied to the clasp.
42. The system of any of embodiments 40-41 wherein the releasable knot is tied to the loop of the clasp.
43. The system any of embodiments 40-42 wherein a first passage for the clasp actuation line in the delivery catheter is offset by about 180 degrees from a second passage for a second clasp actuation line in the delivery catheter.
44. The system of any of embodiments 40-43 wherein pulling both the first portion and the second portion of the clasp actuation line results in a 1:1 ratio of movement of the clasp actuation line to a longitudinal change in position of the clasp.
45. A system comprising:
   an implantable device having a pair of anchors that are movable between an open position and a closed position to secure the implantable device to a native valve;
   a delivery catheter;
   a coupler disposed at a distal end of the delivery catheter;
      a collar attached to the device;
   a compressible sleeve disposed between the collar and the coupler which encases a coupling tether;
      an actuation element which extends through the delivery catheter and into the device;
   wherein the actuation element is coupled to the pair of anchors for moving the pair of anchors between the open position and the closed position.
46. The system of embodiment 45 further comprising a coupling tether disposed inside the compressible sleeve that can recouple the device to the coupler after the actuation element has been removed from the device.
47. The system of any of embodiments 45-46 wherein the catheter and the collar are coupled by the coupling tether.
48. The system of any of embodiments 45-47 wherein the compressible sleeve comprises a first end, a second end, and a middle portion, wherein the compressible sleeve is tapered such that at least one of the first and second ends has a greater diameter than the middle portion of the compressible sleeve.
49. The system of any of embodiments 46-48 wherein the coupling tether is looped around the actuation element.
50. The system of any of embodiments 46-49 wherein a looped end of the coupling tether extends from the coupler through the collar and extends back through the collar into the coupler.
51. The system of any of embodiments 46-50 wherein the coupling tether extends from the coupler through the collar and extends back through the collar into the coupler.
52. The system of any of embodiments 46-51 wherein the coupling tether is a first coupling tether, and the coupler and the collar are tied by a second coupling tether.
53. The system any of embodiments 46-52 wherein a first passage for the coupling tether in the delivery catheter is offset by about 180 degrees from a second passage for the coupling tether in the delivery catheter.
54. The system of embodiment 53 wherein a first passage for the first coupling tether in the delivery catheter is offset by about 180 degrees from a second passage for the coupling tether in the delivery catheter and wherein a third passage for the second coupling tether in the delivery catheter is offset by about 180 degrees from a fourth passage for the second coupling tether in the delivery catheter.
55. The system of embodiment 54 wherein the first passage and the third passage are offset by about 90 degrees.
56. The system of any of embodiments 45-55 wherein the collar extends into the coupler.
57. The system of any of embodiments 45-56 wherein the coupler extends into the collar.
58. The system of any of embodiments 46-57 wherein the coupling tether extends from an end of the coupler.
59. The system of any of embodiments 46-57 wherein the coupling tether extends from a side of the coupler.
60. The system of any of embodiments 46-59 wherein the coupling tether extends into an end of the collar.
61. The system of embodiment any of embodiments 45-60 wherein an outer diameter of the compressible sleeve is less than or equal to an outer diameter of the coupler when the compressible sleeve is compressed between the coupler and the collar.
62. The system of any of embodiments 45-61 further comprising a telescoping actuator coupled to the pair of anchors.
63. The system of any of embodiments 46-62 wherein the coupling tether is connected to the implantable device by a releasable knot.
64. The system of embodiment 63 wherein the releasable knot is configured such that pulling a first end of the coupling tether tightens the releasable knot and pulling a second end of the coupling tether unties the releasable knot.
65. The system of any of embodiments 45-64 wherein the actuation element includes a tapered recapturing feature.
66. The system of any of embodiments 45-65 wherein the collar includes a plurality of recapturing holes.
67. The system of any of embodiments 45-66 wherein the collar includes a penetrable recapturing surface that is configured to be penetrated by the actuation element.
68. The system of embodiment 45-67 wherein the penetrable recapturing surface comprises a wire mesh.
69. The system of any of embodiments 45-68 wherein the collar includes a coupling tether routing passage.
70. The system of embodiment 69 wherein the tether routing passage comprises a tube.
71. The system of embodiment 70 wherein the tether routing passage is defined inside the collar.
72. The system of embodiment 71 wherein the collar comprises a plurality of stacked plates.
73. The system of any of embodiments 46-72 wherein the coupler, the collar, and coupling tether are configured such that pulling two ends of the coupling tether results in a 2:1 ratio of movement of the two ends to a longitudinal change in position of the collar.
74. An implantable device having a pair of paddles that are movable between an open position and a closed position and a pair of gripping clasps that secure the implantable device to the native valve;
   a delivery catheter;
   a coupler disposed at a distal end of the delivery catheter;
   a collar attached to the device; and
   an actuation element which extends through the delivery catheter and into the device;
   wherein the delivery catheter and the pair of gripping clasps are coupled via a pair of clasp actuation lines; and
   wherein the clasp actuation lines each extend through the delivery catheter, through a fastener on one of the pair of gripping clasps, around the actuation element, through the fastener, and back through the delivery catheter.
75. The system of embodiment 74 wherein the coupling tether is a first coupling tether, and the coupler and the collar are tied by a second coupling tether.
76. The system any of embodiments 74-75 wherein a first passage for the coupling tether in the delivery catheter is offset by about 180 degrees from a second passage for the coupling tether in the delivery catheter.
77. The system of any of embodiments 74-76 wherein a first passage for the first coupling tether in the delivery catheter is offset by about 180 degrees from a second passage for the coupling tether in the delivery catheter and wherein a third passage for the second coupling tether in the delivery catheter is offset by about 180 degrees from a fourth passage for the second coupling tether in the delivery catheter.
78. The system of embodiment 77 wherein the first passage and the third passage are offset by about 90 degrees.
79. The system of any of embodiments 74-78 wherein the collar extends into the coupler.
80. The system of any of embodiments 74-78 wherein the coupler extends into the collar.
81. The system of any of embodiments 74-80 wherein the coupling tether extends from an end of the coupler.
82. The system of any of embodiments 74-80 wherein the coupling tether extends from a side of the coupler.
83. The system of any of embodiments 74-82 wherein the coupling tether extends into an end of the collar.
84. The system of any of embodiments 74-82 wherein the coupling tether extends into a side of the collar.
85. The system of any of embodiments 74-84 further comprising a compressible sleeve around the coupling tether between the coupler and the collar.
86. The system of embodiment 85 wherein the compressible sleeve is tapered.
87. The system of embodiment any of embodiments 85-86 wherein an outer diameter of the compressible sleeve is less than or equal to an outer diameter of the coupler when the compressible sleeve is compressed between the coupler and the collar.
88. The system of any of embodiments 74-87 further comprising a telescoping actuator coupled to the pair of anchors.
89. The system of any of embodiments 74-88 wherein the coupling tether is connected to the implantable device by a releasable knot.
90. The system of embodiment 89 wherein the releasable knot is configured such that pulling a first end of the coupling tether tightens the releasable knot and pulling a second end of the coupling tether unties the releasable knot.
91. The system of any of embodiments 74-90 wherein the actuation element includes a tapered recapturing feature.
92. The system of any of embodiments 74-91 wherein the collar includes a plurality of recapturing holes.
93. The system of any of embodiments 74-91 wherein the collar includes a penetrable recapturing surface that is configured to be penetrated by the actuation element.
94. The system of embodiment 93 wherein the penetrable recapturing surface comprises a wire mesh.
95. The system of any of embodiments 74-94 wherein the collar includes a coupling tether routing passage.
96. The system of embodiment 95 wherein the tether routing passage comprises a tube.
97. The system of embodiment 95 wherein the tether routing passage is defined inside the collar.
98. The system of embodiment 95 wherein the collar comprises a plurality of stacked plates.
99. The system of any of embodiments 74-98 wherein the coupler, the collar, and coupling tether are configured such that pulling two ends of the coupling tether results in a 2:1 ratio of movement of the two ends to a longitudinal change in position of the collar.
100. A method for observing and recoupling a previously implanted valve repair device from a native valve of a patient, the previously implanted valve repair device having a pair of anchors that are movable between an open position and a closed position to secure the implantable device to a native valve, and a collar, the method comprising:
   retracting an actuation elements, such as an actuation wire, from the implanted valve repair device;
   retracting a delivery catheter and a coupler from the implanted valve repair device and introducing slack to a coupling tether that couples the coupler and a collar of the implanted valve repair device;
   observing the condition of the implanted valve repair device;
   pulling the coupling tether and advancing the coupler to bring the coupler back to the collar;
   advancing the actuation element into the implanted valve repair device;
   opening the valve repair device with the actuation element;
   repositioning the valve repair device;
   moving the pair of anchors to the closed position;
   retracting the actuation element from the implanted valve repair device;
   retracting the delivery catheter and the coupler from the implanted valve repair device;
   decoupling the coupling tether from the valve repair device; and
   removing the delivery catheter, the actuation element, the coupler, the coupling tether, and the clasp actuation line.
101. An implantable prosthetic device comprising:
   a plurality of paddles;
   a cap or other attachment portion connected to each of the paddles, configured such that movement of the cap or other attachment portion relative to a proximal end of the device opens and closes the plurality of paddles;
   an extendable coupler affixed to the cap or other attachment portion such that the extendable coupler extends when the cap or other attachment portion moves away from the proximal end.
102. The implantable prosthetic device of embodiment 101, further comprising a coaption element connected to each of the paddles, wherein movement of the cap or other attachment portion relative to the coaption portion also opens and closes the plurality of paddles.
103. The implantable prosthetic device of embodiment 102 wherein the extendable coupler extends through the coaption portion.
104. The implantable prosthetic device of any one of embodiments 101-103 wherein the extendable coupler comprises an inner shaft and an outer sleeve, wherein the inner shaft is extendably disposed in the outer sleeve.
105. An implantable prosthetic device comprising:
   a pair of anchors that are movable between an open position and a closed position to secure the implantable device to a native valve;
   a coaption element connected to the pair of anchors;
   wherein the coaption element comprises a plurality of wires;
   a collar connected to the coaption element;
   wherein a plurality of ends of the plurality of wires are gathered into a plurality of bunches and the plurality of bunches are secured in a plurality of openings in the collar to connect the collar to the coaption element.
106. The implantable prosthetic device of embodiment 105 wherein the plurality openings in the collar each have a serpentine shape.
107. The implantable prosthetic device of embodiment 105 wherein the plurality openings in the collar each have four sides.
108. The implantable prosthetic device of any of embodiments 105-107 wherein the plurality of openings in the collar consist of two openings.
109. The implantable prosthetic device of any of embodiments 105-107 wherein the plurality of openings in the collar consist of four openings.
110. The implantable prosthetic device of any of embodiments 105-107 further comprising a coupling tether that is looped through the collar.
111. The implantable prosthetic device of embodiment 110 wherein the coupling tether is looped through tether openings through the collar that are between the plurality of bunches of wire ends.
112. The implantable prosthetic device of embodiment 110 wherein the coupling tether is looped through tether openings through the collar that are between the plurality of bunches of wire ends.
113. The implantable prosthetic device of any of embodiments 105-112 wherein the bunches of wire ends are welded to the collar.
114. The implantable prosthetic device of any of embodiments 105-113 wherein the collar includes a plurality of recapturing holes.
115. The implantable prosthetic device of any of embodiments 105-114 wherein the collar includes a penetrable recapturing surface that is configured to be penetrated by the actuation element.
116. The implantable prosthetic device of embodiment 115 wherein the penetrable recapturing surface comprises a wire mesh.
117. The implantable prosthetic device of any of embodiments 105-116 wherein the collar includes a coupling tether routing passage.
118. The implantable prosthetic device of any of embodiments 105-117 wherein the tether routing passage is defined inside the collar.
119. The implantable prosthetic device of any of embodiments 105-118 wherein the collar comprises a plurality of stacked plates.
120. A system comprising:
   an implantable device having a pair of anchors that are movable between an open position and a closed position to secure the implantable device to a native valve;
   a delivery catheter;
   an actuation element, such as an actuation wire, which extends through the delivery catheter and is coupled to the device; and
   wherein at least one of the actuation element and the device includes a recapturing feature configured to reconnect the actuation element to the device after an initial disconnection of the actuation element from the device.
121. The system of embodiment 120 wherein the actuation element includes the recapturing feature.
122. The system of any of embodiments 120-121 wherein the device includes a proximal collar or proximal end that includes the recapturing feature.
123. The system of any of embodiments 120-121 wherein the device includes a distal cap or distal end that includes the recapturing feature.
124. The system of any of embodiments 120-123 wherein the recapturing feature includes a tapered surface.
125. The system of any of embodiments 120-124 wherein the recapturing feature is pointed.
126. The system of any of embodiments 120-125 wherein the recapturing feature includes a penetrable surface.
127. The system of embodiment 126 wherein the penetrable surface comprises a wire mesh.
128. The system of any of embodiments 120-125 wherein the implantable device and the catheter are tied by a coupling tether which can recouple the device to the catheter after the actuation element has been removed from the device.
129. The system of any of embodiments 120-128 wherein the recapturing feature comprises a plurality of recapturing holes.
130. The system of embodiment 120 wherein the recapturing feature comprises a penetrable recapturing surface on one of a cap of the device and a collar of the device, wherein the penetrable recapturing surface is configured to be penetrated by the actuation element.
131. The system of embodiment 130 wherein the penetrable recapturing surface comprises a wire mesh.
132. The system of any of embodiments 120-131 further comprising a coupling tether that connects the implantable catheter to the implantable device.
133. The system of embodiment 132 wherein the coupling tether is looped around the actuation element.
134. The system of any of embodiments 132-133 wherein the coupling tether is a first coupling tether, and the catheter and the implantable device are tied by a second coupling tether.
135. The system of any of embodiments 132-134 wherein the coupling tether is connected to the implantable device by a releasable knot.
136. The system of any of embodiments 132-134wherein the releasable knot is configured such that pulling a first end of the coupling tether tightens the releasable knot and pulling a second end of the coupling tether unties the releasable knot.
137. The system of any of embodiments 132-135 wherein the implantable device includes a tether routing passage.
138. The system of embodiment 137 wherein the tether routing passage comprises a tube.
139. The system of embodiment 137 wherein the tether routing passage is defined inside a collar of the implantable device.
140. An implantable prosthetic device comprising:
   a pair of anchors that are movable between an open position and a closed position to secure the implantable device to a native valve;
   a coaption element connected to the pair of anchors;
   a collar connected to the coaption element;
   a cover disposed over one or more of the pair of anchors, the coaption element and the collar;
   wherein at least an element of the cover is folded into a tube; and
   a coupling tether that extends through the tube.
141. The implantable prosthetic device of embodiment 140 further comprising a telescoping actuator coupled to the pair of anchors.
142. The implantable prosthetic device of any of embodiments 140-141 wherein the coupling tether is connected to the implantable device by a releasable knot.
143. The implantable prosthetic device of embodiment 142 wherein the releasable knot is configured such that pulling a first end of the coupling tether tightens the releasable knot and pulling a second end of the coupling tether unties the releasable knot.
144. The implantable prosthetic device of any of embodiments 140-143 wherein the collar includes a plurality of recapturing holes.
145. The implantable prosthetic device of any of embodiments 140-144 wherein the collar includes a penetrable recapturing surface that is configured to be penetrated by the actuation element.
146. The implantable prosthetic device of embodiment 145 wherein the penetrable recapturing surface comprises a wire mesh.

## Claims

1. An implantable prosthetic device (100) comprising:
a plurality of paddles (120, 122);
a cap or other attachment portion (114) connected to each of the paddles (120, 122), configured such that movement of the cap or other attachment portion (114) relative to a proximal end of the device opens and closes the plurality of paddles (120, 122);
an extendable coupler (3502) affixed to the cap or other attachment portion (114) such that the extendable coupler (3502) extends when the cap or other attachment portion (114) moves away from the proximal end.

2. The implantable prosthetic device (100) of claim 1, further comprising a coaption element (110) connected to each of the paddles (120, 122), wherein movement of the cap or other attachment portion (114) relative to the coaption portion also opens and closes the plurality of paddles (120, 122).

3. The implantable prosthetic device (100) of claim 2, wherein the extendable coupler (3502) extends through the coaption portion.

4. The implantable prosthetic device (100) of any one of claims 1-3, wherein the extendable coupler (3502) comprises an inner shaft (3606) and an outer sleeve (3602), wherein the inner shaft (3606) is extendably disposed in the outer sleeve (3602).

5. The implantable prosthetic device (100) of claim 4, wherein the inner shaft (3606) comprises a coupling structure (3612) for attachment to the cap at a distal end of the inner shaft (3606).

6. The implantable prosthetic device (100) of any one of claims 4 or 5, wherein the inner shaft (3606) comprises a first radially outwardly extending step (3702) at a proximal end of the inner shaft (3606).

7. A system comprising:
the implantable prosthetic device (100) of any one of claims 4-6;
a delivery catheter (102);
an actuation element (112) that extends through the delivery catheter (102) and is connected to the extendable coupler (3502).

8. The system of claim 7, wherein extending and retracting the actuation element (112) increases and decreases the spacing between the proximal end of the implantable prosthetic device (100) and the distal end of the implantable prosthetic device (100).

9. The system of claim 8, wherein, when the inner shaft (3606) is at least partially extended from the outer sleeve (3602), retraction of the actuation element (112) draws the inner shaft (3606) into the outer sleeve (3602).

10. The system of any one of claims 8 or 9, wherein, when the inner shaft (3606) is at least partially retracted into the outer sleeve (3602), extension of the actuation element (112) extends the inner shaft (3606) outwardly from the outer sleeve (3602).

11. The system of any one of claims 7-10, wherein the implantable prosthetic device (100) comprises a pair of gripping clasps (130) for securing the implantable prosthetic device (100) to a native valve.

12. The system of claim 11, wherein each of the clasps (130) comprises a fixed arm (132), a moveable arm (134), barbs (136), and a joint portion (138).

13. The system of claim 12, wherein the clasps (130) can be opened by applying tension to actuation lines attached to the moveable arms (134), thereby causing the moveable arms (134) to articulate, flex, or pivot around the joint portions (138).

14. The system of any one of claims 12 or 13, wherein the plurality of paddles (120, 122) include inner paddles (122) and outer paddles (120).

15. The system of claim 14, wherein the fixed arms (132) are attached to the inner paddies (122).
